# EUROPEAN PATENT SPECIFICATION

(11) **EP 4 369 900 B1**
(45) Date of publication and mention of the grant of the patent: **20.05.2026**
(21) Application number: 22837955.8
(22) Date of filing: 06.07.2022
(51) Int. Cl.: H10K 85/60, C07D 209/86, C07D 405/14, C07D 409/14, H10K 50/11, H10K 101/00, H10K 101/10

(54) **COMPOSITION FOR ORGANIC OPTOELECTRONIC DEVICE, ORGANIC OPTOELECTRONIC DEVICE AND DISPLAY DEVICE**
ZUSAMMENSETZUNG FÜR ORGANISCHE OPTOELEKTRONISCHE VORRICHTUNG, ORGANISCHE OPTOELEKTRONISCHE VORRICHTUNG UND ANZEIGEVORRICHTUNG
COMPOSITION POUR DISPOSITIF OPTOÉLECTRONIQUE ORGANIQUE, DISPOSITIF OPTOÉLECTRONIQUE ORGANIQUE ET DISPOSITIF D'AFFICHAGE

(30) Priority: 06.07.2021 KR 20210088618; 05.07.2022 KR 20220082644
(43) Date of publication of application: 15.05.2024
(73) Proprietor: Samsung SDI Co., Ltd., Yongin-si, Gyeonggi-do 17084 (KR)
(72) Inventor: LUI, Jinhyun, Suwon-si, Gyeonggi-do 16678 (KR); KIM, Jonghoon, Suwon-si, Gyeonggi-do 16678 (KR); LEE, Mijin, Suwon-si, Gyeonggi-do 16678 (KR); LIM, Youngmook, Suwon-si, Gyeonggi-do 16678 (KR); JUNG, Ho Kuk, Suwon-si, Gyeonggi-do 16678 (KR); JO, Youngkyoung, Suwon-si, Gyeonggi-do 16678 (KR); KIM, Hyung Sun, Suwon-si, Gyeonggi-do 16678 (KR); LYU, Seungchul, Suwon-si, Gyeonggi-do 16678 (KR); SHIN, Jihun, Suwon-si, Gyeonggi-do 16678 (KR); JUNG, Kyunghag, Suwon-si, Gyeonggi-do 16678 (KR); JUNG, Sung-Hyun, Suwon-si, Gyeonggi-do 16678 (KR); CHO, Pyeongseok, Suwon-si, Gyeonggi-do 16678 (KR); HUH, Dalho, Suwon-si, Gyeonggi-do 16678 (KR)
(74) Representative: Michalski Hüttermann & Partner Patentanwälte mbB
(86) International application number: PCT/KR2022/009716
(87) International publication number: WO 2023/282603

(56) References cited:
- EP-A1- 4 254 528
- EP-A1- 4 349 812
- EP-A2- 4 369 901
- CN-A- 110 746 409
- KR-A- 20210 041 166
- KR-A- 20210 041 166
- KR-A- 20220 017 374
- US-A1- 2020 203 631
- US-A1- 2021 122 765

## Description

### [Technical Field]

A composition for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric device, an organic light emitting diode, an organic solar cell, and an organic photoconductor drum.

Among them, organic light emitting diodes (OLEDs) are attracting much attention in recent years due to increasing demands for flat panel display devices. The organic light emitting diode is a device that converts electrical energy into light, and the performance of the organic light emitting diode is greatly influenced by an organic material between electrodes.

Organic optoelectronic devices are inter alia known from KR 202100411666 A, US 2014/197386 A1, US 2015/0001488 A1, EP 2709181 A1, post published documents EP 4349811 A1, EP 4349812 A1, EP 4254528 A1, as well as document EP 4369901 A2 having the same priority date as the present application.

### [Disclosure]

The invention is defined by the features of the independent claims. An embodiment provides a composition for an organic optoelectronic device that can implement a low-driving, high-efficiency, and long-life organic optoelectronic device.

Another embodiment provides an organic optoelectronic device including the composition for an organic optoelectronic device.

Another embodiment provides a display device including the organic optoelectronic device.

According to an embodiment, provided is a composition for an organic optoelectronic device including a first compound represented by Chemical Formula 1, and a second compound represented by a combination of Chemical Formula 2 and Chemical Formula 3.

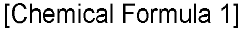

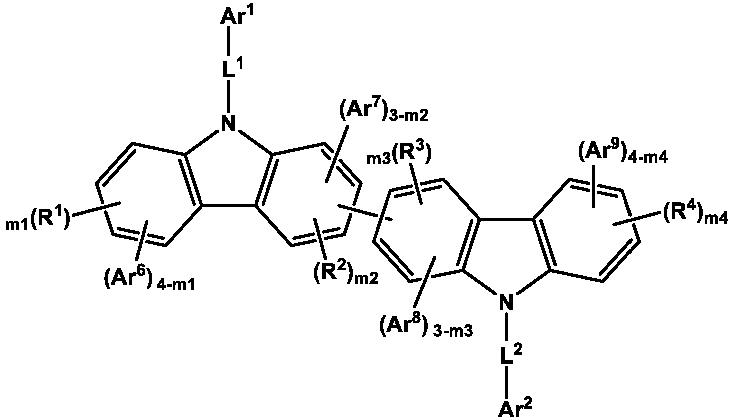

In Chemical Formula 1,
L¹ and L² are each independently a single bond or a substituted or unsubstituted C6 to C30 arylene group,
Ar¹ and Ar² are each independently a substituted or unsubstituted C6 to C30 aryl group or a substituted or unsubstituted C2 to C30 heterocyclic group,
R¹ to R⁴ are each independently hydrogen, deuterium, or a substituted or unsubstituted C6 to C30 aryl group,
Ar⁶ to Ar⁹ are each independently hydrogen or a substituted or unsubstituted C6 to C30 aryl group,
m1 and m4 are each independently one of integers of 1 to 4,
m2 and m3 are each independently one of integers of 1 to 3, and
Chemical Formula 1 simultaneously satisfies the following conditions (i) and (ii),
   (i) at least one of Ar¹ and Ar² is a C6 to C30 aryl group substituted with at least one deuterium or a C2 to C30 heterocyclic group substituted with at least one deuterium, and
   (ii) at least one of R¹ to R⁴ is deuterium; 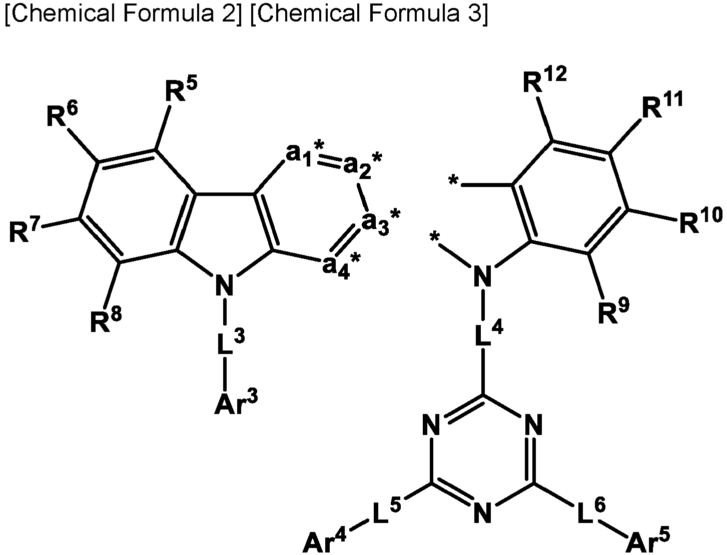
      wherein, in Chemical Formula 2 and Chemical Formula 3,
      Ar³ to Ar⁵ are each independently a substituted or unsubstituted C6 to C20 aryl group or a substituted or unsubstituted C2 to C30 heterocyclic group,
      a₁* to a₄* are each independently a linking carbon (C) or C-L^{a}-R^{a},
      the two adjacent of a₁* to a₄* in Chemical Formula 2 are linked to * in Chemical Formula 3,
      L² and L³ to L⁶ are each independently a single bond, a substituted or unsubstituted C6 to C20 arylene group or a substituted or unsubstituted C2 to C20 heteroarylene group, and
      R^{a} and R⁵ to R¹² are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group.

In the claimed invention, for the case that the first compound is selected from one of compounds D1-1 to D1-8 as defined in claim 1 and the second compound is selected from one of the compounds D2-1 to D2-7 as defined in claim 1, the following combinations are excluded: D1-1 with D2-1, D1-2 with D2-1, D1-3 with D2-1, D1-4 with D2-1, D1-5 with D2-1, D1-1 with D2-2, D1-2 with D2-2, D1-3 with D2-2, D1-4 with D2-2, D1-5 with D2-2, D1-1 with D2-3, D1-2 with D2-3, D1-3 with D2-3, D1-4 with D2-3, D1-5 with D2-3, D1-6 with D2-4, D1-7 with D2-5, D1-8 with D2-6, and D1-6 with D2-7.

According to another embodiment, provided is an organic optoelectronic device including an anode and a cathode facing each other, and at least one organic layer between the anode and the cathode, wherein the organic layer includes the composition for an organic optoelectronic device.

According to another embodiment, a display device including the organic optoelectronic device is provided.

An organic optoelectronic device with low driving, high efficiency, and long life-span can be implemented.

### [Description of the Drawings]

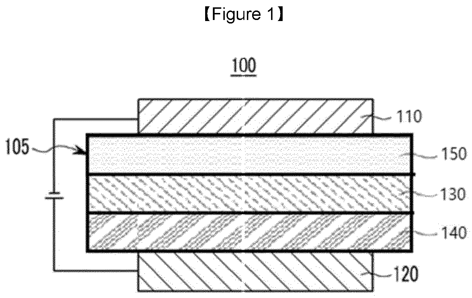

FIG. 1 is a cross-sectional view showing an organic light emitting diode according to an embodiment.

### [Mode for Invention]

Hereinafter, embodiments of the present invention are described in detail. However, these embodiments are exemplary, the present invention is not limited thereto and the present invention is defined by the scope of claims.

As used herein, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In specific example of the present invention, the "substituted" refers to replacement of at least one hydrogen of a substituent or a compound by deuterium, a C1 to C20 alkyl group, a C6 to C30 aryl group, or a cyano group. In specific example of the present invention, the "substituted" refers to replacement of at least one hydrogen of a substituent or a compound by deuterium, a C1 to C5 alkyl group, a C6 to C18 aryl group, or a cyano group. In specific example of the present invention, the "substituted" refers to replacement of at least one hydrogen of a substituent or a compound by deuterium, a cyano group, a methyl group, an ethyl group, a propyl group, a butyl group, a phenyl group, a biphenyl group, a terphenyl group, or a naphthyl group.

In the present specification, "unsubstituted" refers to non-replacement of a hydrogen atom by another substituent and remaining of the hydrogen atom.

In the present specification, "deuterium substituted (-D) "may include "tritium substituted (-T)".

As used herein, when a definition is not otherwise provided, "hetero" refers to one including one to three heteroatoms selected from **N,** O, S, P, and Si, and remaining carbons in one functional group.

As used herein, "an aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quarterphenyl group, and the like, and two or more hydrocarbon aromatic moieties are fused directly or indirectly to provide a non-aromatic fused ring, for example a fluorenyl group.

The aryl group may include a monocyclic, polycyclic, or fused ring polycyclic (i.e., rings sharing adjacent pairs of carbon atoms) functional group.

As used herein, "a heterocyclic group" is a generic concept of a heteroaryl group, and may include at least one heteroatom selected from N, O, S, P, and Si instead of carbon (C) in a cyclic compound such as an aryl group, a cycloalkyl group, a fused ring thereof, or a combination thereof. When the heterocyclic group is a fused ring, the entire ring or each ring of the heterocyclic group may include one or more heteroatoms.

For example, "a heteroaryl group" may refer to an aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted triphenylene group, a substituted or unsubstituted perylenyl group, a substituted or unsubstituted fluorenyl group, a substituted or unsubstituted indenyl group, a substituted or unsubstituted furanyl group, or a combination thereof, but is not limited thereto.

More specifically, the substituted or unsubstituted C2 to C30 heterocyclic group may be a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, a substituted or unsubstituted dibenzothiophenyl group, a substituted or unsubstituted benzofuranpyrimidinyl group, a substituted or unsubstituted benzothiophenepyrimidinyl group, or a combination thereof, but is not limited thereto.

As used herein, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a composition for an organic optoelectronic device according to an embodiment is described.

The composition for an organic optoelectronic device according to an embodiment includes a first compound represented by Chemical Formula 1 and a second compound represented by a combination of Chemical Formula 2 and Chemical Formula 3.

The first compound may be represented by Chemical Formula 1.

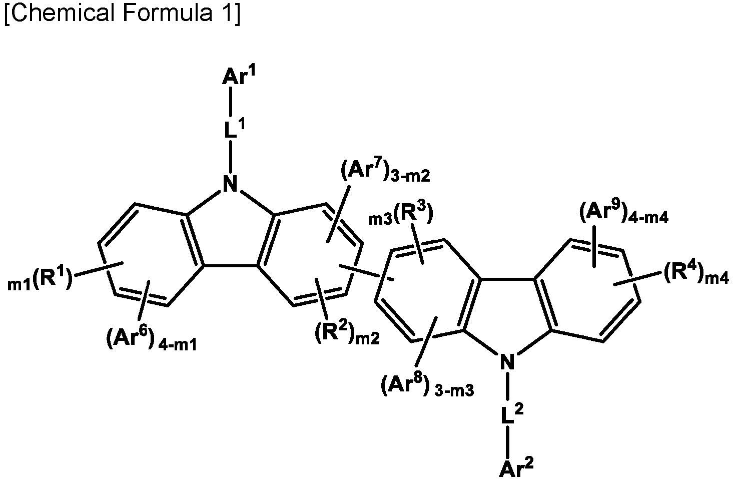

In Chemical Formula 1,
L¹ and L² are each independently a single bond or a substituted or unsubstituted C6 to C30 arylene group,
Ar¹ and Ar² are each independently a substituted or unsubstituted C6 to C30 aryl group or a substituted or unsubstituted C2 to C30 heterocyclic group,
R¹ to R⁴ are each independently hydrogen, deuterium, or a substituted or unsubstituted C6 to C30 aryl group,
m1 and m4 are each independently one of integers of 1 to 4,
m2 and m3 are each independently one of integers of 1 to 3,
Ar⁶ to Ar⁹ are each independently hydrogen or a substituted or unsubstituted C6 to C30 aryl group, and
Chemical Formula 1 simultaneously satisfies the following conditions (i) and (ii).
   (i) at least one of Ar¹ and Ar² is a C6 to C30 aryl group substituted with at least one deuterium or a C2 to C30 heterocyclic group substituted with at least one deuterium, and
   (ii) at least one of R¹ to R⁴ is deuterium.

The first compound represented by Chemical Formula 1 has biscarbazole as the basic skeleton, and has a structure in which the benzene moiety of the carbazole is substituted with at least one deuterium, and at least one of the 9th (N-direction) substituents Ar¹ and Ar² of carbazole is substituted with at least one deuterium.

As the benzene moiety of carbazole and the 9th (N-direction) substituent of carbazole are simultaneously substituted with deuterium, a zero point energy and vibration energy of the compound may be further lowered. As a result, the energy of the ground state is further lowered, and the interaction between molecules is weakened, making it possible to make the thin film formed from this in an amorphous state, which improves heat resistance and life-span. In other words, if this is applied, it is possible to implement an organic light emitting diode with low driving, high efficiency, and especially long life-span.

Chemical Formula 1 may be expressed as, for example, any one of Chemical Formulas 1-1 to 1-10, depending on the linking position of carbazole. 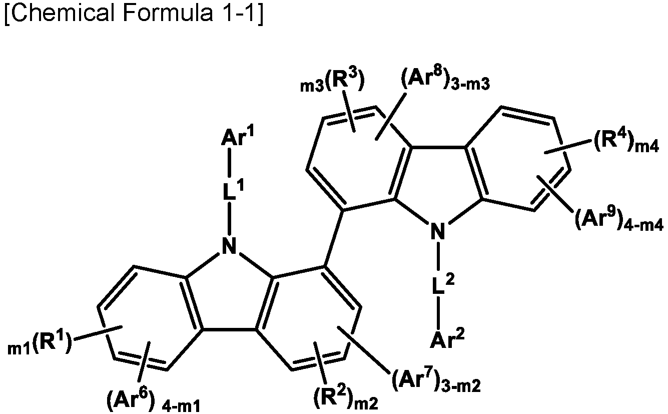 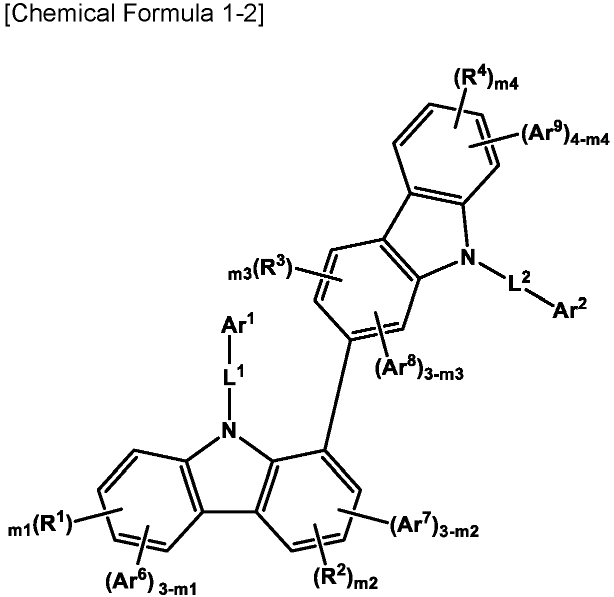 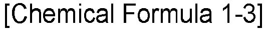 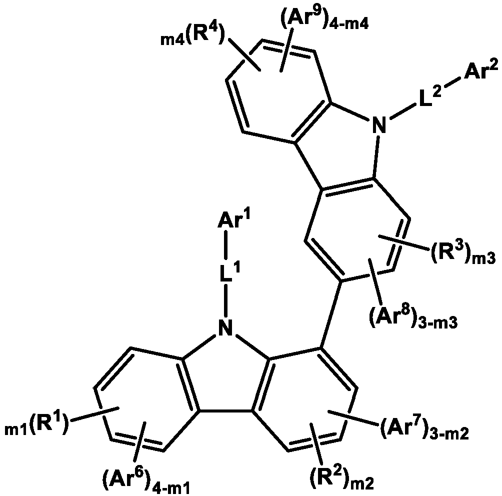 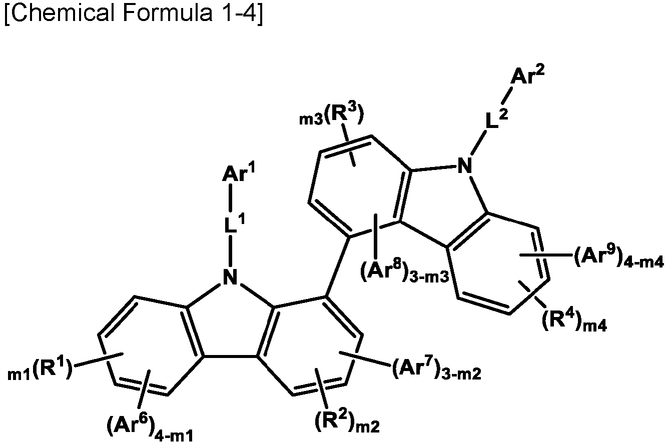 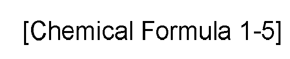 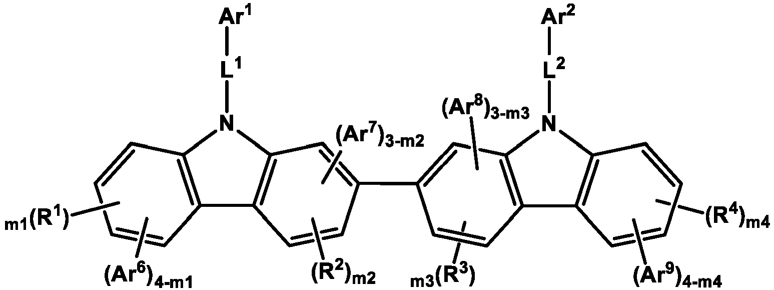 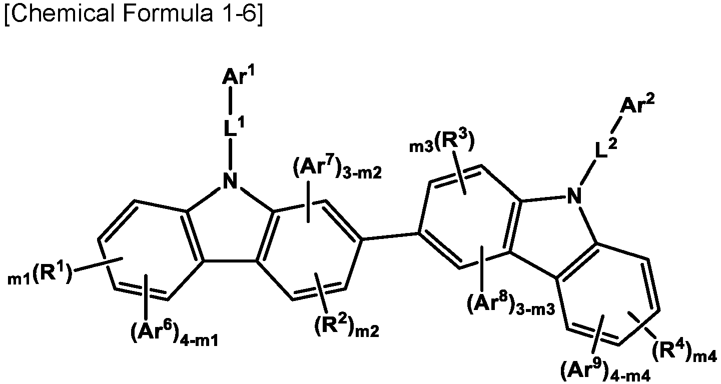 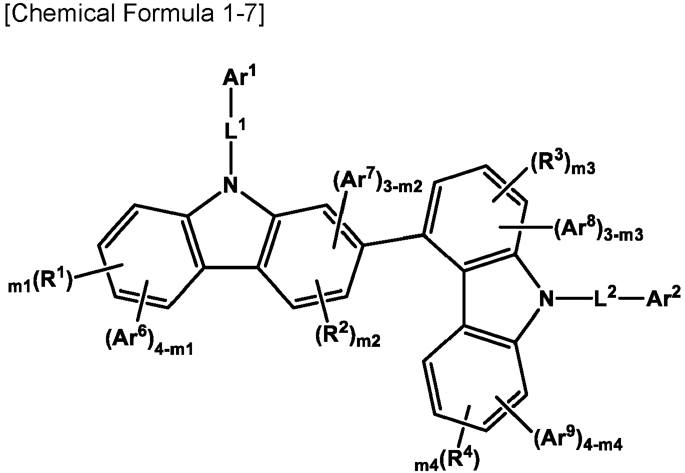 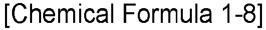 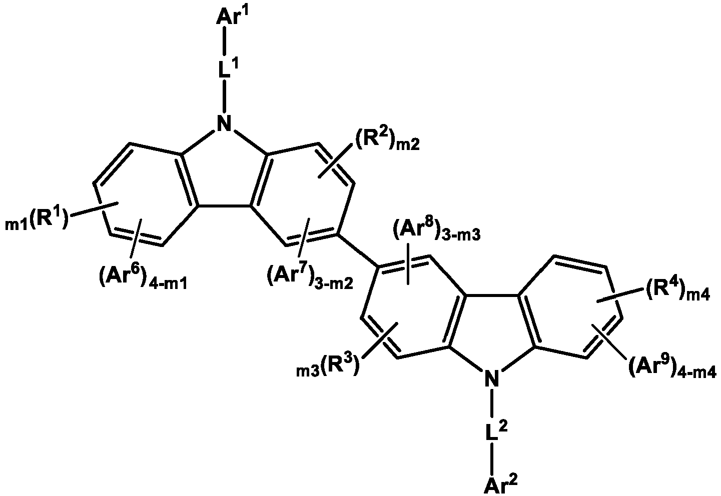 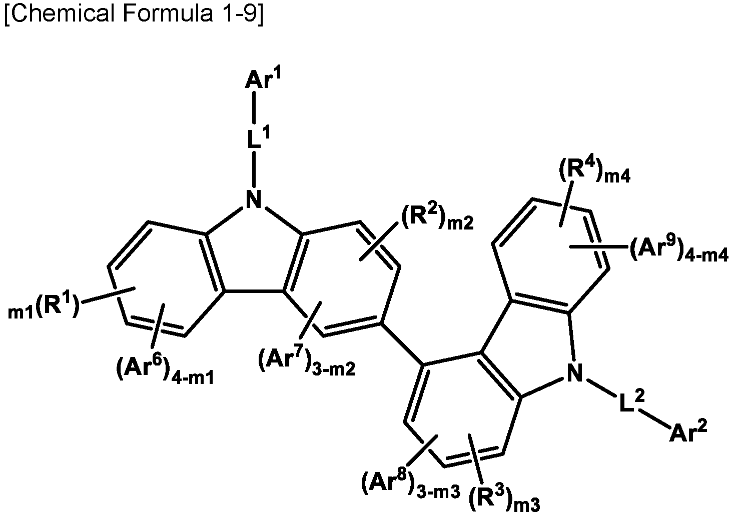 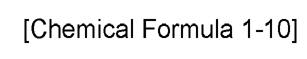 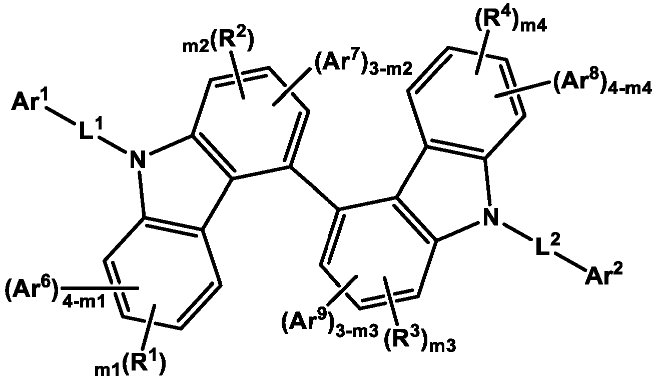
wherein, in Chemical Formula 1-1 to Chemical Formula 1-10,
L¹, L², Ar¹, Ar², Ar⁶ to Ar⁹, R¹ to R⁴, and m¹ to m⁴ are the same as described above.

When R¹ is greater than or equal to 2, each R¹ may be the same as or different from each other.

When R² is greater than or equal to 2, each R² may be the same as or different from each other.

When R³ is greater than or equal to 2, each R³ may be the same as or different from each other.

When R⁴ is greater than or equal to 2, each R⁴ may be the same as or different from each other.

When Ar⁶ is greater than or equal to 2, each Ar⁶ may be the same as or different from each other.

When Ar⁷ is greater than or equal to 2, each Ar⁷ may be the same as or different from each other.

When Ar⁸ is greater than or equal to 2, each Ar⁸ may be the same as or different from each other.

When Ar⁹ is greater than or equal to 2, each Ar⁹ may be the same as or different from each other.

For example, at least two of R¹ to R⁴ may be deuterium.

For example, R¹ to R⁴ may each be deuterium, m1 and m4 may each be an integer of 4, and m2 and m3 may each be an integer of 3.

For example, R¹ and R² may each be deuterium, m1 may be an integer from 1 to 4, m2 may be an integer from 1 to 3, and R³ and R⁴ may each be hydrogen.

For example, R³ and R⁴ may each be deuterium, m3 may be an integer from 1 to 3, m4 may be an integer from 1 to 4, and R¹ and R² may each be hydrogen.

For example, R¹ and R⁴ may each be deuterium, m1 and m4 may each be an integer from 1 to 4, and R² and R³ may each be hydrogen.

For example, R¹ to R³ may each be deuterium, m2 and m3 may each be an integer of 1 to 3, m1 may be an integer of 1 to 4, and R⁴ may be deuterium or a C6 to C30 aryl group substituted or unsubstituted with deuterium.

As an example, depending on the substitution position of the deuterium substituted for R¹ to R⁴, Chemical Formula 1 may be represented by any of Chemical Formulas 1a to 1e.

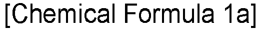

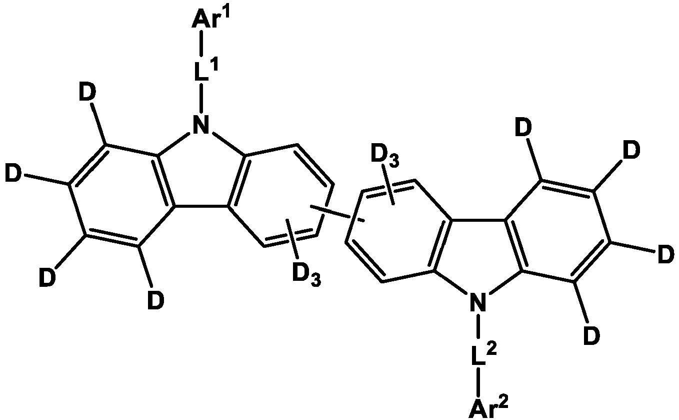

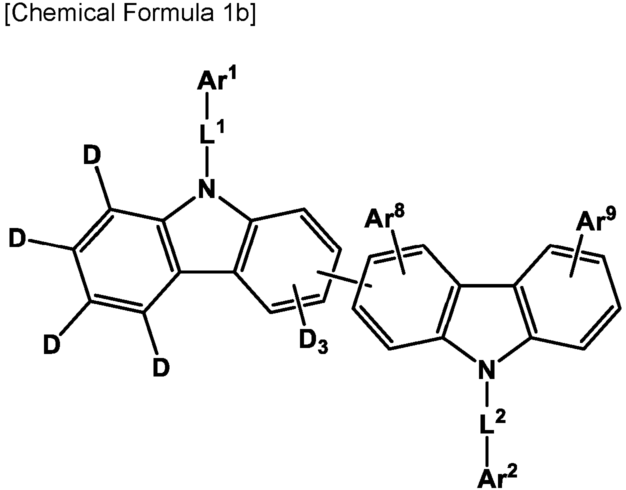

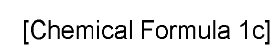

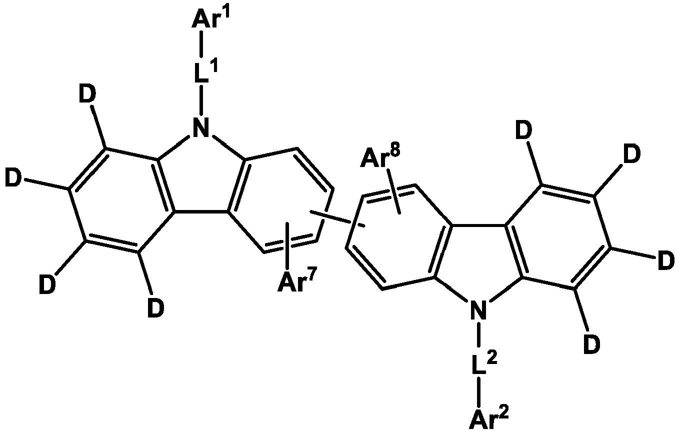

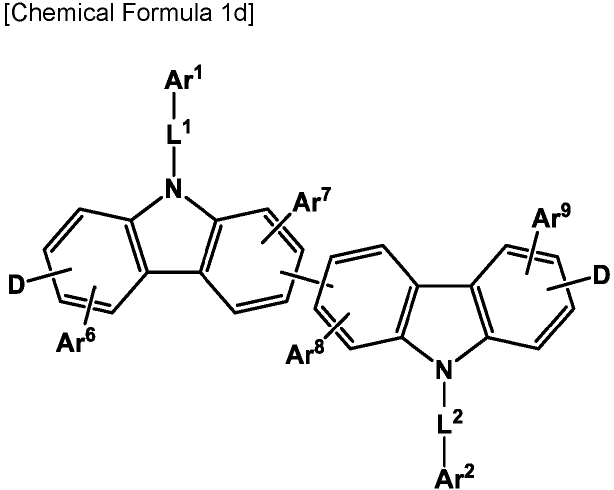

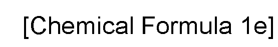

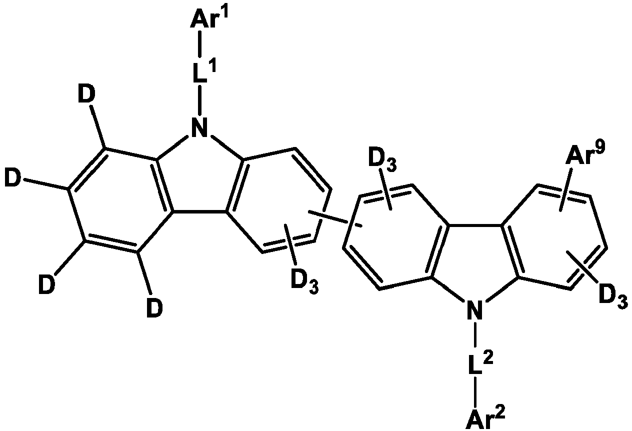

In Chemical Formula 1a to Chemical Formula 1e, L¹, L², Ar¹, Ar², and Ar⁶ to Ar⁹ are the same as described above.

Ar6 to Ar9 are each independently a C6 to C30 aryl group substituted or unsubstituted with hydrogen or deuterium,
D₃ refers to replacement of three deuterium.

For example, at least one of Ar¹ and Ar² may be a phenyl group substituted with at least one deuterium, a biphenyl group substituted with at least one deuterium, a terphenyl group substituted with at least one deuterium, a naphthyl group substituted with at least one deuterium, an anthracenyl group substituted with at least one deuterium, a phenanthrenyl group substituted with at least one deuterium, a triphenylene group substituted with at least one deuterium, a fluorenyl group substituted with at least one deuterium, a dibenzofuranyl group substituted with at least one deuterium, or a dibenzothiophenyl group substituted with at least one deuterium.

As a specific example, at least one of Ar¹ and Ar² may be a phenyl group substituted with at least one deuterium, a biphenyl group substituted with at least one deuterium, a terphenyl group substituted with at least one deuterium, a triphenylene group substituted with at least one deuterium, a dibenzofuranyl group substituted with at least one deuterium, or a dibenzothiophenyl group substituted with at least one deuterium.

As a specific example, Ar⁶ to Ar⁹ may each independently be a C6 to C20 aryl group unsubstituted or substituted with hydrogen or deuterium.

For example, Ar⁶ to Ar⁹ may each independently be hydrogen, a phenyl group substituted or unsubstituted with deuterium, a biphenyl group substituted or unsubstituted with deuterium, a terphenyl group substituted or unsubstituted with deuterium, a naphthyl group substituted or unsubstituted with deuterium, a phenanthrenyl group substituted or unsubstituted with deuterium, an anthracenyl group substituted or unsubstituted with deuterium, a triphenylene group substituted or unsubstituted with deuterium, or a fluorenyl group substituted or unsubstituted with deuterium.

For example, L¹-Ar¹ and L²-Ar² in Chemical Formula 1 may each independently be selected from the substituents listed in Group I -1 and Group I -2, and at least one of L¹-Ar¹ and L²-Ar² may be selected from among the listed substituents in Group I -2.

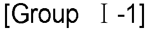

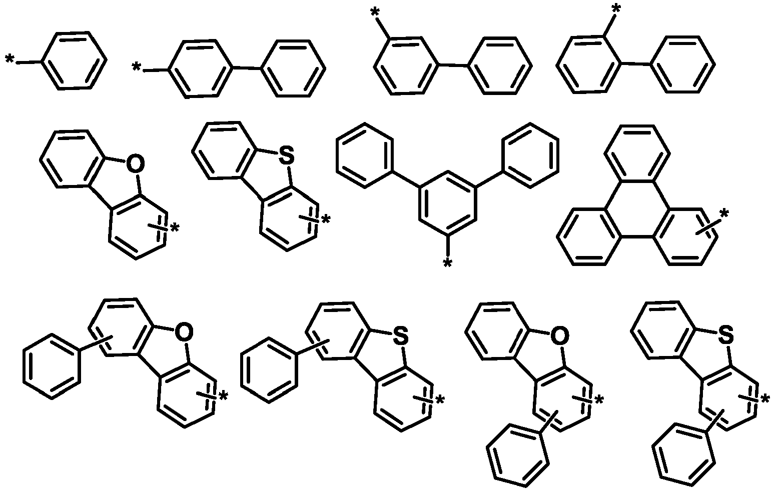

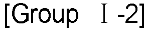

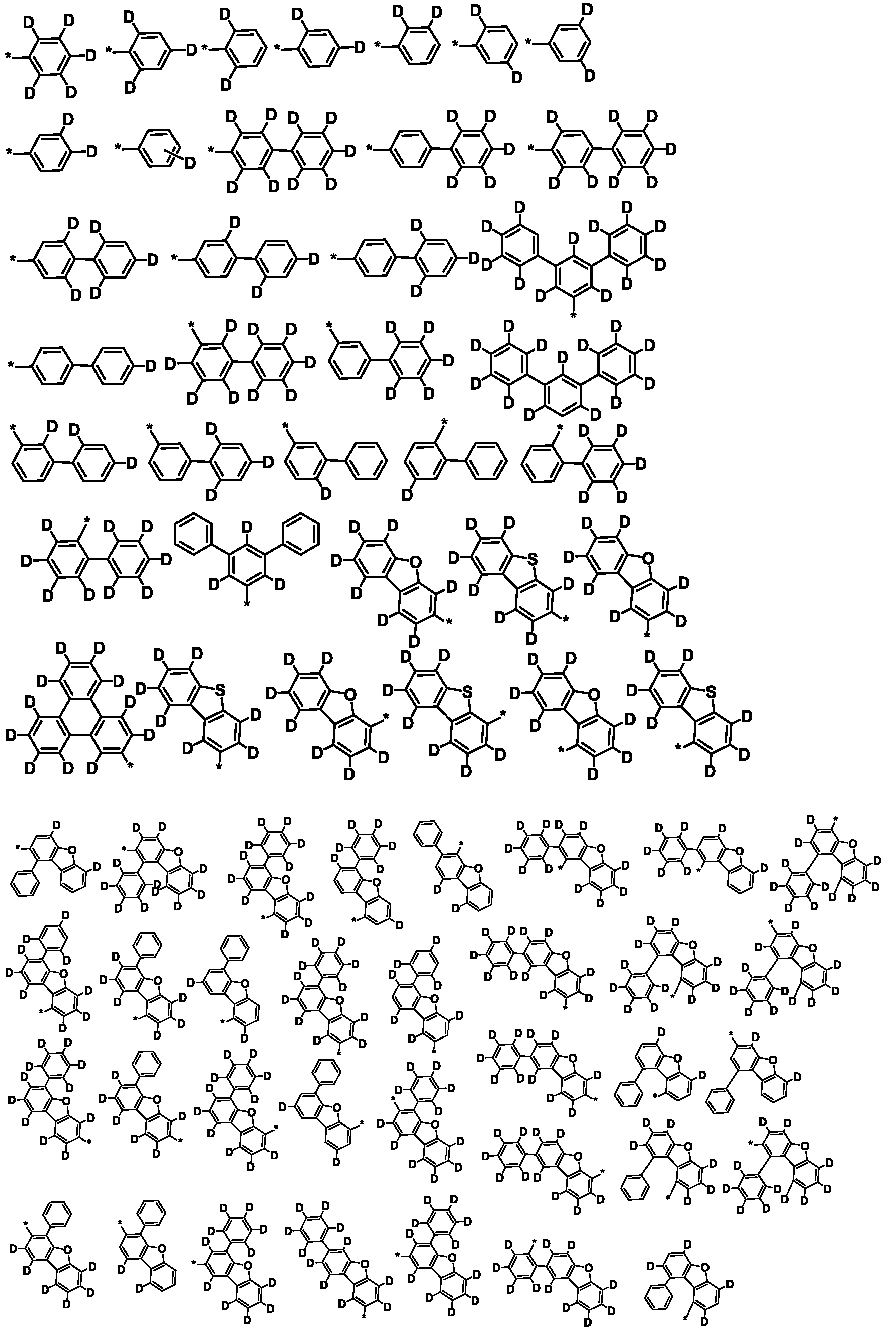

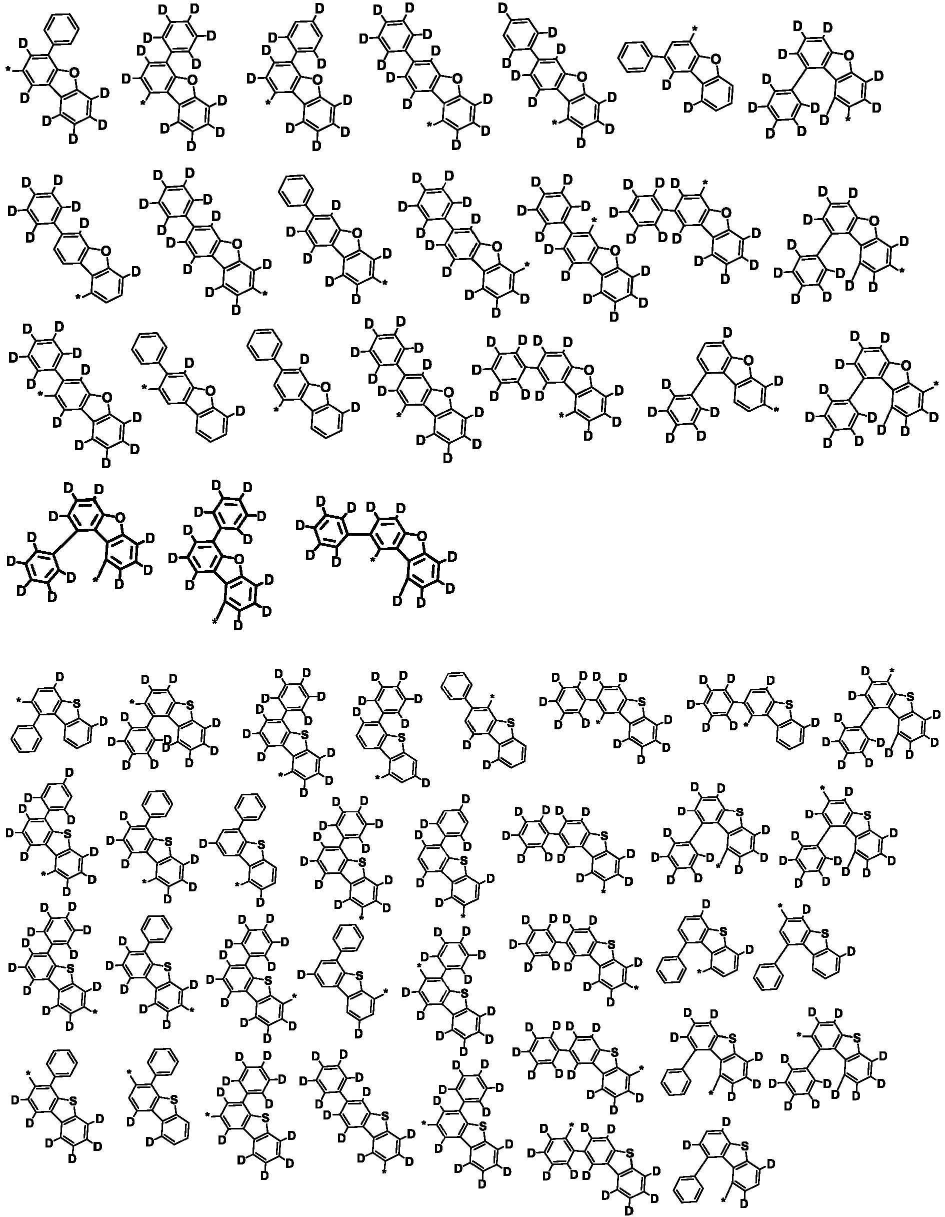

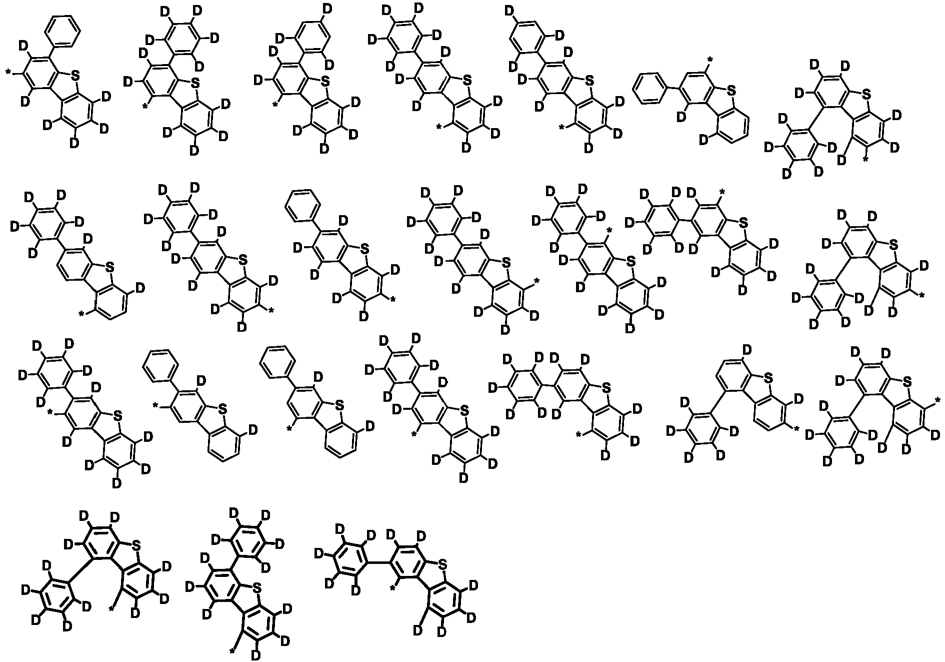

In Group I -1 and Group I -2, * is a linking point.

For example, Chemical Formula 1 may be represented by Chemical Formula 1-8a or Chemical Formula 1-8e.

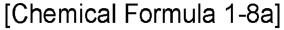

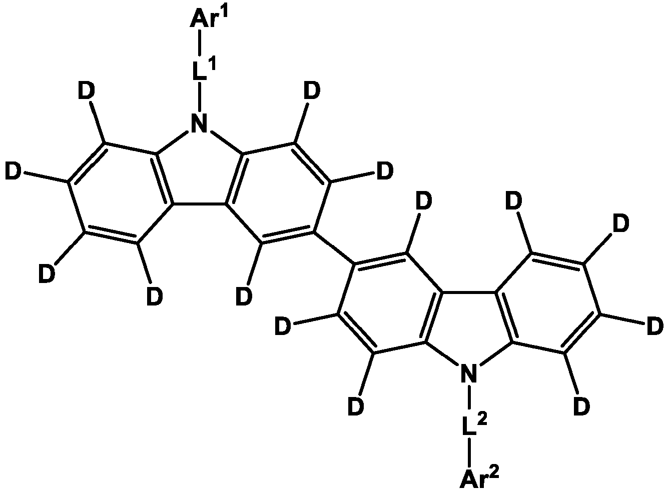

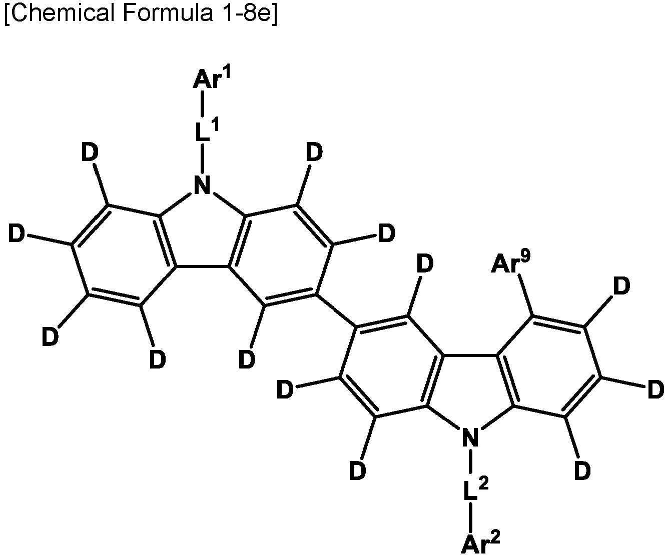

In Chemical Formula 1-8a and Chemical Formula 1-8e,
L¹, L², Ar¹, and Ar² are the same as described above, and Ar⁹ is a C6 to C30 aryl group substituted or unsubstituted with deuterium.

For example, Ar⁹ may be a phenyl group substituted or unsubstituted with deuterium, a biphenyl group substituted or unsubstituted with deuterium, a terphenyl group substituted or unsubstituted with deuterium, a naphthyl group substituted or unsubstituted with deuterium, a phenanthrenyl group substituted or unsubstituted with deuterium, an anthracenyl group substituted or unsubstituted with deuterium, a triphenylene group substituted or unsubstituted with deuterium, or a fluorenyl group substituted or unsubstituted with deuterium.

For example, the compound for an organic optoelectronic device represented by Chemical Formula 1 may be one selected from the compounds listed in Group 1, but is not limited thereto.

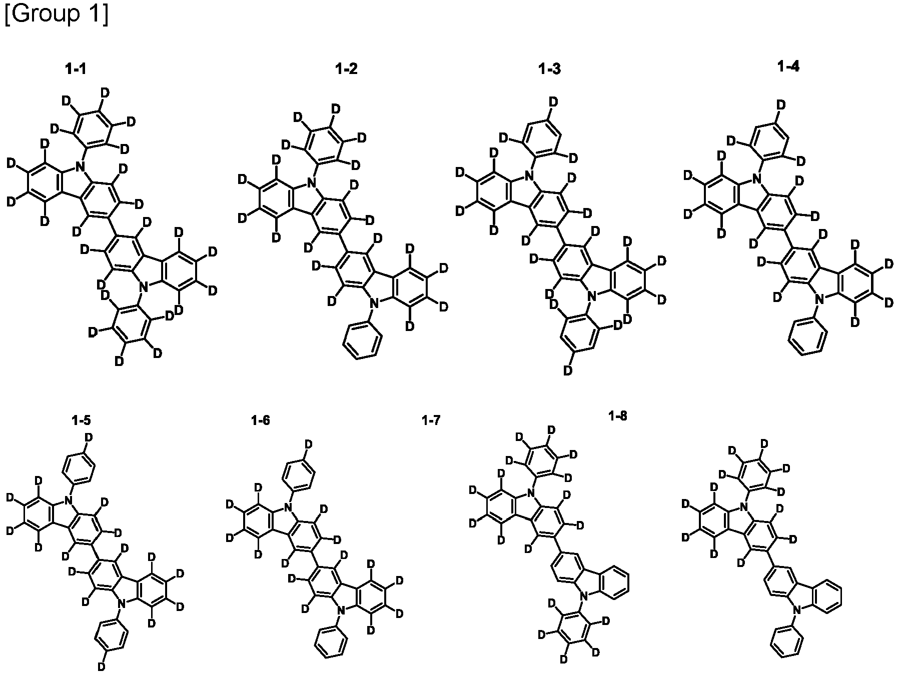

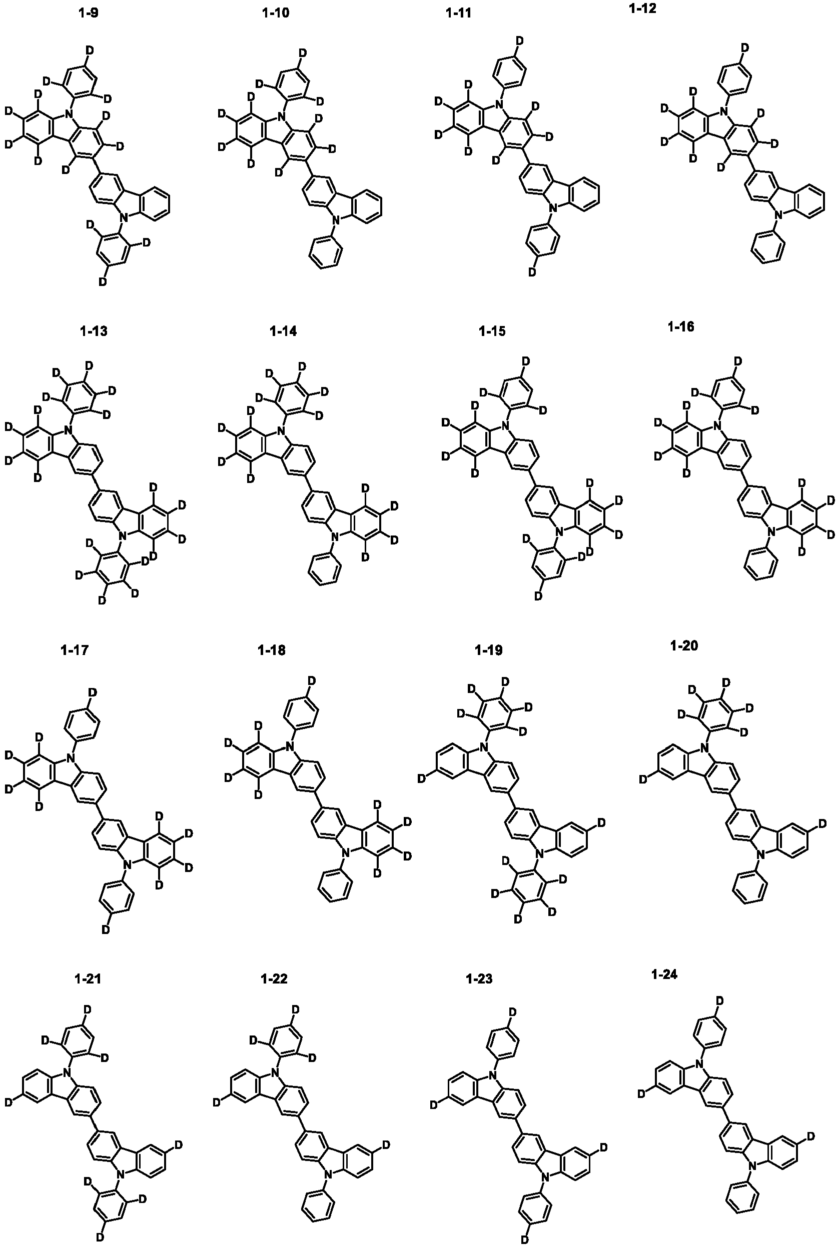

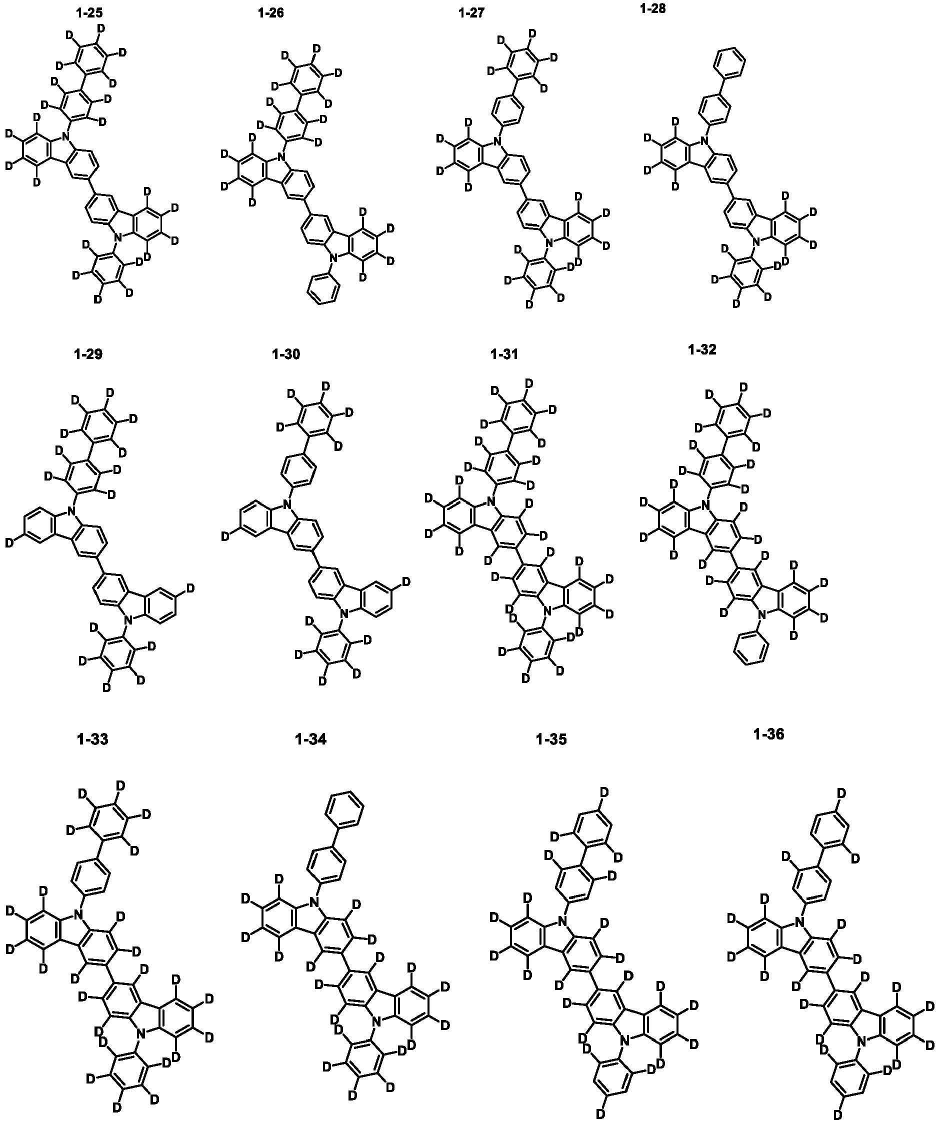

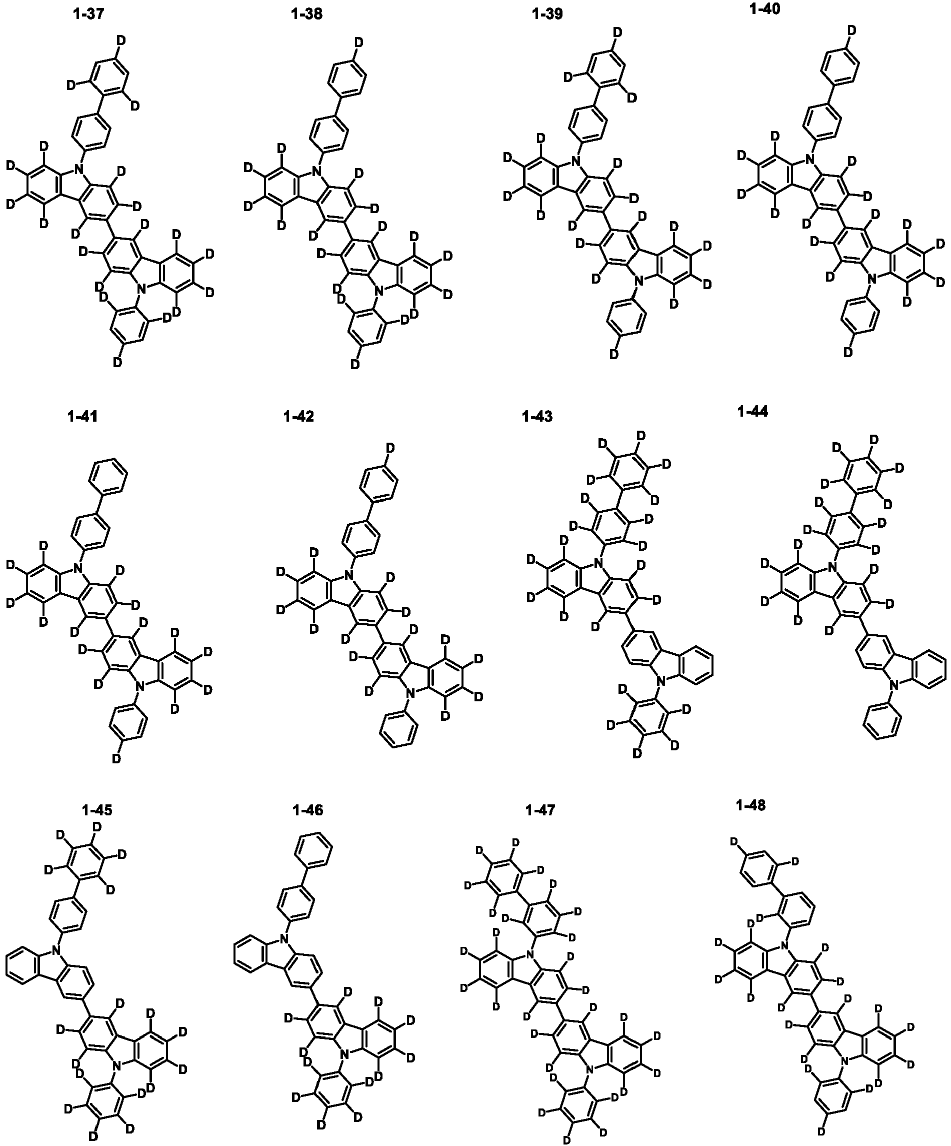

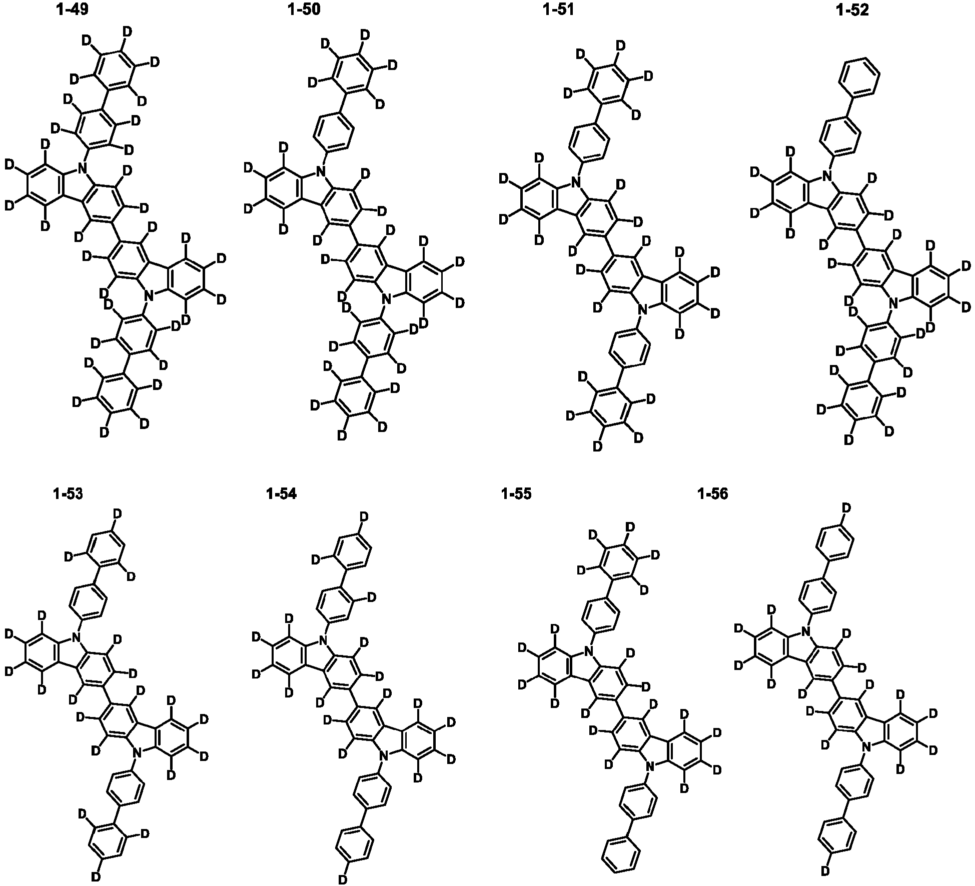

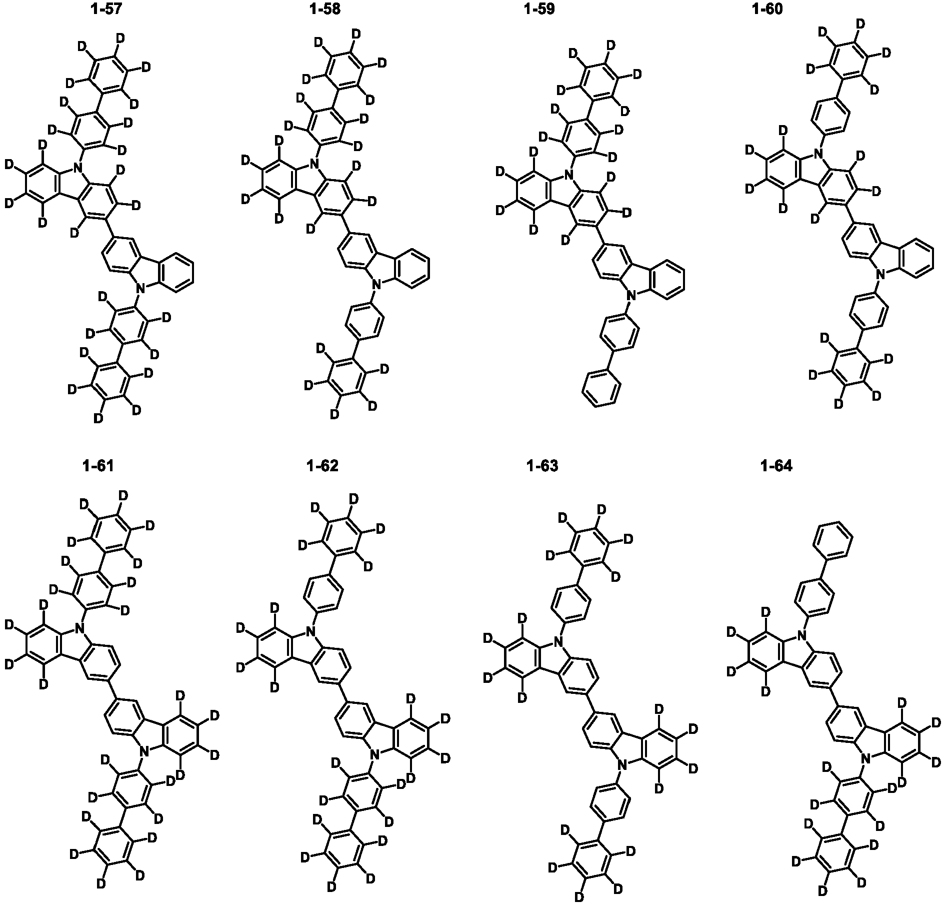

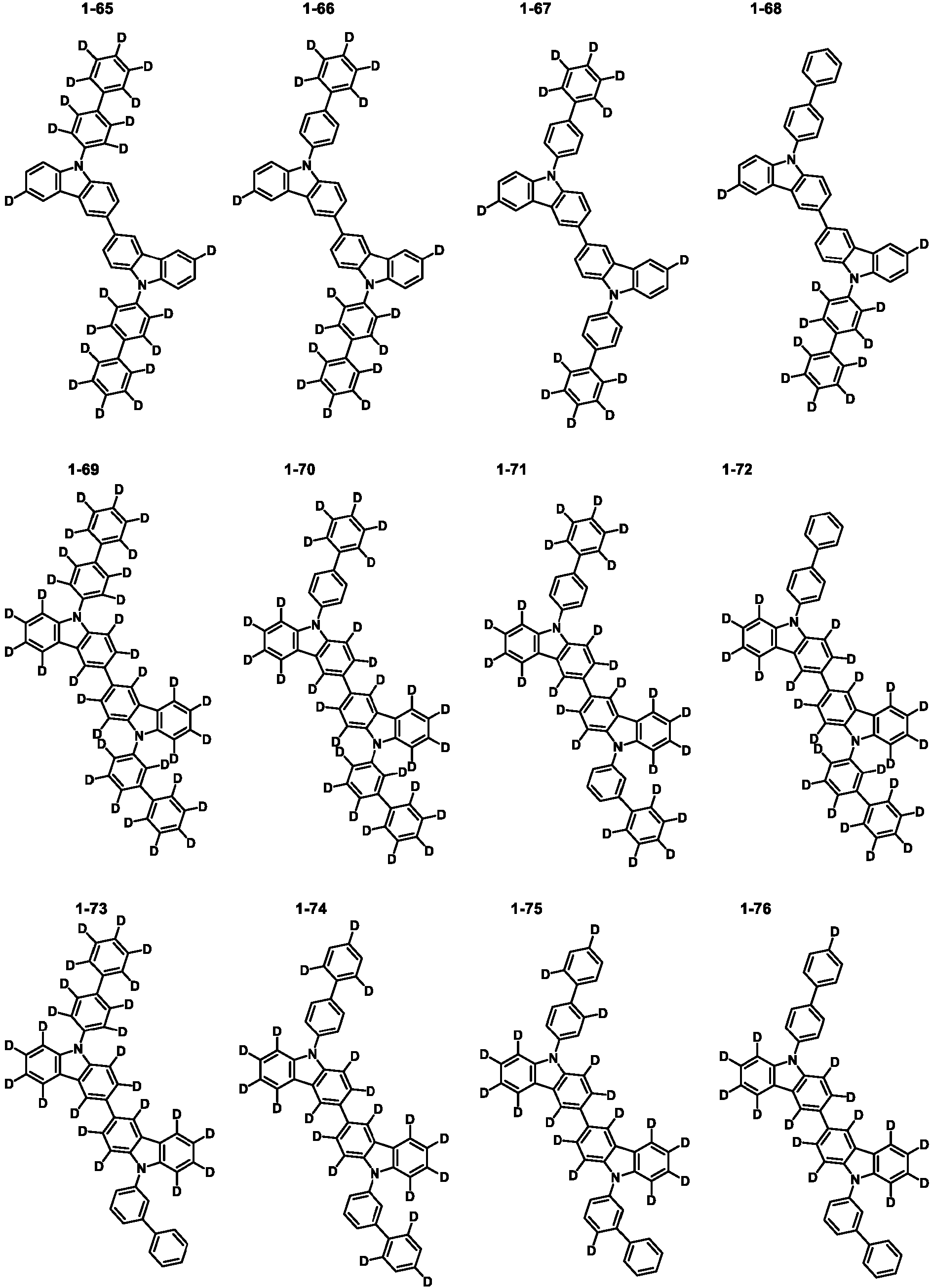

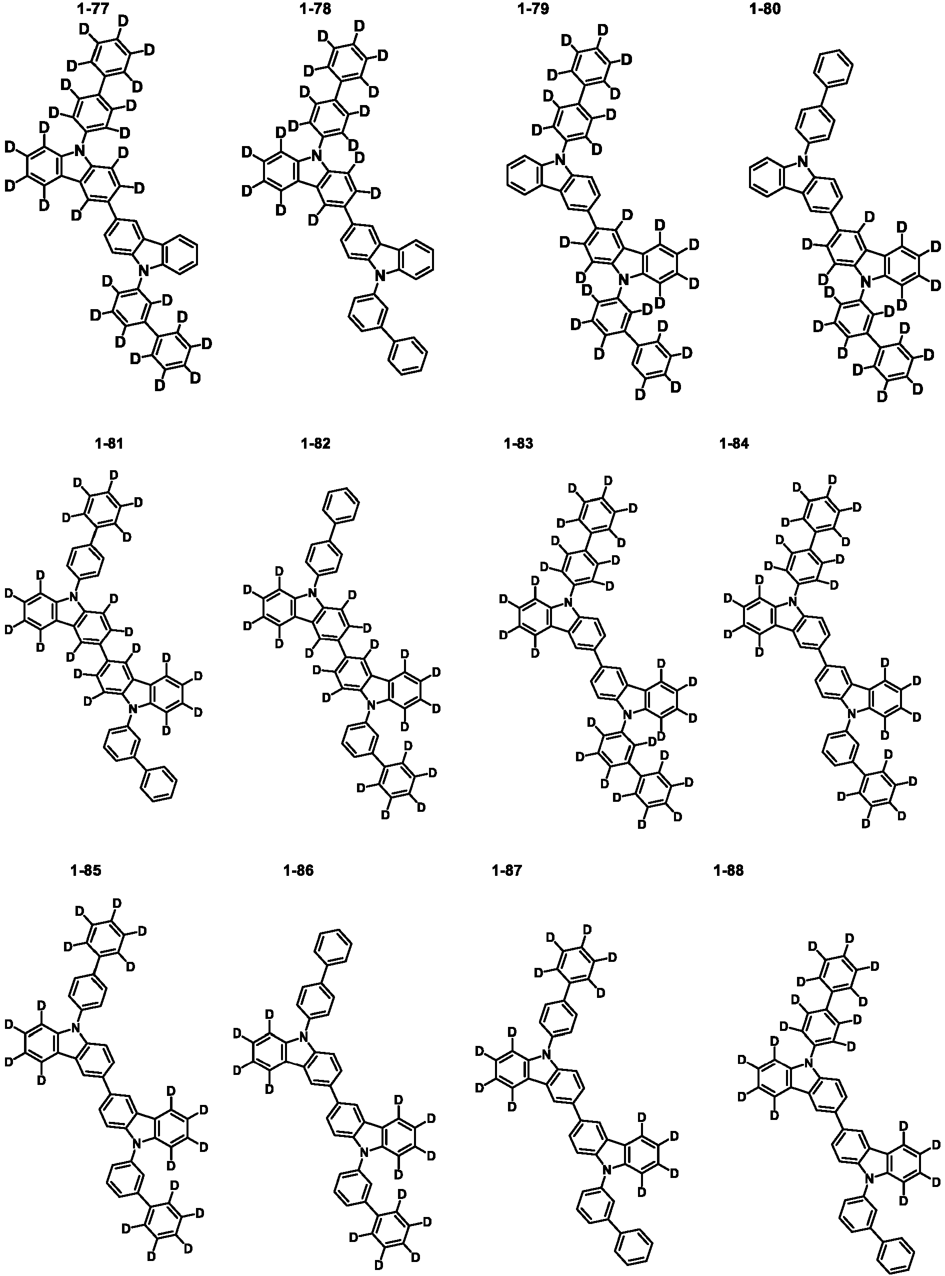

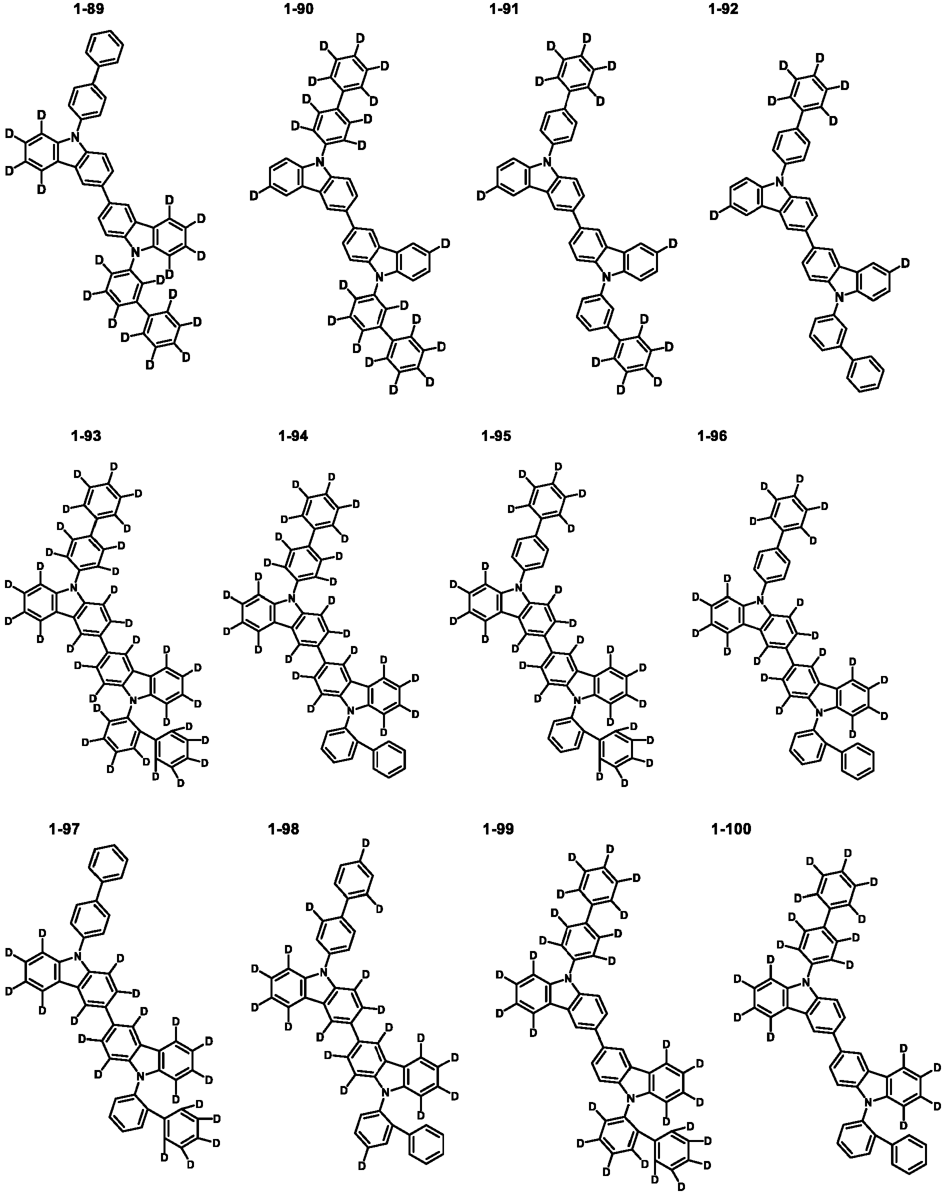

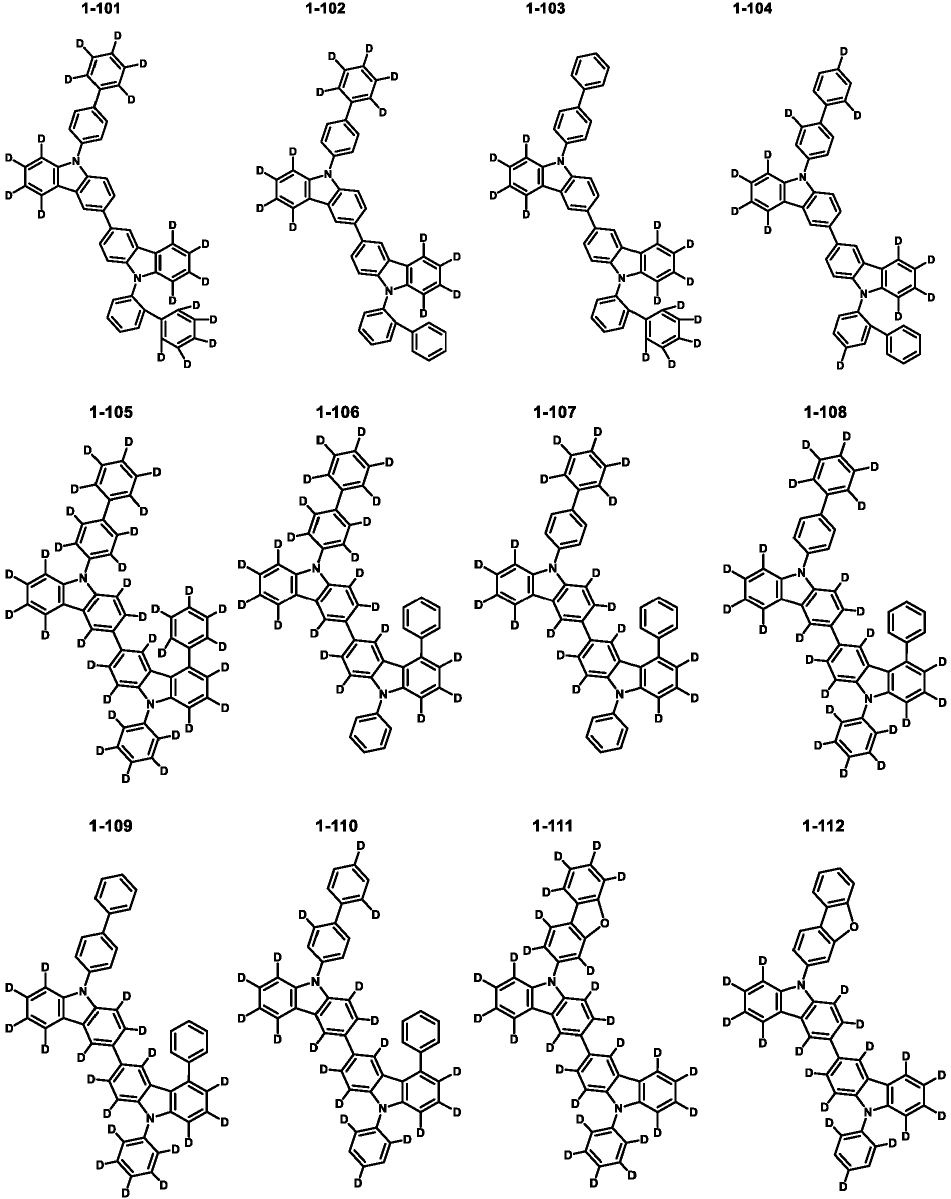

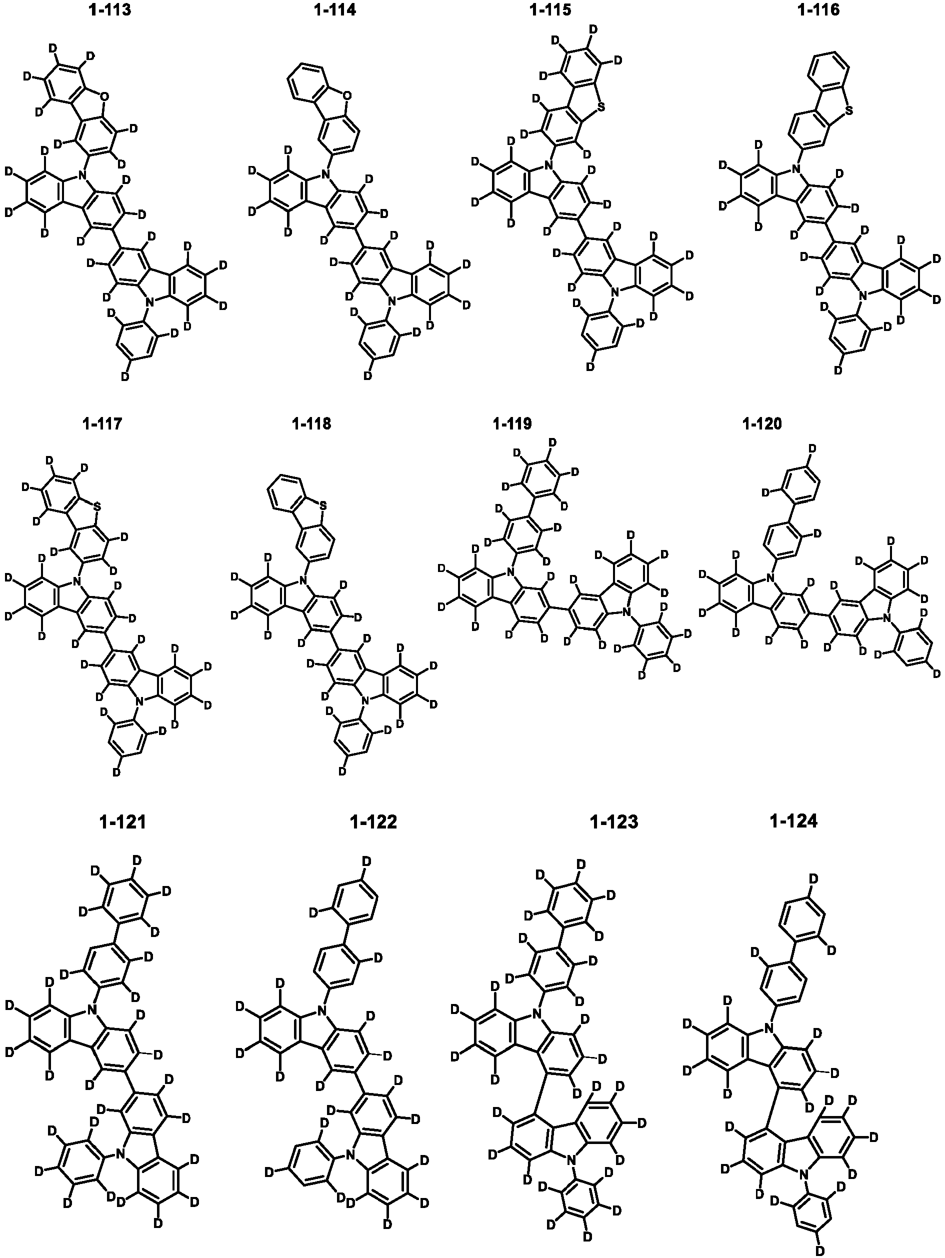

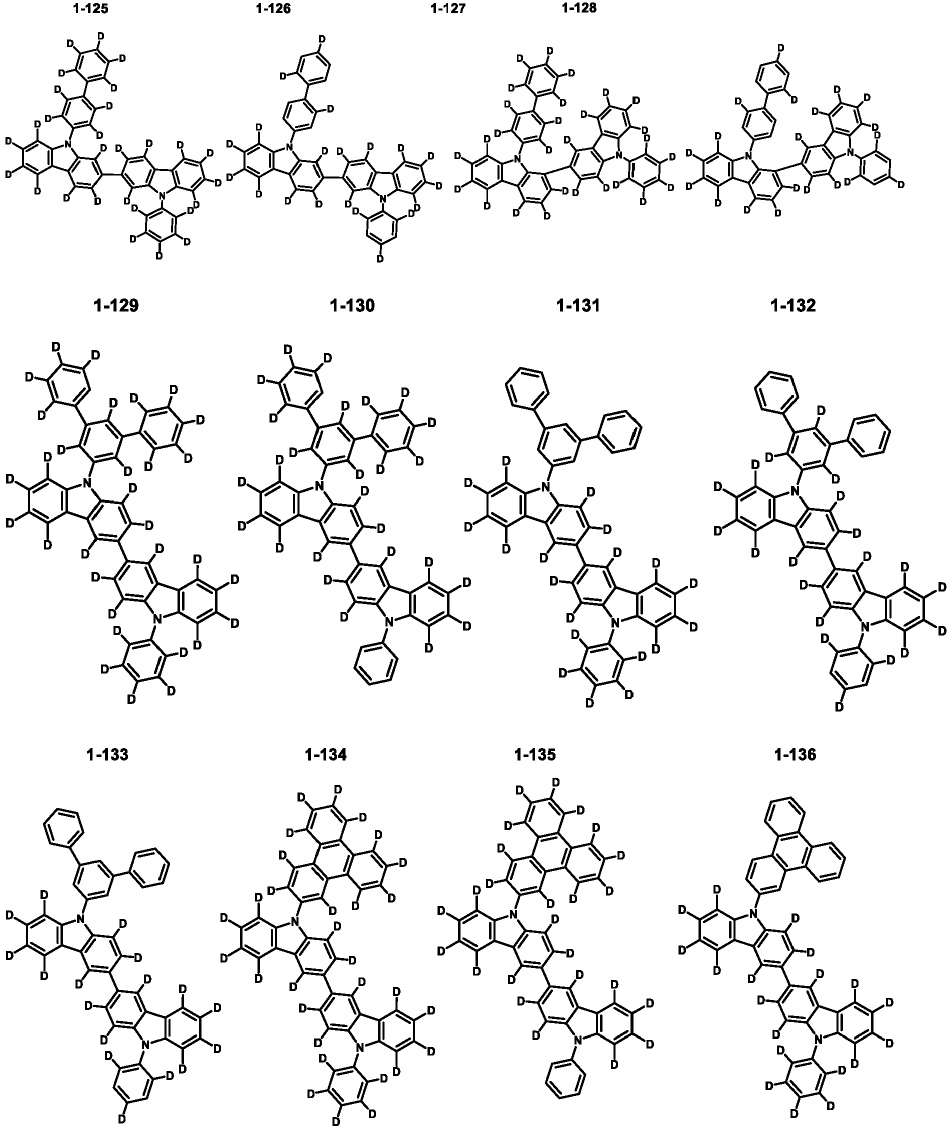

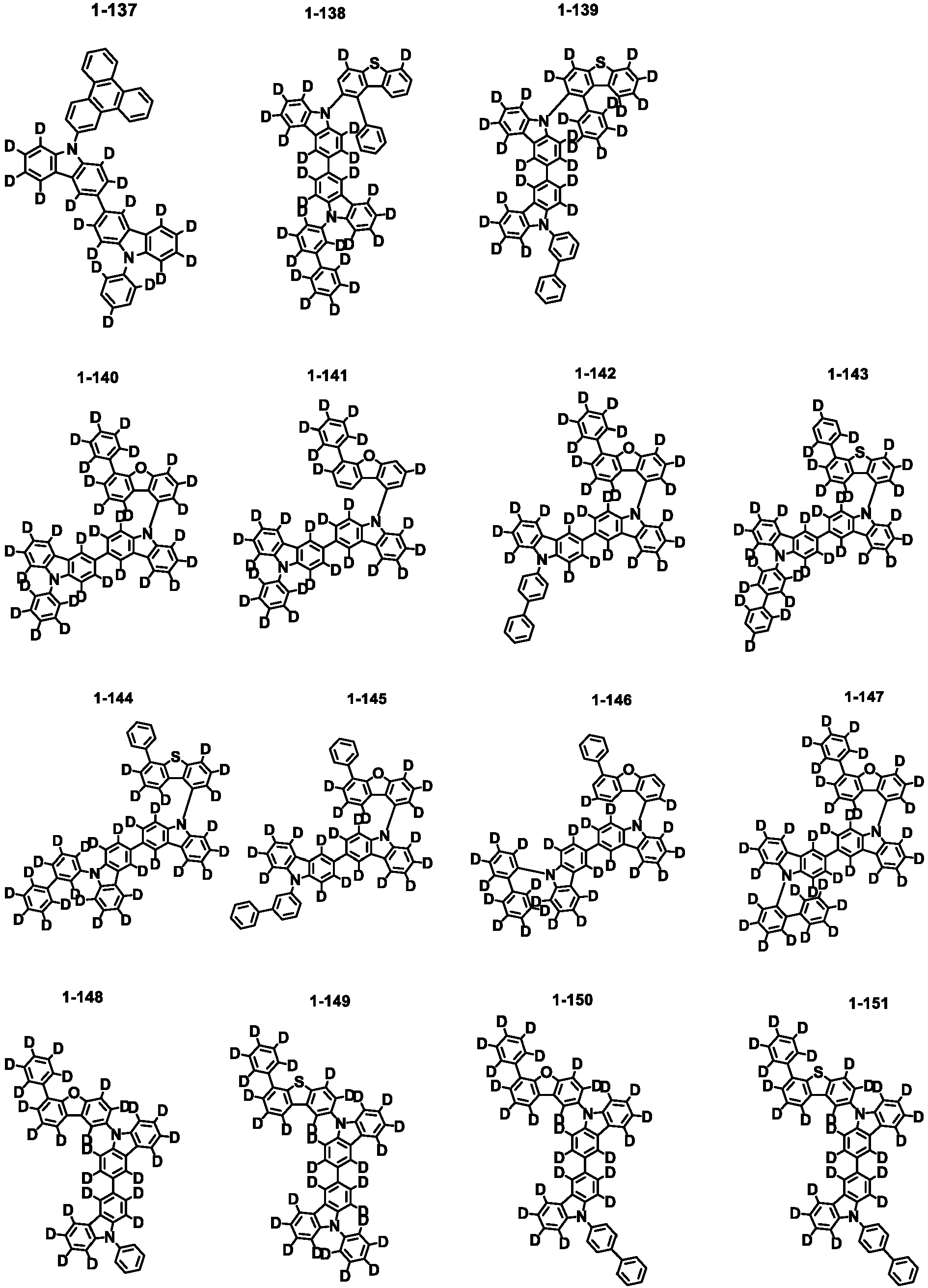

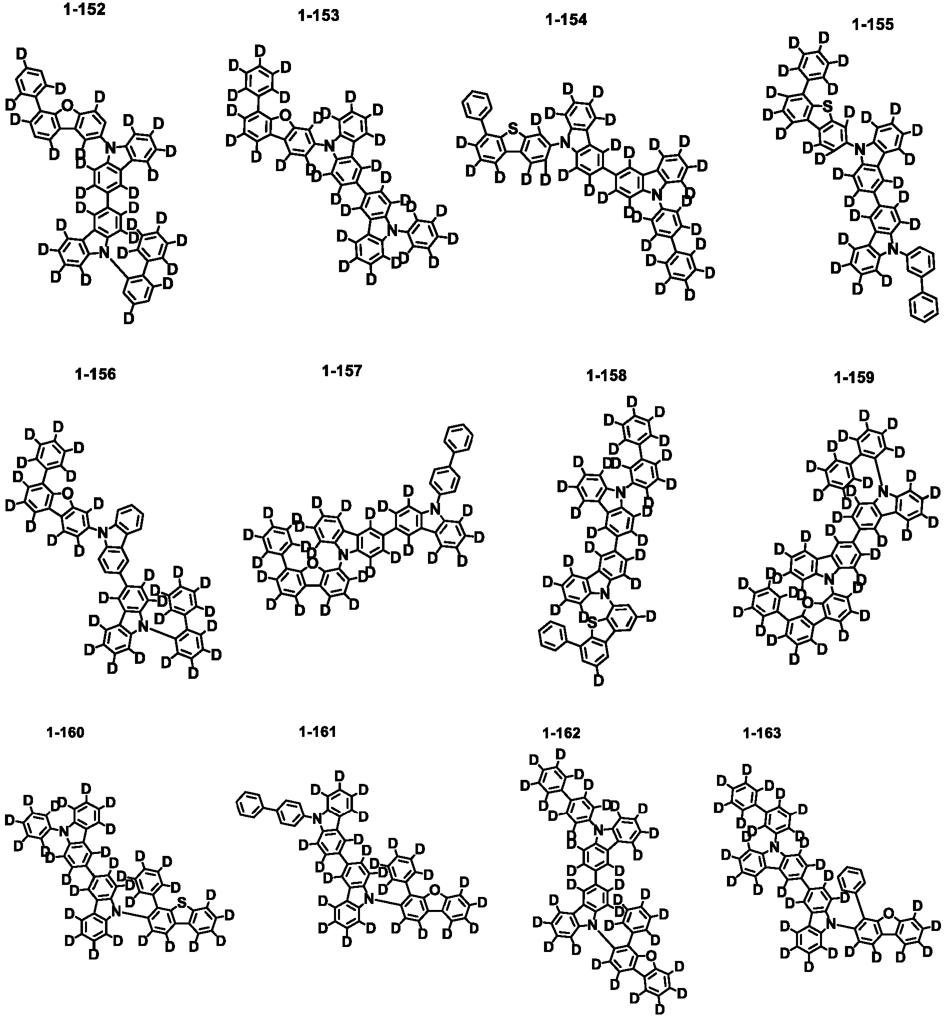

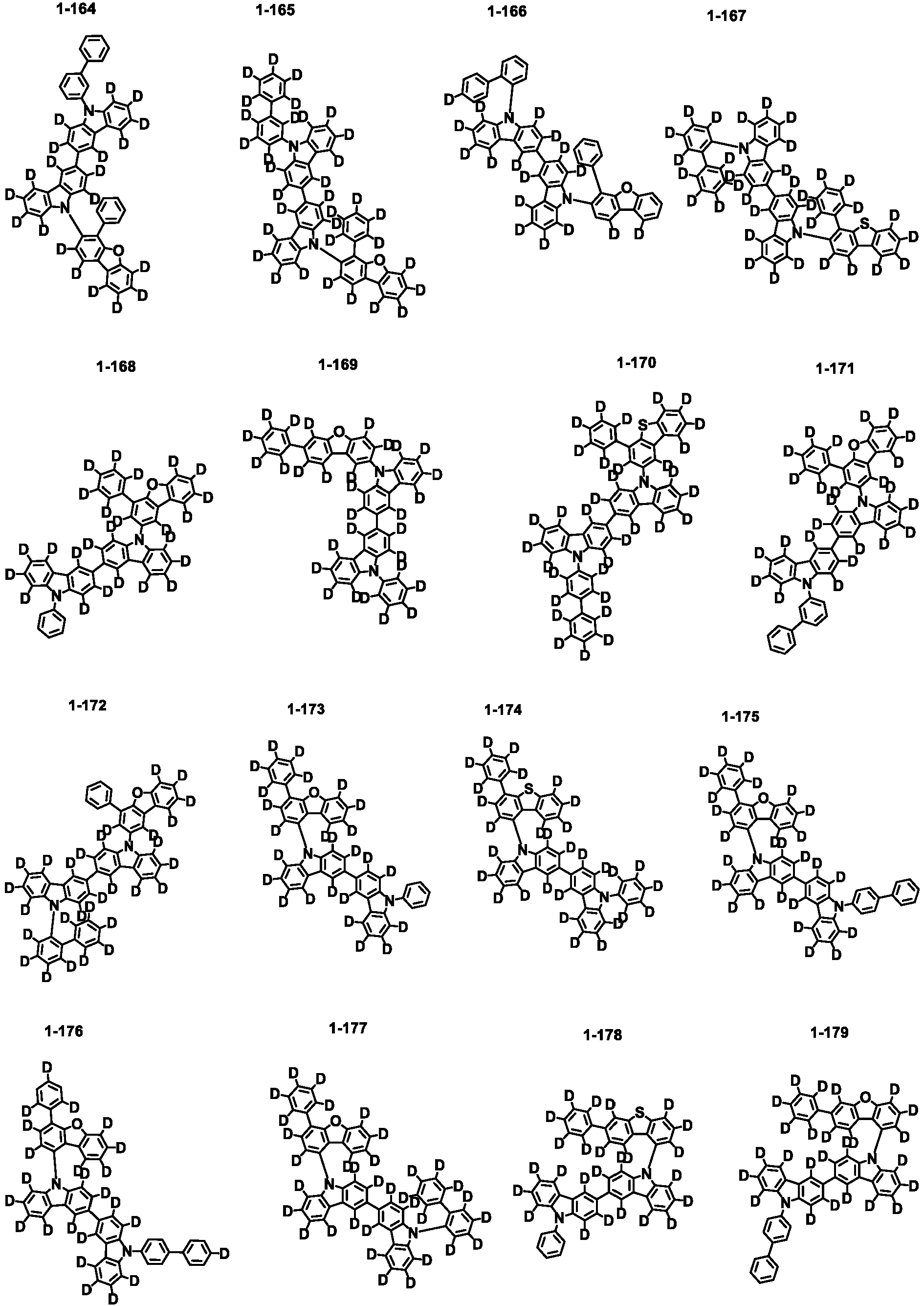

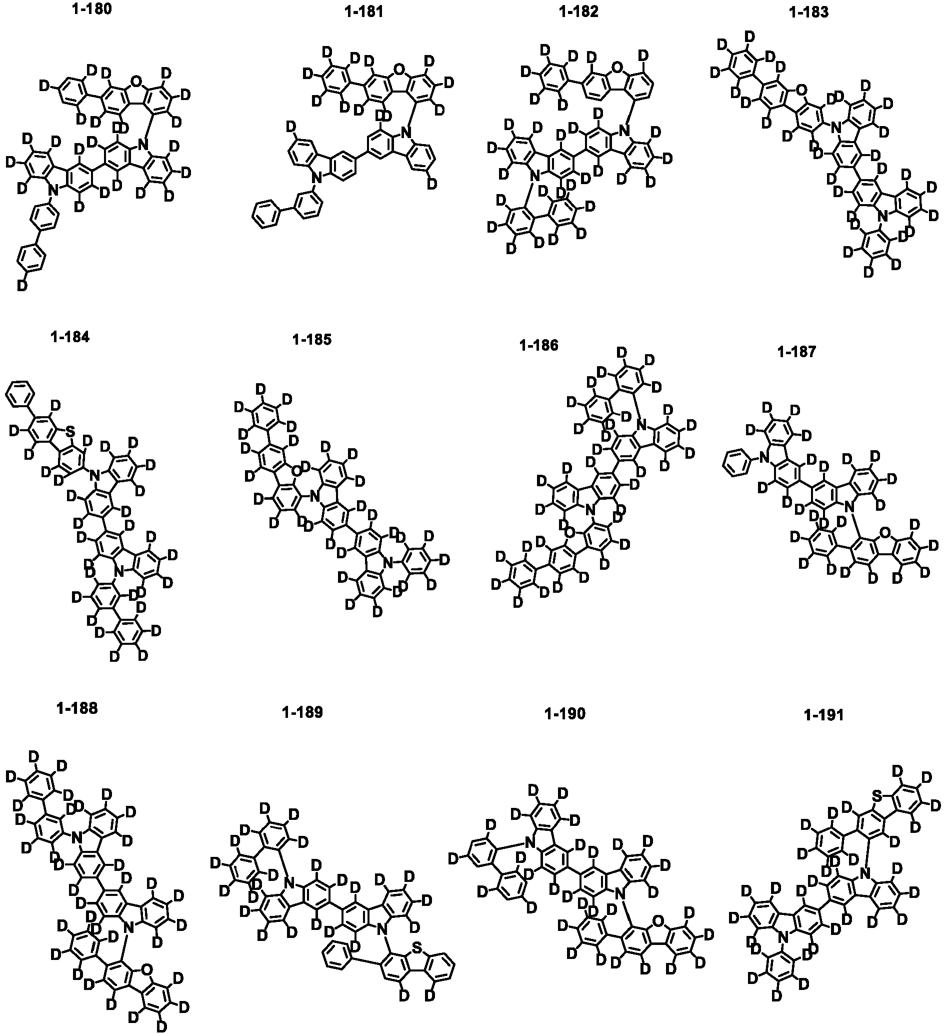

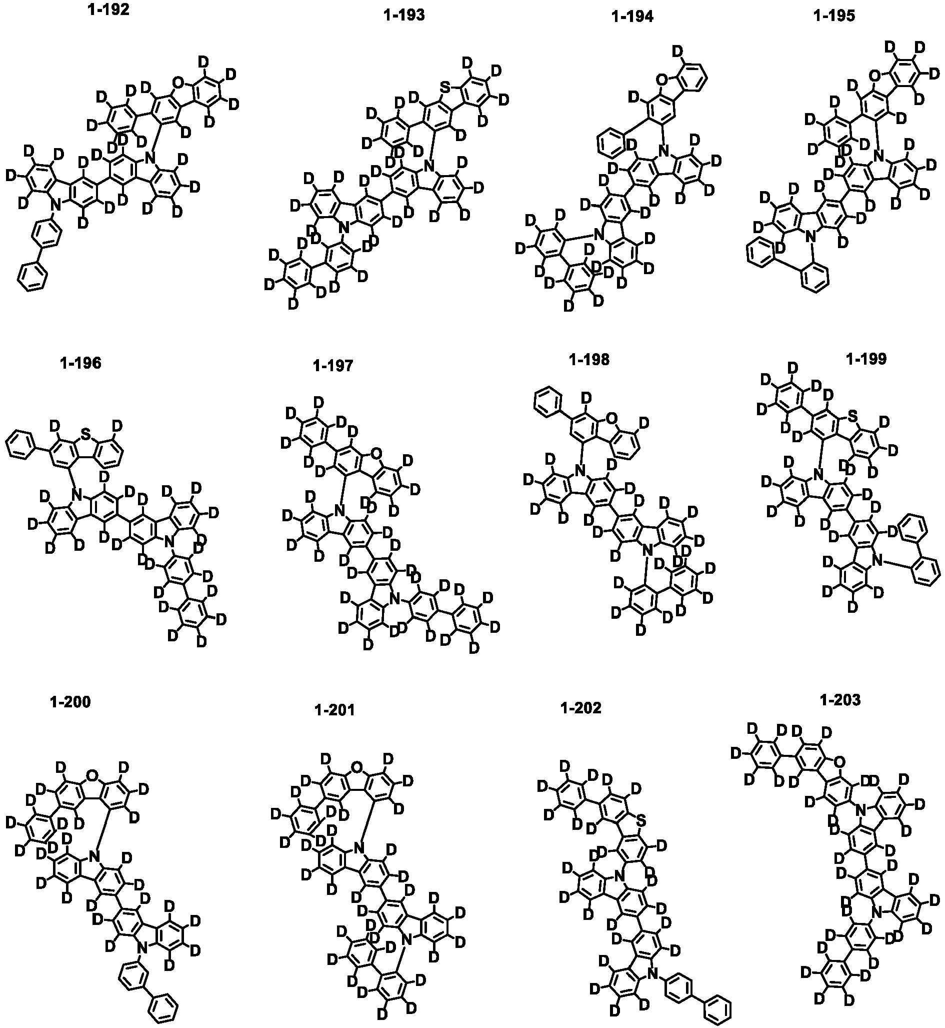

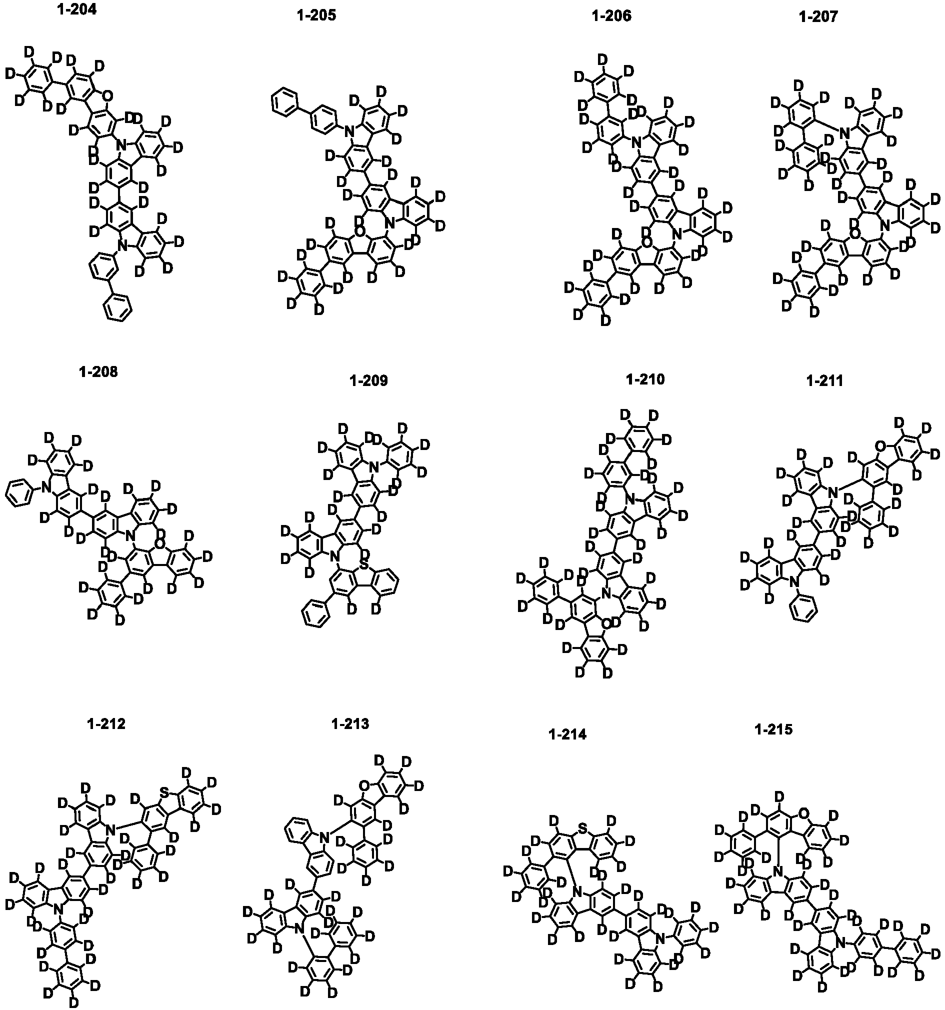

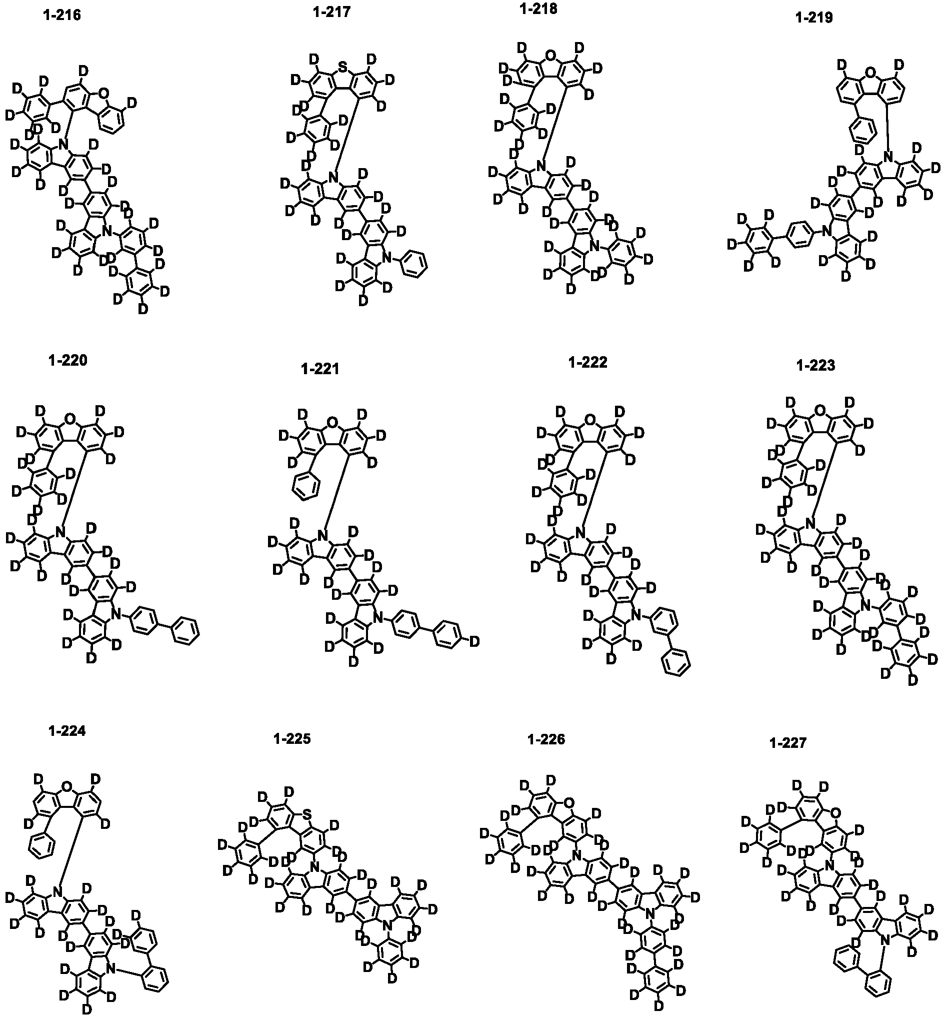

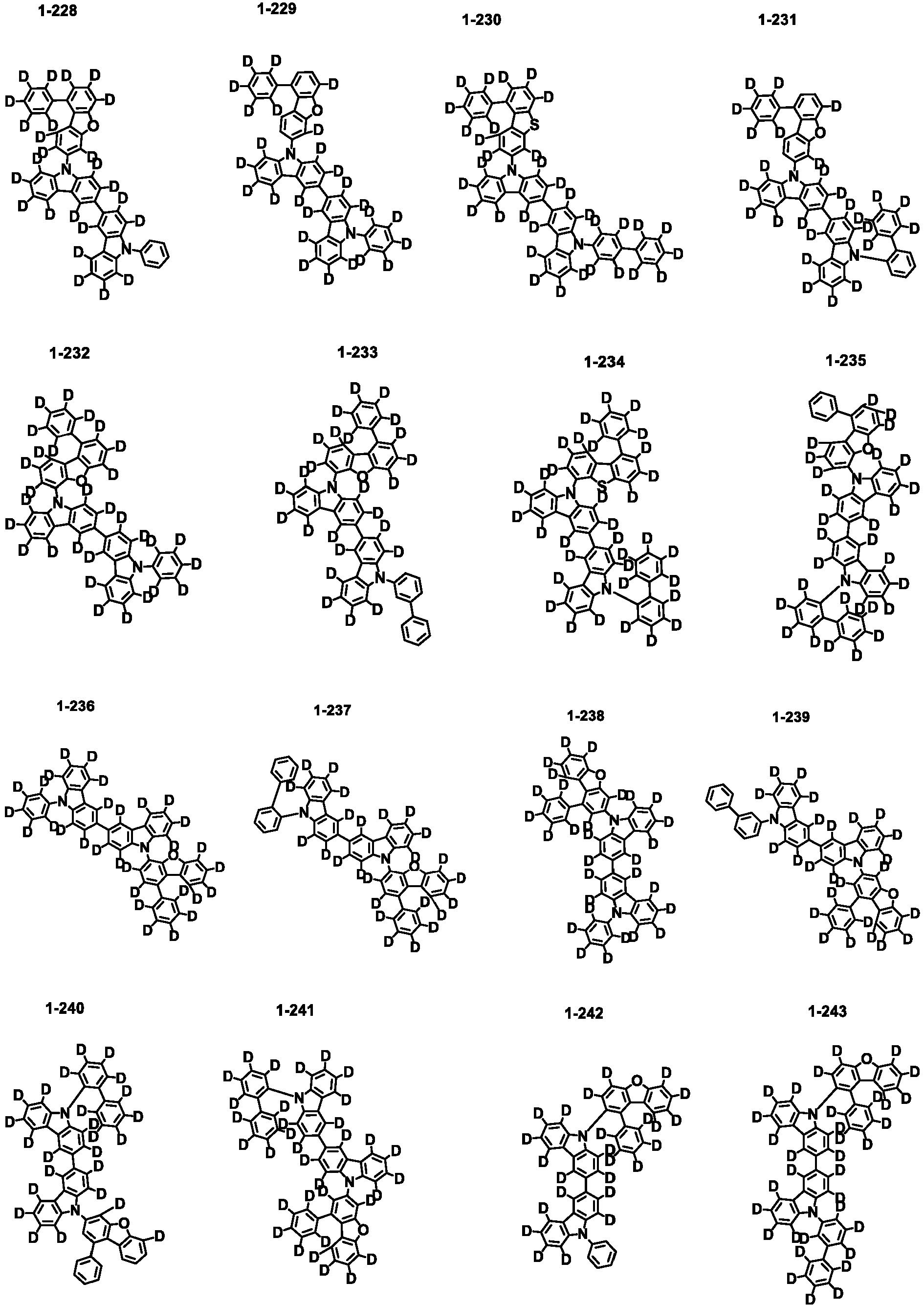

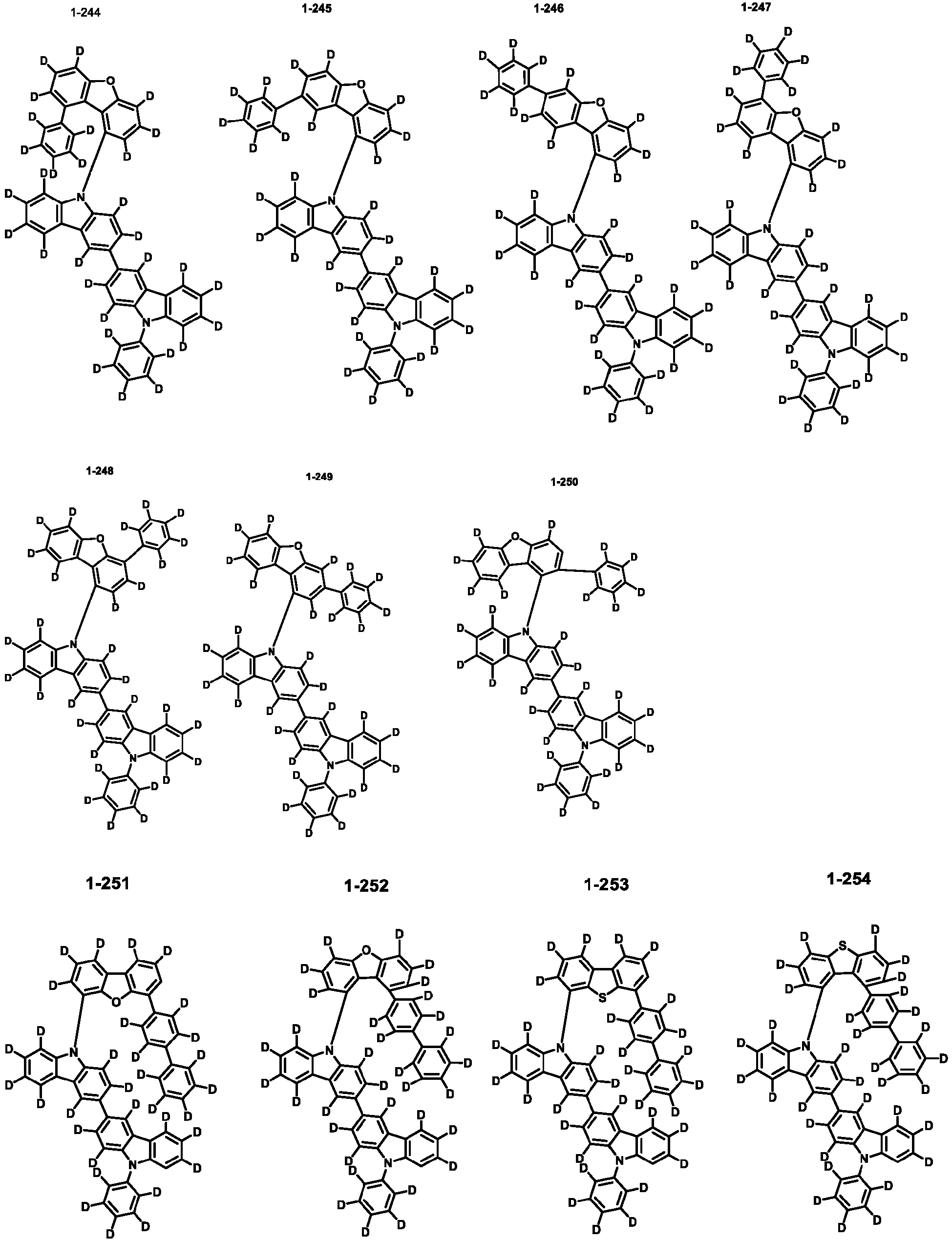

As a more specific example, the compound for an organic optoelectronic device according to the present invention may be represented by Chemical Formula 1-8a,

L¹ and L² may be a single bond or a substituted or unsubstituted phenylene group, and

Ar¹ and Ar² may each be a phenyl group substituted with deuterium, a biphenyl group substituted with deuterium, a terphenyl group substituted with deuterium, or a triphenylene group substituted with deuterium.

The second compound may be represented by a combination of Chemical Formula 2 and Chemical Formula 3.

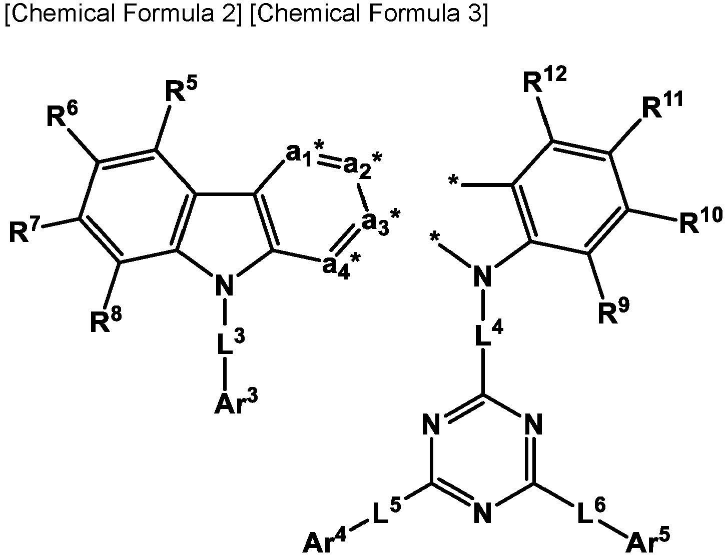

In Chemical Formula 2 and Chemical Formula 3,
Ar³ to Ar⁵ are each independently a substituted or unsubstituted C6 to C20 aryl group or a substituted or unsubstituted C2 to C30 heterocyclic group,
a1 * to a4* in Chemical Formula 2 are each independently a linking carbon (C) or C-L^{a}-R^{a},
adjacent two of a₁* to a₄* in Chemical Formula 2 are each linked to * in Chemical Formula 3,
L², L³, to L⁶ are each independently a single bond, a substituted or unsubstituted C6 to C20 arylene group or a substituted or unsubstituted C2 to C20 heteroarylene group, and
R^{a} and R⁶ to R¹² are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group.

The second compound can be used in the light emitting layer together with the first compound to improve luminous efficiency and life-span characteristics by increasing charge mobility and stability.

For example, the second compound may be represented by any one of Chemical Formula 2A, Chemical Formula 2B, Chemical Formula 2C, Chemical Formula 2D, Chemical Formula 2E, and Chemical Formula 2F.

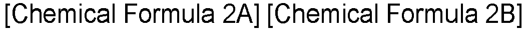

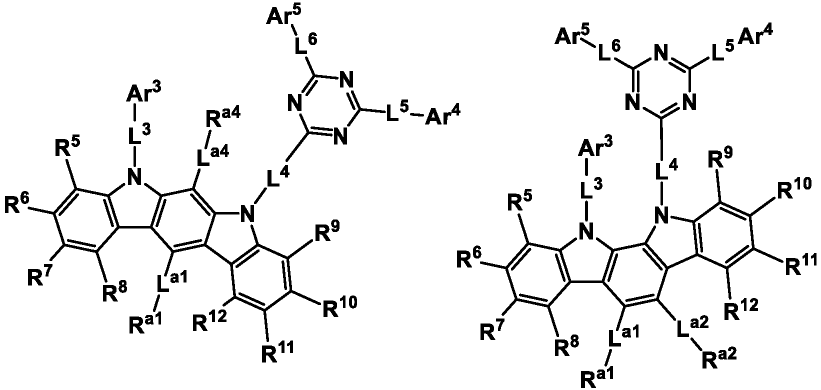

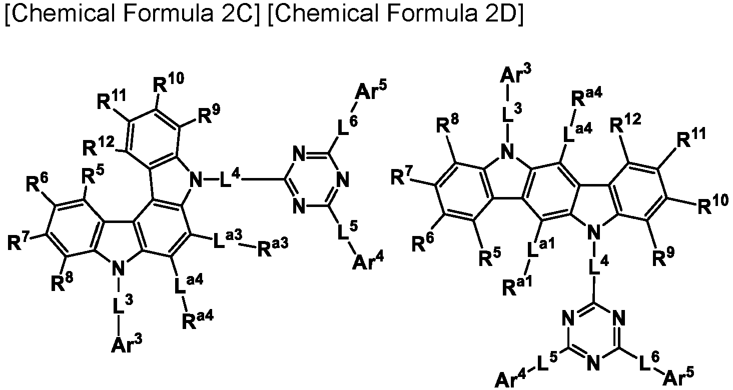

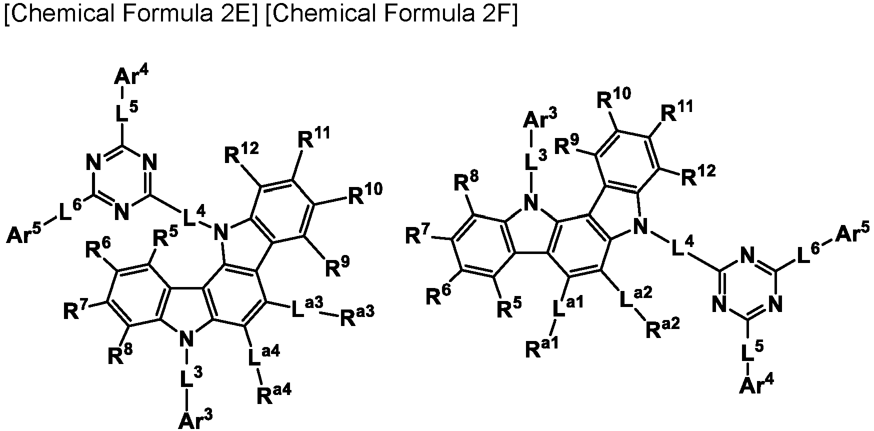

In Chemical Formula 2A to Chemical Formula 2F, Ar³ to Ar⁵, L³ to L⁶, and R⁵ to R¹² are the same as described above,
L^{a1} to L^{a4} are the same as the definitions of L³ to L⁶,
R^{a1} to R^{a4} are the same as the definitions of R⁵ to R¹²

For example, Ar³ to Ar⁵ of Chemical Formulas 2 and 3 may each independently be a substituted or unsubstituted phenyl group, a substituted or unsubstituted biphenyl group, a substituted or unsubstituted terphenyl group, a substituted or unsubstituted quaterphenyl group, a substituted or unsubstituted triphenylene group, a substituted or unsubstituted fluorenyl group, a substituted or unsubstituted pyridinyl group, a substituted or unsubstituted carbazolyl group, a substituted or unsubstituted dibenzofuranyl group, or a substituted or unsubstituted dibenzothiophenyl group,

R^{a1} to R^{a4} and R⁵ to R¹² may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, Ar³ to Ar⁵ in Chemical Formula 2 and Chemical Formula 3 may each be independently selected from the substituents listed in Group II.

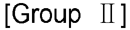

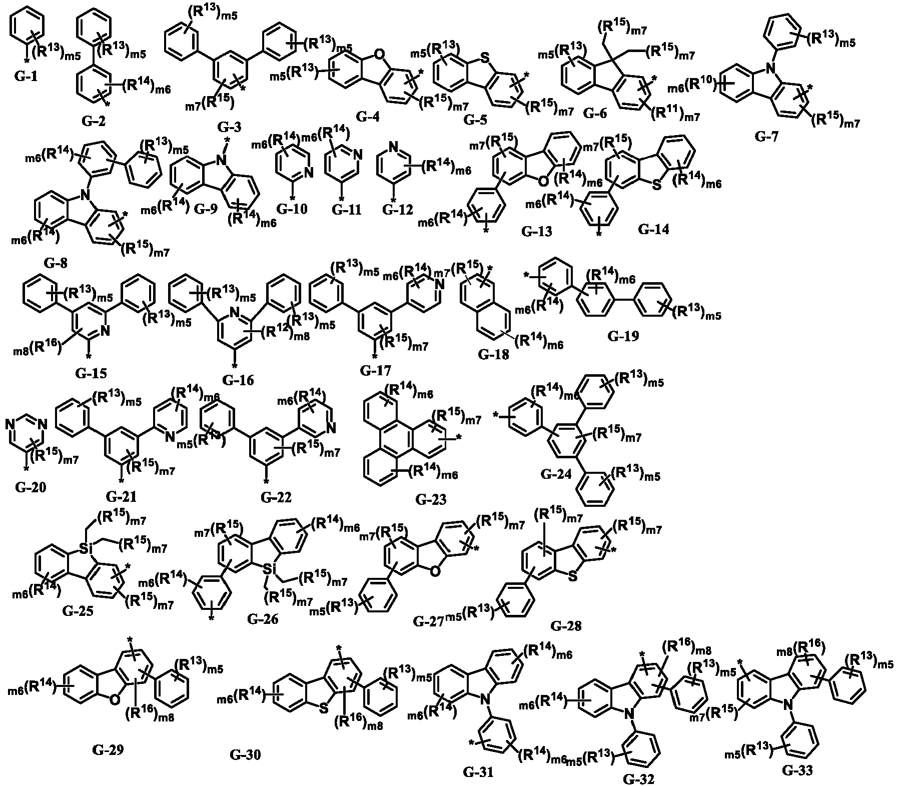

In Group II,
R¹³ to R¹⁶ are each independently hydrogen, deuterium, a cyano group, a substituted or unsubstituted C1 to C10 alkyl group, or a substituted or unsubstituted C6 to C12 aryl group, and
* is a linking point.

In an example embodiment, R^{a1} to R^{a4} and R⁵ to R¹² may independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, R^{a1} to R^{a4} and R⁵ to R¹² may each independently be hydrogen, deuterium, a cyano group, or a substituted or unsubstituted phenyl group.

In a more specific embodiment, R⁵ to R¹² may each independently be hydrogen, deuterium, or a cyano group,
wherein, L³ to L⁶ may be a single bond, a substituted or unsubstituted phenylene group, a substituted or unsubstituted biphenylene group, or a substituted or unsubstituted pyridinylene group, and
Ar³ to Ar⁵ may each independently be a substituted or unsubstituted phenyl group, a substituted or unsubstituted biphenyl group, a substituted or unsubstituted triphenylene group, a substituted or unsubstituted fluorenyl group, a substituted or unsubstituted dibenzofuranyl group, or a substituted or unsubstituted dibenzothiophenyl group.

In a specific embodiment of the present invention, the second compound may be represented by Chemical Formula 2B, wherein in Chemical Formula 2B, L^{a1} and L^{a2} may be a single bond, L³ to L⁶ may each independently be a single bond ora substituted or unsubstituted C6 to C12 arylene group, R⁵ to R¹², R^{a1}, and R^{a2} may each independently be hydrogen, deuterium or a substituted or unsubstituted phenyl group, and Ar³ to Ar⁵ may each independently be a substituted or unsubstituted phenyl group, a substituted or unsubstituted biphenyl group, or a substituted or unsubstituted terphenyl group.

For example, the second compound may be one selected from the compounds of Group 2, but is not limited thereto.

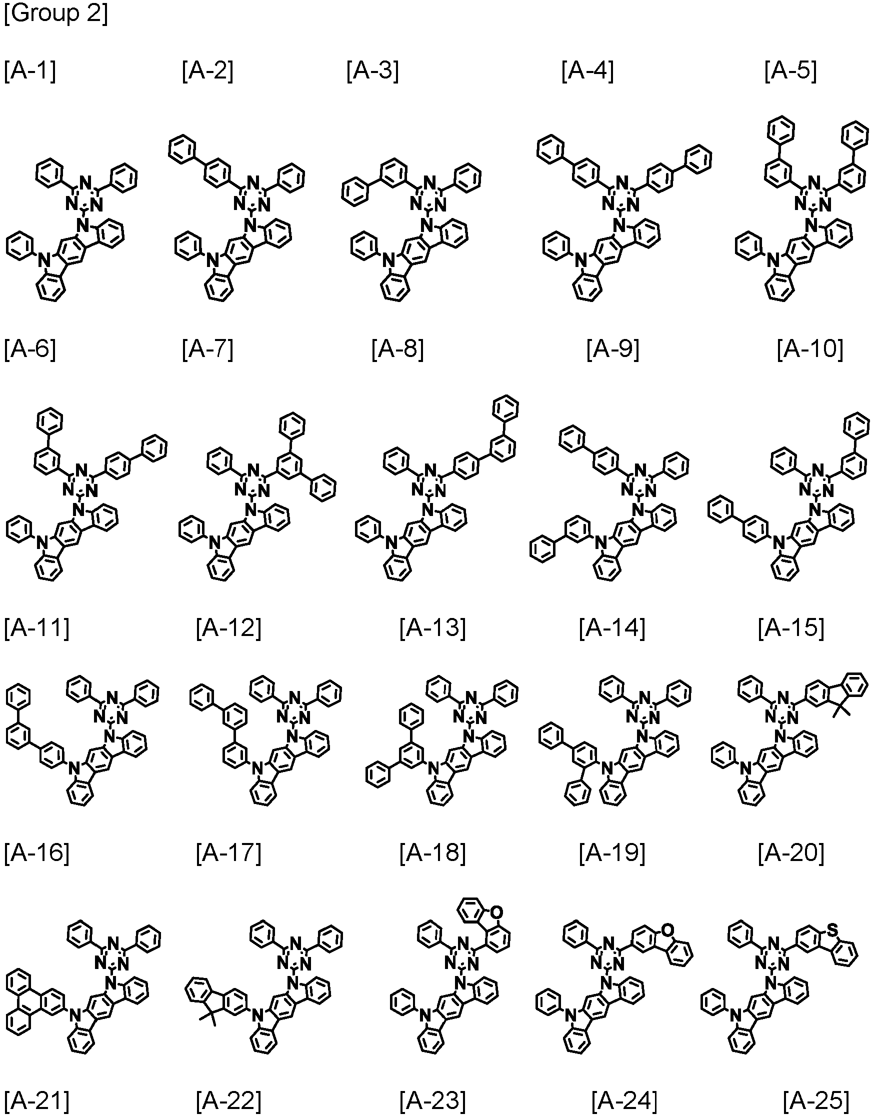

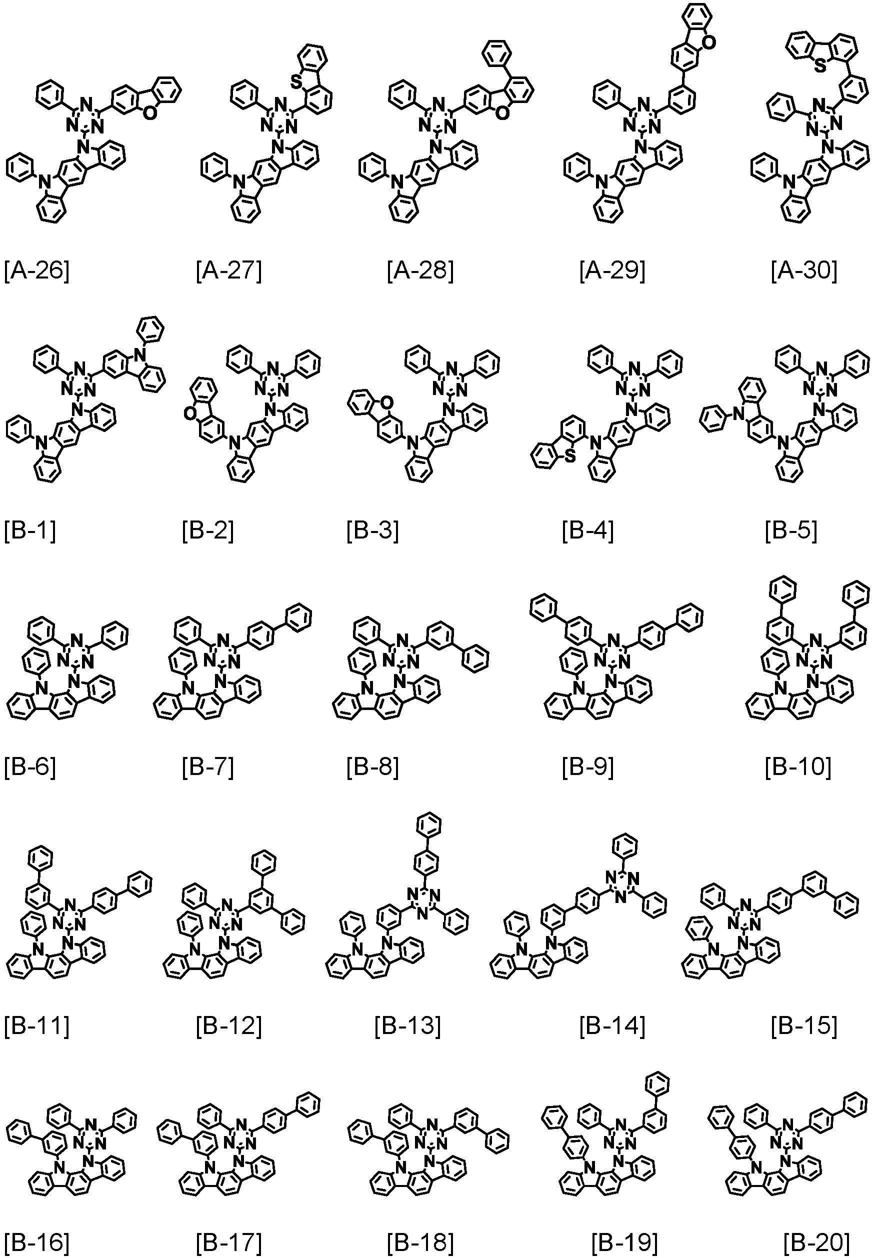

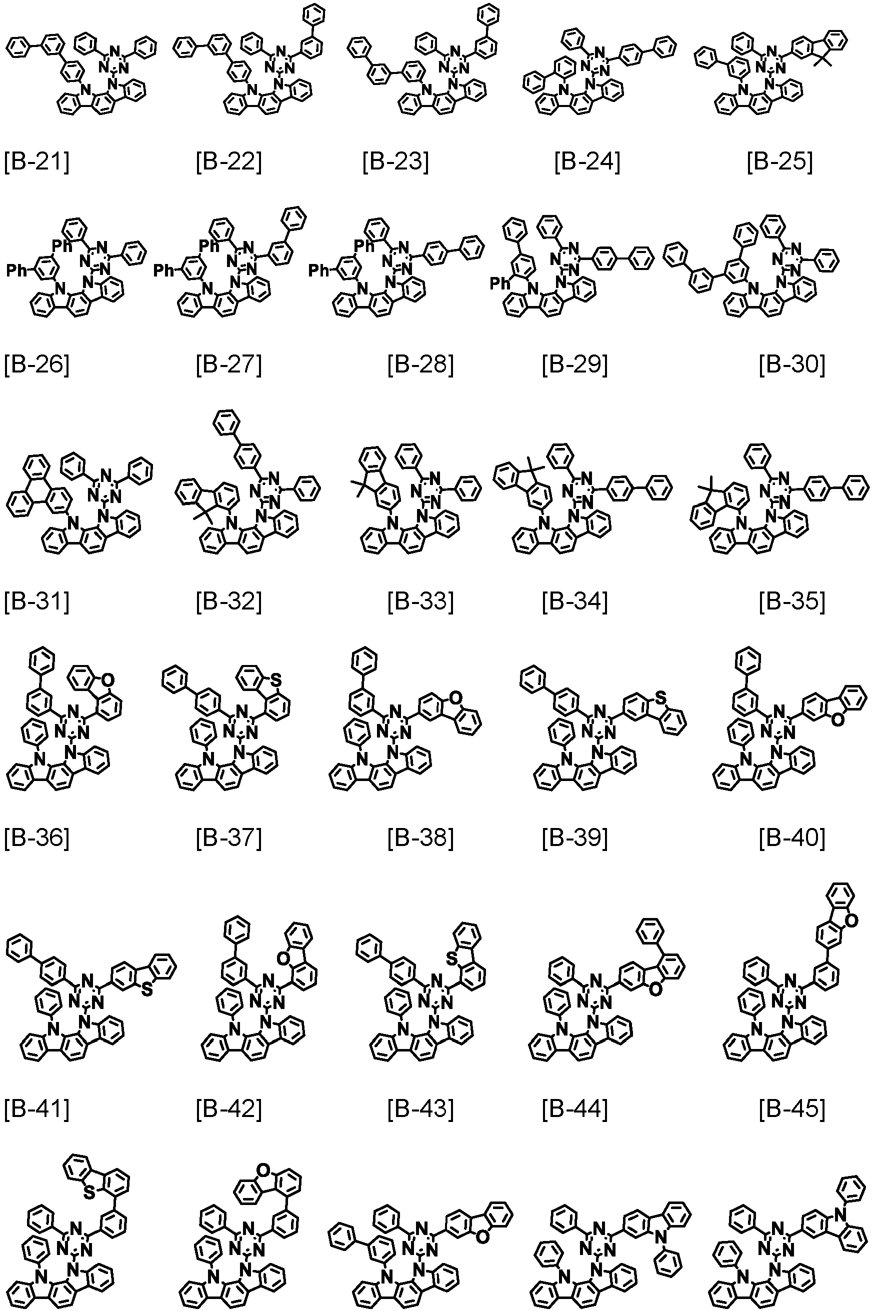

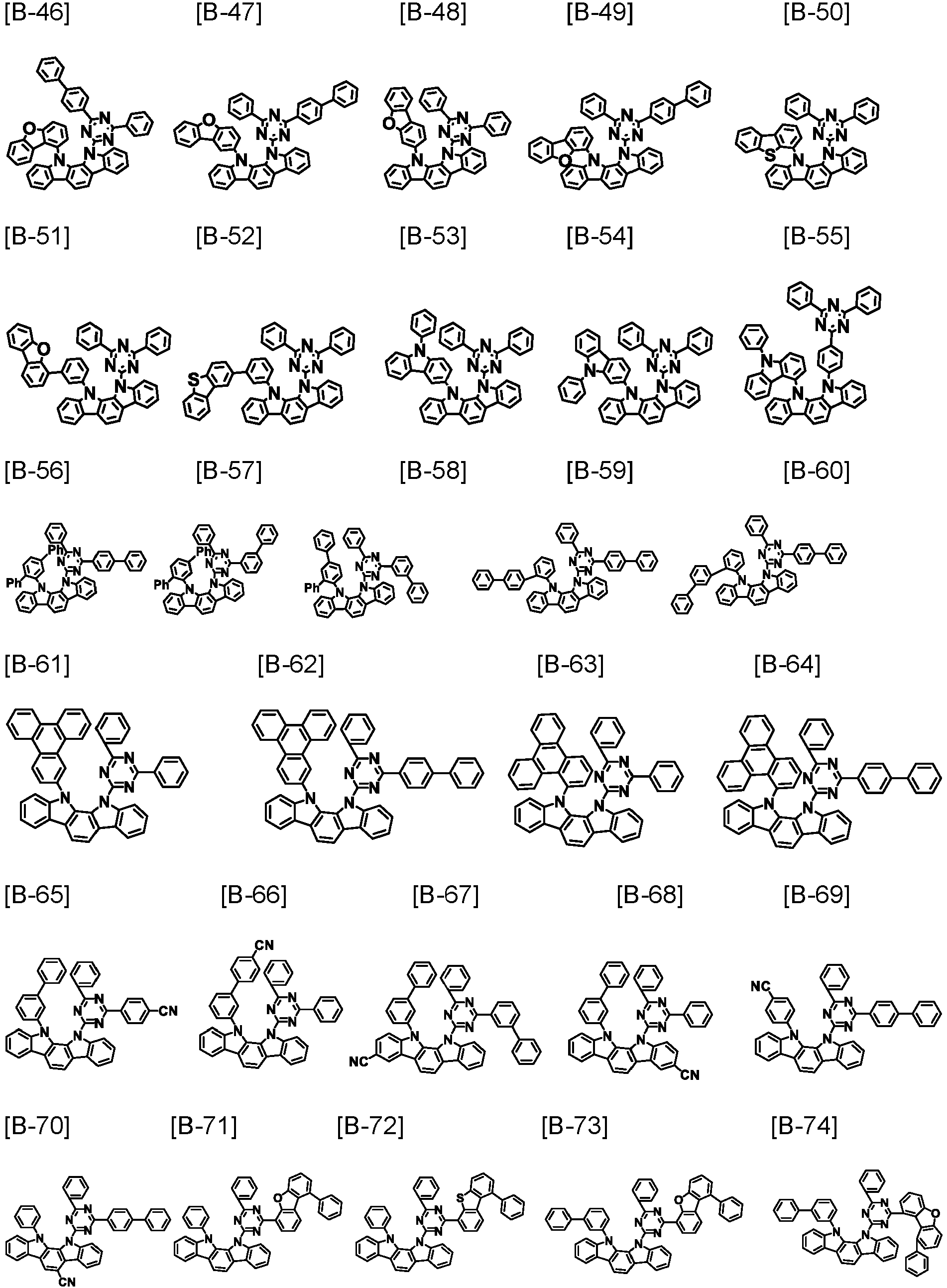

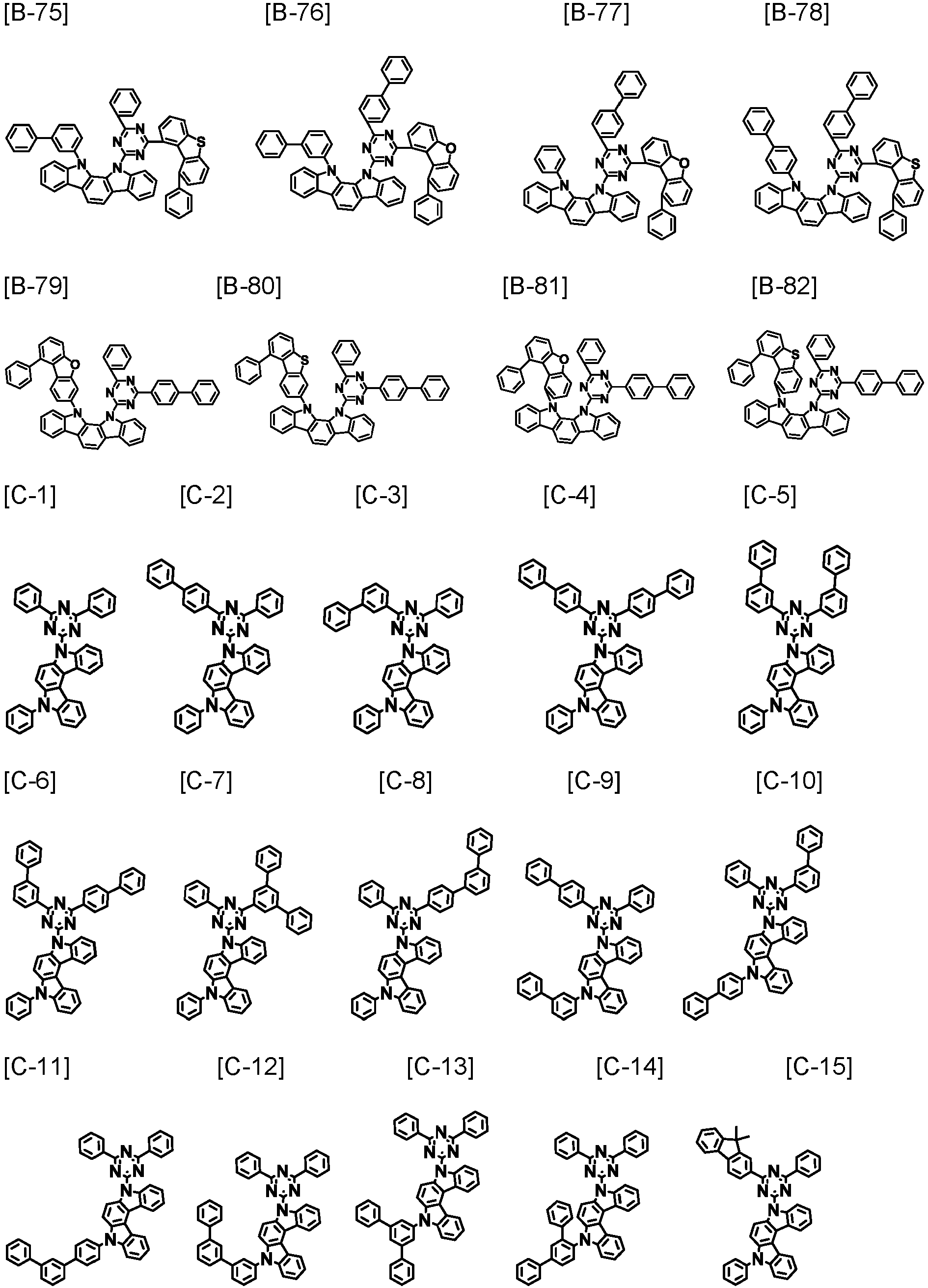

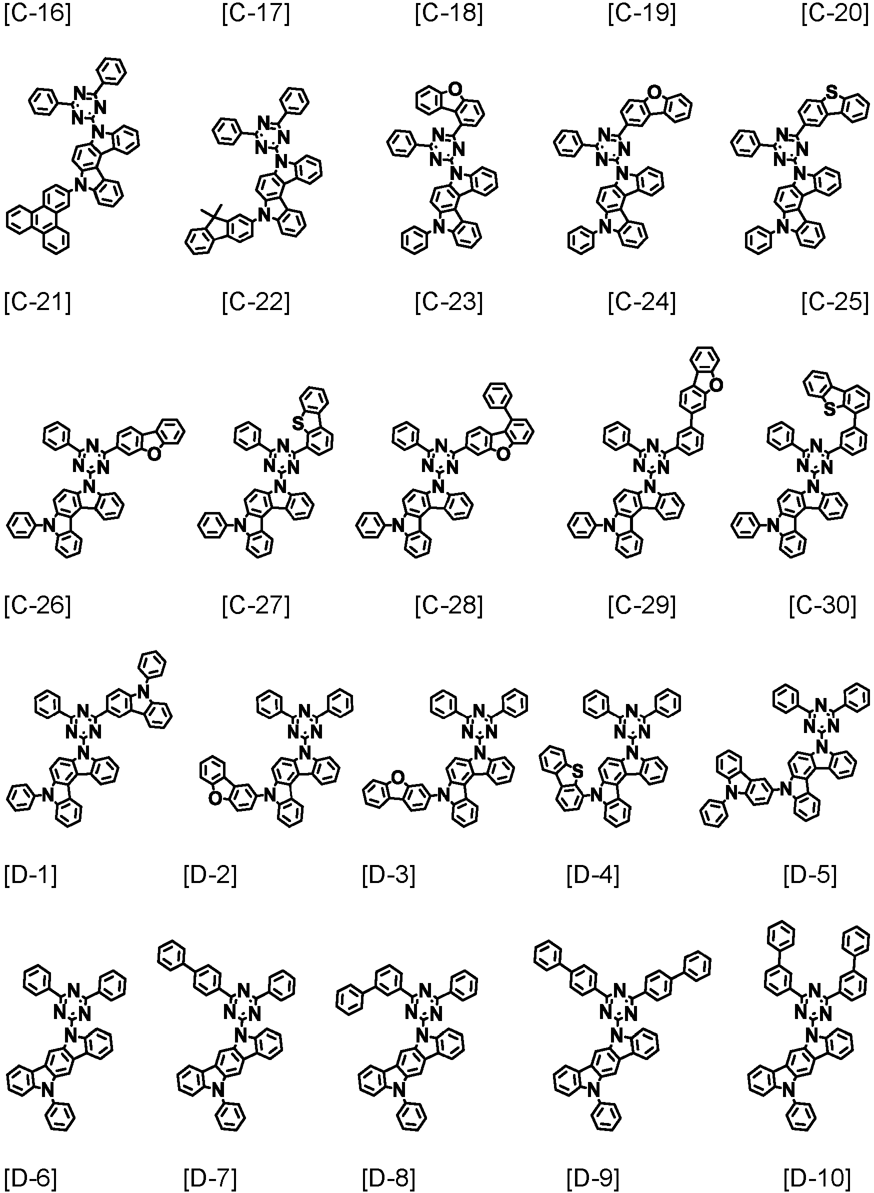

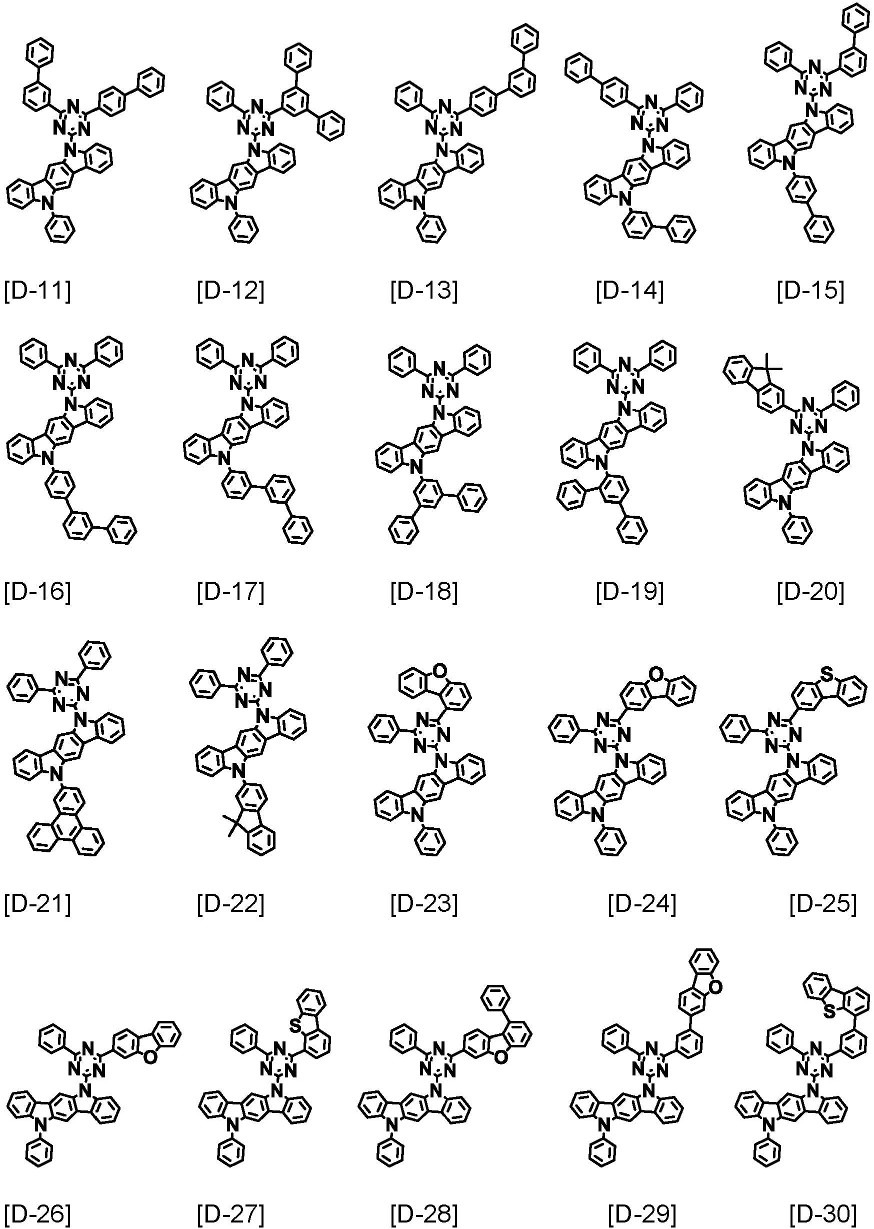

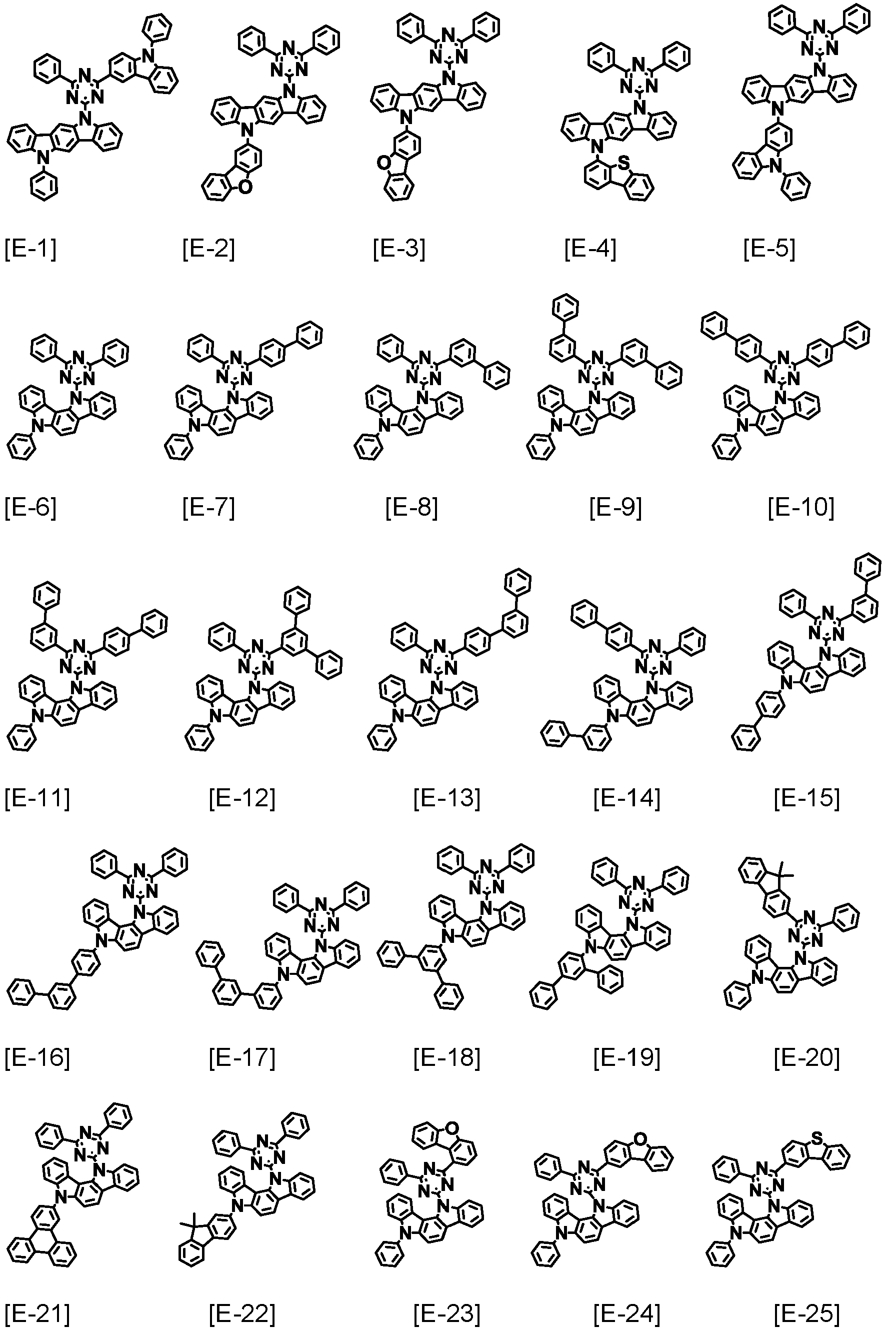

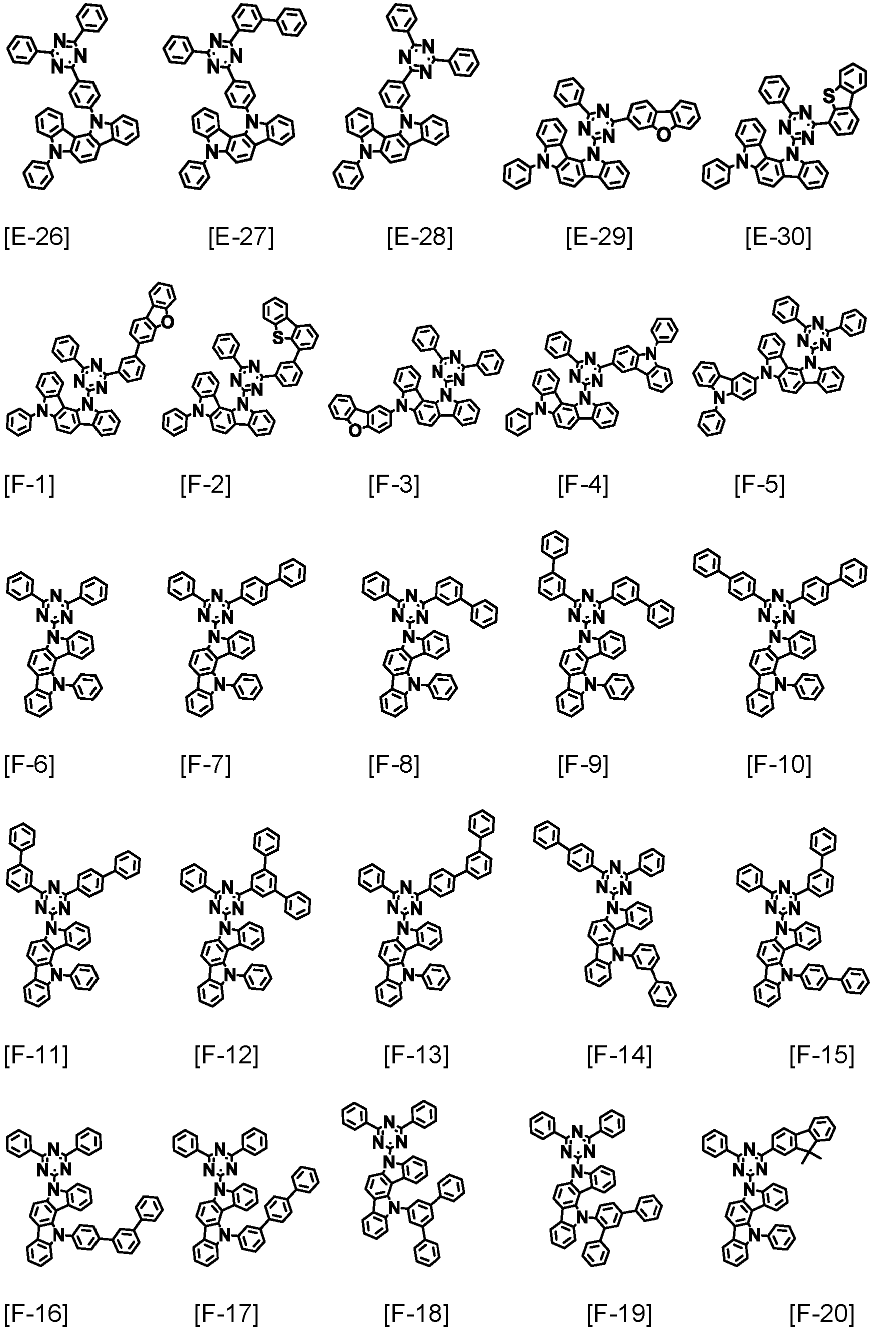

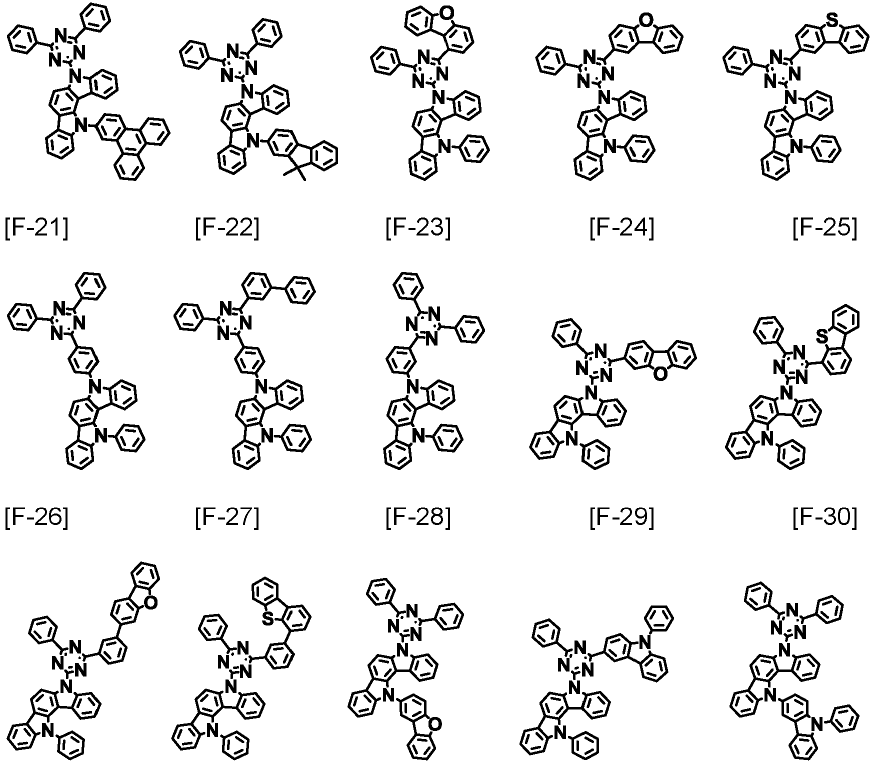

In a more specific embodiment of the present invention, the first compound may be represented by Chemical Formula 1-8a, and the second compound may be represented by Chemical Formula 2B.

The first compound and the second compound may be included in a weight ratio of, for example, 1:99 to 99:1. Within the range, a desirable weight ratio may be adjusted using an electron transport capability of the first compound and a hole transport capability of the second compound to realize bipolar characteristics and thus to improve efficiency and life-span. Within the range, they may be for example included in a weight ratio of 10:90 to 90:10, 20:80 to 80:20, for example, 20:80 to 70:30, 20:80 to 60:40, or 30:70 to 60:40. As a specific example, it may be included in a weight ratio of 40:60, 50:50, or 60:40.

In addition to the first and second compounds described above, one or more compounds may be further included.

For example, the composition for an organic optoelectronic device described above may further include a dopant.

The dopant may be, for example, a phosphorescent dopant, for example a phosphorescent dopant of red, green or blue, and may be, for example, a red phosphorescent dopant.

The dopant is a material mixed with the composition for an organic optoelectronic device in a small amount to cause light emission and may be generally a material such as a metal complex that emits light by multiple excitation into a triplet or more. The dopant may be, for example, an inorganic, organic, or organic-inorganic compound, and may include one or two or more types.

An example of the dopant may be a phosphorescent dopant, and examples of the phosphorescent dopant may include an organometallic compound including Ir, Pt, Os, Ti, Zr, Hf, Eu, Tb, Tm, Fe, Co, Ni, Ru, Rh, Pd, or a combination thereof. The phosphorescent dopant may be, for example, a compound represented by Chemical Formula Z, but is not limited thereto.

[Chemical Formula Z] L⁷MX

In Chemical Formula Z, M is a metal, and L⁷ and X are the same or different, and are a ligand to form a complex compound with M.

The M may be for example Ir, Pt, Os, Ti, Zr, Hf, Eu, Tb, Tm, Fe, Co, Ni, Ru, Rh, Pd, or a combination thereof, and L⁷ and X may be, for example a bidendate ligand.

Examples of the ligands represented by L⁷ and X may be selected from the formulas listed in Group A, but are not limited thereto.

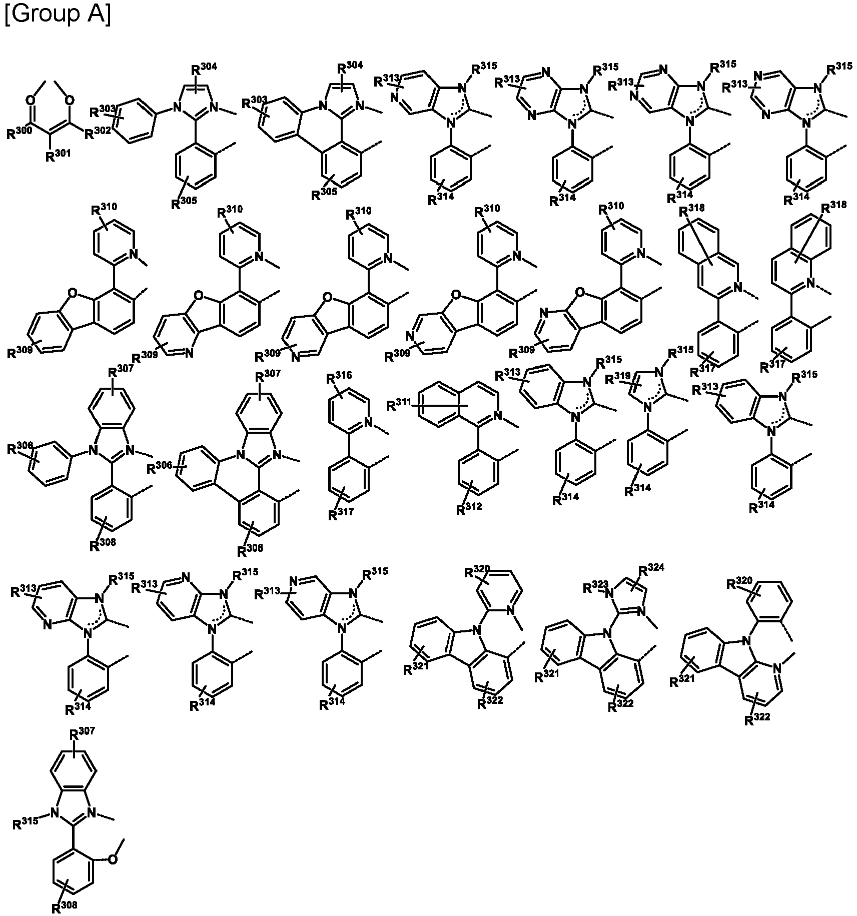

In Group A,
R³⁰⁰ to R³⁰² are each independently hydrogen, deuterium, a C1 to C30 alkyl group that is substituted or unsubstituted with a halogen, a C6 to C30 aryl group that is substituted or unsubstituted with a C1 to C30 alkyl substitution or a halogen, and
R³⁰³ to R³²⁴ are each independently hydrogen, deuterium, a halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, SFs, a trialkylsilyl group having a substituted or unsubstituted C1 to C30 alkyl group, a dialkylarylsilyl group having a substituted or unsubstituted C1 to C30 alkyl group and C6 to C30 aryl group, or a triarylsilyl group having a substituted or unsubstituted C6 to C30 aryl group.

For example, a dopant represented by Chemical Formula V may be included.

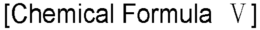

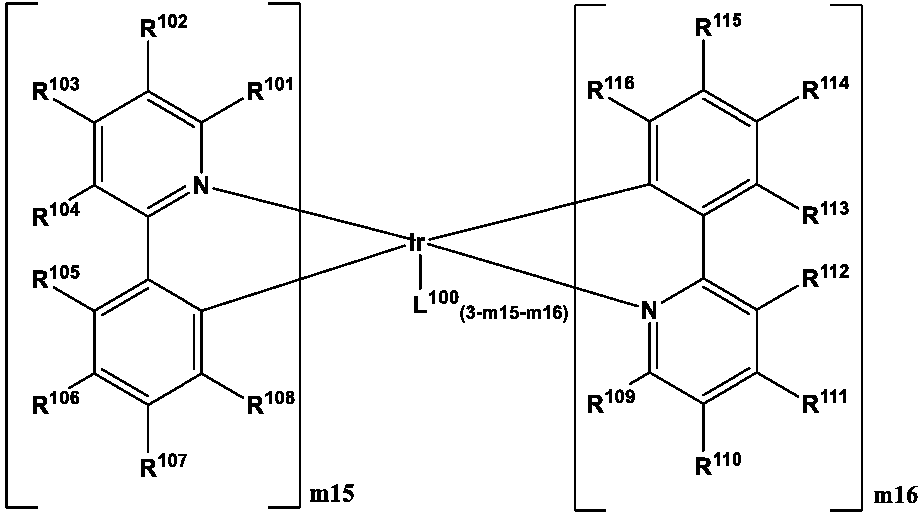

In Chemical Formula V,
R¹⁰¹ to R¹¹⁶ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group,
at least one of R¹⁰¹ to R¹¹⁶ is a functional group represented by Chemical Formula V-1,
L¹⁰⁰ is a bidentate ligand of a monovalent anion, which coordinates to iridium through a lone pair of electrons on carbon or a heteroatom, and
m15 and m16 are each independently any one of integers of 0 to 3, and m15 + m16 is any one of integers of 1 to 3, 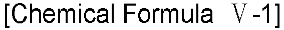 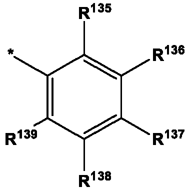
wherein, in Chemical Formula V-1,
R¹³⁵ to R¹³⁹ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group, and
* refers to the portion linked to the carbon atom.

For example, the dopant represented by Chemical Formula Z-1 may be included.

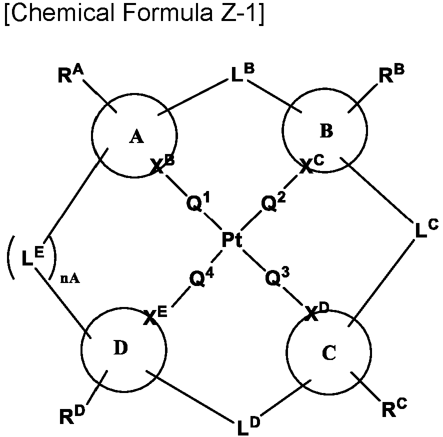

In Chemical Formula Z-1, rings A, B, C, and D are each independently 5- or 6-membered carbocyclic or heterocyclic ring;
R^{A}, R^{B}, R^{C}, and R^{D} each independently represent mono-, di-, tri-, or tetra-substitution, or unsubstitution;
L^{B}, L^{C}, and L^{D} are each independent selected from a direct bond, BR, NR, PR, O, S, Se, C=O, S=O, SO₂, CRR', SiRR', GeRR', and a combination thereof;
when nA is 1, L^{E} is selected from a direct bond, BR, NR, PR, O, S, Se, C=O, S=O, SO₂, CRR', SiRR', GeRR', and a combination thereof; when nA is 0, L^{E} does not exist; and
R^{A}, R^{B}, R^{C}, R^{D}, R, and R' are each independently selected from hydrogen, deuterium, halogen, an alkyl group, a cycloalkyl group, a heteroalkyl group, an arylalkyl group, an alkoxy group, an aryloxy group, an amino group, a silyl group, an alkenyl group, a cycloalkenyl group, a heteroalkenyl group, an alkynyl group, an aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, and a combination thereof; any adjacent R^{A}, R^{B}, R^{C}, R^{D}, R, and R' are optionally linked to form a ring; X^{B}, X^{C}, X^{D}, and X^{E} are each independently selected from carbon and nitrogen; and Q¹, Q², Q³, and Q⁴ each represents an oxygen or a direct bond.

The dopant according to an embodiment may be a platinum complex, and may be represented by Chemical Formula VI.

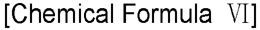

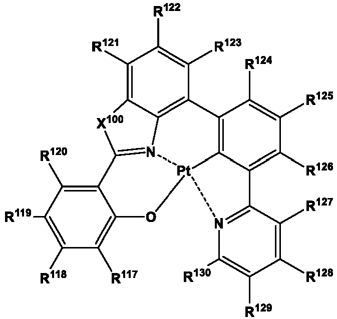

In Chemical Formula VI,
X¹⁰⁰ is selected from O, S and NR¹³¹,
R¹¹⁷ to R¹³¹ are each independently hydrogen, deuterium, a substituted or unsubstituted C1 to C10 alkyl group, a substituted or unsubstituted C6 to C20 aryl group, or - SiR¹³²R¹³³R¹³⁴,
R¹³² to R¹³⁴ are each independently a C1 to C6 alkyl group, and
at least one of R¹¹⁷ to R¹³¹ is -SiR¹³²R¹³³R¹³⁴ or tert-butyl group.

Hereinafter, an organic optoelectronic device to which the aforementioned compound for an organic optoelectronic device is applied will be described.

The organic optoelectronic device may be any device to convert electrical energy into photoenergy and vice versa without particular limitation, and may be, for example an organic photoelectric device, an organic light emitting diode, an organic solar cell, and an organic photo-conductor drum.

Herein, an organic light emitting diode as one example of an organic optoelectronic device is described referring to drawings.

FIG. 1 is a cross-sectional view showing organic light emitting diodes according to embodiments.

Referring to FIG. 1, an organic light emitting diode 100 according to an embodiment includes an anode 120 and a cathode 110 facing each other and an organic layer 105 disposed between the anode 120 and cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a small work function to help electron injection, and may be for example a metal, a metal oxide, and/or a conductive polymer. The cathode 110 may be for example a metal such as magnesium, calcium, sodium, potassium, titanium, indium, yttrium, lithium, gadolinium, aluminum silver, tin, lead, cesium, barium, and the like, or an alloy thereof; a multi-layer structure material such as LiF/Al, LiO₂/Al, LiF/Ca, and BaF₂/Ca, but is not limited thereto.

The organic layer 105 may include the aforementioned composition for an organic optoelectronic device.

The organic layer 105 may include the light emitting layer 130, and the light emitting layer 130 may include the aforementioned composition for an organic optoelectronic device.

The composition for an organic optoelectronic device further including a dopant may be, for example, a red light emitting composition.

The light emitting layer 130 may include, for example, the aforementioned composition for an organic optoelectronic device as a phosphorescent host.

The organic layer may further include a charge transport region in addition to the light emitting layer.

The charge transport region may be, for example, the hole transport region 140.

The hole transport region 140 may further increase hole injection and/or hole mobility between the anode 120 and the light emitting layer 130 and block electrons.

Specifically, the hole transport region 140 may include a hole transport layer between the anode 120 and the light emitting layer 130, and a hole transport auxiliary layer between the light emitting layer 130 and the hole transport layer, and at least one of the compounds of Group B may be included in at least one of the hole transport layer and the hole transport auxiliary layer.

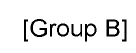

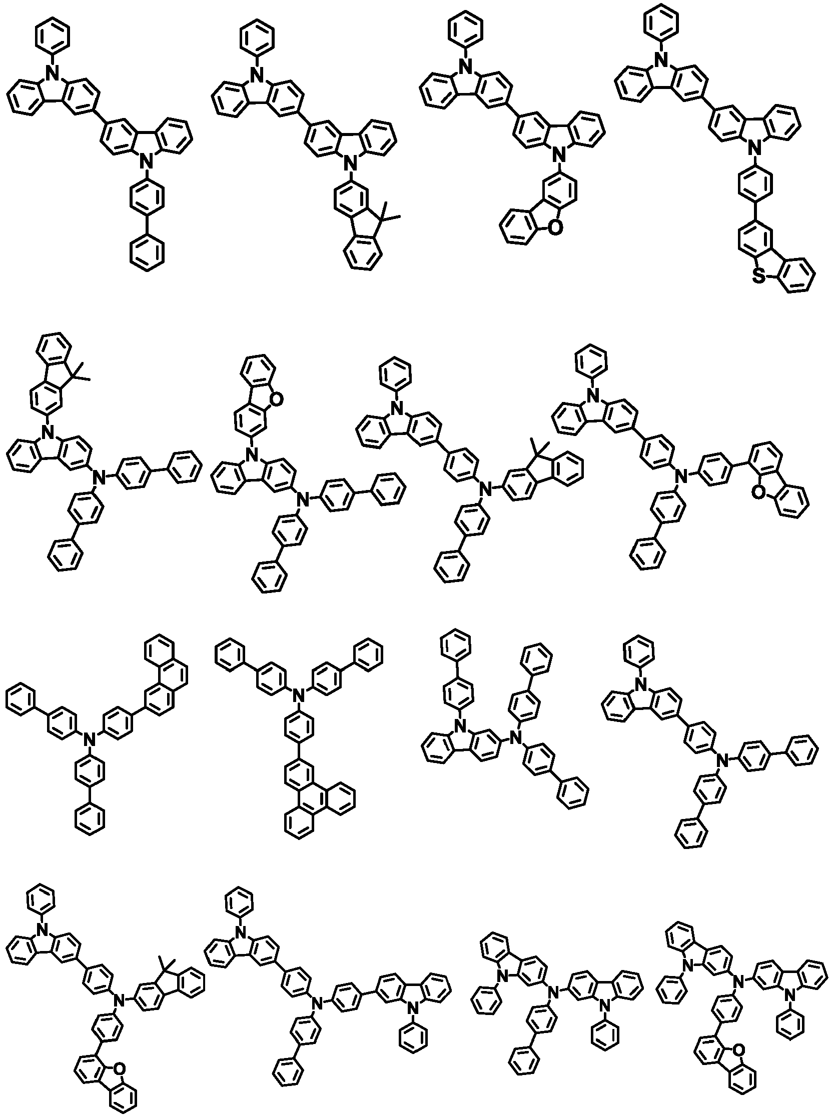

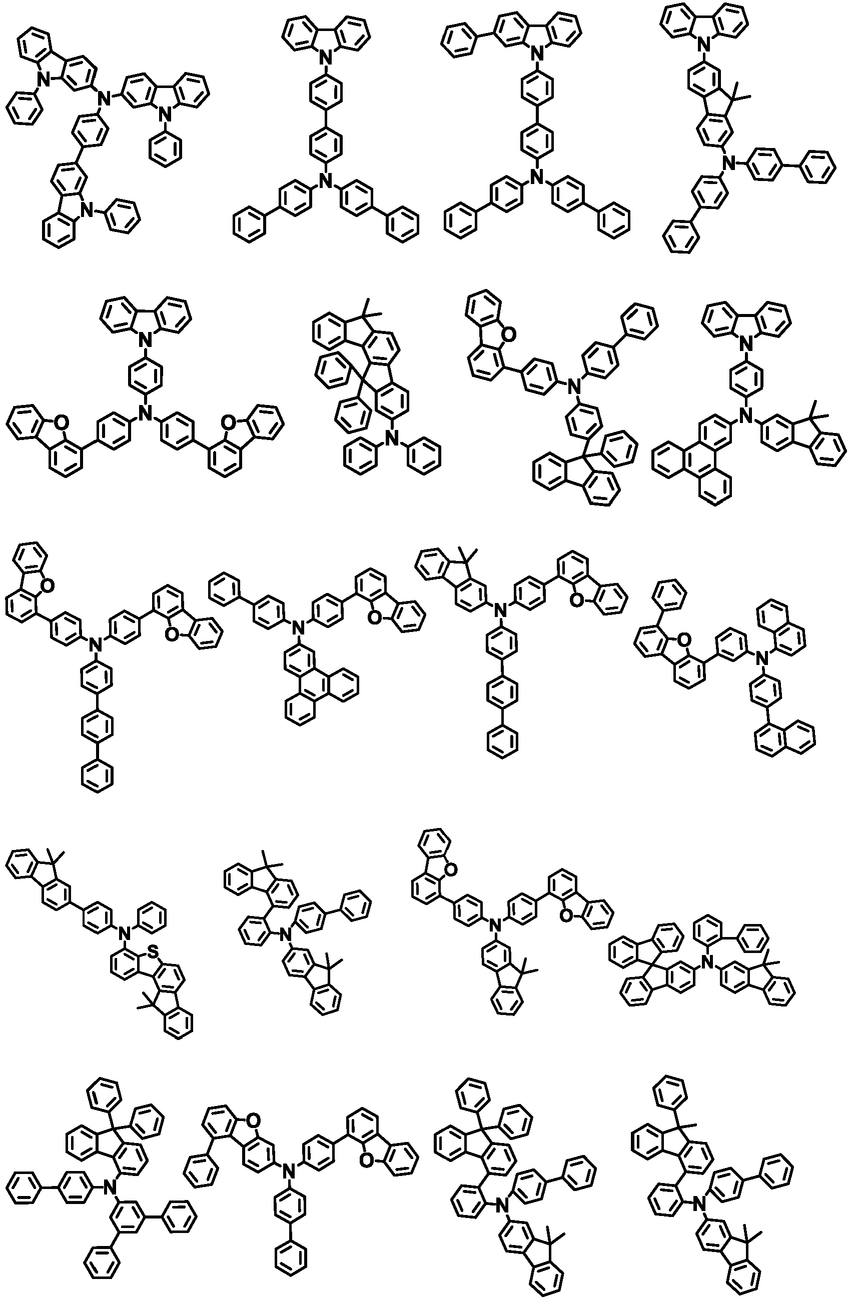

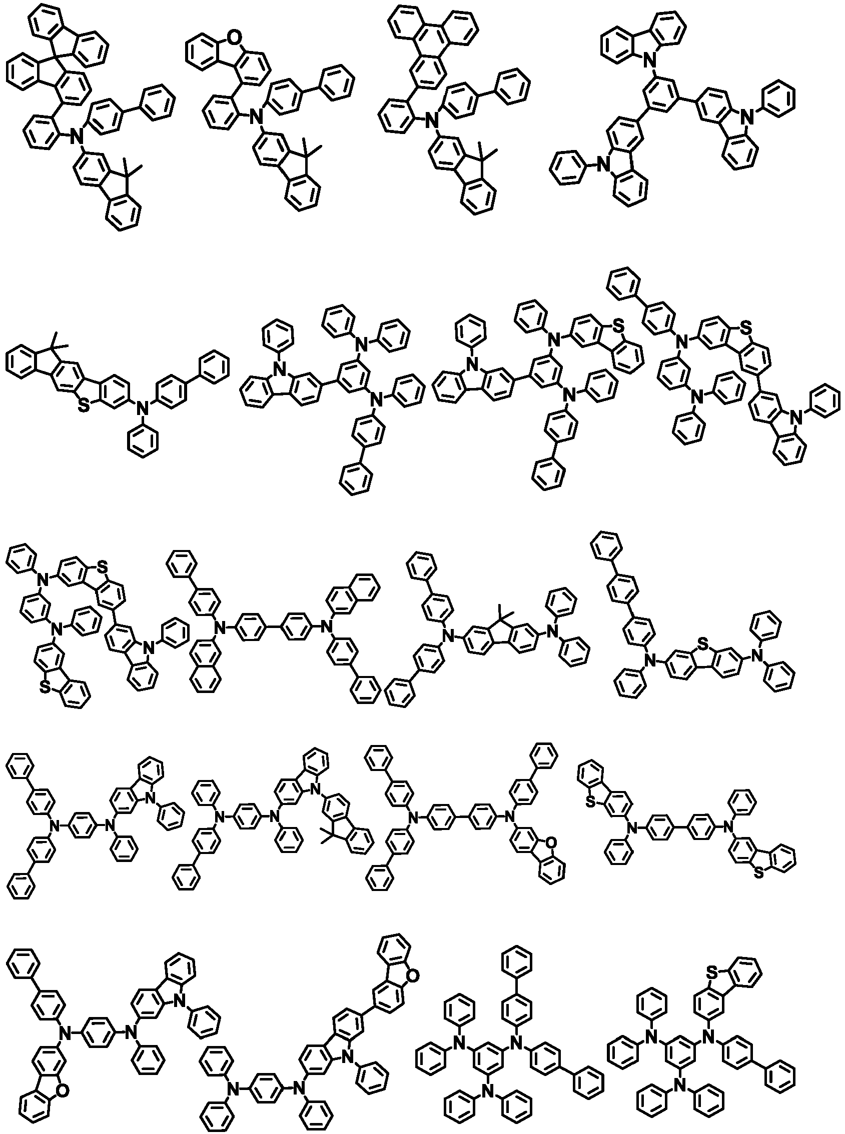

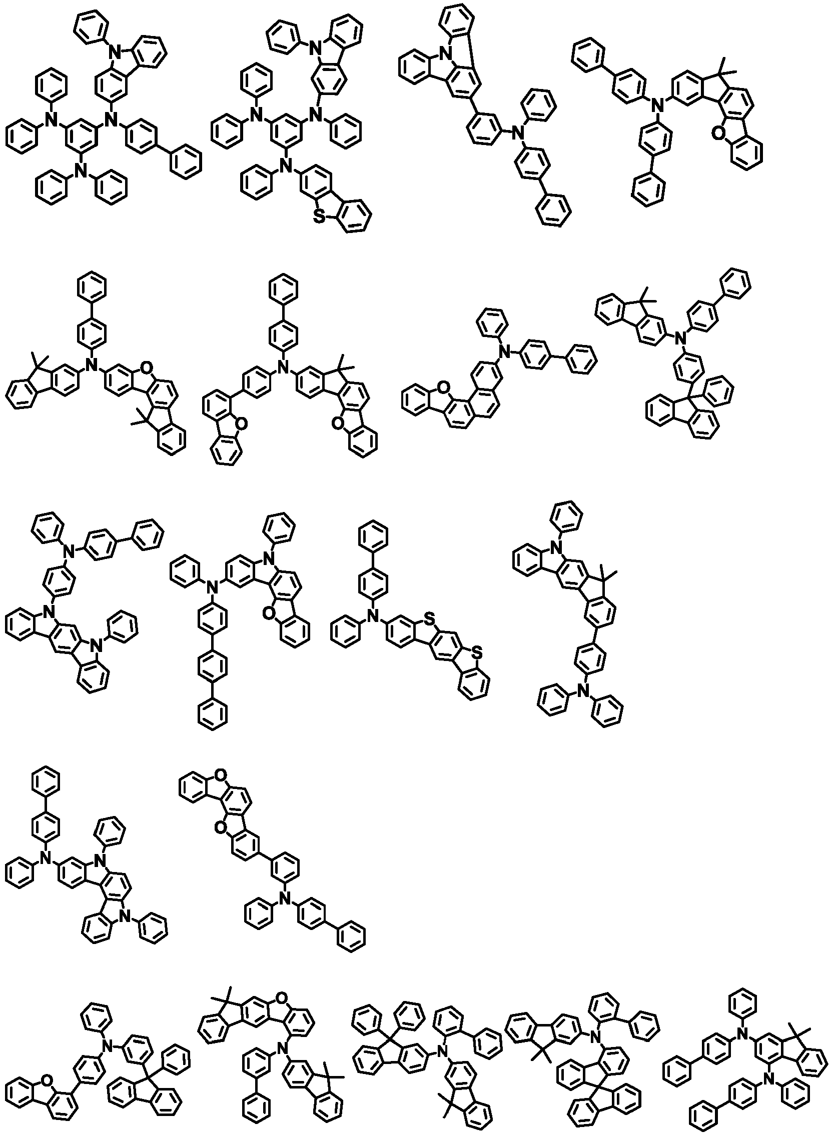

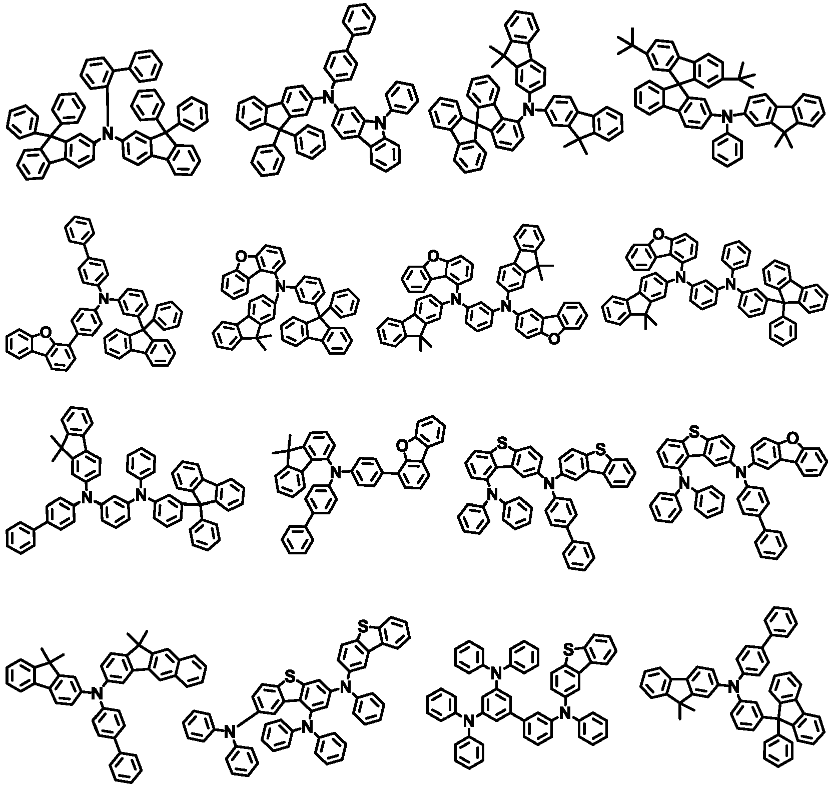

In the hole transport region, in addition to the compounds described above, known compounds disclosed in US5061569A, JP1993-009471A, WO1995-009147A1, JP1995-126615A, JP1998-095973A, etc. and compounds having a similar structure may also be used.

Also, the charge transport region may be, for example, the electron transport region 150.

The electron transport region 150 may further increase electron injection and/or electron mobility and block holes between the cathode 110 and the light emitting layer 130.

Specifically, the electron transport region 150 may include an electron transport layer between the cathode 110 and the light emitting layer 130, and an electron transport auxiliary layer between the light emitting layer 130 and the electron transport layer, and at least one of the compounds of Group C may be included in at least one of the electron transport layer and the electron transport auxiliary layer.

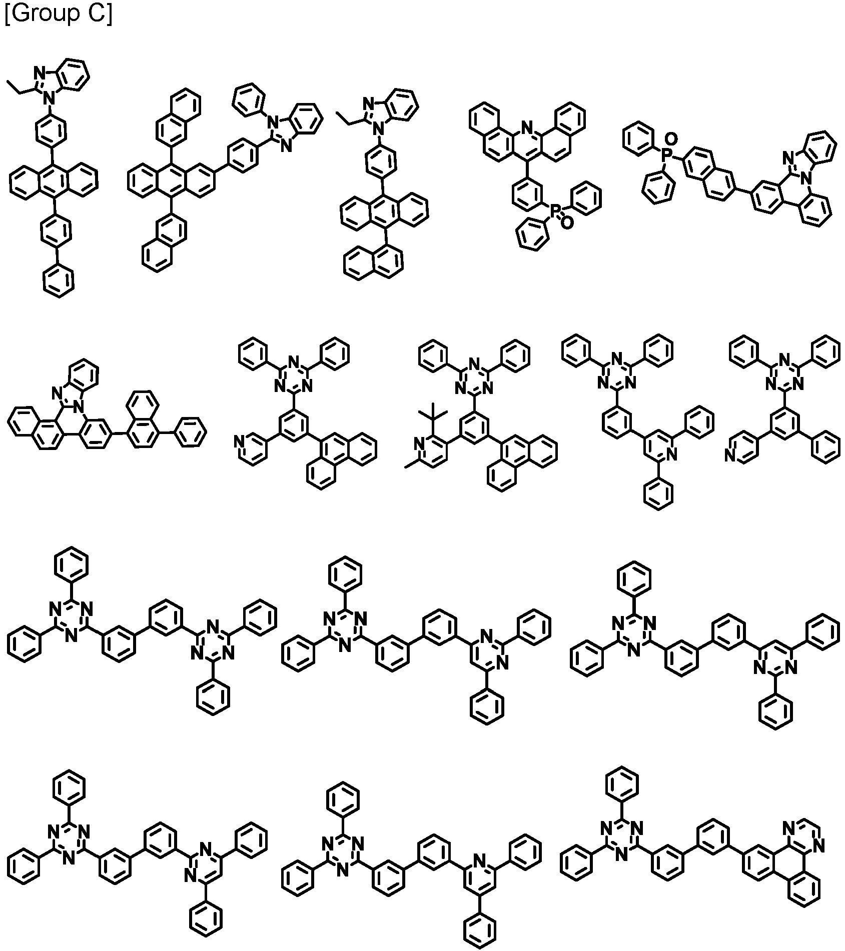

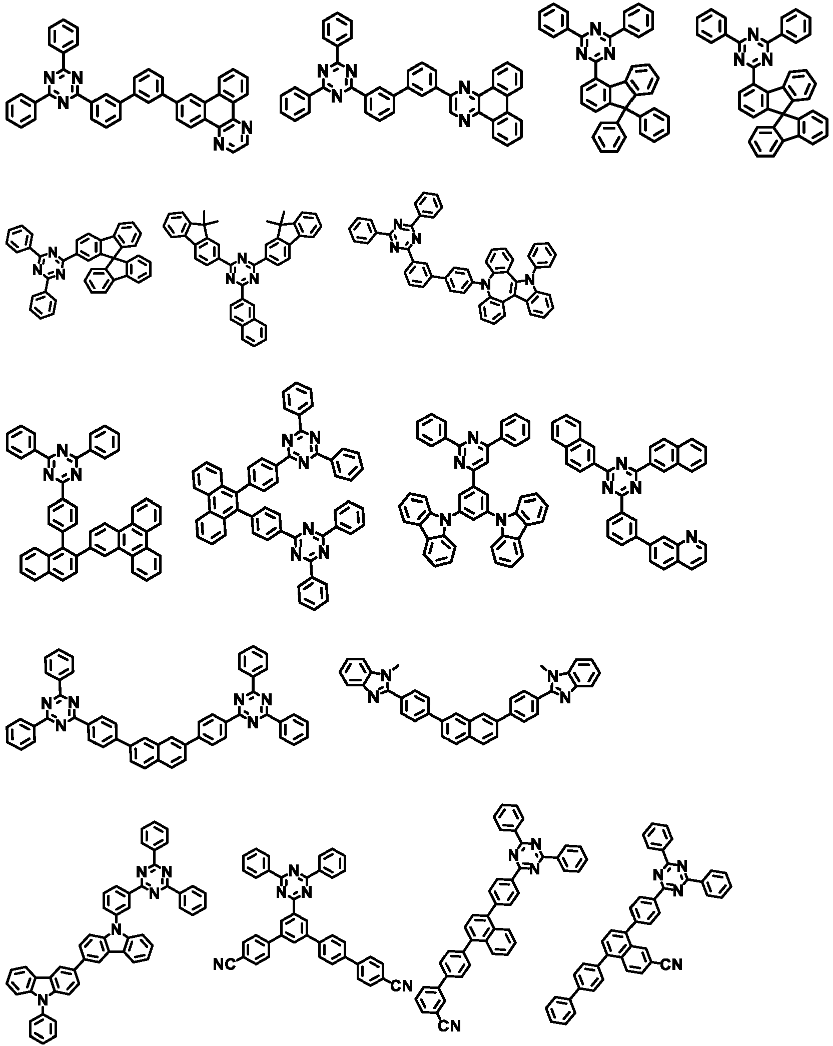

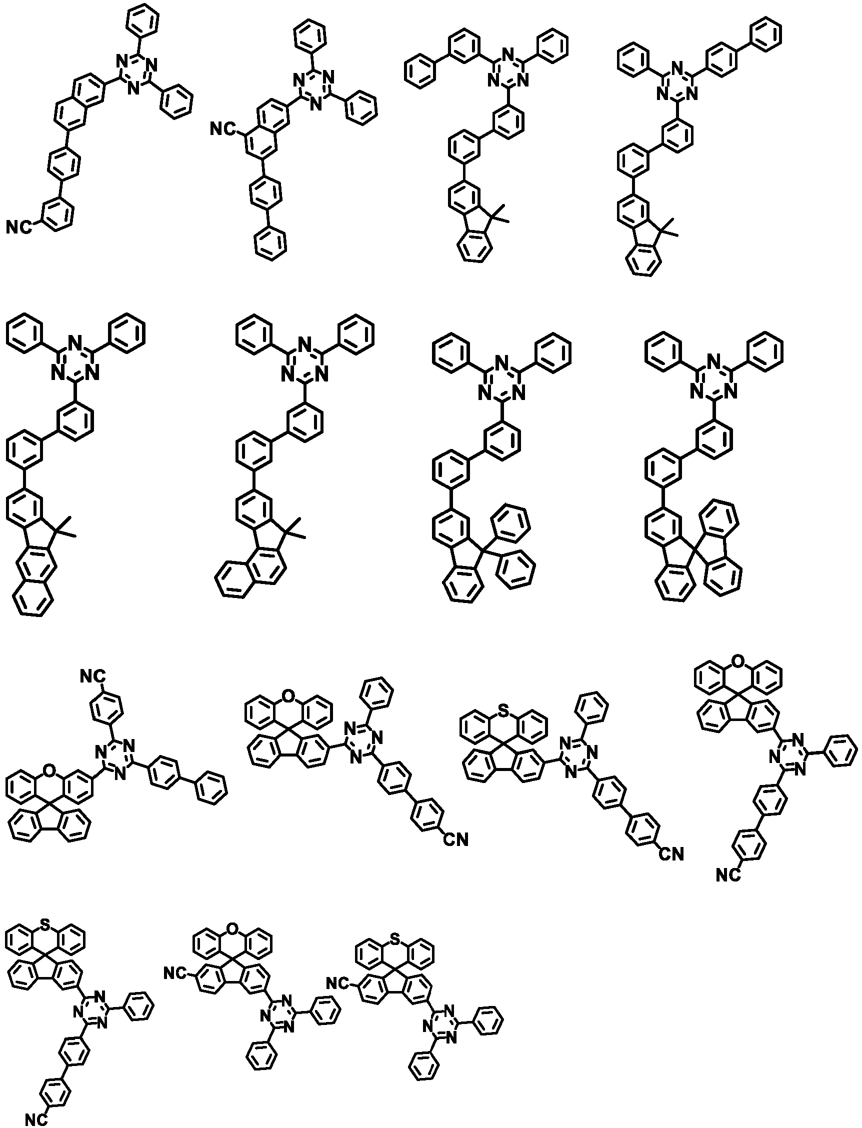

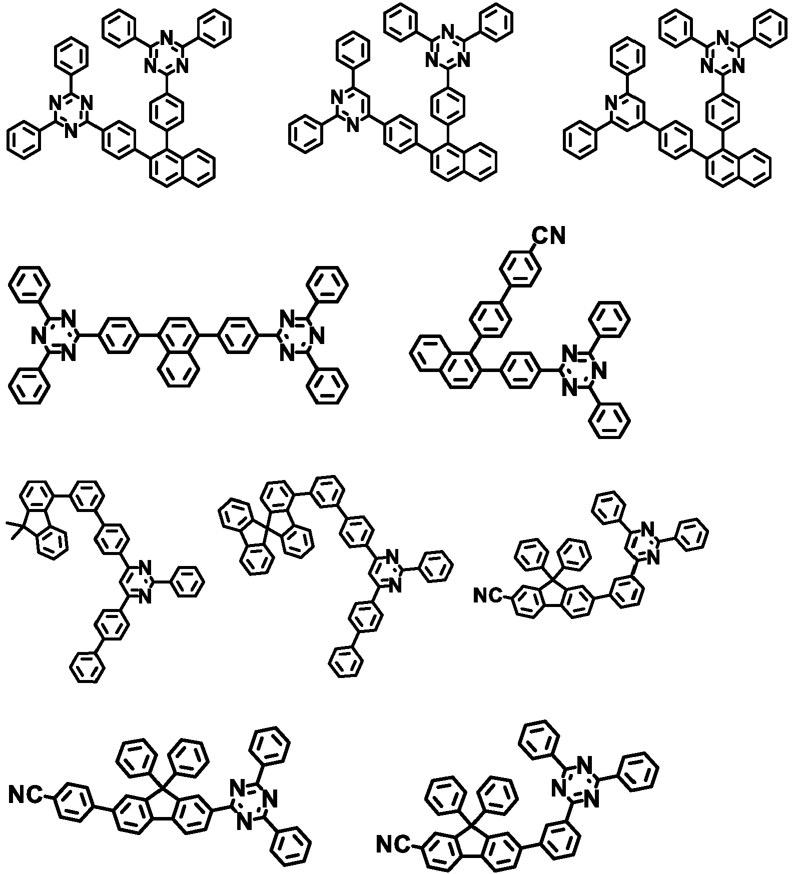

An embodiment of the present invention may provide an organic light emitting diode including the light emitting layer as the organic layer.

Another embodiment of the present invention may provide an organic light emitting diode including a hole transport region and the light emitting layer as the organic layer.

Another embodiment of the present invention may provide an organic light emitting diode including an electron transport region and the light emitting layer as the organic layer.

An embodiment of the present invention may provide an organic light emitting diode including a hole transport region 140 and an electron transport region 150 in addition to the light emitting layer 130 as the organic layer 105, as shown in FIG. 1.

In another embodiment of the present invention, an organic light emitting diode may further include an electron injection layer (not shown), a hole injection layer (not shown), etc. in addition to the light emitting layer as the organic layer.

The organic light emitting diodes may be manufactured by forming an anode or a cathode on a substrate, and then forming an organic layer by a dry film method such as vacuum deposition, sputtering, plasma plating and ion plating, and forming a cathode or an anode thereon.

The organic light emitting diode may be applied to an organic light emitting display device.

Hereinafter, the embodiments are illustrated in more detail with reference to examples. However, these examples are exemplary, and the scope of claims is not limited thereto.

Hereinafter, starting materials and reactants used in examples and synthesis examples were purchased from Sigma-Aldrich Co. Ltd., TCI Inc., Tokyo chemical industry, or P&H tech as far as there is no particular comment or were synthesized by known methods.

### (Preparation of Compound for Organic Optoelectronic Device)

The compounds presented as a more specific example of the compound of the present invention were synthesized through the following steps.

### (Synthesis of First Compound)

The compounds presented as a more specific example of the compound of the present invention were synthesized through the following steps.

### Synthesis Example 1: Synthesis of Compound 1-38

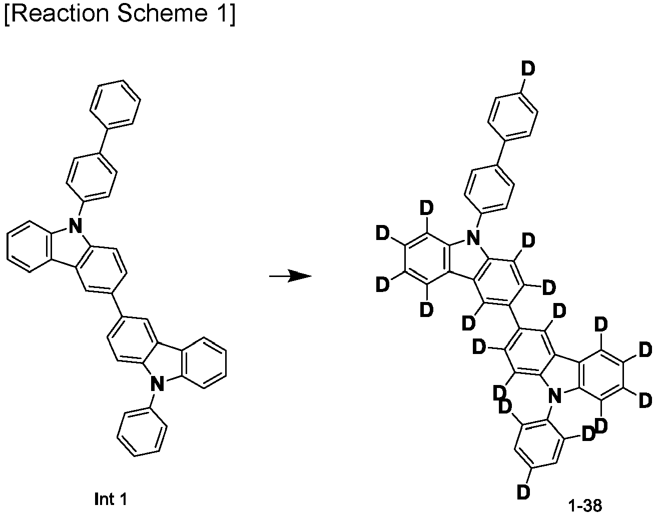

### 1st step: Synthesis of Compound Int 1

Compound Int 1 was synthesized by referring to the method disclosed in Korean Publication No. 10-2016-0049842.

### 2nd step: Synthesis of Compound 1-38

30 g (0.0535 mol) of Compound Int 1, 40 g (0.267 mol) of trifluoromethanesulfonic acid, and 282 g (3.35 mol) of D₆-benzene were put and then, stirred at 10 °C for 24 hours. Subsequently, purified water was thereto and then, neutralized with a saturated K₃PO₄ solution. An organic layer therefrom was concentrated and column-purified to obtain 18 g of Compound 1-38 (a white solid, LC-Mass Mz 578.79, C42H10D18N2).

### Comparative Synthesis Example 1: Synthesis of Compound Y1

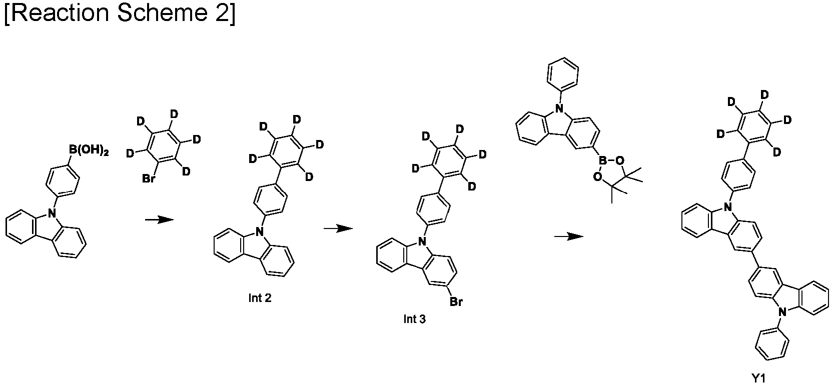

35 g (0.095 mol) of (phenyl-4-boronic acid)-9H-carbazole, 17 g (0.105 mol) of bromobenzene-Ds, 3.3 g (0.0028 mol) of Pd(PPh₃)₄, 32.7 g (0.237 mol) of K₂CO₃, 120 ml of purified water, and 320 ml of THF were put and then, stirred under reflux. When a reaction was completed, purified water was added thereto after cooling to separate an organic layer, and the organic layer was concentrated. The concentrated product was column-purified to obtain 25 g of Int 2 (a molecular weight: 324.43).

20 g (0.062 mol) of Int 2 was dissolved in 200 ml of DMF, and 11.5 g (0.065 mol) of NBS was slowly added thereto at 0 °C. The obtained mixture was stirred at room temperature to complete a reaction, and purified water was added thereto to produce a solid. The solid was column-purify to obtain 23 g of Int 3 (a molecular weight: 403.33).

20 g (0.0496 mol) of Int 3, 22 g (0.06 mol) of phenyl-9H-carbazole-3-boronic ester, 1.72 g (0.0015 mol) of Pd(PPh₃)₄, 13.7 g (0.099 mol) of K₂CO₃, 50 ml of purified water, and 165 ml of THF were put and stirred under reflux. When a reaction was completed, purified water thereto after cooling was added thereto to separate an organic layer, and then, the organic layer was concentrated. The concentrated product was column-purified to obtain 11.5 g of Compound Y1 (a molecular weight: 565.72).

### Comparative Synthesis Example 2: Synthesis of Compound Y2

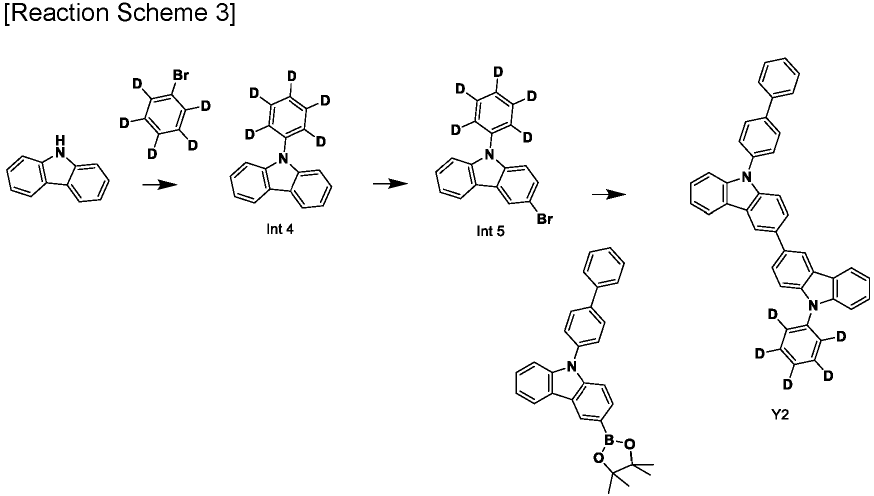

47 g (0.281 mol) of carbazole, 50 g (0.310 mol) of bromobenzene-Ds, 53 g (0.028 mol) of Cul, 58 g (0.42 mol) of K₂CO₃, 5 g (0.028 mol) of 1,10-phenanthroline, and 560 ml of DMF were put and stirred under reflux. When a reaction was completed, a solid was produced by adding purified water thereto after cooling to room temperature. The solid was column-purified to obtain 62 g of Int 4 (a molecular weight: 248.33).

62 g (0.25 mol) of Int 4 was added to DMF and dissolved therein. Subsequently, 45 g (0.25 mol) of NBS was slowly added thereto at 0 °C and then, stirred at room temperature to complete a reaction. Subsequently, purified water was added to the reaction solution to produce crystals, and the solids were column-purified to obtain 80 g of Int 5 (a molecular weight: 327.23).

80 g (0.245 mol) of Int 5, 120 g (0.27 mol) of 4-biphenyl-carbazole-3-boronic ester, 68 g (0.49 mol) of K₂CO₃, 14 g (0.0122 mol) of Pd(PPh₃)₄, 320 ml of purified water, and 490 ml of THF were put and stirred under reflux. When a reaction was completed, an organic layer was extracted by adding purified water thereto and then, concentrated. The mixture was column-purified to obtain 90 g of Compound Y2 (a molecular weight: 565.72).

### (Synthesis of Second Compound)

### Synthesis Example 2: Synthesis of Compound B-12

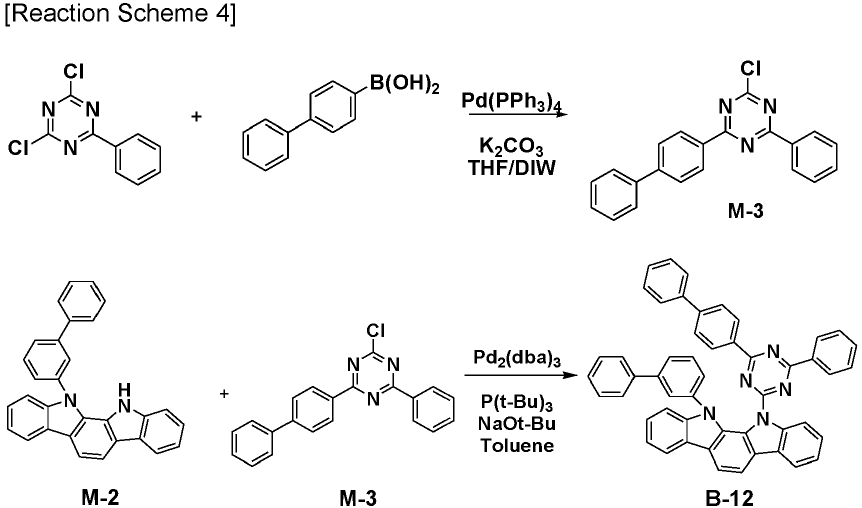

### 1st step: Synthesis of Intermediate M-2

11,12-dihydroindolo[2,3-a]carbazole (78.35 g, 305.69 mmol, CAS No. 60511-85-5), 3-bromobiphenyl (59.38 g, 254.74 mmol), NaOt-Bu (26.93 g, 280.22 mmol), and Pd₂(dba)₃ (7 g, 7.64 mmol) were suspended in 1,400 ml of toluene, and P(t-Bu)₃ (3.64 ml, 15.28 mmol) was added thereto and then, stirred under reflux for 12 hours. Subsequently, distilled water was added to the reaction solution to separate the mixture. The obtained product was purified through silica gel column to obtain Intermediate M-2 (68.7 g, 57%).

### 2nd step: Synthesis of Intermediate M-3

2,4-dichloro-6-phenyl-1,3,5-triazine (74.50 g, 329.56 mmol) and 4-biphenylboronic acid (55.47 g, 280.12 mmol) were dissolved in 0.7 L of a mixed solution of tetrahydrofuran (THF) and distilled water (3:1), and sodium tert-butoxide (68.32 g, 494.34 mmol) was added thereto and then, stirred under reflux for 12 hours. After cooling the reaction solution and separating layers, an organic layer therefrom was collected therefrom and concentrated. The concentrated residue was purified through silica gel column to obtain Intermediate M-3 (75.9 g, 67%).

### 3rd step: Synthesis of Compound B-12

Compound B-12 was obtained in the same manner as in the method of synthesizing Intermediate M-2 by using Intermediate M-2 and Intermediate M-3.

### (Manufacture of Organic Light Emitting Diode)

### Example 1

A glass substrate coated with ITO (indium tin oxide) was washed with distilled water ultrasonic wave. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This prepared ITO transparent electrode was used as an anode, Compound A doped with 3% NDP-9 (Novaled GmbH) was vacuum-deposited on the ITO substrate to form a 100 Å-thick hole injection layer, and Compound A was deposited on the hole injection layer to a thickness of 1350 Å to form a hole transport layer. Compound B was deposited on the hole transport layer to a thickness of 350 Å to form a hole transport auxiliary layer. On the hole transport auxiliary layer, Compound 1-38 and Compound B-12 obtained in the above synthesis examples were simultaneously used as hosts at a weight ratio of 4:6, and 10 wt% of PhGD was doped as a dopant to form a 330Å-thick light emitting layer by vacuum deposition. Subsequently, Compound C was deposited on the light emitting layer to form a 50 Å-thick electron transport auxiliary layer, and Compound D and Liq were simultaneously vacuum deposited in a weight ratio of 1:1 to form a 300 Å-thick electron transport layer. 15 Å of LiQ and 1,200 Å of Al were sequentially vacuum-deposited on the electron transport layer to form a cathode, manufacturing an organic light emitting diode.

The structure was ITO/Compound A (3% NDP-9 doping, 100 Å)/ Compound A (1350 Å) / Compound B (350 Å) / EML[90 wt% of host (Compound 1-38: Compound B-12=4:6 w/w): 10 wt% of PhGD] (330 Å)/Compound C (50 Å)/ Compound D:LiQ (300 Å) /LiQ (15 Å)/Al (1200 Å).
Compound A: N-(biphenyl-4-yl)-9,9-dimethyl-N-(4-(9-phenyl-9H-carbazol-3-yl)phenyl)-9H-fluoren-2-amine
Compound B: N,N-bis(9,9-dimethyl-9H-fluoren-4-yl)-9,9-spirobi(fluorene)-2-amine
Compound C: 2-[3'-(9,9-Dimethyl-9H-fluoren-2-yl)[1,1'-biphenyl]-3-yl]-4,6-diphenyl-1,3,5-triazine
Compound D: 2-(Biphenyl-4-yl)-4-(9,9-diphenyl-9H-fluoren-4-yl)-6-phenyl-1,3,5-triazine

### [PhGD]

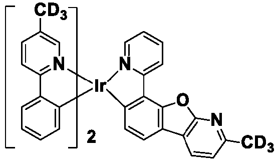

### Comparative Examples 1 to 3

Organic light emitting diodes according to Comparative Examples 1 to 3 were manufactured in the same manner as in Example 1, except that the host was changed as shown in Table 1.

### Evaluation

### (1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

Luminous efficiency (cd/A) at the same current density (10 mA/cm²) were calculated by using the luminance and current density from the items (1) and (2).

### (4) Measurement of Life-span

The results were obtained by maintaining the luminance (cd/m²) at 24000 cd/m² and measuring the time for the luminous efficiency (cd/A) to decrease to 95%.

The values shown in Table 1 are relative values based on the values of Comparative Example 2, respectively.

**(Table 1)**

| Nos. | Compound (first host) | Compound (second host) | Life-span ratio (T95@24K) |
|---|---|---|---|
| Example 1 | 1-38 | B-12 | 124% |
| Comparative Example 1 | Int 1 | B-12 | 95% |
| Comparative Example 2 | Y1 | B-12 | 100% |
| Comparative Example 3 | Y2 | B-12 | 100% |

Referring to Table 1, life-span characteristics of the organic light emitting diode according to the example of the present invention are significantly improved compared to the organic light emitting diodes according to the comparative examples.

While this disclosure has been described in connection with what is presently considered to be practical example embodiments, it is to be understood that the invention is not limited to the disclosed embodiments.

### <Description of symbols>

- 100:: organic light emitting diode
- 105:: organic layer
- 110:: cathode
- 120:: anode
- 130:: light emitting layer
- 140:: hole transport region
- 150:: electron transport region

## Claims

1. A composition for an organic optoelectronic device, comprising
a first compound represented by Chemical Formula 1, and
a second compound represented by a combination of Chemical Formula 2 and Chemical Formula 3: 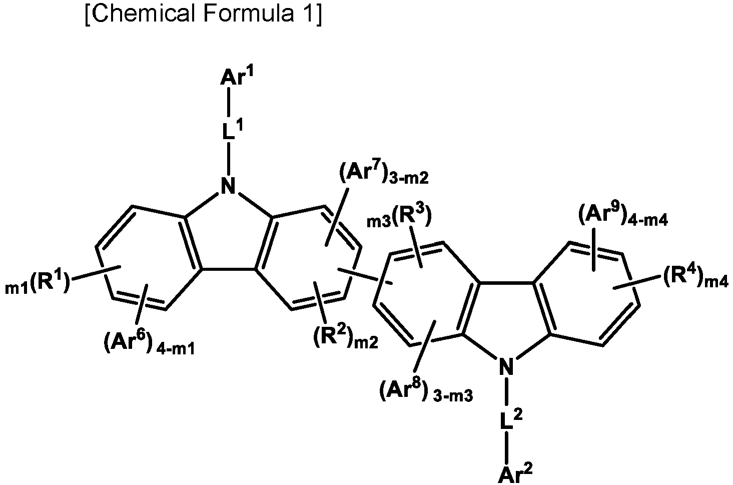
wherein, in Chemical Formula 1,
L¹ and L² are each independently a single bond or a substituted or unsubstituted C6 to C30 arylene group,
Ar¹ and Ar² are each independently a substituted or unsubstituted C6 to C30 aryl group or a substituted or unsubstituted C2 to C30 heterocyclic group,
R¹ to R⁴ are each independently hydrogen, deuterium, or a substituted or unsubstituted C6 to C30 aryl group,
Ar⁶ to Ar⁹ are each independently hydrogen or a substituted or unsubstituted C6 to C30 aryl group, and
m1 and m4 are each independently one of integers of 1 to 4,
m2 and m3 are each independently one of integers of 1 to 3, and
Chemical Formula 1 simultaneously satisfies the following conditions (i) and (ii),
(i) at least one of Ar¹ and Ar² is a C6 to C30 aryl group substituted with at least one deuterium or a C2 to C30 heterocyclic group substituted with at least one deuterium, and
(ii) at least one of R¹ to R⁴ is deuterium; 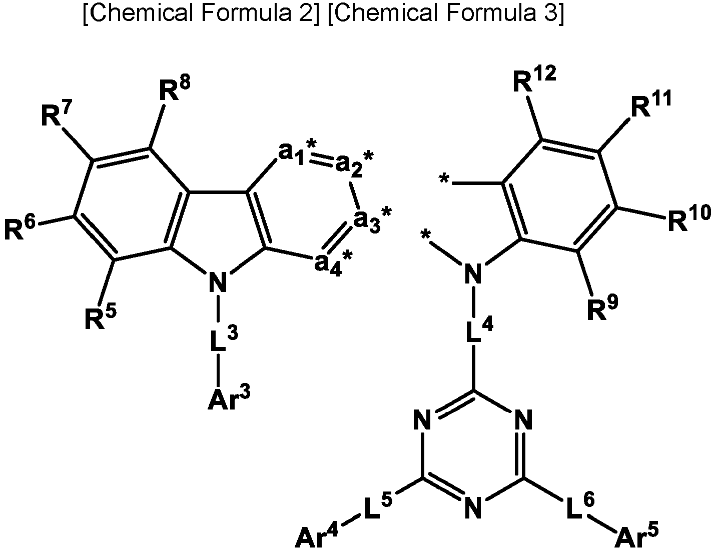
wherein, in Chemical Formula 2 and Chemical Formula 3,
Ar³ to Ar⁵ are each independently a substituted or unsubstituted C6 to C20 aryl group or a substituted or unsubstituted C2 to C30 heterocyclic group,
a₁ * to a₄* are each independently a linking carbon (C) or C-L^{a}-R^{a},
the two adjacent of a₁* to a₄* in Chemical Formula 2 are linked to * in Chemical Formula 3,
L^{a}, L³, to L⁶ are each independently a single bond, a substituted or unsubstituted C6 to C20 arylene group or a substituted or unsubstituted C2 to C20 heteroarylene group, and
R^{a} and R⁵ to R¹² are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group,
wherein for the case that the first compound is selected from one of the following compounds D1-1 to D1-8 and the second compound is selected from one of the following compounds D2-1 to D2-7, the following combinations are excluded
D1-1 with D2-1, D1-2 with D2-1, D1-3 with D2-1, D1-4 with D2-1, D1-5 with D2-1,
D1-1 with D2-2, D1-2 with D2-2, D1-3 with D2-2, D1-4 with D2-2, D1-5 with D2-2,
D1-1 with D2-3, D1-2 with D2-3, D1-3 with D2-3, D1-4 with D2-3, D1-5 with D2-3,
D1-6 with D2-4, D1-7 with D2-5, D1-8 with D2-6, and D1-6 with D2-7

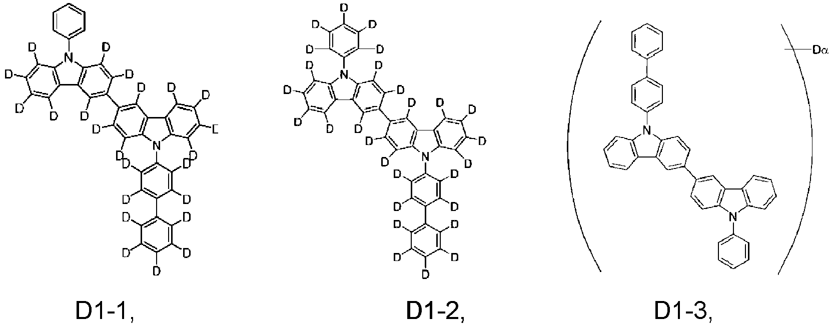

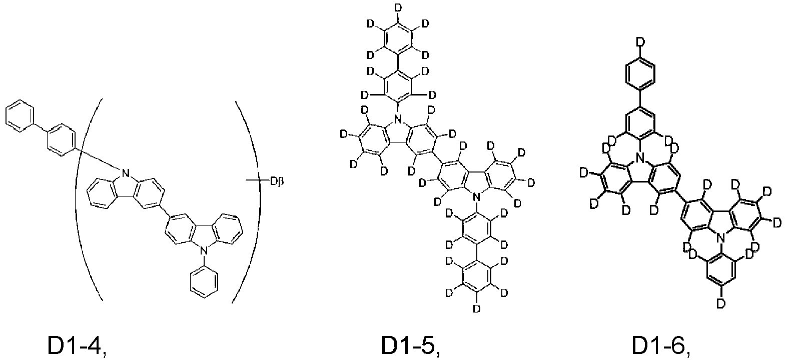

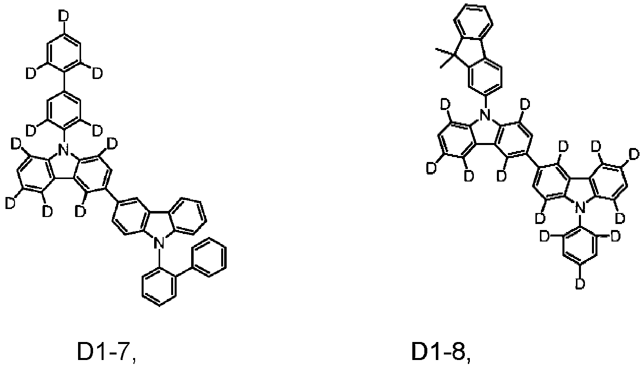

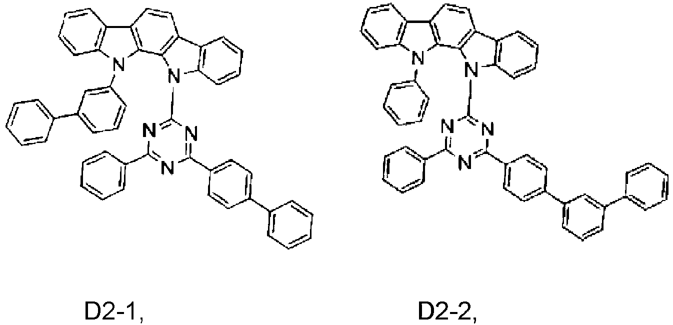

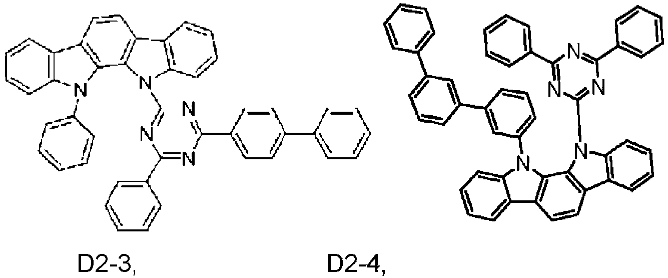

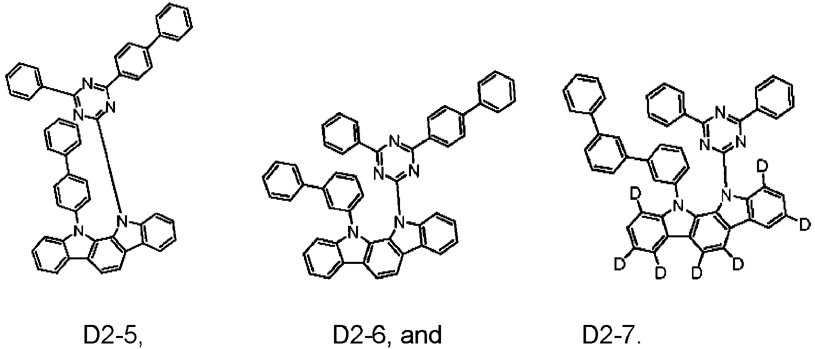

2. The composition for the organic optoelectronic device of claim 1, wherein the first compound is represented by any one of Chemical Formula 1-1 to Chemical Formula 1-10: 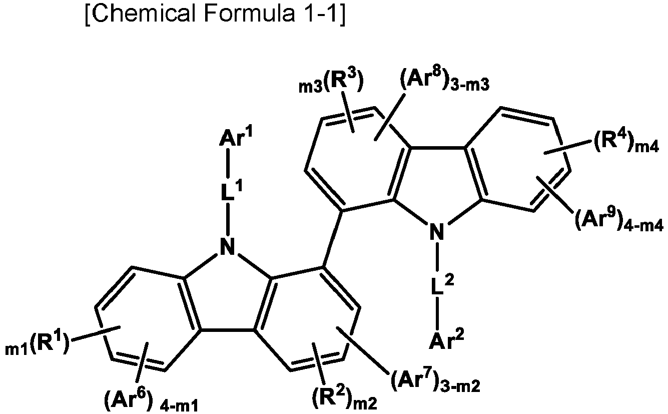 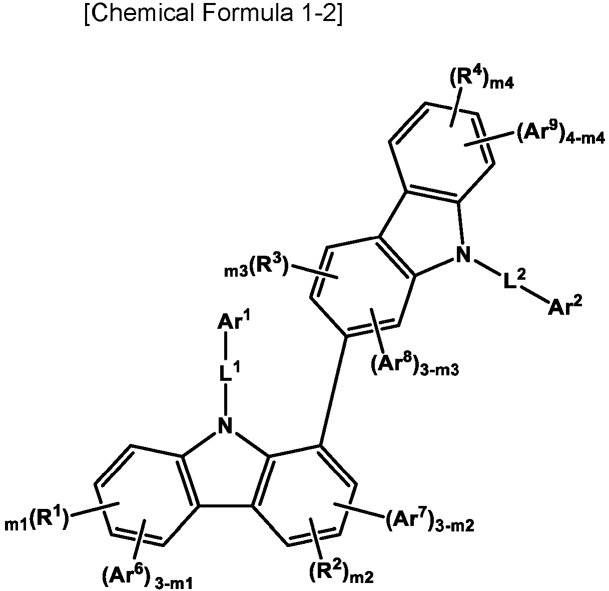 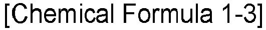 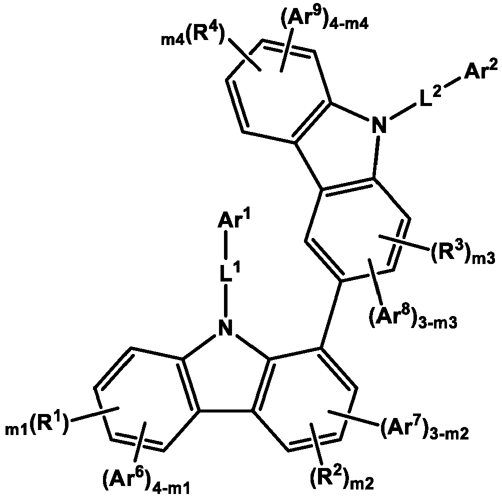 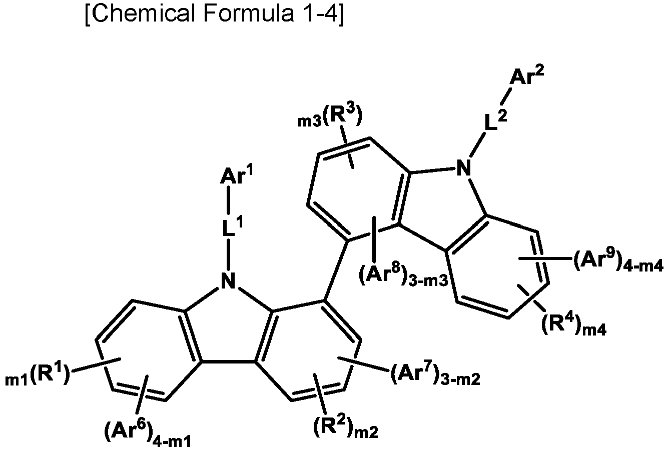 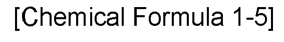 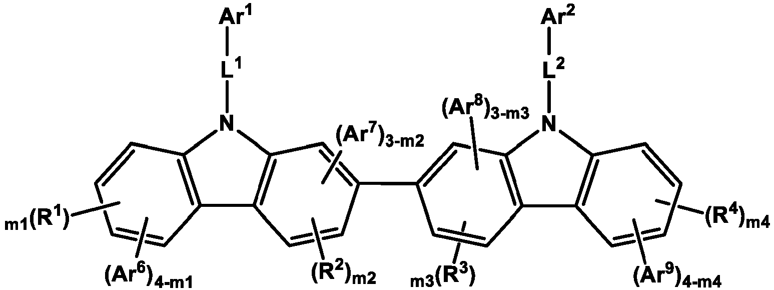 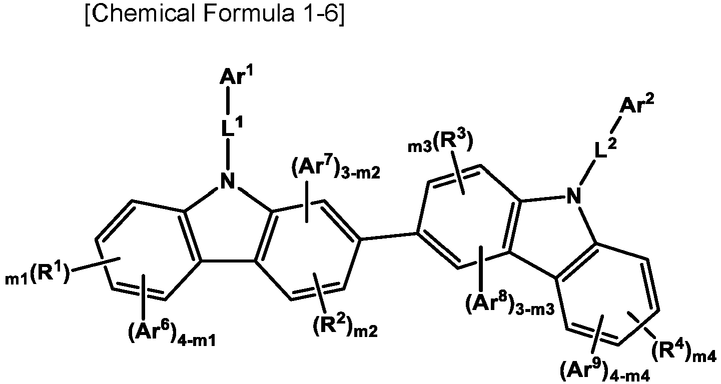 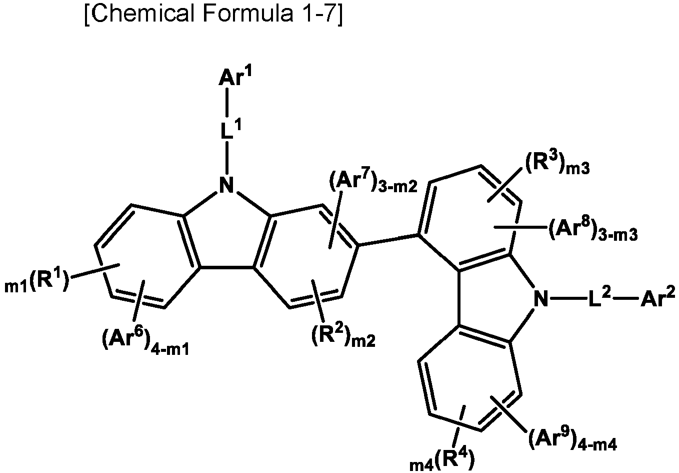 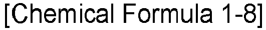 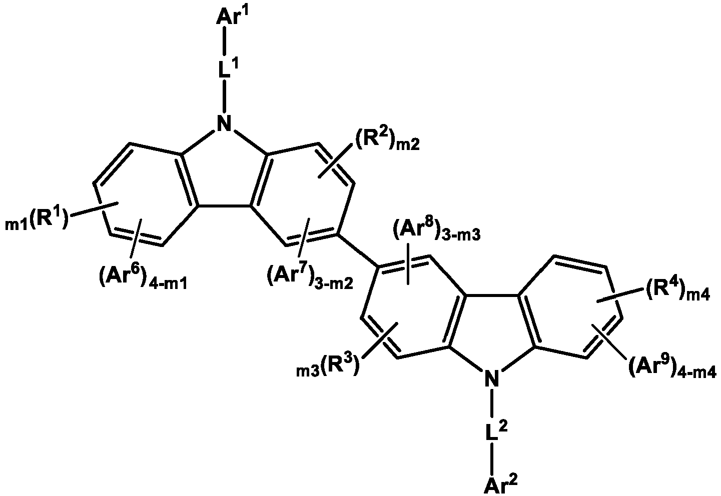 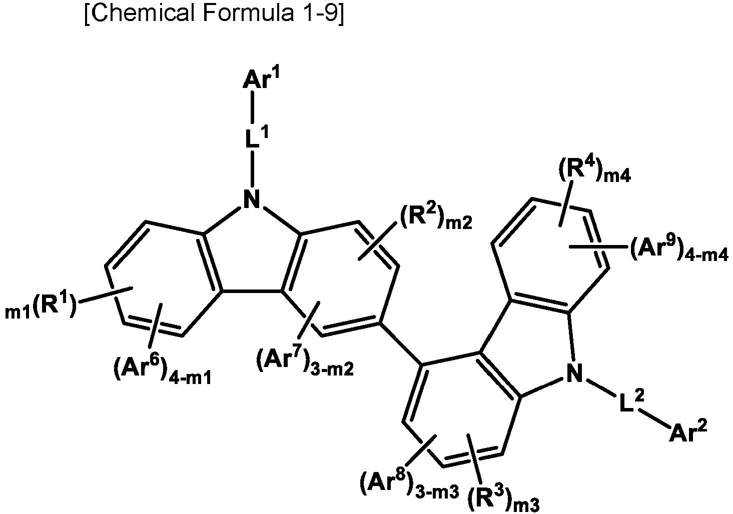 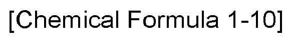 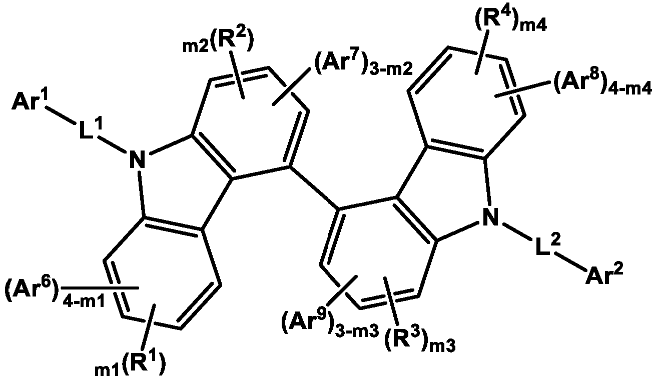
wherein, in Chemical Formula 1-1 to Chemical Formula 1-10,
L¹, L², Ar¹, Ar², Ar⁶ to Ar⁹, R¹ to R⁴, and m¹ to m⁴ are the same as described in claim 1.

3. The composition for the organic optoelectronic device of claim 1, wherein
at least one of Ar¹ and Ar² of Chemical Formula 1 is a phenyl group substituted with at least one deuterium, a biphenyl group substituted with at least one deuterium, a terphenyl group substituted with at least one deuterium, a naphthyl group substituted with at least one deuterium, an anthracenyl group substituted with at least one deuterium, a phenanthrenyl group substituted with at least one deuterium, a triphenylene group substituted with at least one deuterium, a fluorenyl group substituted with at least one deuterium, a dibenzofuranyl group substituted with at least one deuterium, or a dibenzothiophenyl group substituted with at least one deuterium.

4. The composition for the organic optoelectronic device of claim 1, wherein
L¹-Ar¹ and L²-Ar² of Chemical Formula 1 are each independently selected from the substituents listed in Group I -1 and Group I -2, and
at least one of L¹-Ar¹ and L²-Ar² is selected from among the listed substituents in Group I -2: 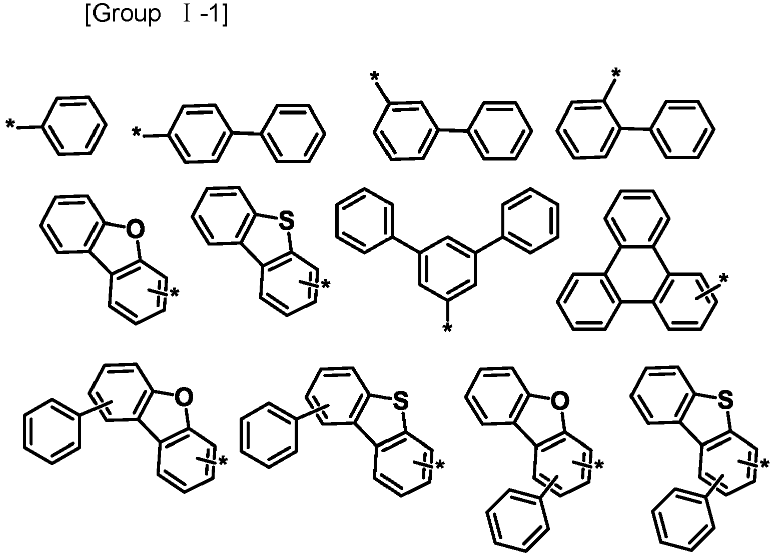 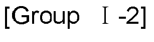 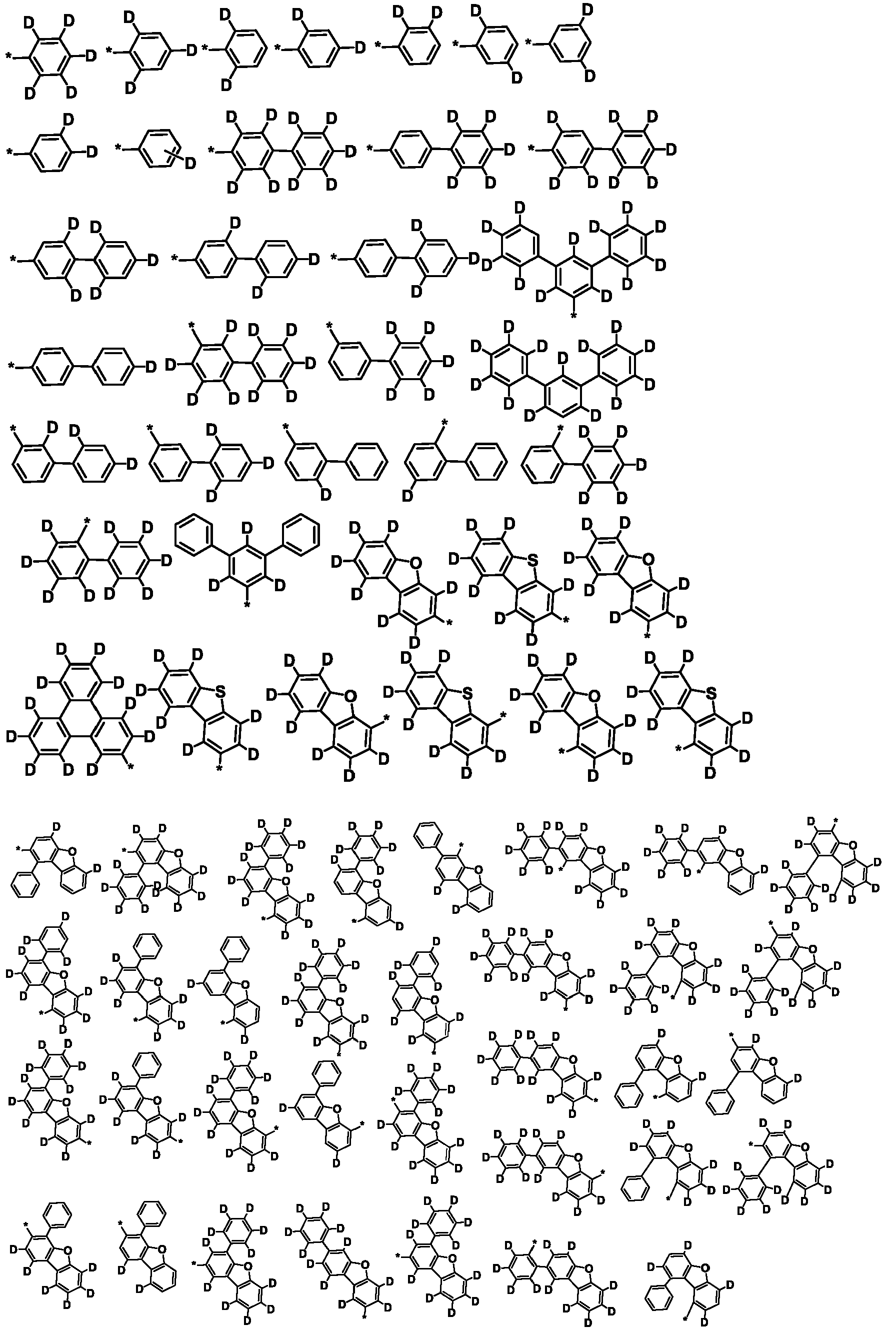 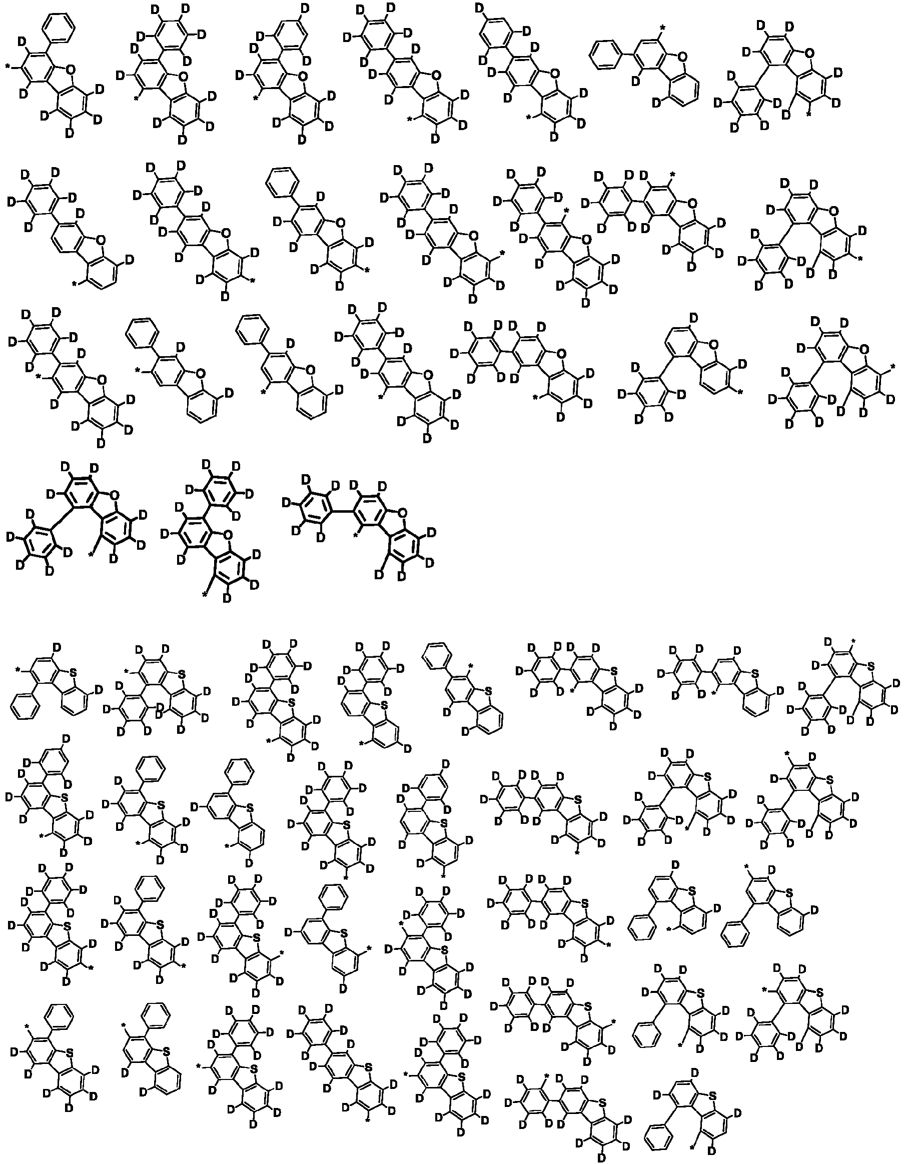 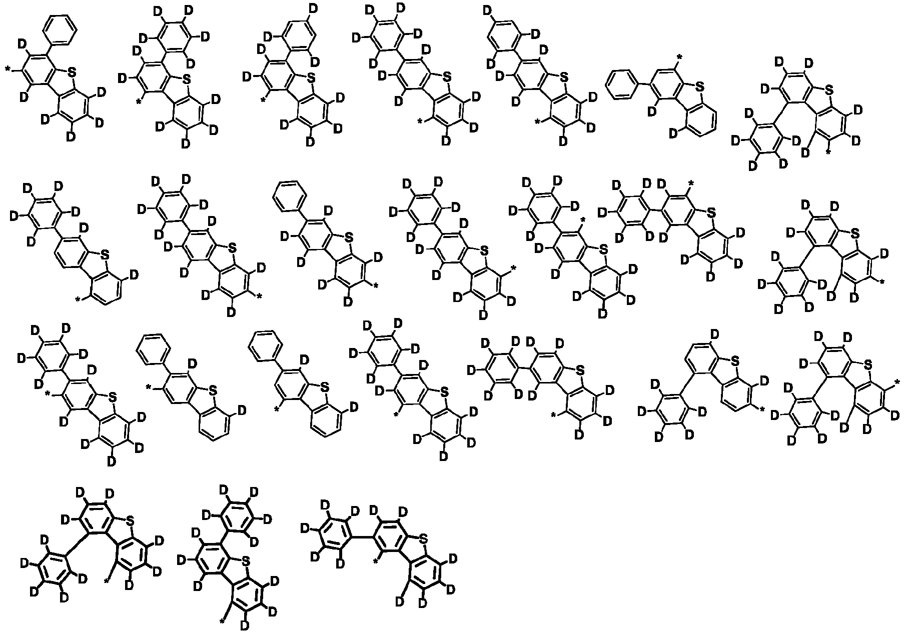
wherein, in Group I -1 and Group I -2, * is a linking point.

5. The composition for the organic optoelectronic device of claim 1, wherein
the first compound is represented by Chemical Formula 1-8a: 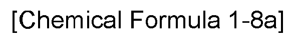 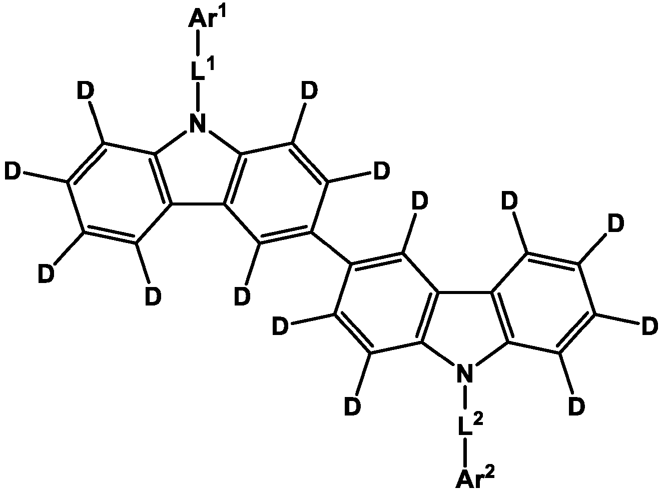
wherein, in Chemical Formula 1-8a,
L¹, L², Ar¹, and Ar² are as the same as defined in claim 1.

6. The composition for the organic optoelectronic device of claim 1, wherein
the first compound is one selected from the compounds listed in Group 1:

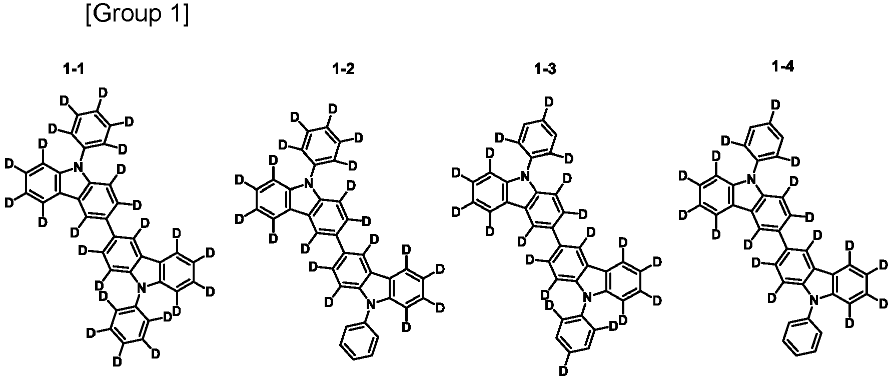

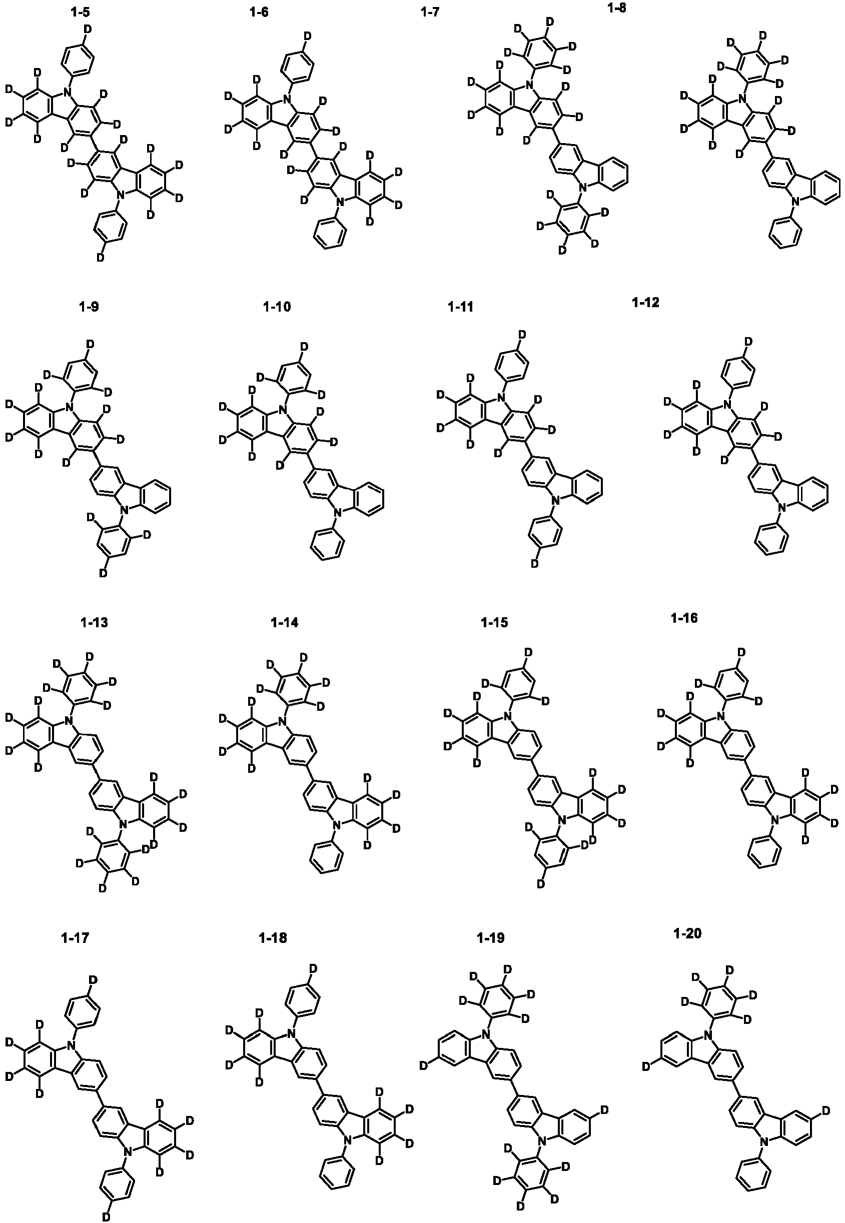

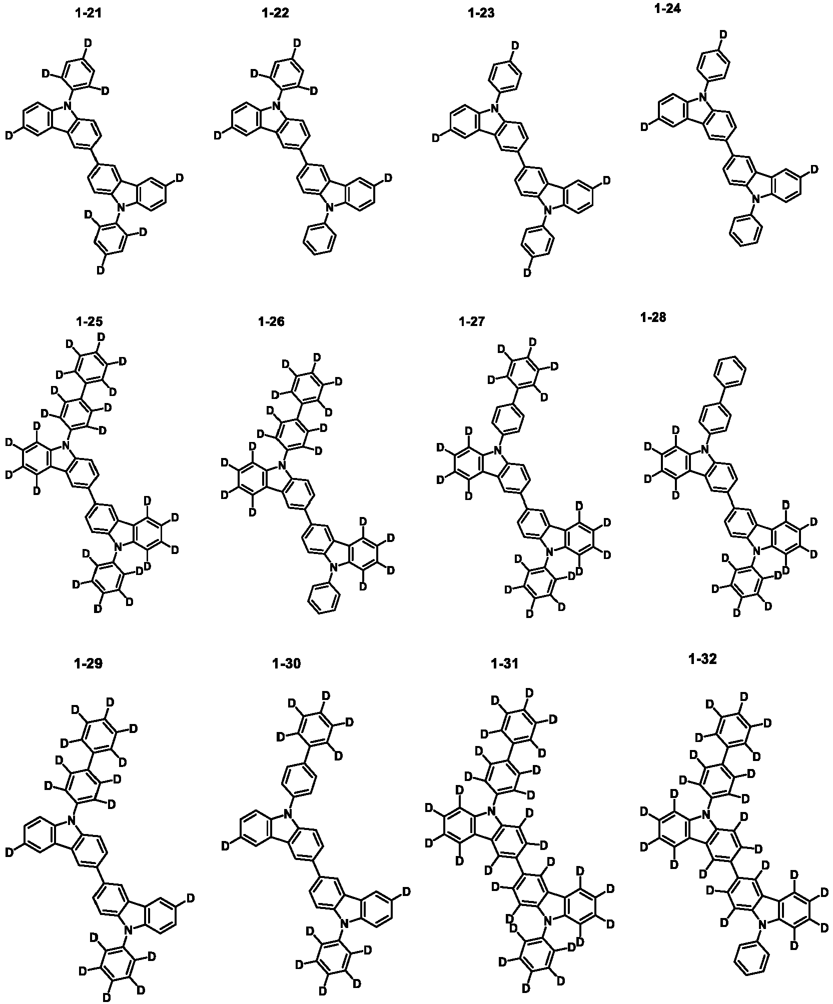

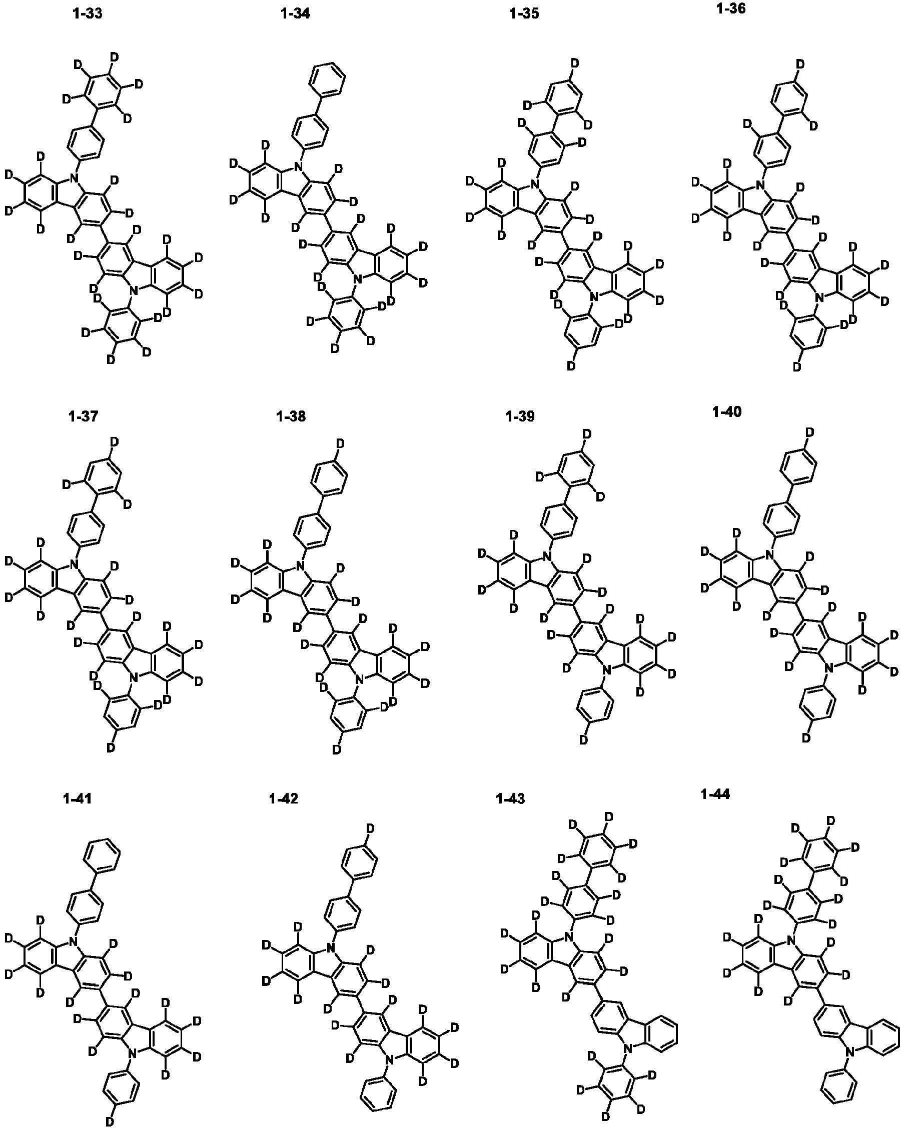

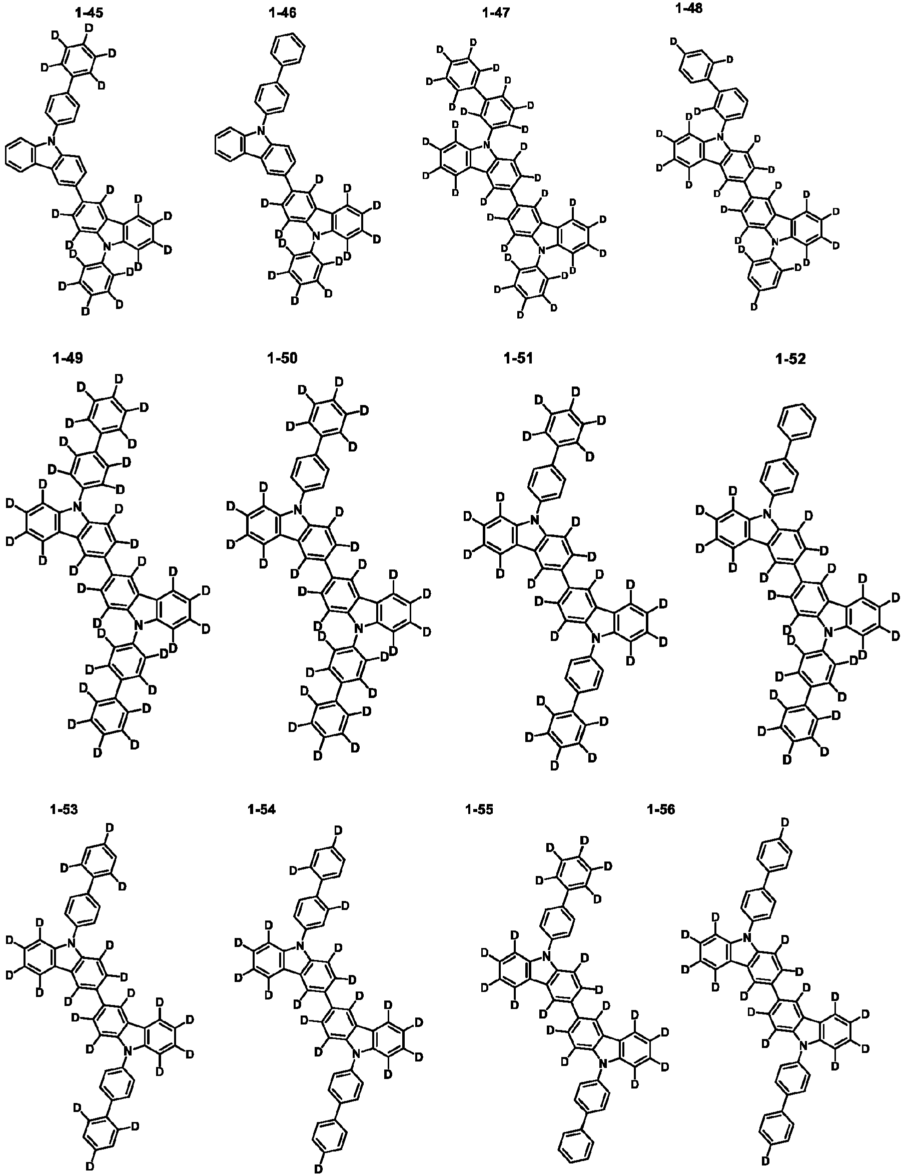

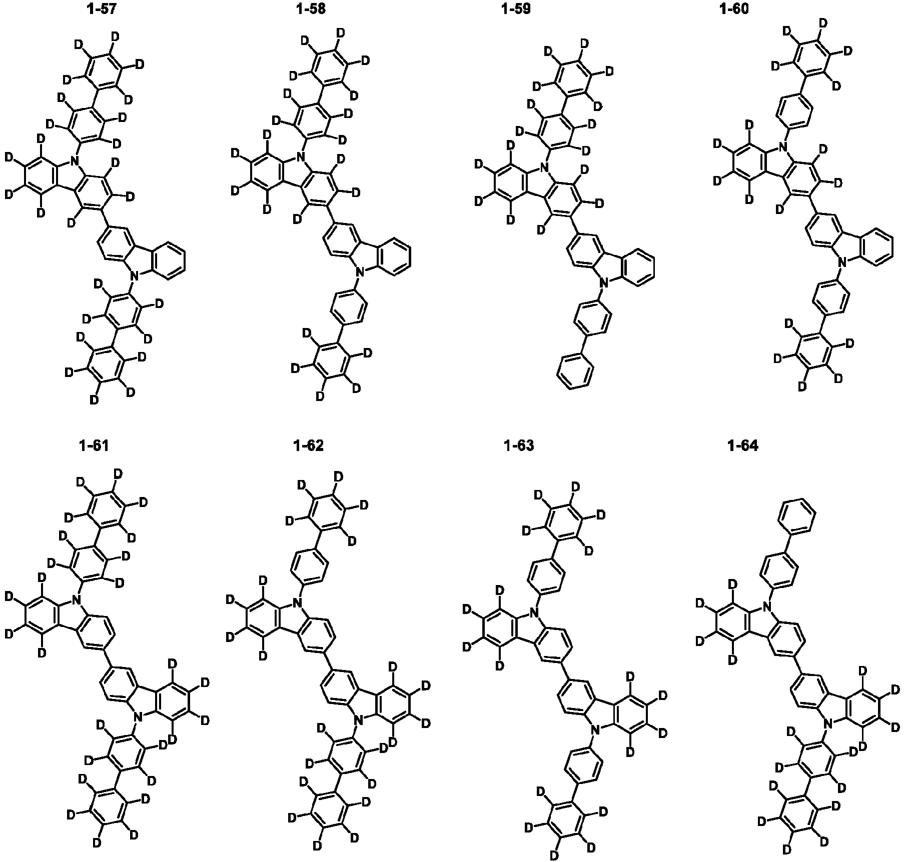

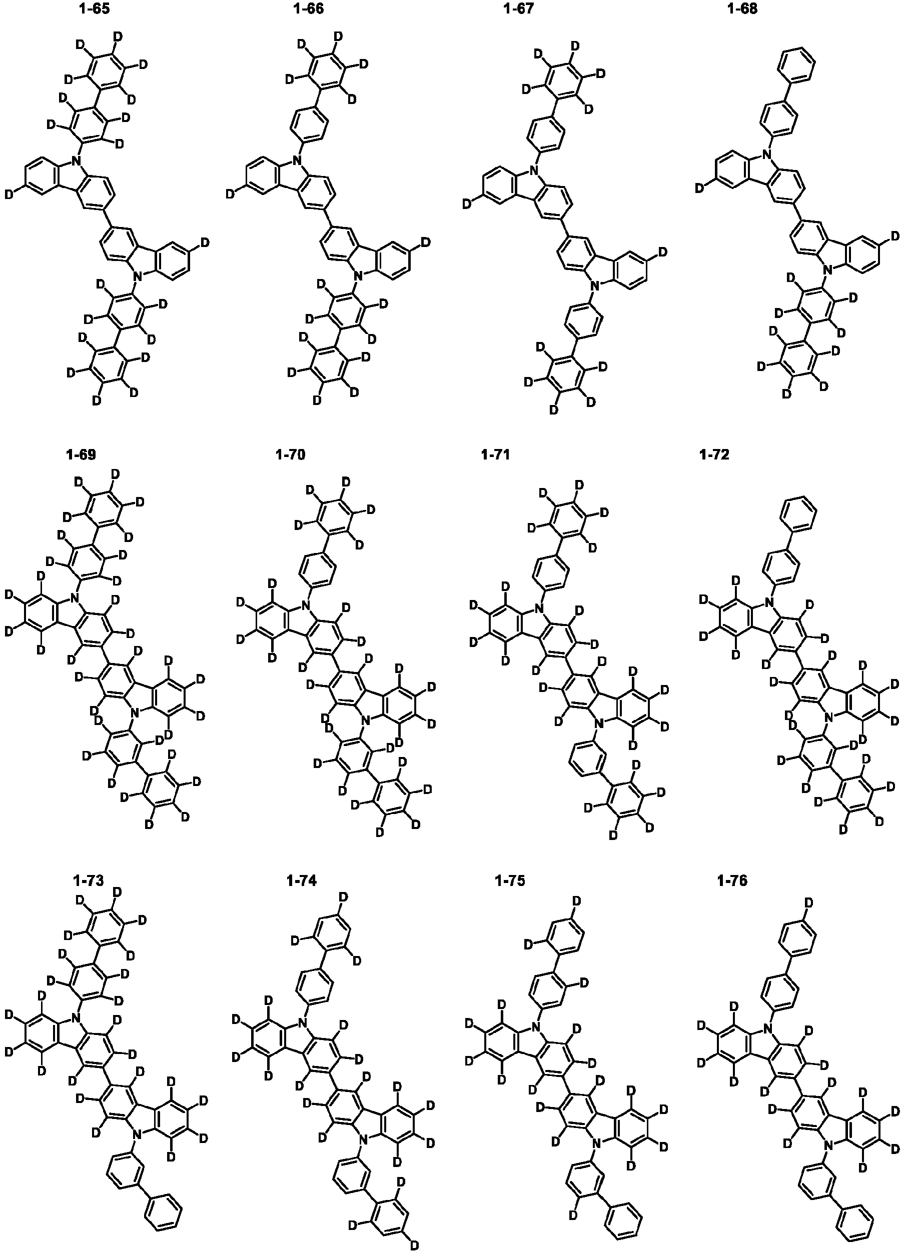

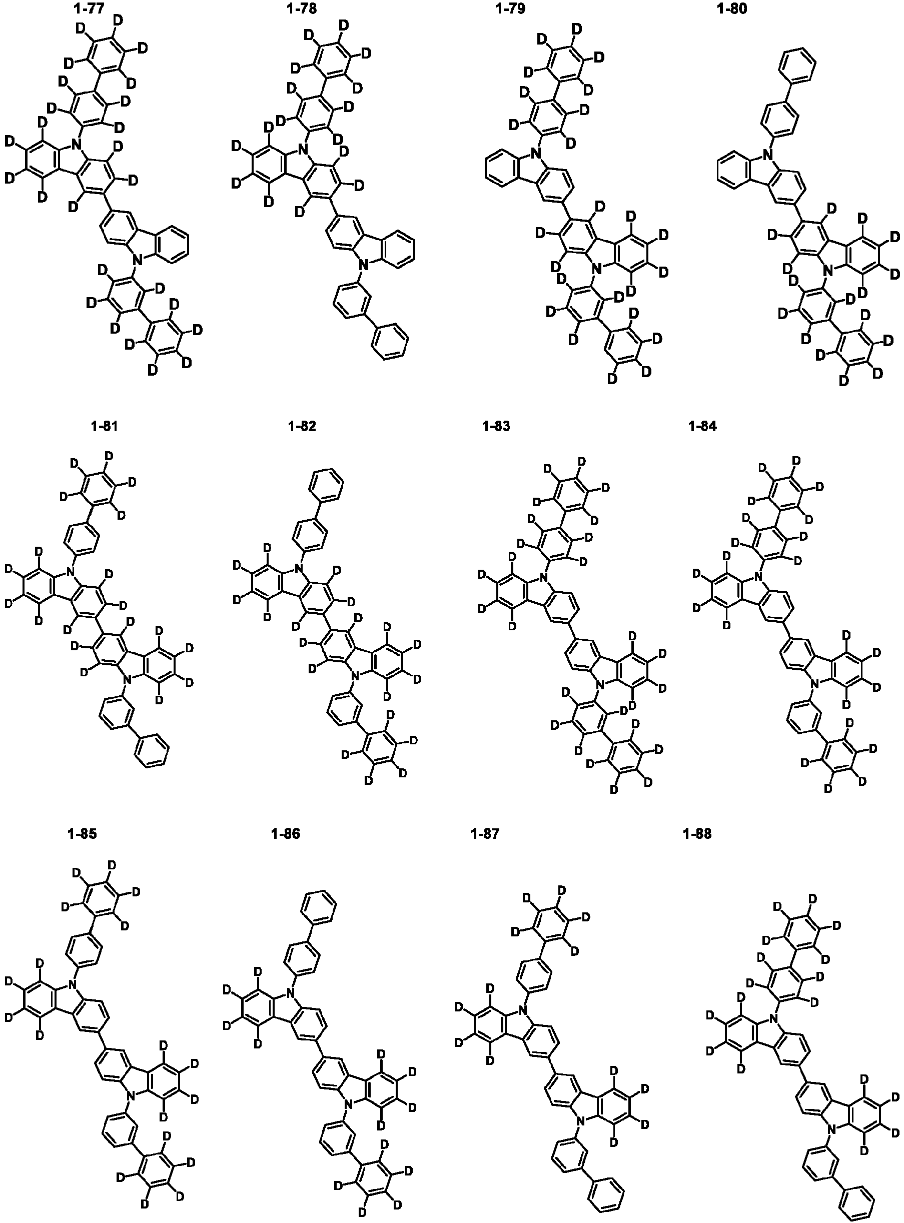

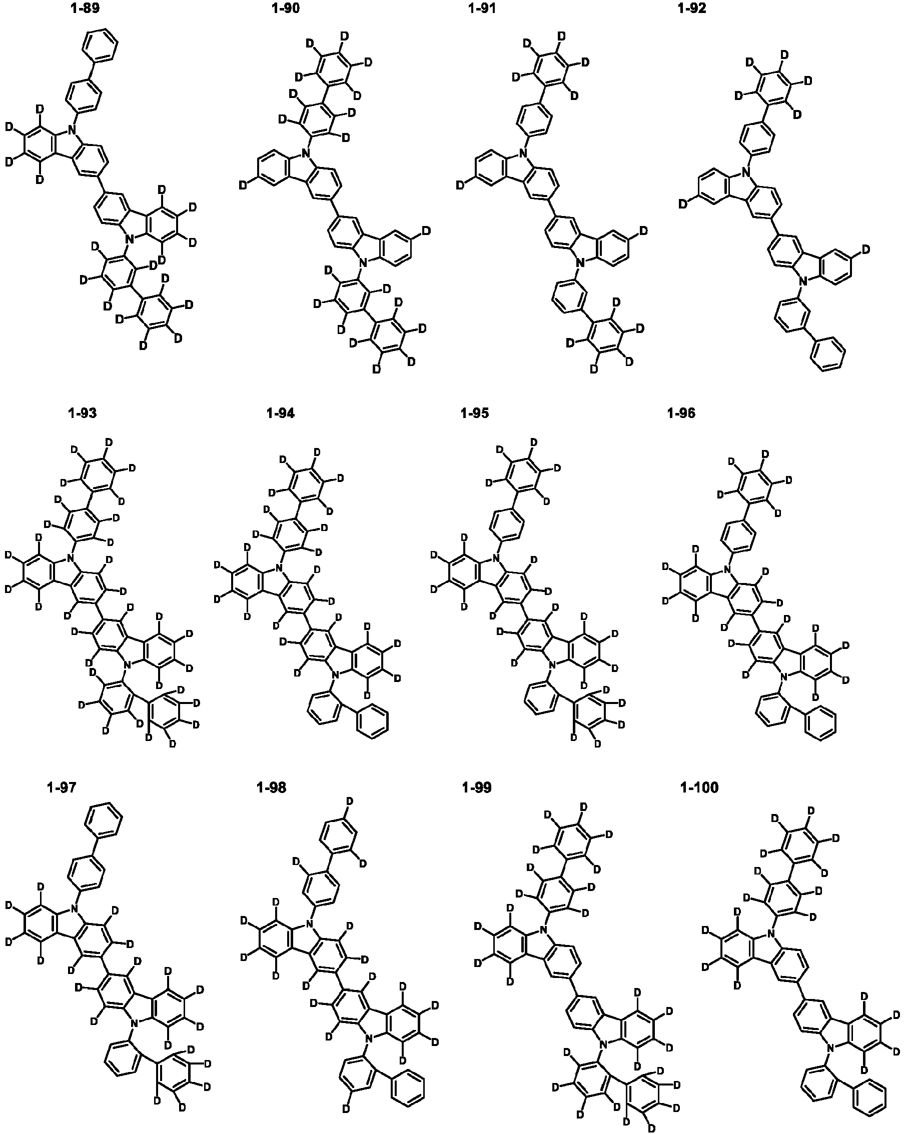

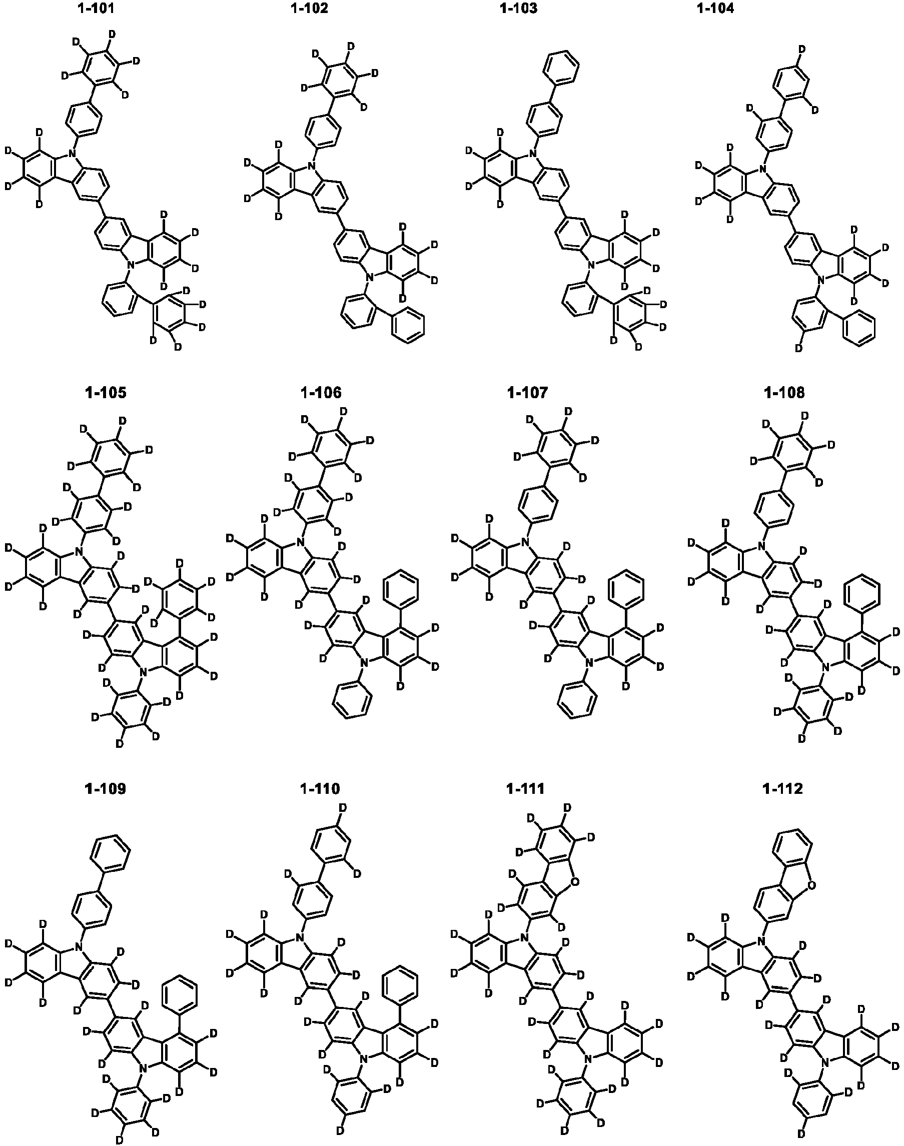

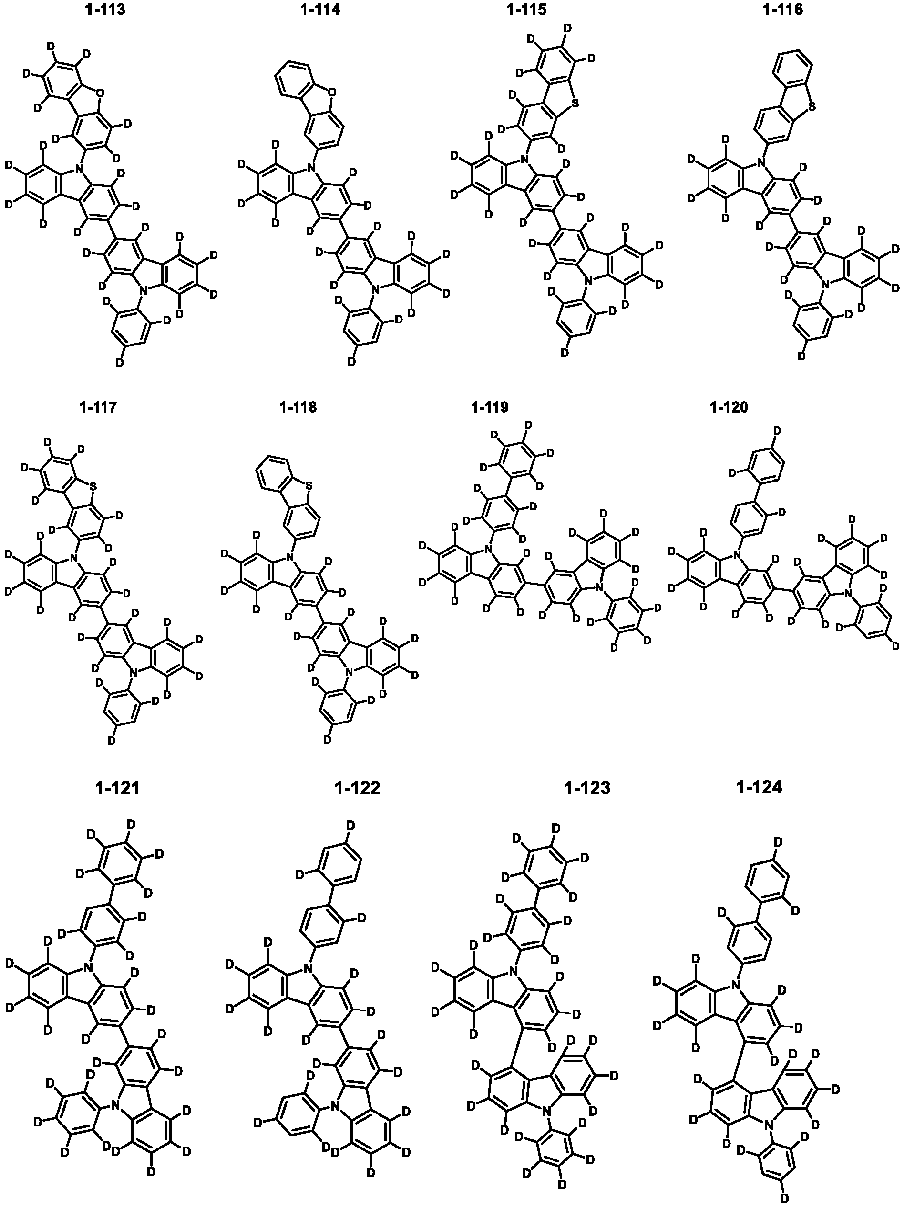

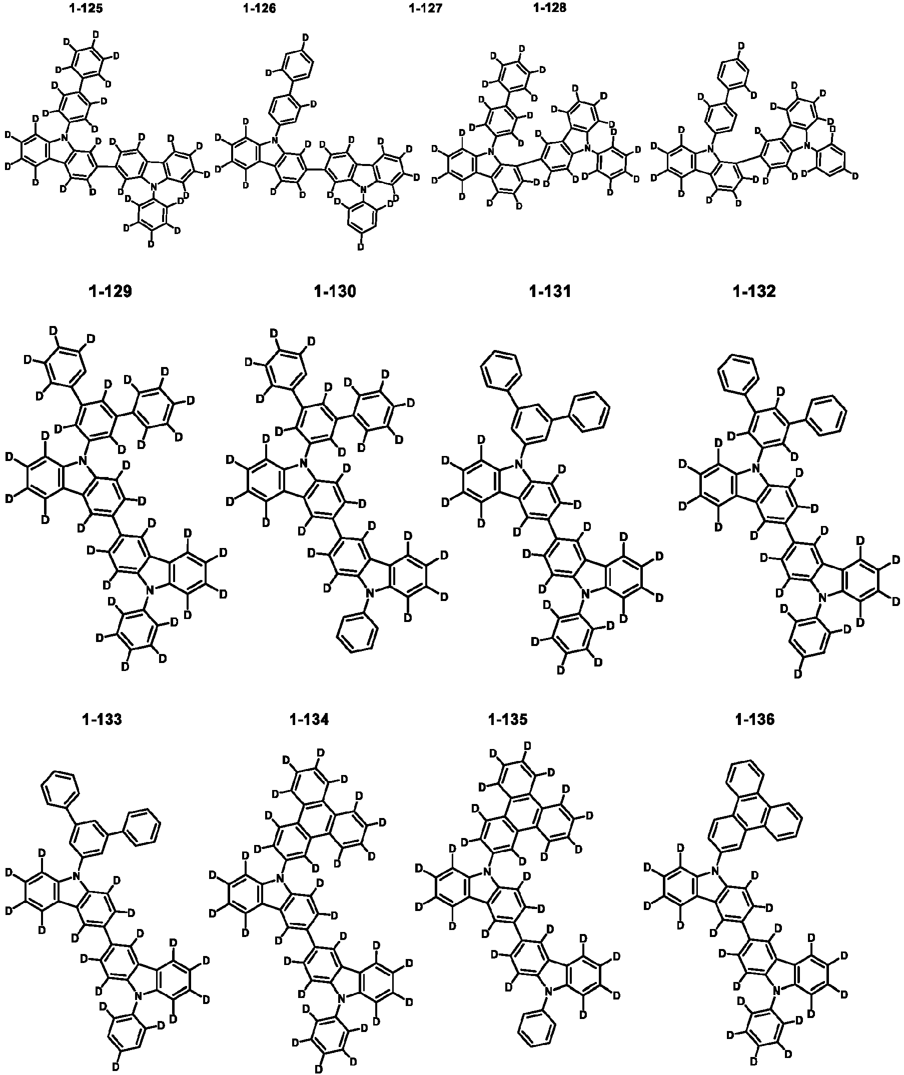

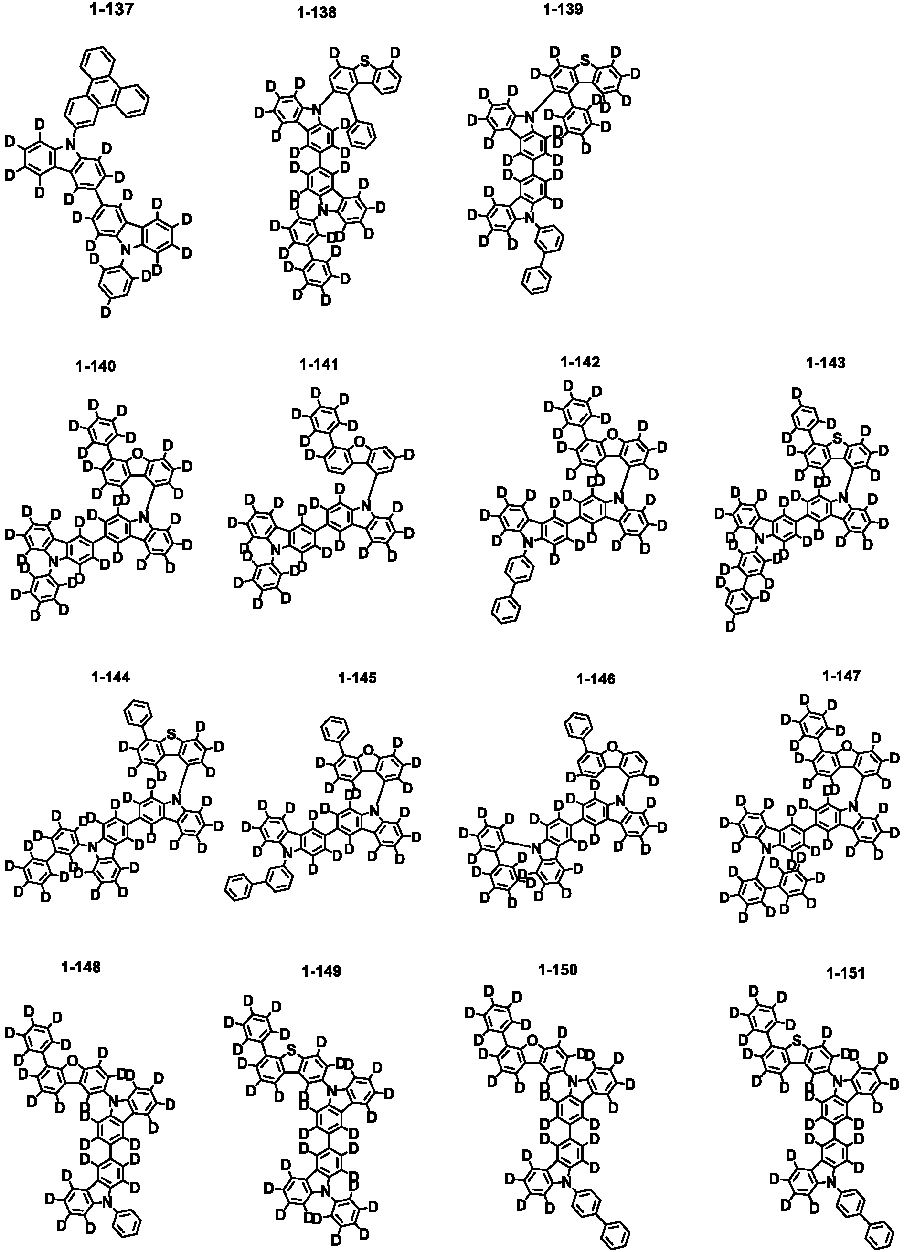

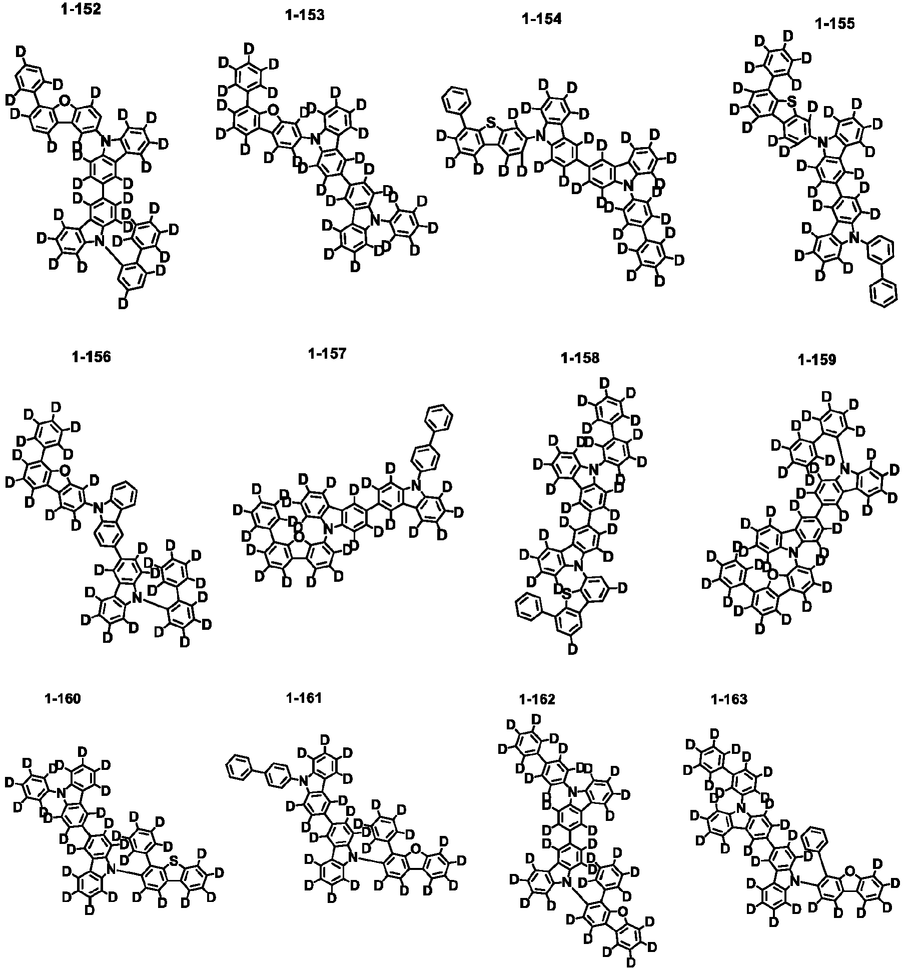

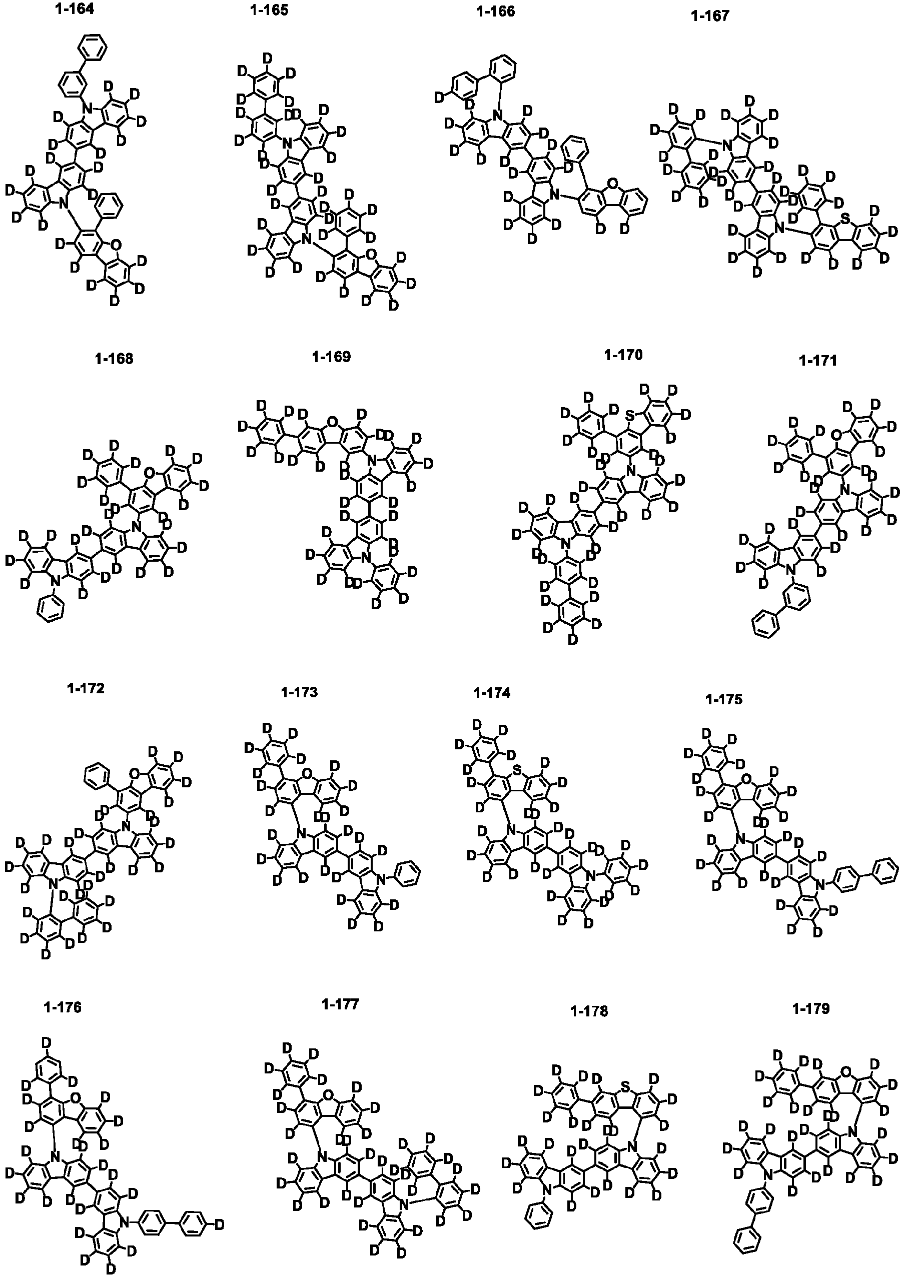

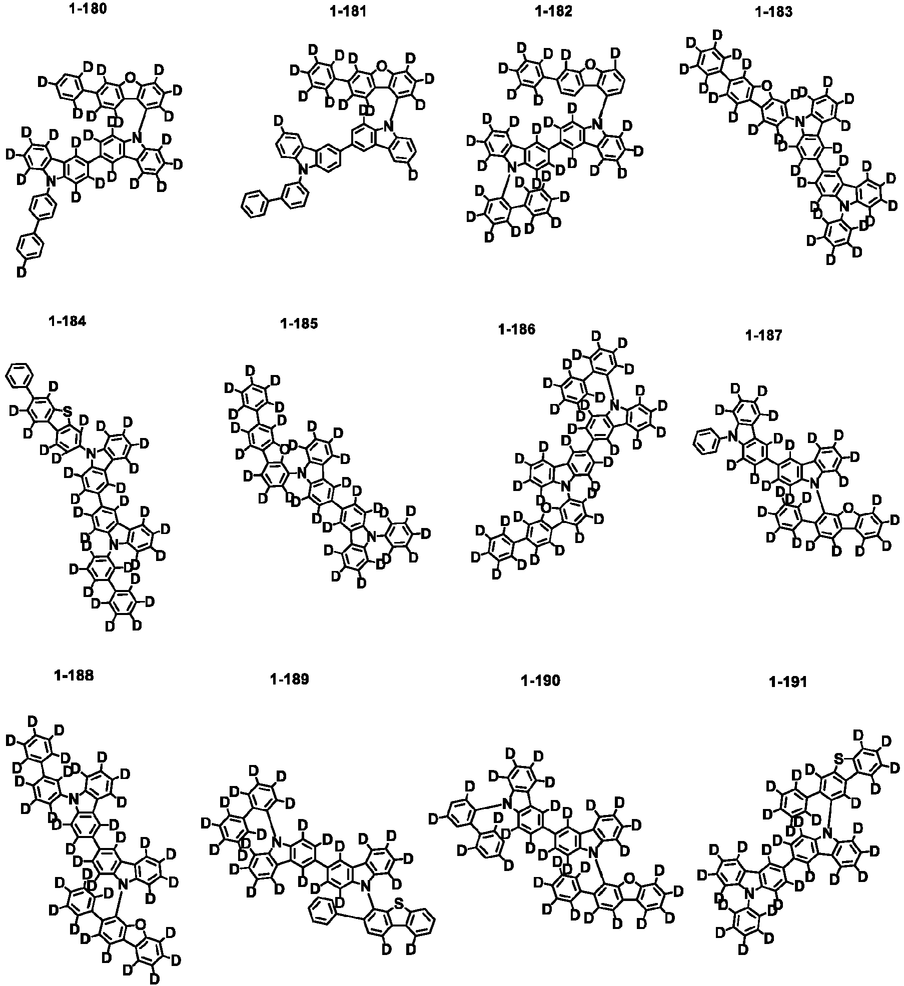

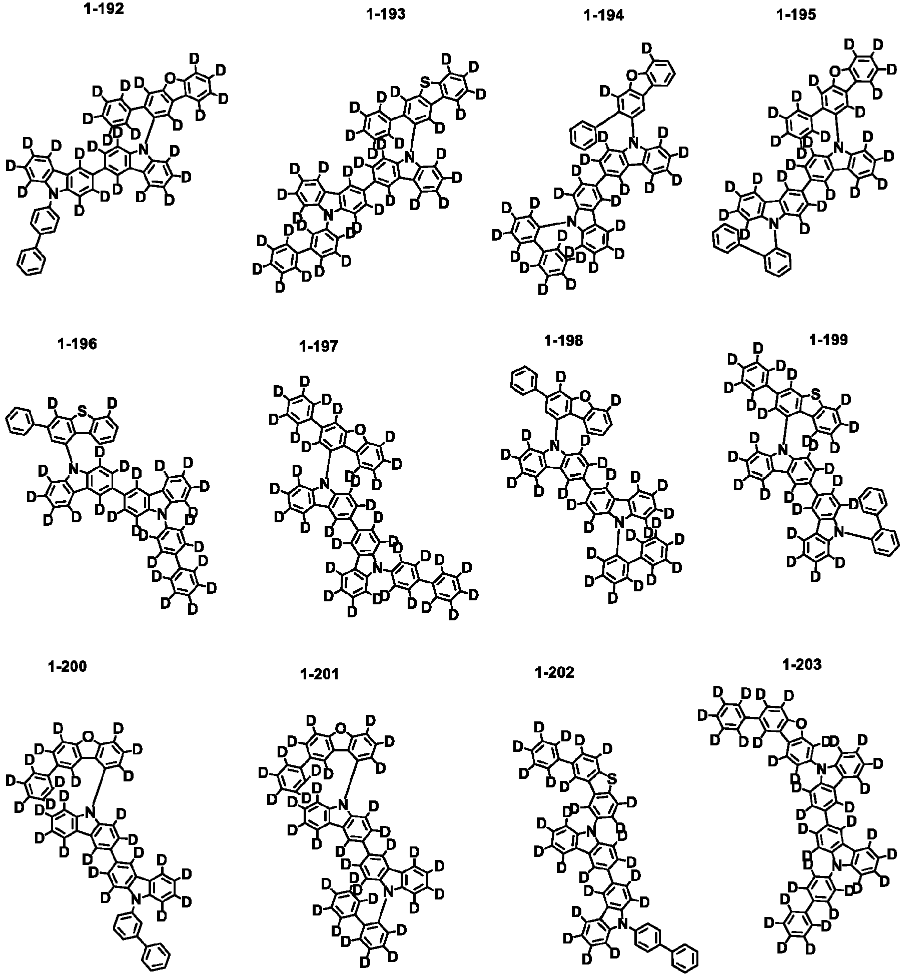

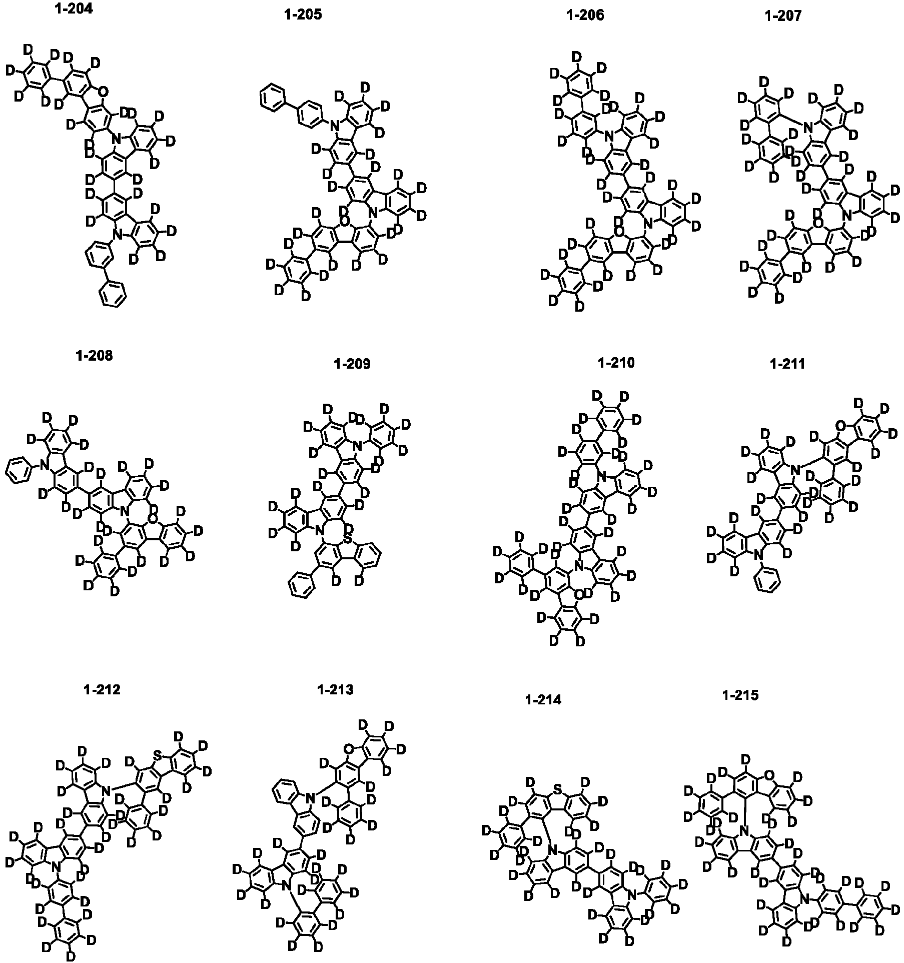

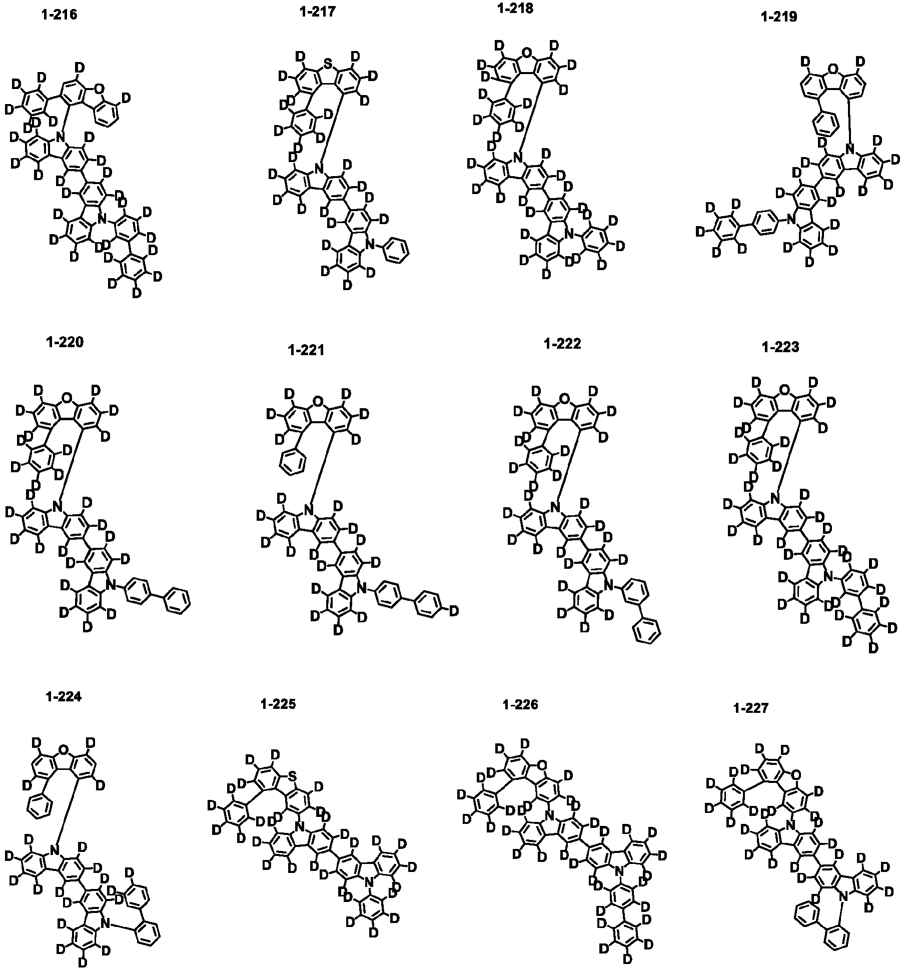

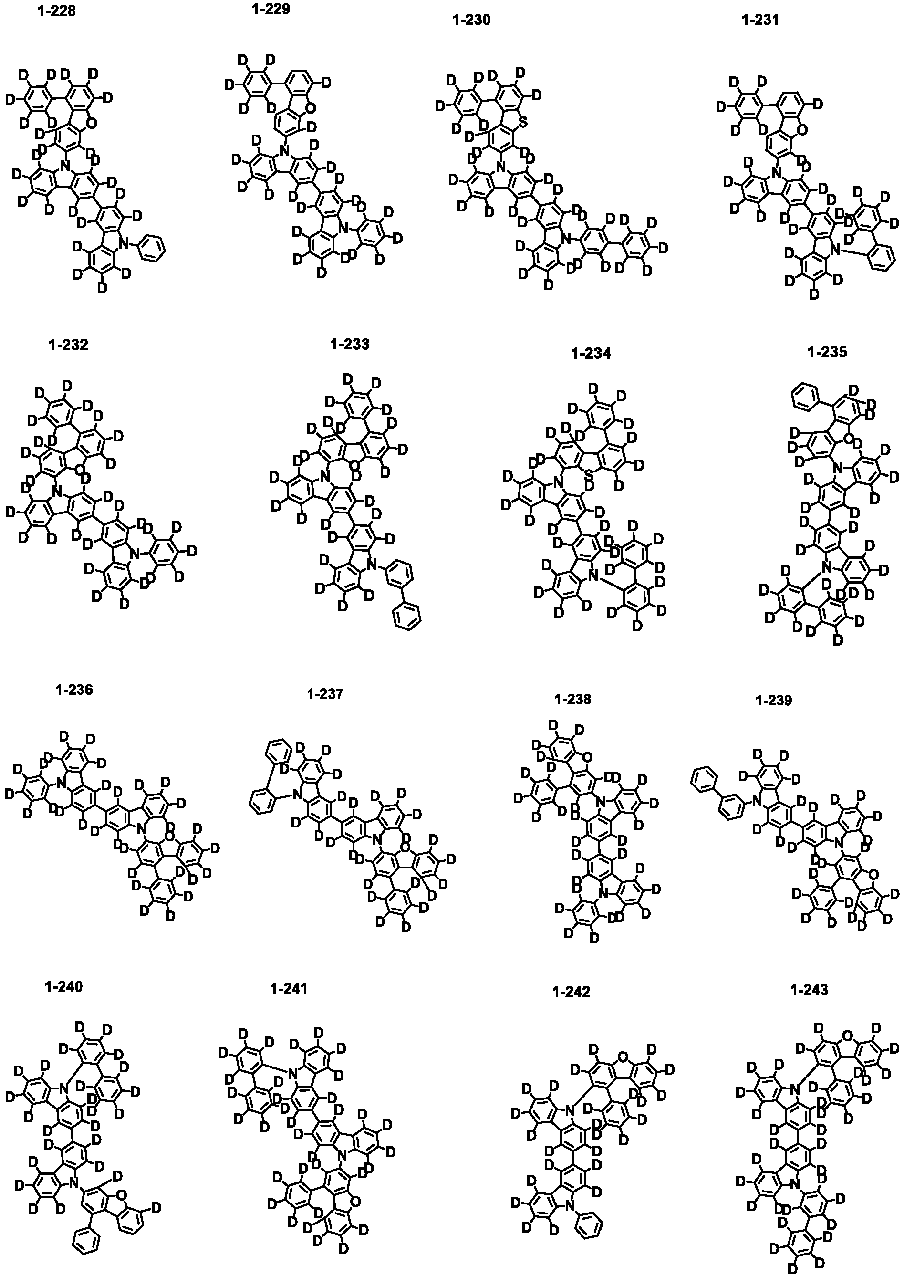

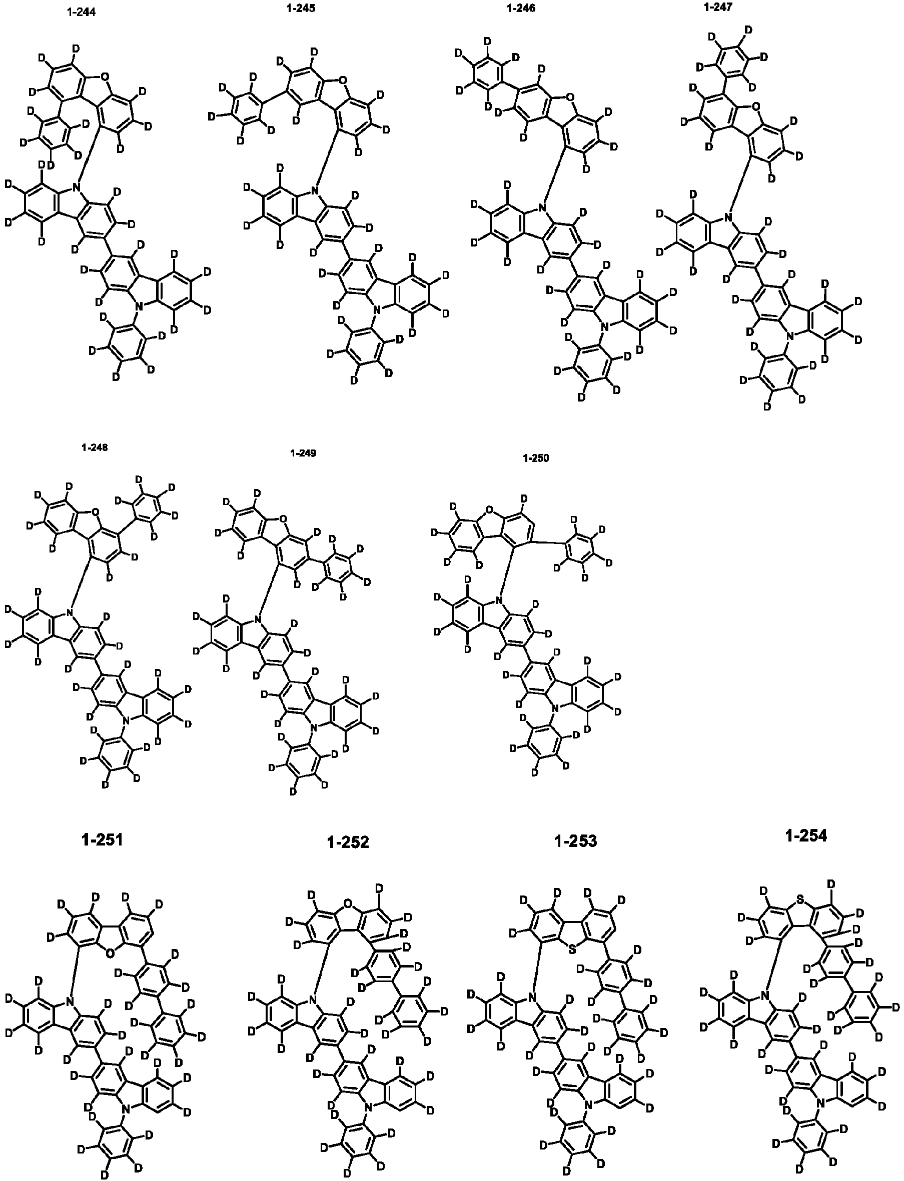

7. The composition for the organic optoelectronic device of claim 1, wherein
the second compound is represented by any one of Chemical Formula 2A to Chemical Formula 2F: 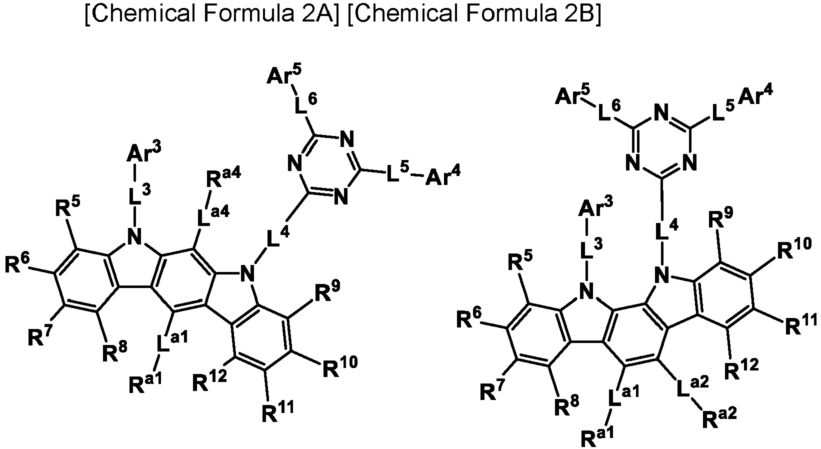 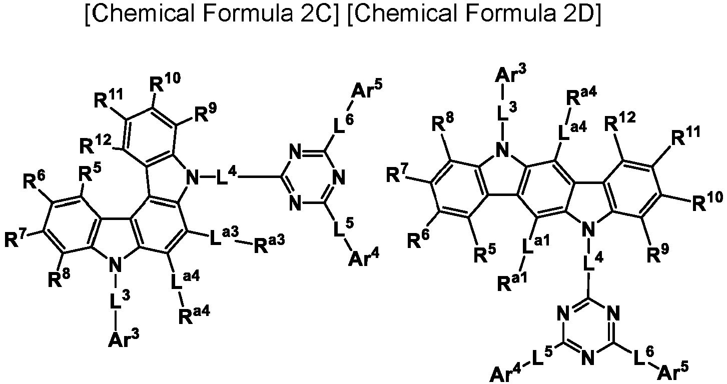 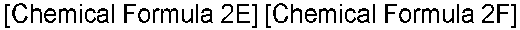 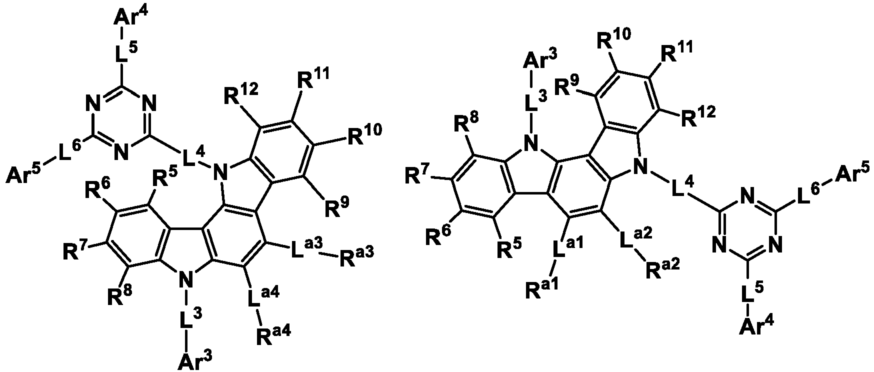
wherein, in Chemical Formula 2A to Chemical Formula 2F,
Ar³ to Ar³, L³ to L⁶, and R⁵ to R¹² are the same as described in claim 1,
L^{a1} to L^{a4} are the same as the definition of L^{a}, and
R^{a1} to R^{a4} are the same as the definition of R^{a}.

8. The composition for the organic optoelectronic device of claim 1, wherein
R⁵ to R¹² in Chemical Formulas 2 and 3 are each independently hydrogen, deuterium, or a cyano group,
L³ to L⁶ are single bond, a substituted or unsubstituted unsubstituted phenylene group, a substituted or unsubstituted unsubstituted biphenylene group, or a substituted or unsubstituted unsubstituted pyridinylene group, and
Ar³ to Ar⁵ are each independently a substituted or unsubstituted unsubstituted phenyl group, a substituted or unsubstituted unsubstituted biphenyl group, a substituted or unsubstituted unsubstituted triphenylene group, a substituted or unsubstituted unsubstituted fluorenyl group, a substituted or unsubstituted unsubstituted dibenzofuranyl group, or a substituted or unsubstituted unsubstituted dibenzothiophenyl group.

9. The composition for the organic optoelectronic device of claim 1, wherein
the first compound is represented by Chemical Formula 1-8a, and
the second compound is represented by Chemical Formula 2B: 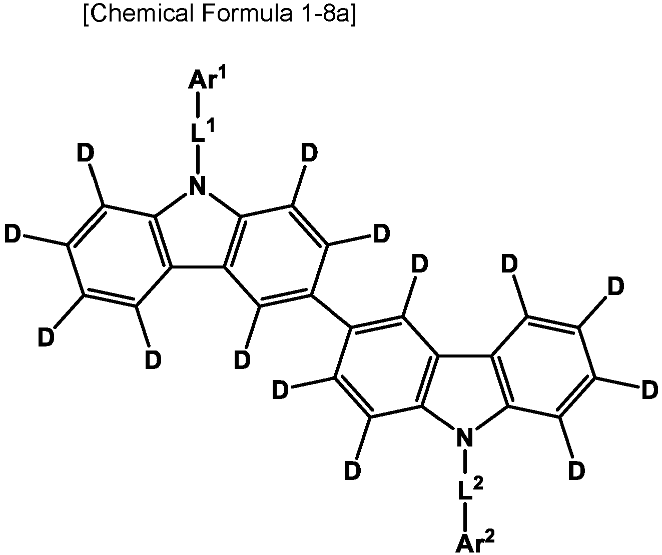
wherein, in Chemical Formula 1-8a,
L¹ and L² are a single bond or a substituted or unsubstituted phenylene group, and
Ar¹ and Ar² are each a phenyl group substituted with deuterium, a biphenyl group substituted with deuterium, a terphenyl group substituted with deuterium, or a triphenylene group substituted with deuterium, 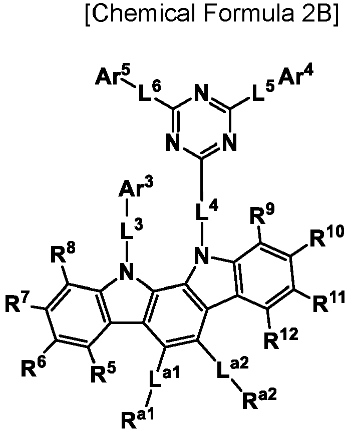
wherein, in Chemical Formula 2B,
L^{a1} and L^{a2} are a single bond,
L³ to L⁶ are each independently a single bond or a substituted or unsubstituted C6 to C12 arylene group,
R⁵ to R¹², R^{a1}, and R^{a2} are each independently hydrogen, deuterium, a cyano group, or a substituted or unsubstituted phenyl group, and
Ar³ to Ar⁵ are each independently a substituted or unsubstituted phenyl group, a substituted or unsubstituted biphenyl group, or a substituted or unsubstituted triphenylene group.

10. An organic optoelectronic device, comprising
an anode and a cathode facing each other,
at least one organic layer between the anode and the cathode, and
the organic layer includes the composition for the organic optoelectronic device of any one of claim 1 to claim 9.

11. The organic optoelectronic device of claim 10, wherein
the organic layer includes a light emitting layer, and
the light emitting layer includes the composition for an organic optoelectronic device.

12. A display device comprising the organic optoelectronic device of claim 10.

## Patentansprüche

1. Zusammensetzung für organische optoelektronische Einrichtung, aufweisend
eine erste Verbindung, dargestellt durch die Chemische Formel 1, und
eine zweite Verbindung, dargestellt durch eine Kombination der Chemischen Formel 2 und der Chemischen Formel 3: 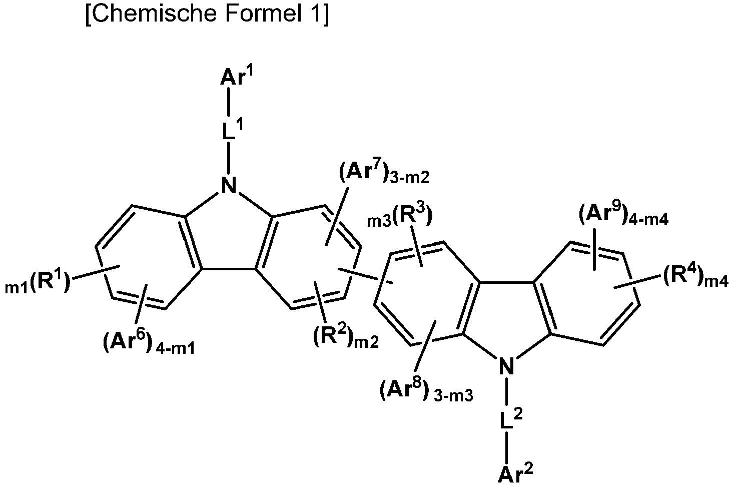
wobei in der Chemischen Formel 1
L¹ und L² jeweils unabhängig eine Einfachbindung oder eine substituierte oder unsubstituierte C6- bis C30-Arylengruppe sind,
Ar¹ und Ar² jeweils unabhängig eine substituierte oder unsubstituierte C6-bis C30-Arylgruppe oder eine substituierte oder unsubstituierte C2- bis C30-heterocyclische Gruppe sind,
R¹ bis R⁴ jeweils unabhängig Wasserstoff, Deuterium oder eine substituierte oder unsubstituierte C6- bis C30-Arylgruppe sind,
Ar⁶ bis Ar⁹ jeweils unabhängig Wasserstoff oder eine substituierte oder unsubstituierte C6- bis C30-Arylgruppe sind,
m1 und m4 jeweils unabhängig eine der Ganzzahlen 1 bis 4 sind,
m2 und m3 jeweils unabhängig eine der Ganzzahlen 1 bis 3 sind und
die Chemische Formel 1 gleichzeitig die folgenden Bedingungen (i) und (ii) erfüllt:
(i) mindestens eines aus Ar¹ und Ar² ist eine mit mindestens einem Deuterium substituierte C6- bis C30-Arylgruppe oder eine mit mindestens einem Deuterium substituierte C2- bis C30-heterocyclische Gruppe und
(ii) mindestens eines aus R¹ bis R⁴ ist Deuterium; 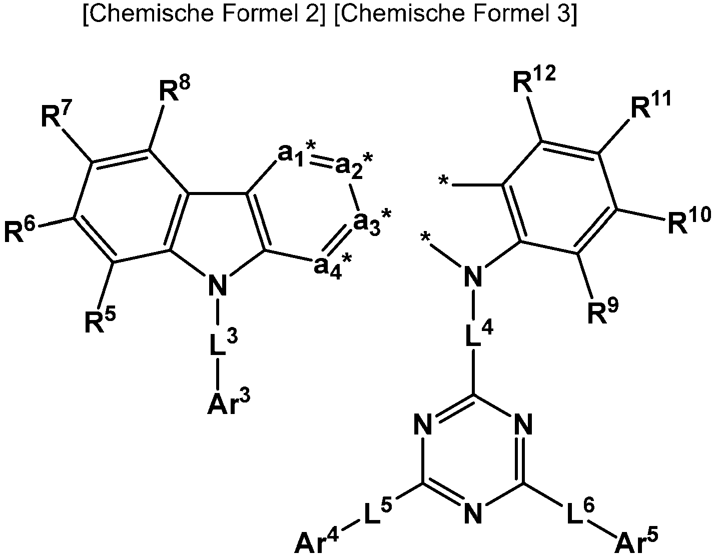
wobei in der Chemischen Formel 2 und der Chemischen Formel 3
Ar³ bis Ar⁵ jeweils unabhängig eine substituierte oder unsubstituierte C6-bis C20-Arylgruppe oder eine substituierte oder unsubstituierte C2- bis C30-heterocyclische Gruppe sind,
a₁* bis a₄* jeweils unabhängig ein Verknüpfungskohlenstoff (C) oder C-L^{a}-R^{a} sind,
zwei benachbarte aus a₁* bis a₄* in der Chemischen Formel 2 mit * in der Chemischen Formel 3 verknüpft sind,
L^{a}, L³ bis L⁶ jeweils unabhängig eine Einfachbindung, eine substituierte oder unsubstituierte C6- bis C20-Arylengruppe oder eine substituierte oder unsubstituierte C2- bis C20-Heteroarylengruppe sind und
R^{a} und R⁵ bis R¹² jeweils unabhängig Wasserstoff, Deuterium, eine Cyanogruppe, ein Halogen, eine substituierte oder unsubstituierte Aminogruppe, eine substituierte oder unsubstituierte C1- bis C30-Alkylgruppe, eine substituierte oder unsubstituierte C6- bis C30-Arylgruppe oder eine substituierte oder unsubstituierte C2- bis C30-heterocyclische Gruppe sind,
wobei für den Fall, dass die erste Verbindung aus einer der folgenden Verbindungen D1-1 bis D1-8 ausgewählt ist und die zweite Verbindung aus einer der folgenden Verbindungen D2-1 bis D2-7 ausgewählt ist, die folgenden Kombinationen ausgeschlossen sind:
D1-1 mit D2-1, D1-2 mit D2-1, D1-3 mit D2-1, D1-4 mit D2-1, D1-5 mit D2-1,
D1-1 mit D2-2, D1-2 mit D2-2, D1-3 mit D2-2, D1-4 mit D2-2, D1-5 mit D2-2,
D1-1 mit D2-3, D1-2 mit D2-3, D1-3 mit D2-3, D1-4 mit D2-3, D1-5 mit D2-3,
D1-6 mit D2-4, D1-7 mit D2-5, D1-8 mit D2-6 und D1-6 mit D2-7

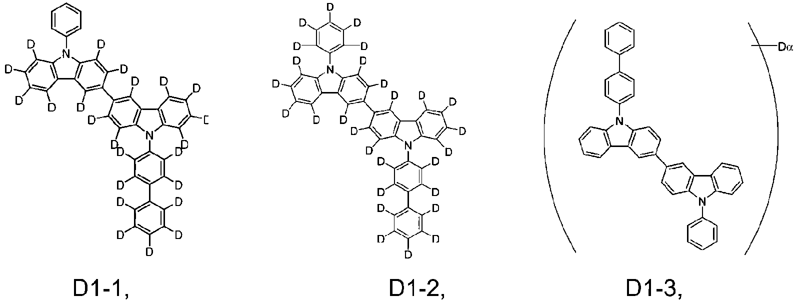

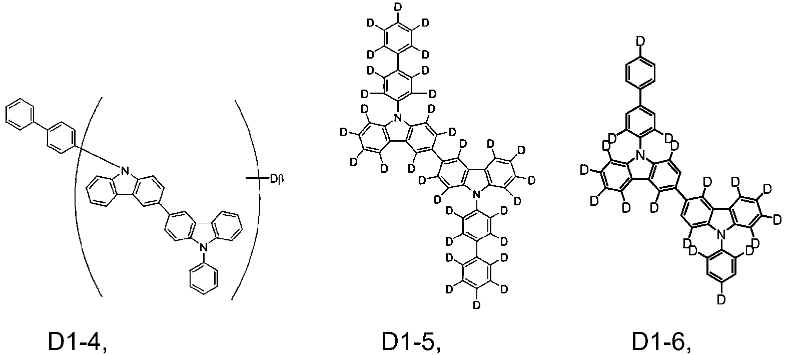

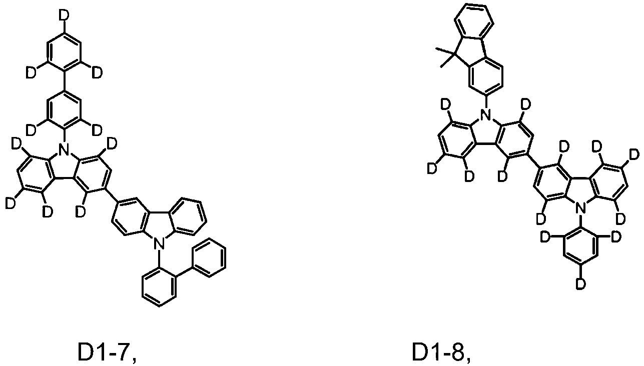

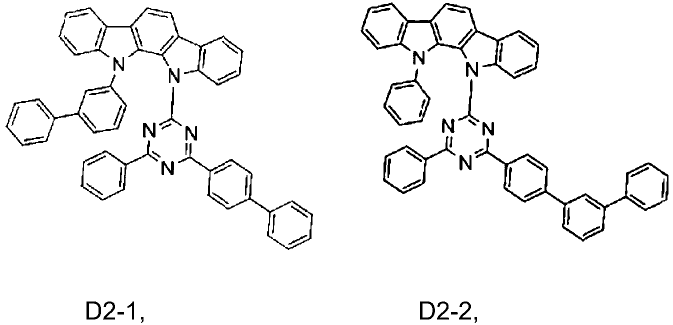

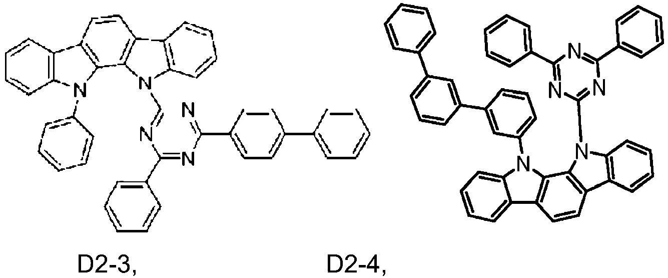

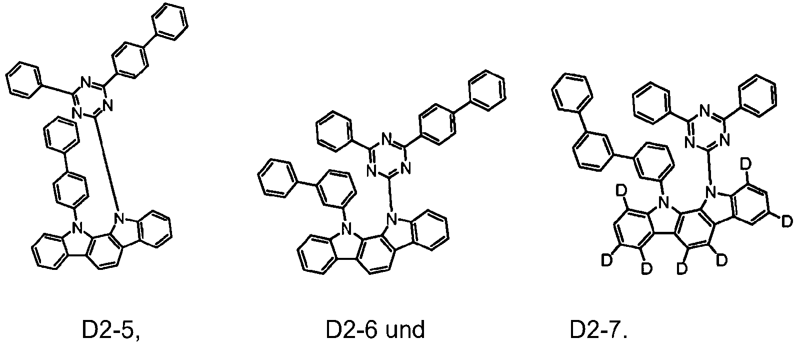

2. Zusammensetzung für organische optoelektronische Einrichtung nach Anspruch 1, wobei
die erste Verbindung durch eine der Chemischen Formeln 1-1 bis 1-10 dargestellt ist: 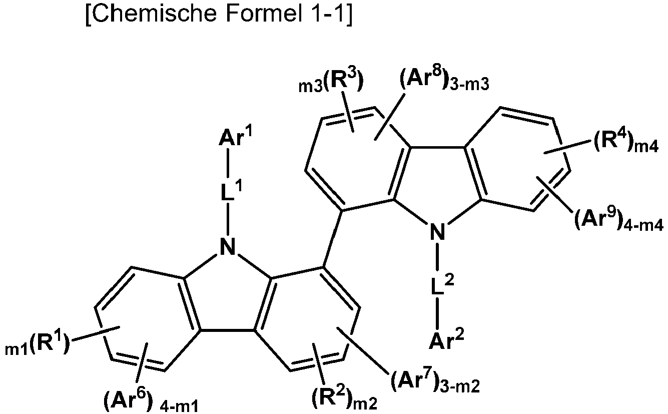 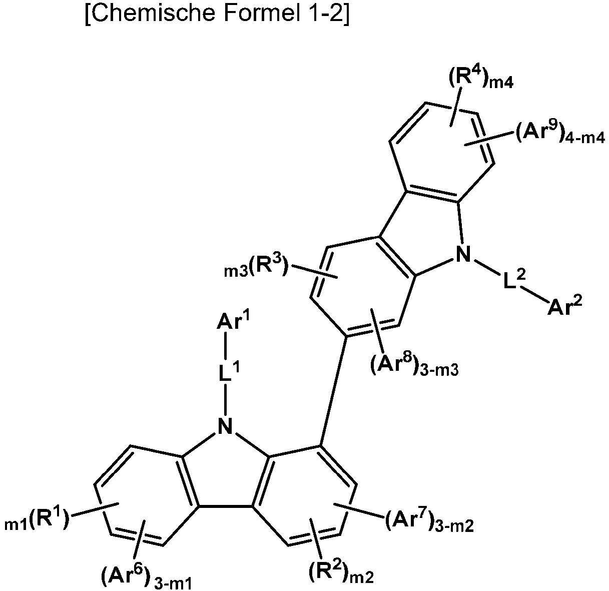 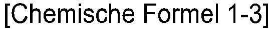 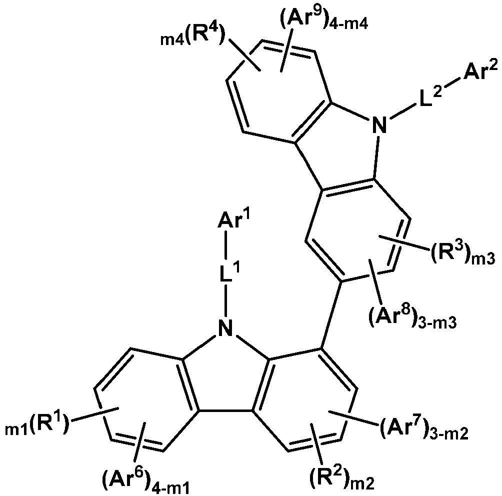 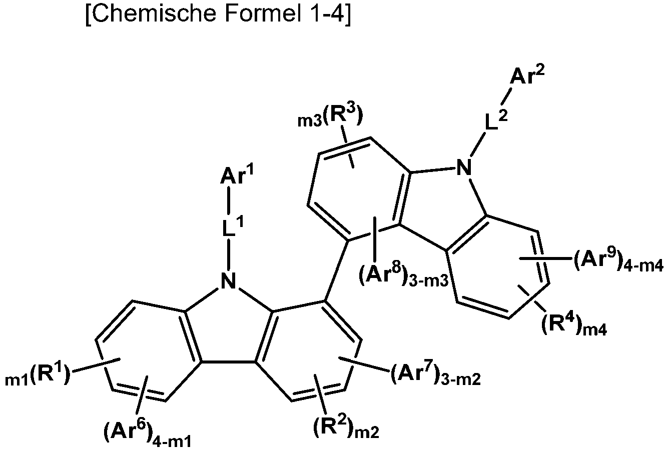 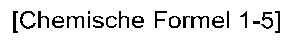 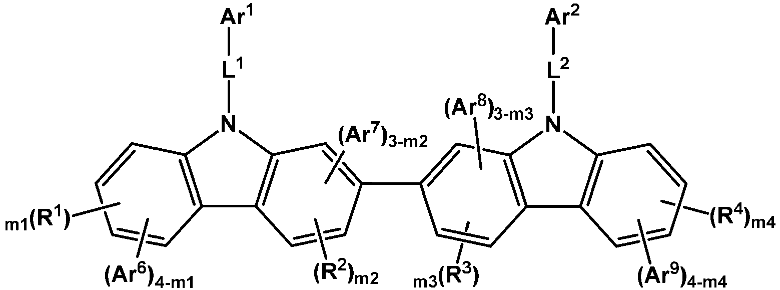 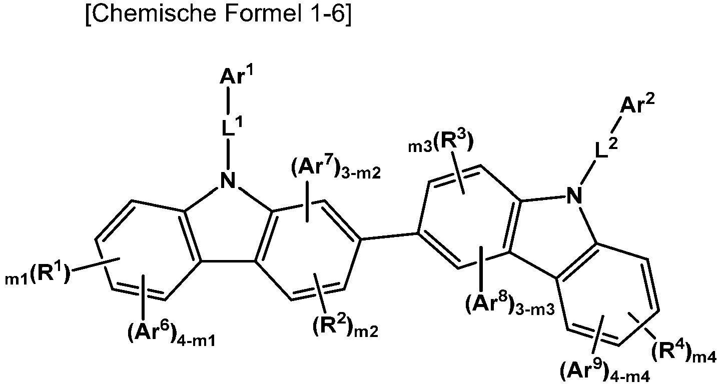 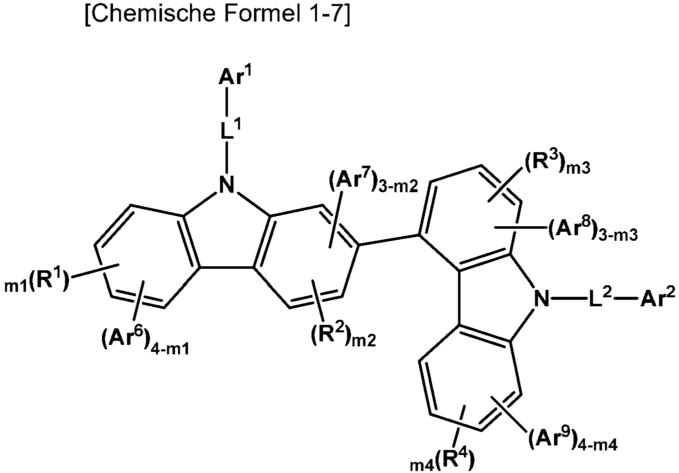 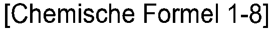 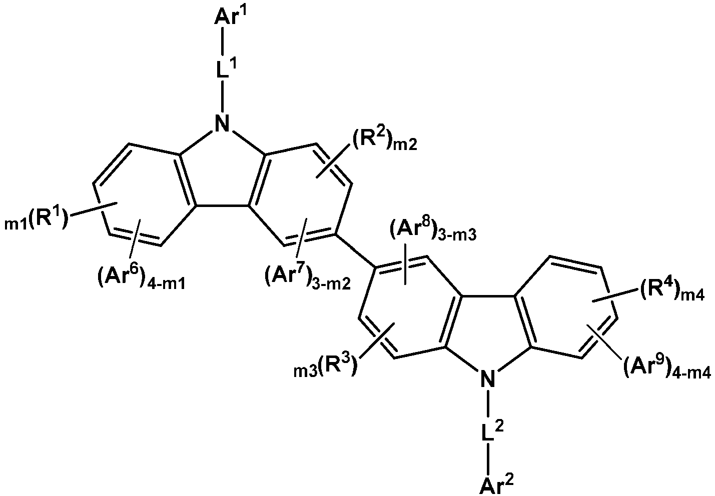 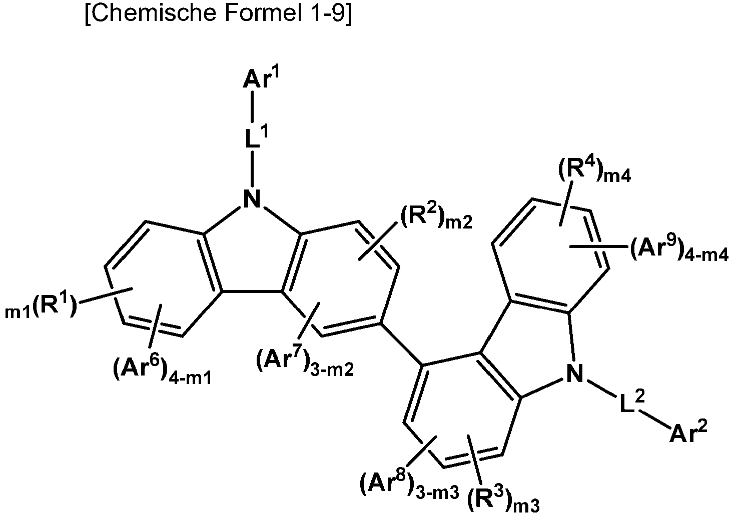 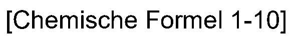 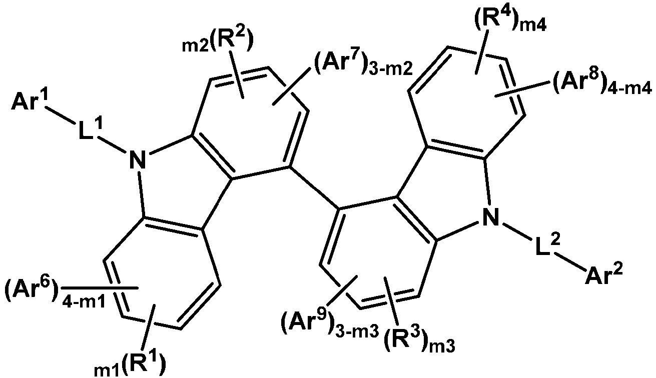
wobei in den Chemischen Formeln 1-1 bis 1-10
L¹, L², Ar¹, Ar², Ar⁶ bis Ar⁹, R¹ bis R⁴ und m¹ bis m⁴ wie in Anspruch 1 beschrieben definiert sind.

3. Zusammensetzung für organische optoelektronische Einrichtung nach Anspruch 1, wobei
mindestens eines aus Ar¹ und Ar² der Chemischen Formel 1 eine mit mindestens einem Deuterium substituierte Phenylgruppe, eine mit mindestens einem Deuterium substituierte Biphenylgruppe, eine mit mindestens einem Deuterium substituierte Terphenylgruppe, eine mit mindestens einem Deuterium substituierte Naphthylgruppe, eine mit mindestens einem Deuterium substituierte Anthracenylgruppe, eine mit mindestens einem Deuterium substituierte Phenanthrenylgruppe, eine mit mindestens einem Deuterium substituierte Triphenylengruppe, eine mit mindestens einem Deuterium substituierte Fluorenylgruppe, eine mit mindestens einem Deuterium substituierte Dibenzofuranylgruppe oder eine mit mindestens einem Deuterium substituierte Dibenzothiophenylgruppe ist.

4. Zusammensetzung für organische optoelektronische Einrichtung nach Anspruch 1, wobei
L¹-Ar¹ und L²-Ar² der Chemischen Formel 1 jeweils unabhängig aus den in Gruppe I -1 und Gruppe I -2 aufgeführten Substituenten ausgewählt sind und
mindestens eines aus L¹-Ar¹ und L²-Ar² aus den aufgeführten Substituenten in Gruppe I -2 ausgewählt ist: 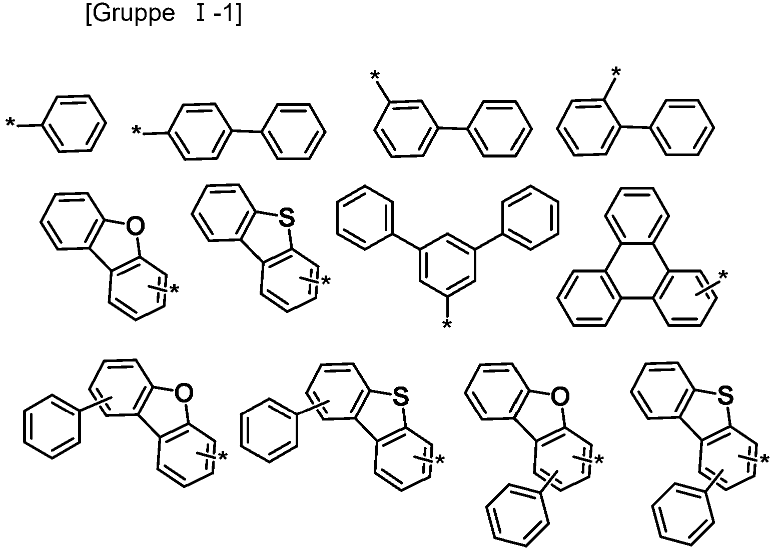 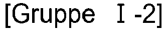 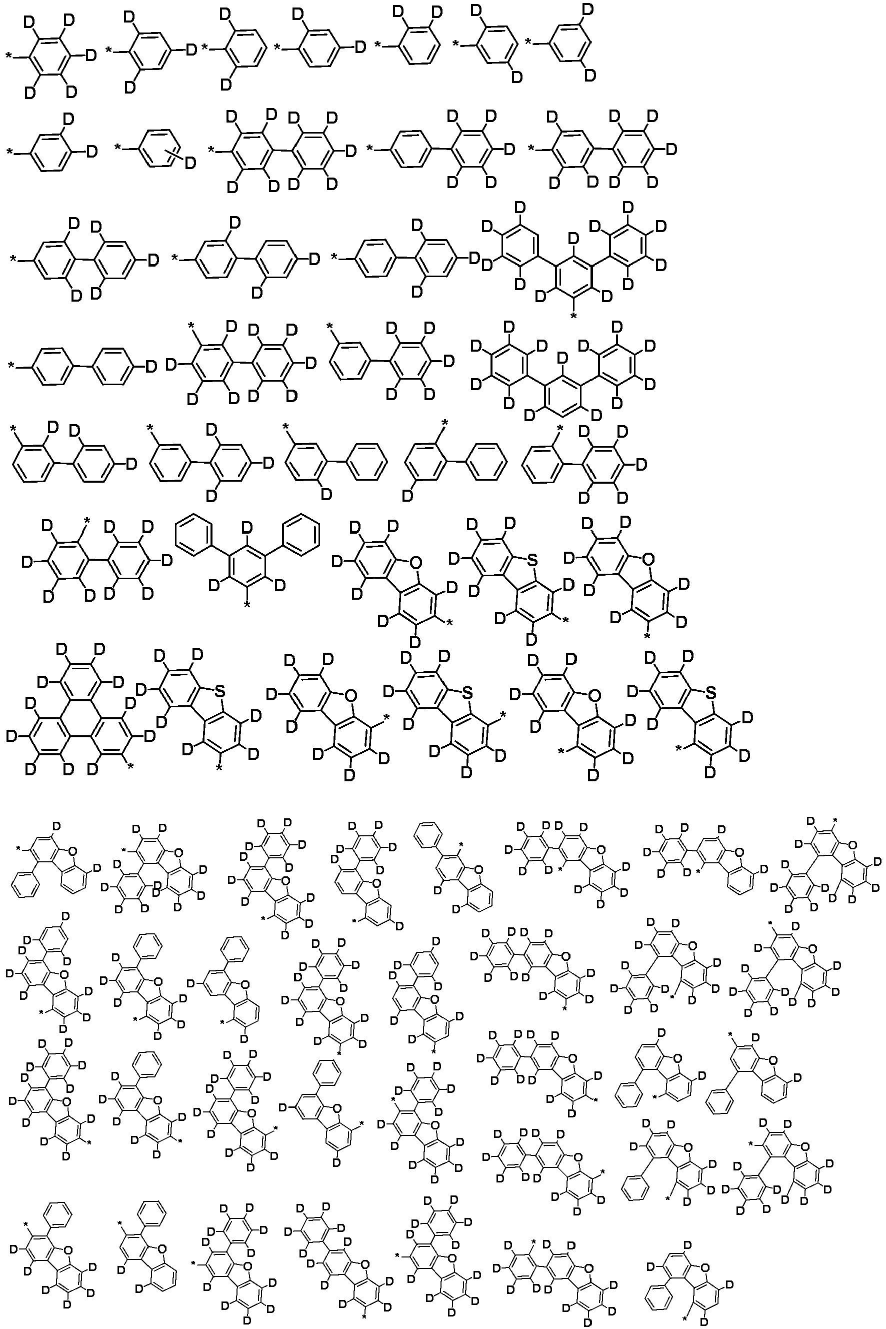 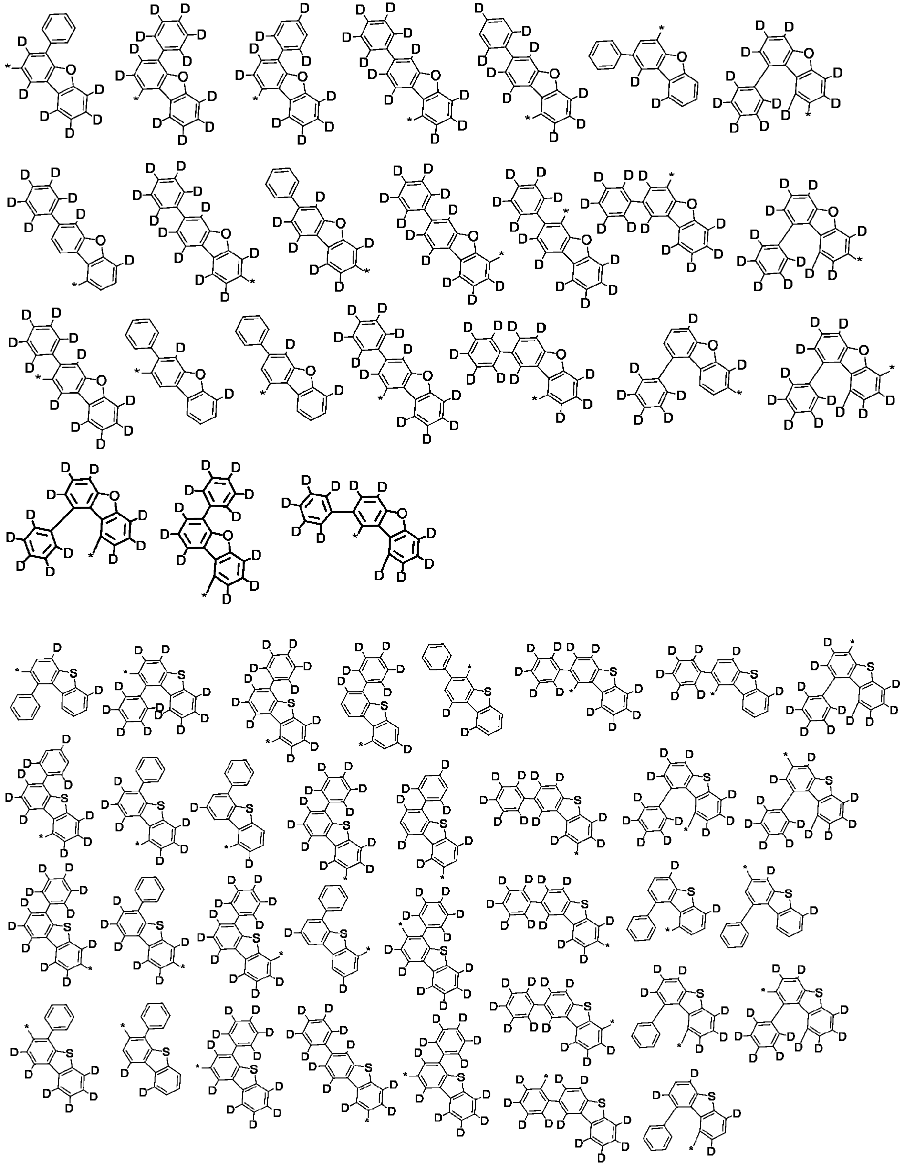 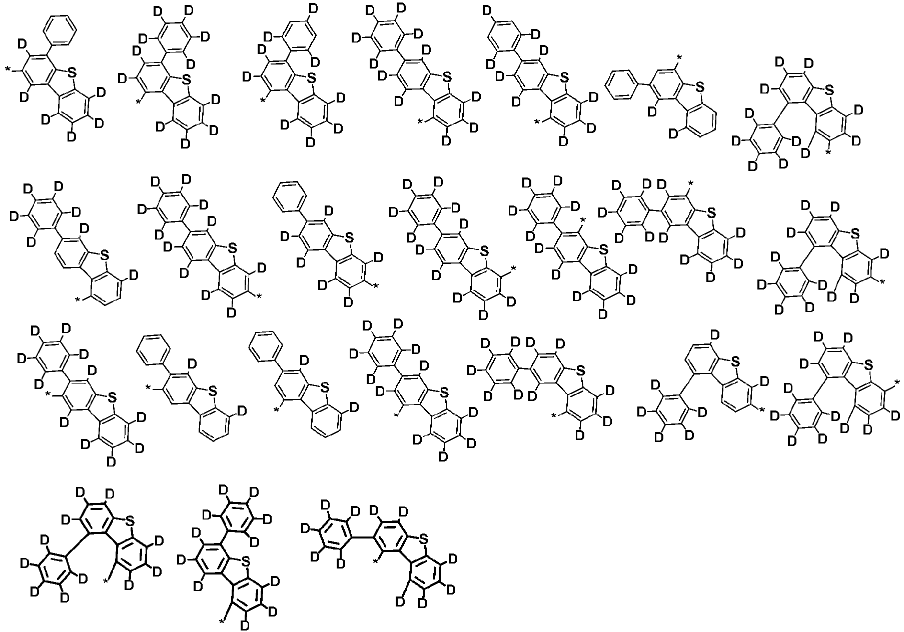
wobei in Gruppe I -1 und Gruppe I -2 * ein Verknüpfungspunkt ist.

5. Zusammensetzung für organische optoelektronische Einrichtung nach Anspruch 1, wobei
die erste Verbindung durch die Chemische Formel 1-8a dargestellt ist: 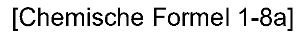 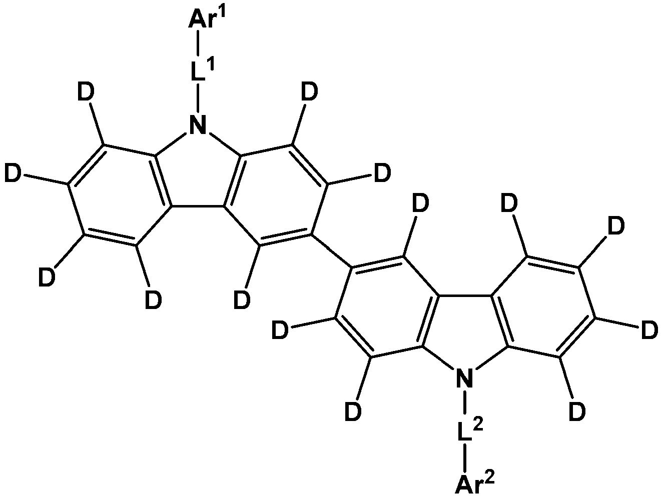
wobei in der Chemischen Formel 1-8a
L¹, L², Ar¹ und Ar² wie in Anspruch 1 definiert sind.

6. Zusammensetzung für organische optoelektronische Einrichtung nach Anspruch 1, wobei
die erste Verbindung eine aus den in Gruppe 1 aufgeführten Verbindungen ausgewählte Verbindung ist:

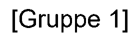

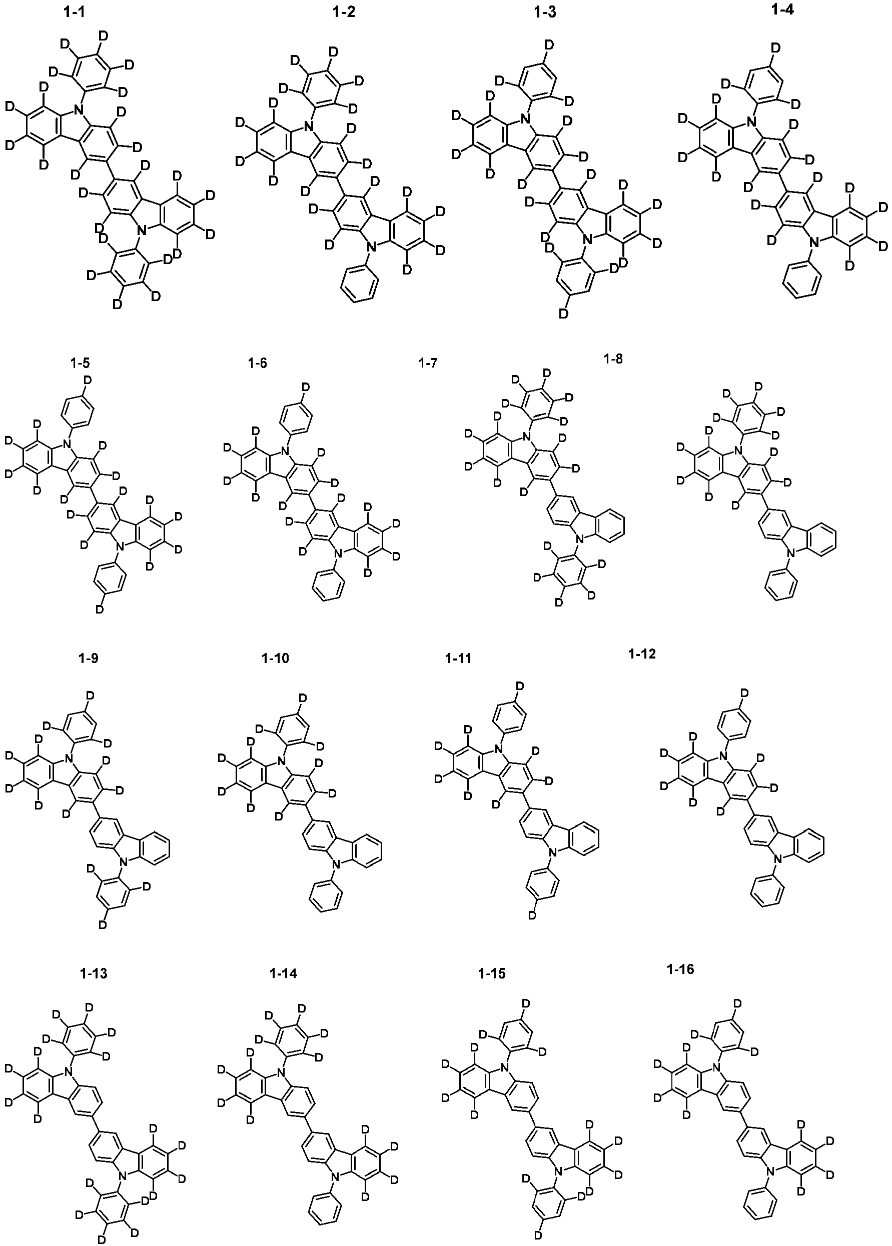

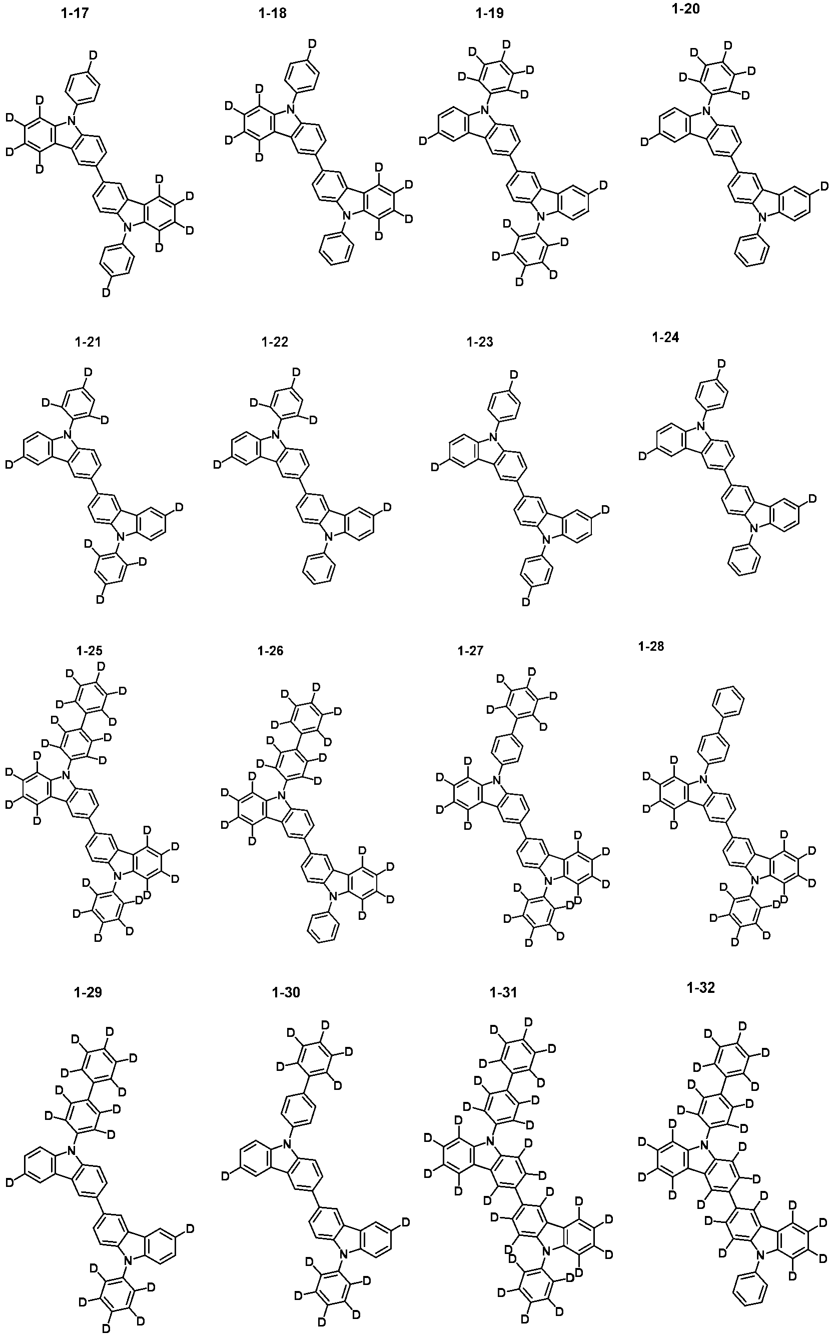

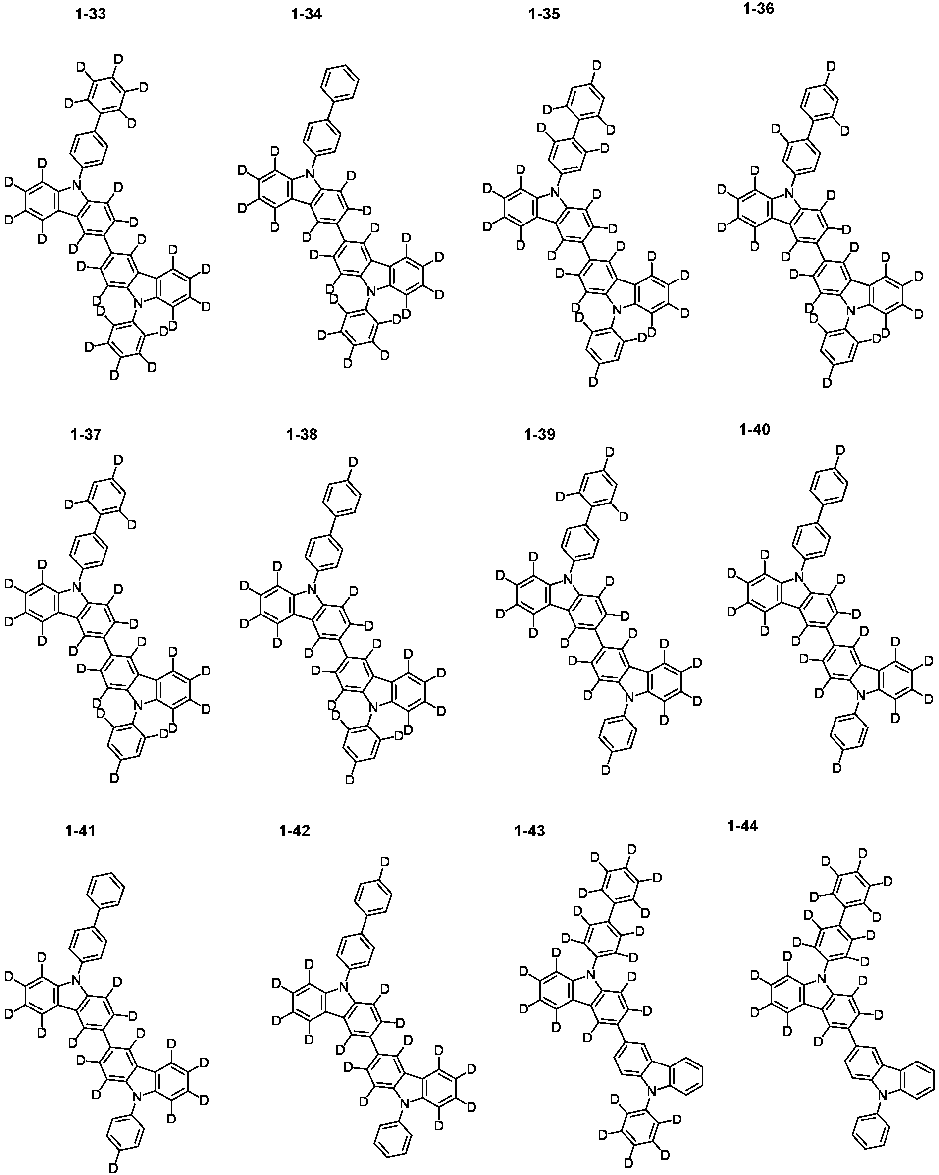

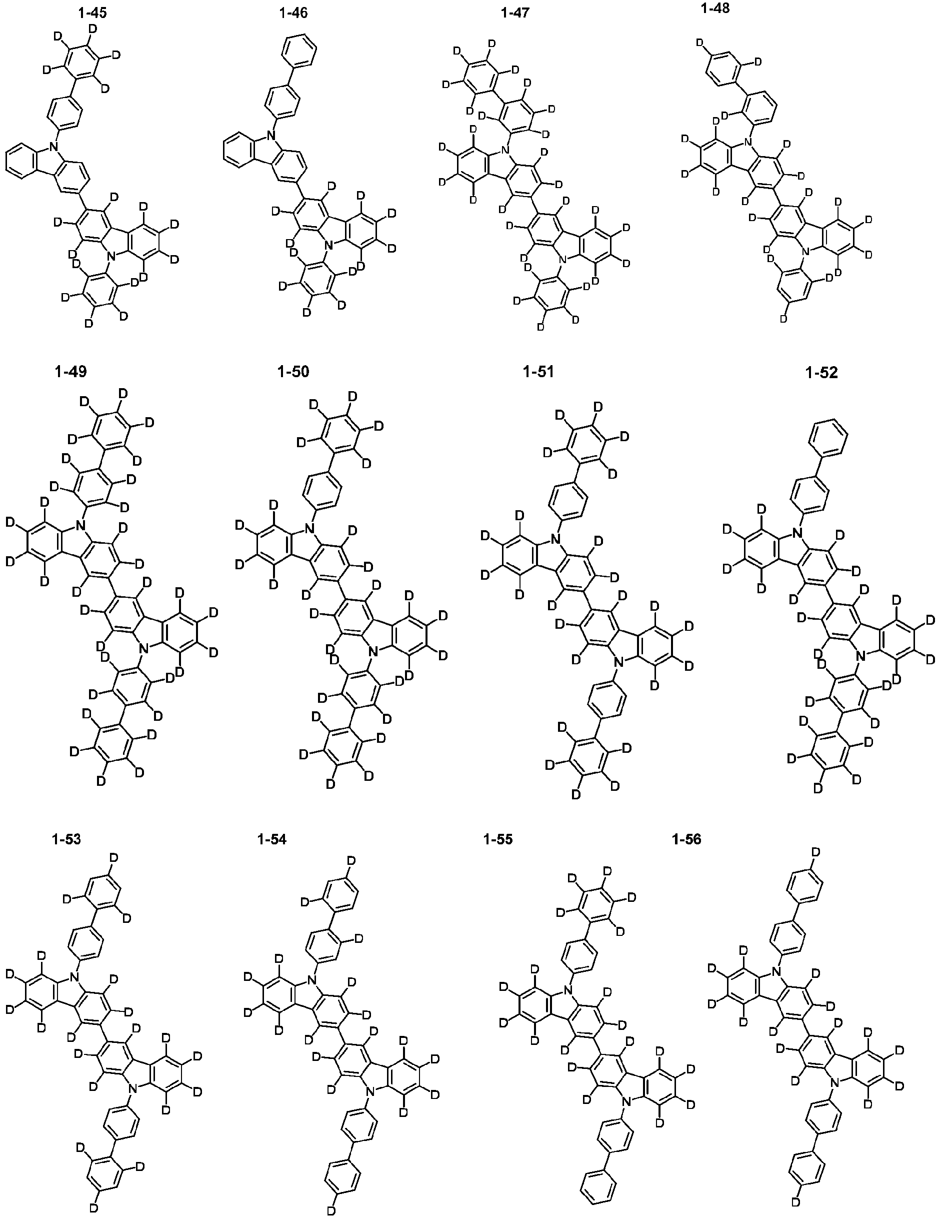

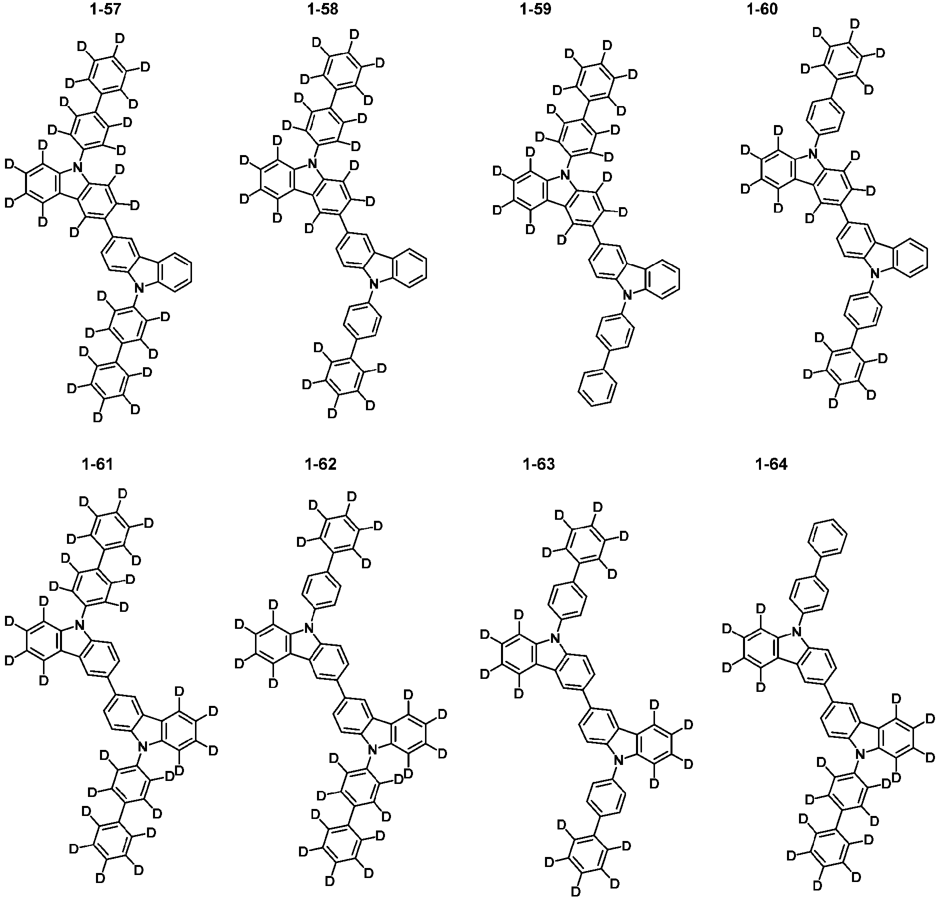

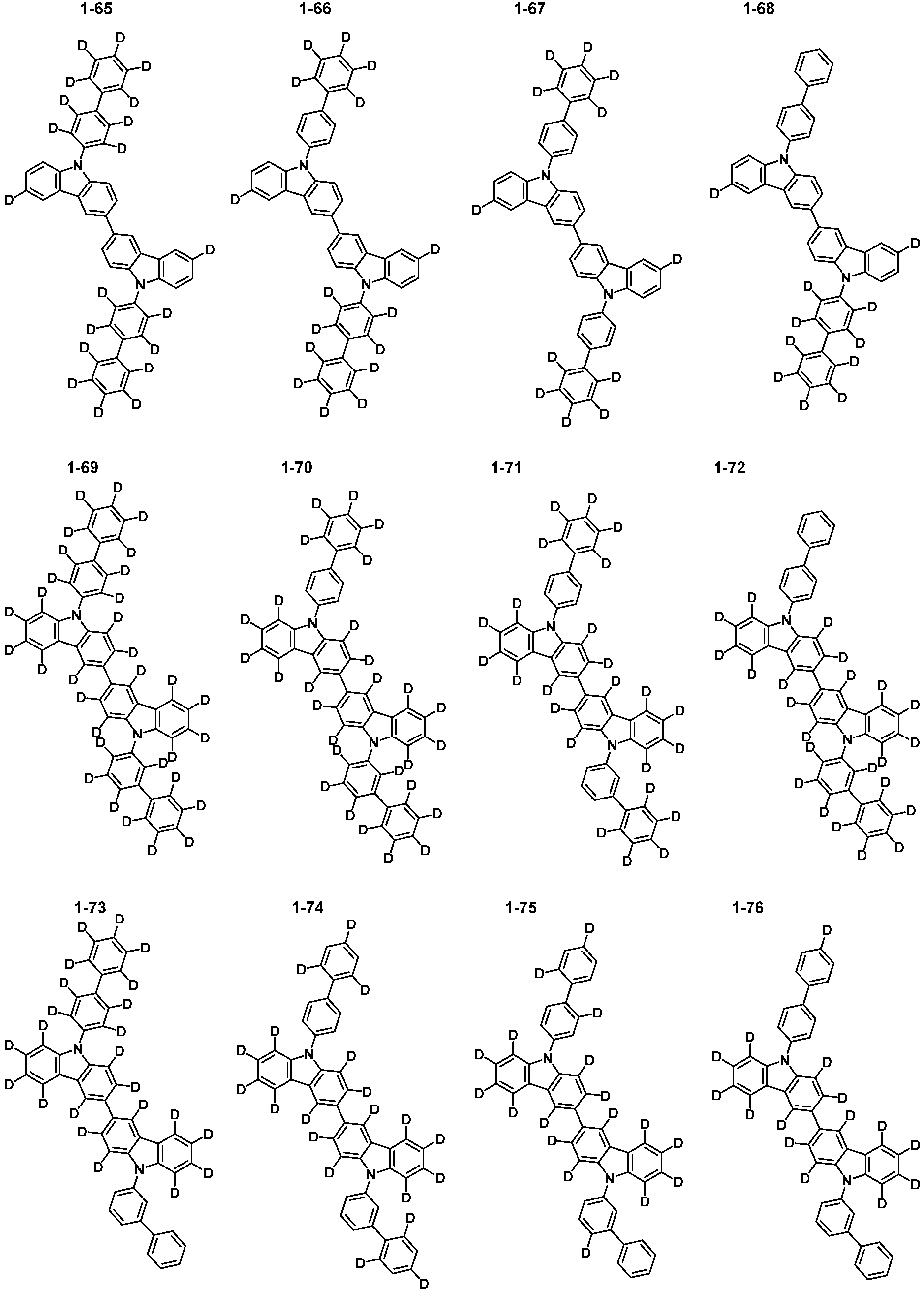

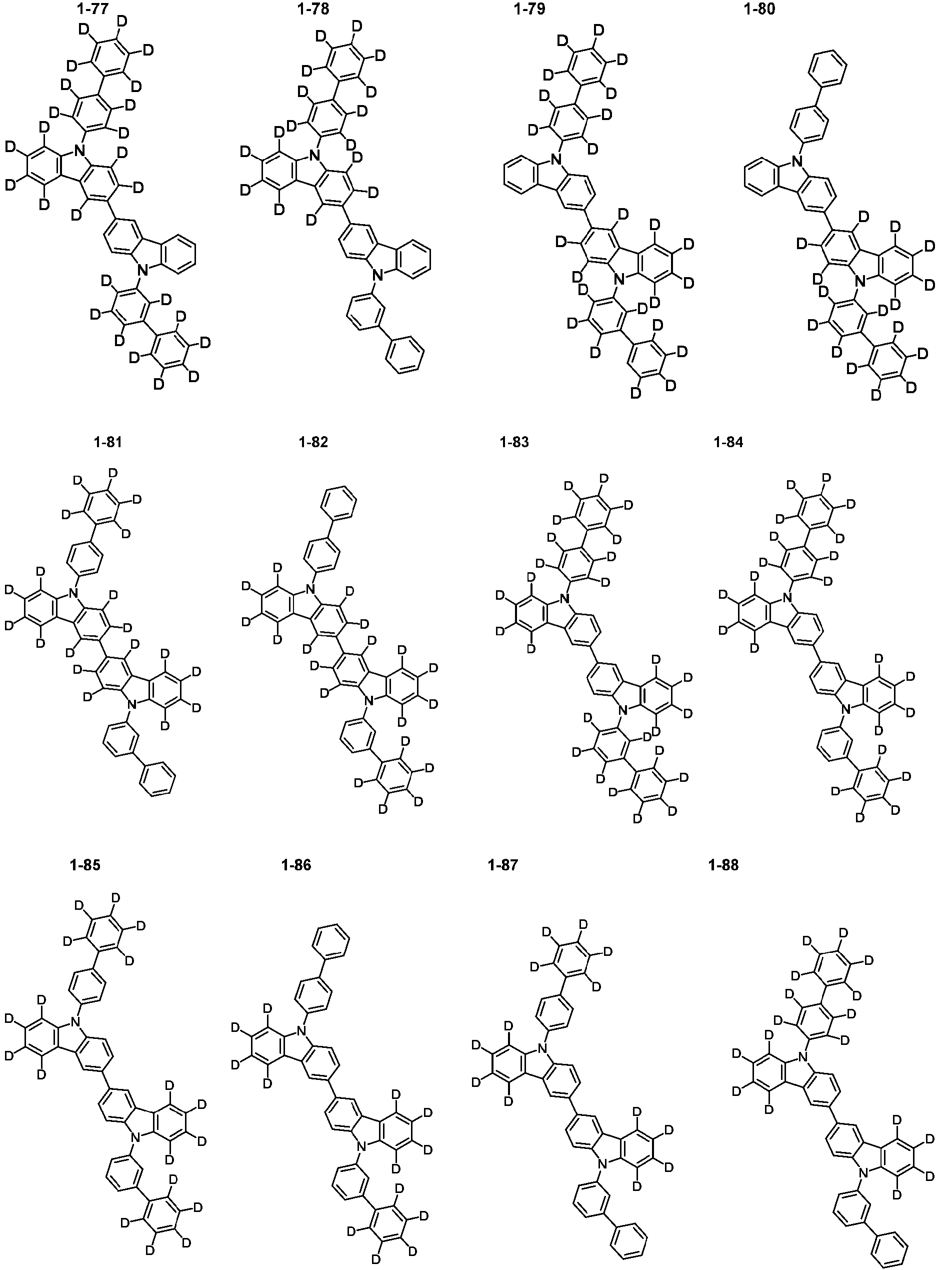

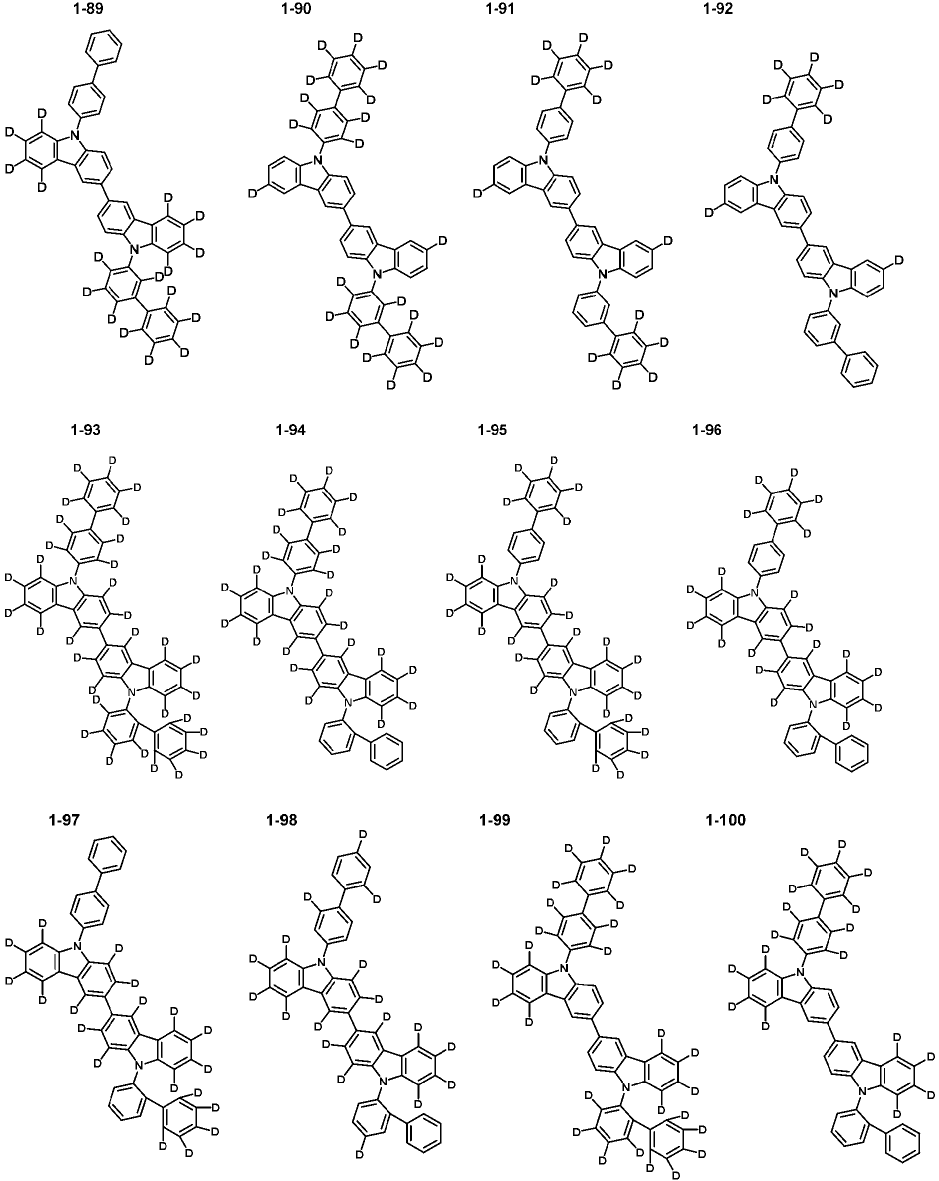

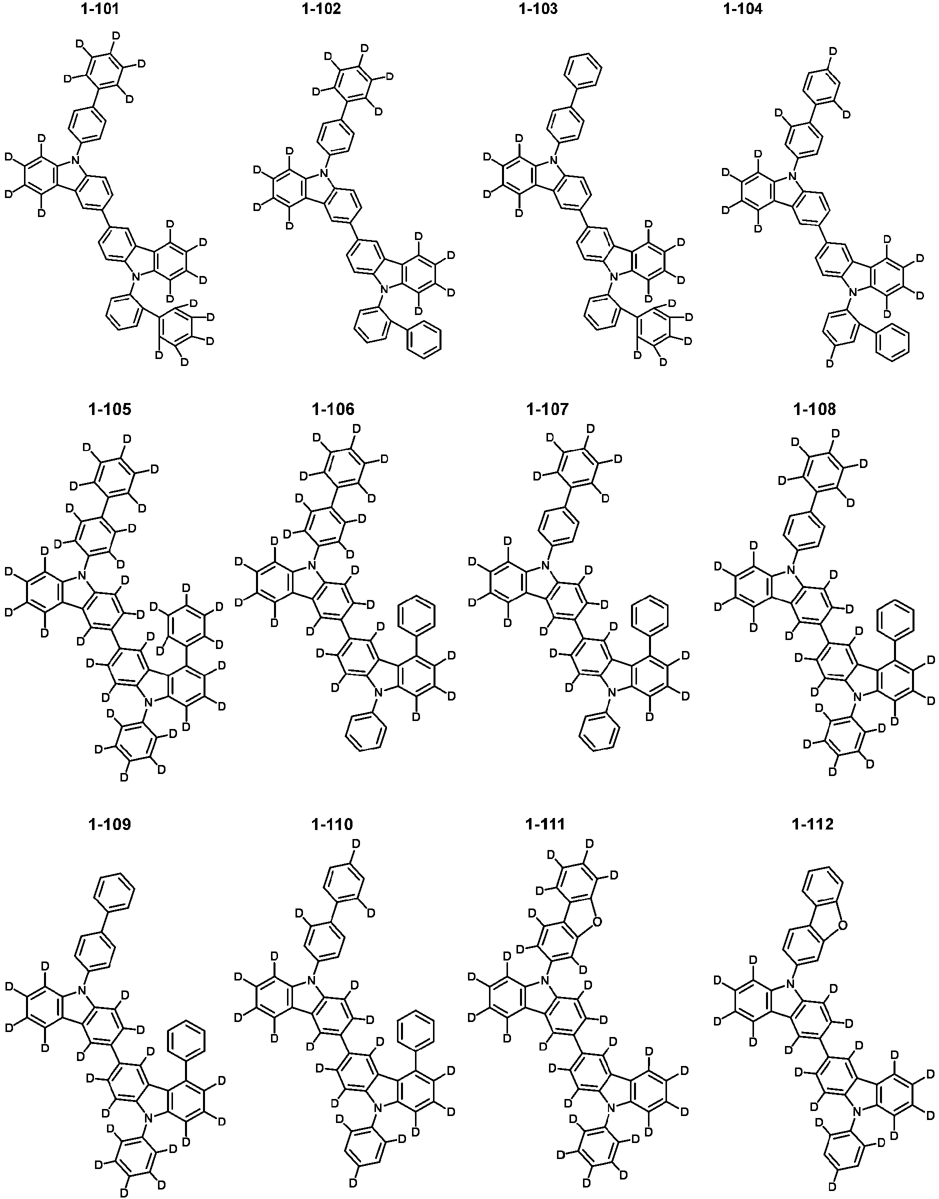

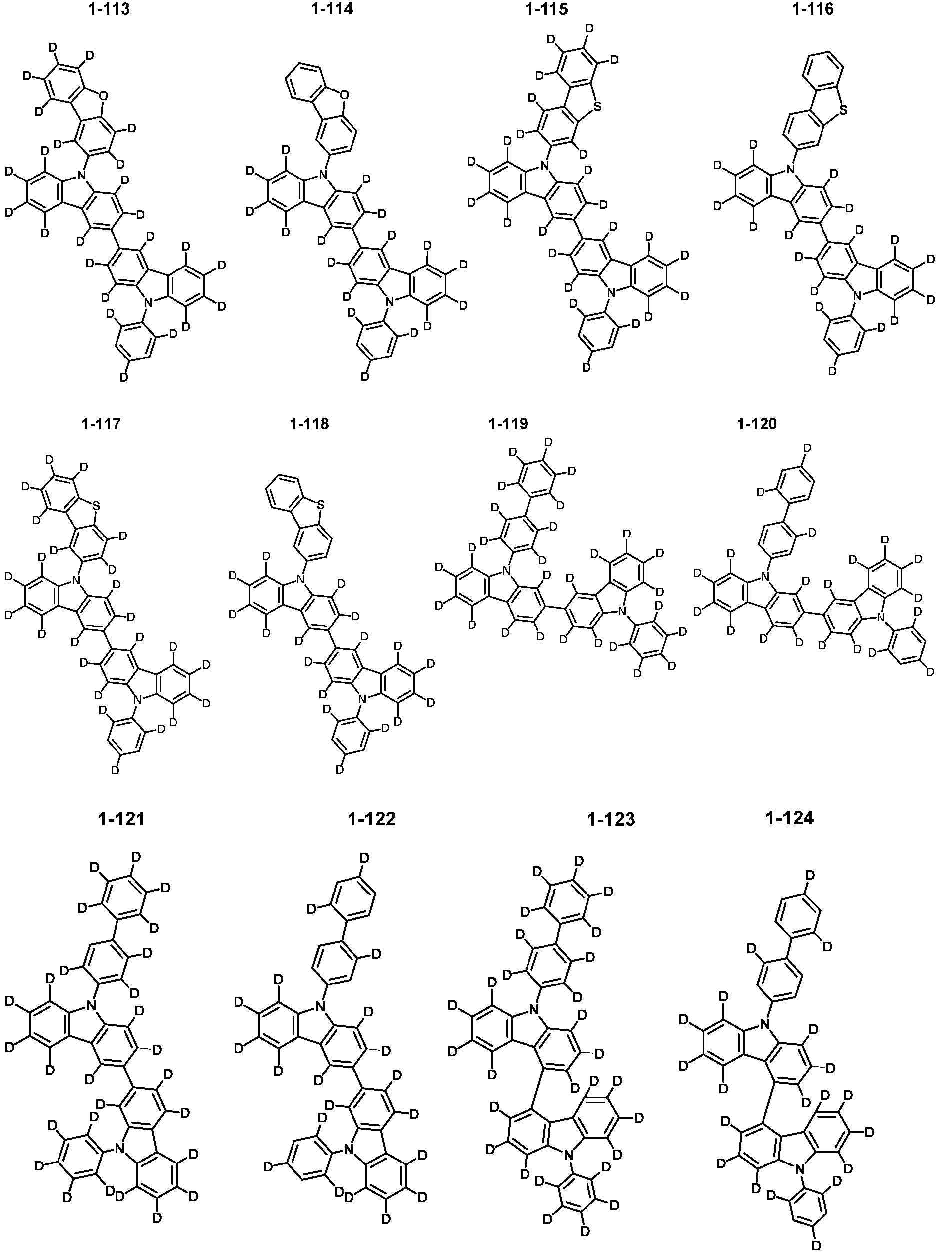

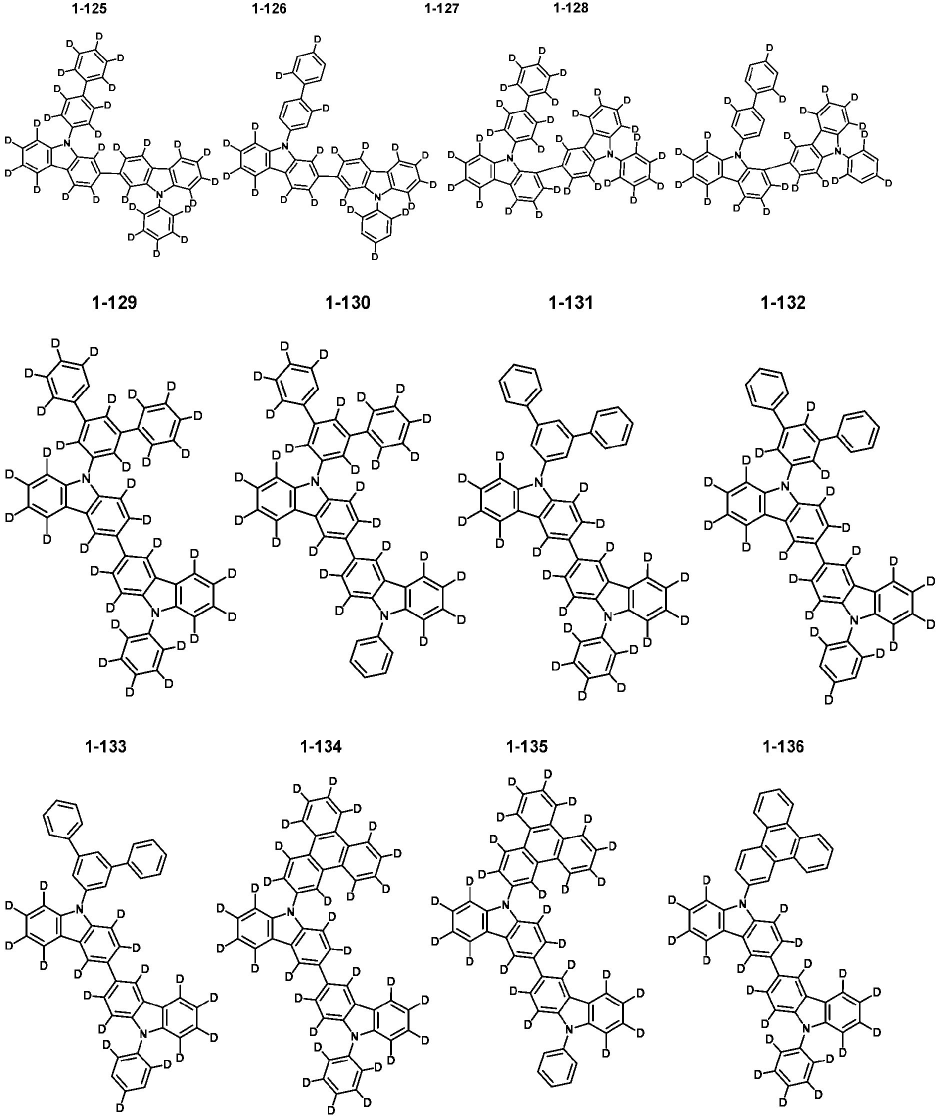

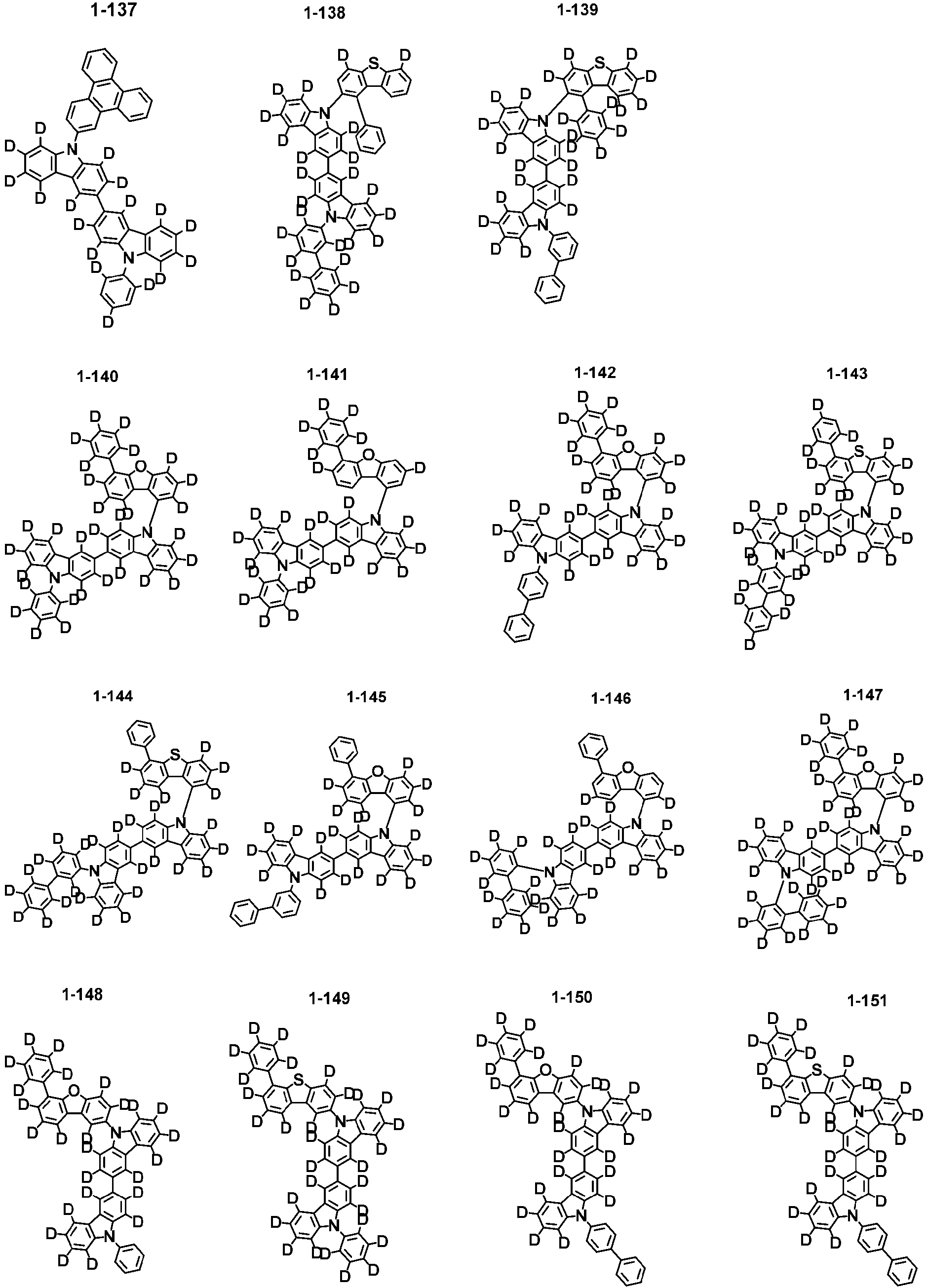

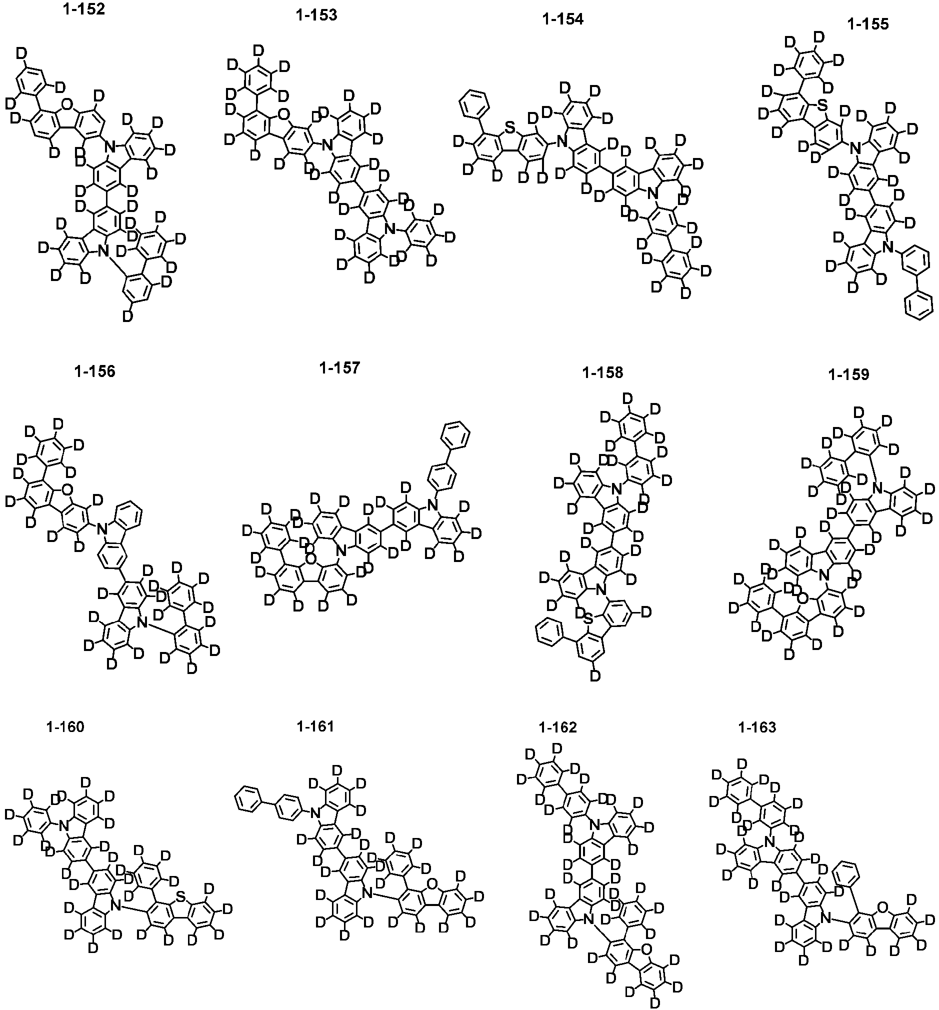

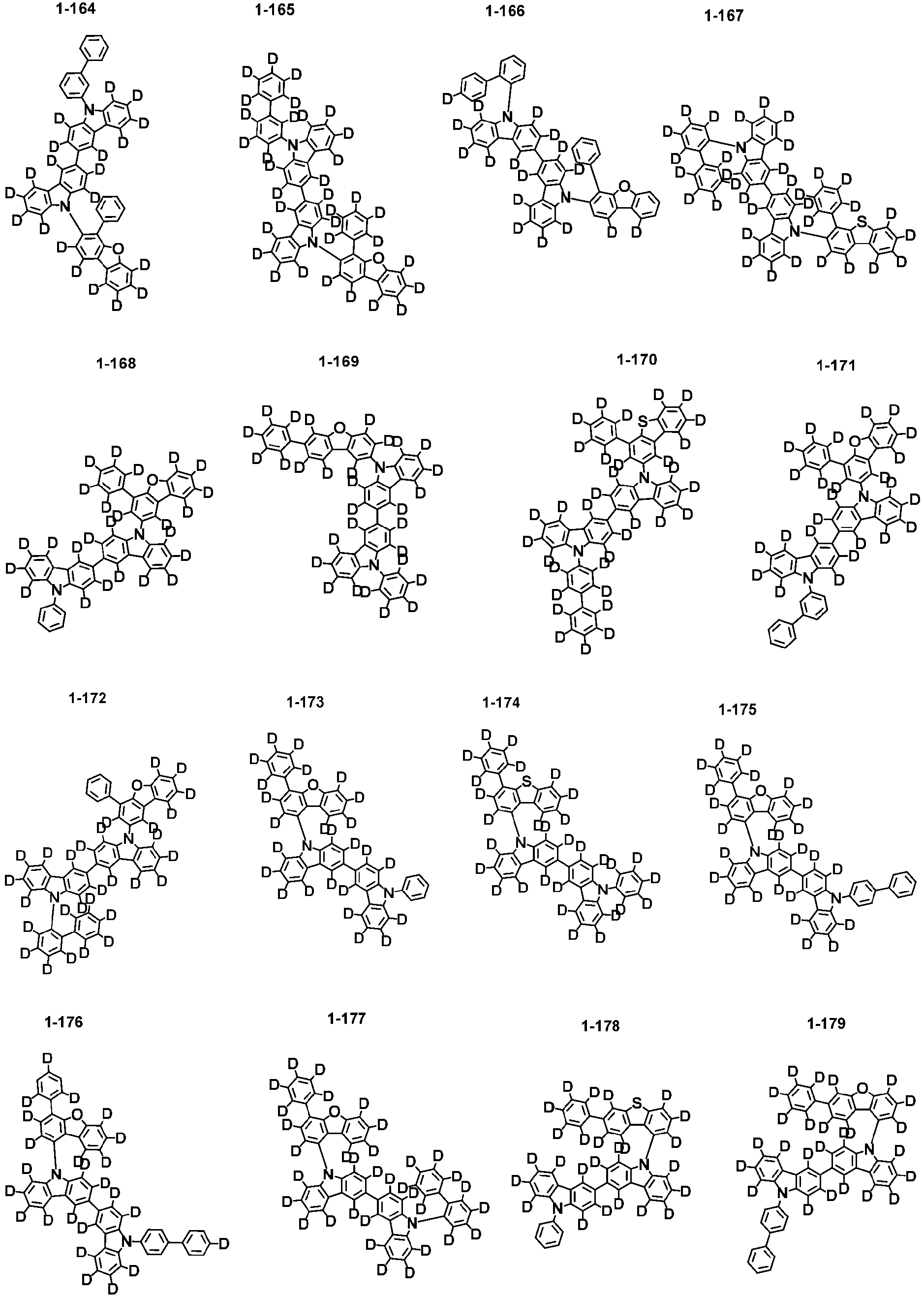

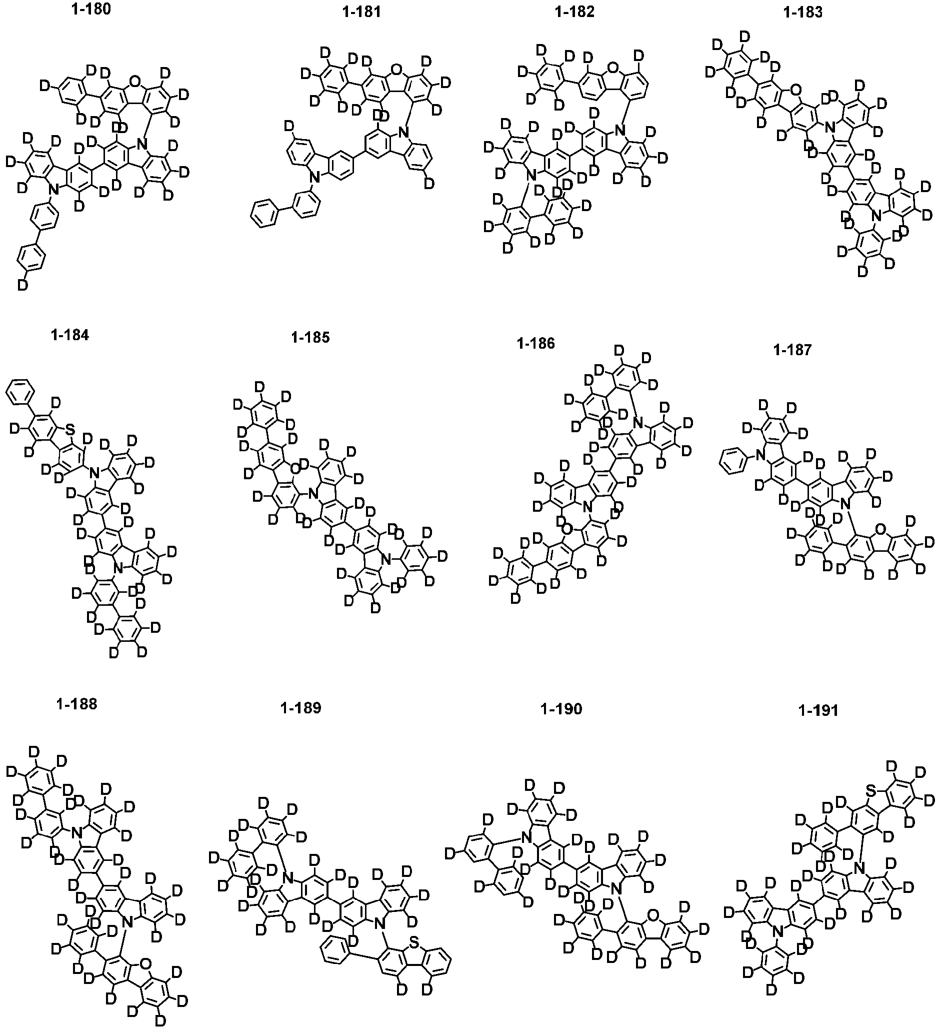

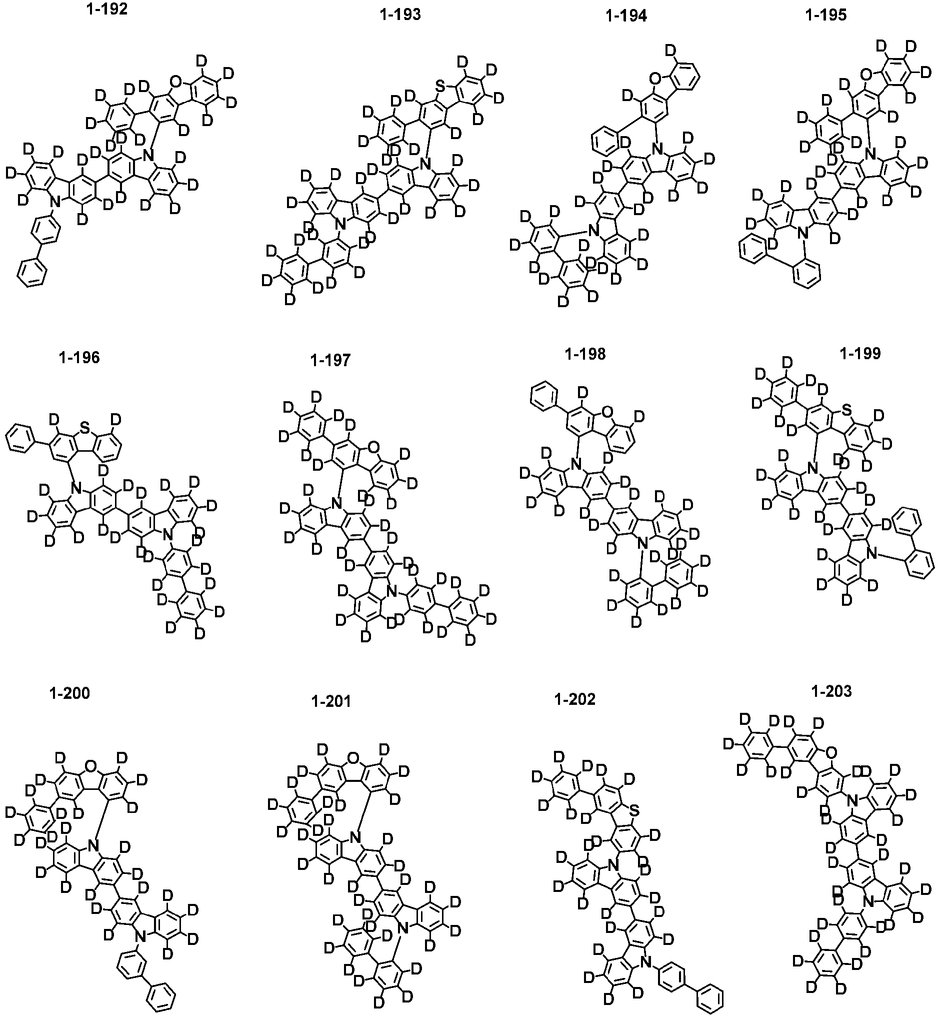

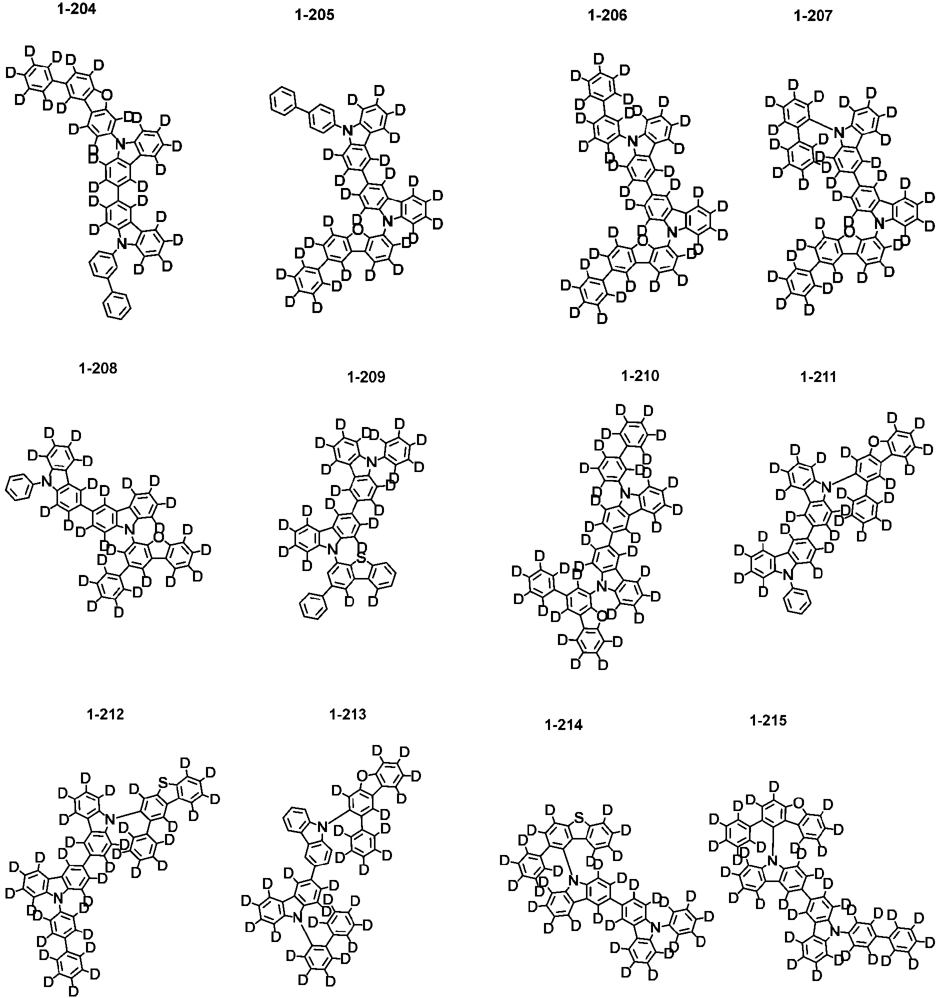

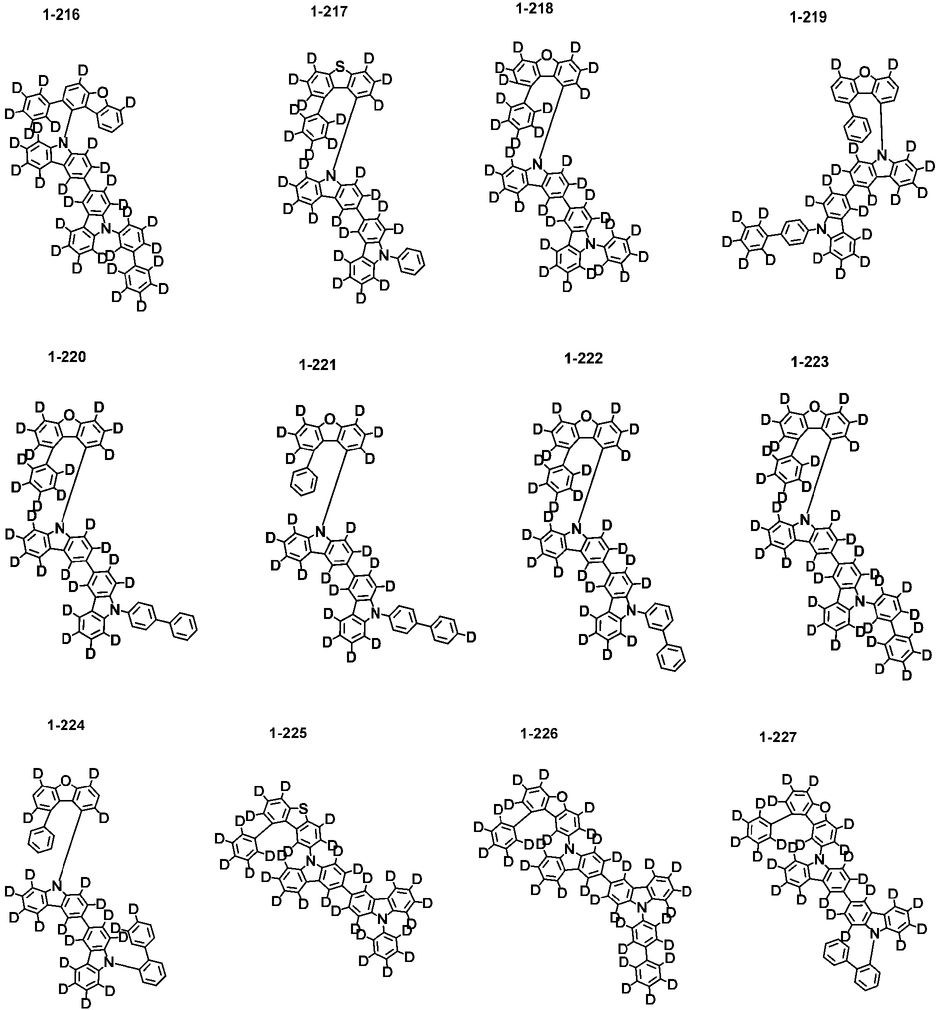

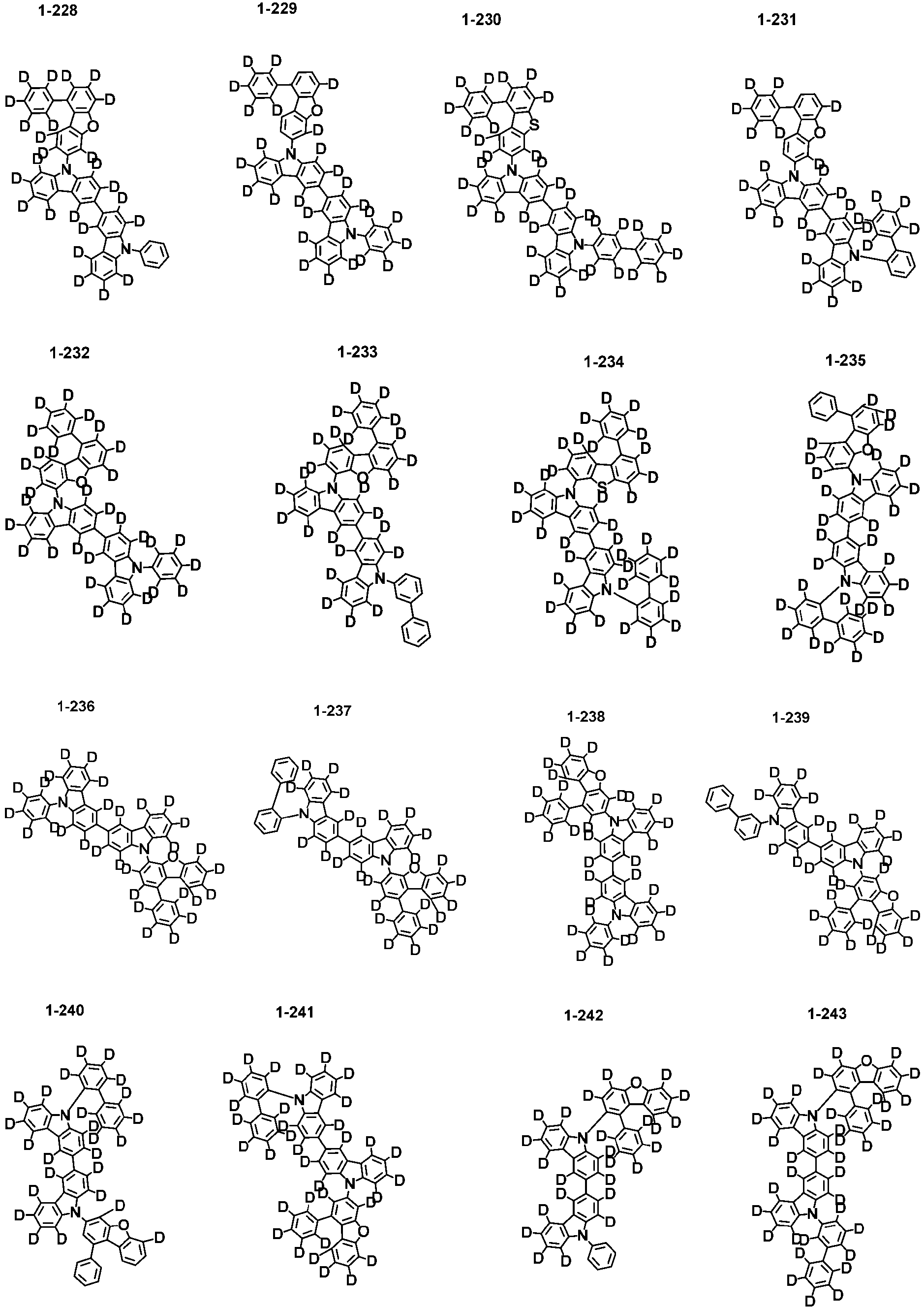

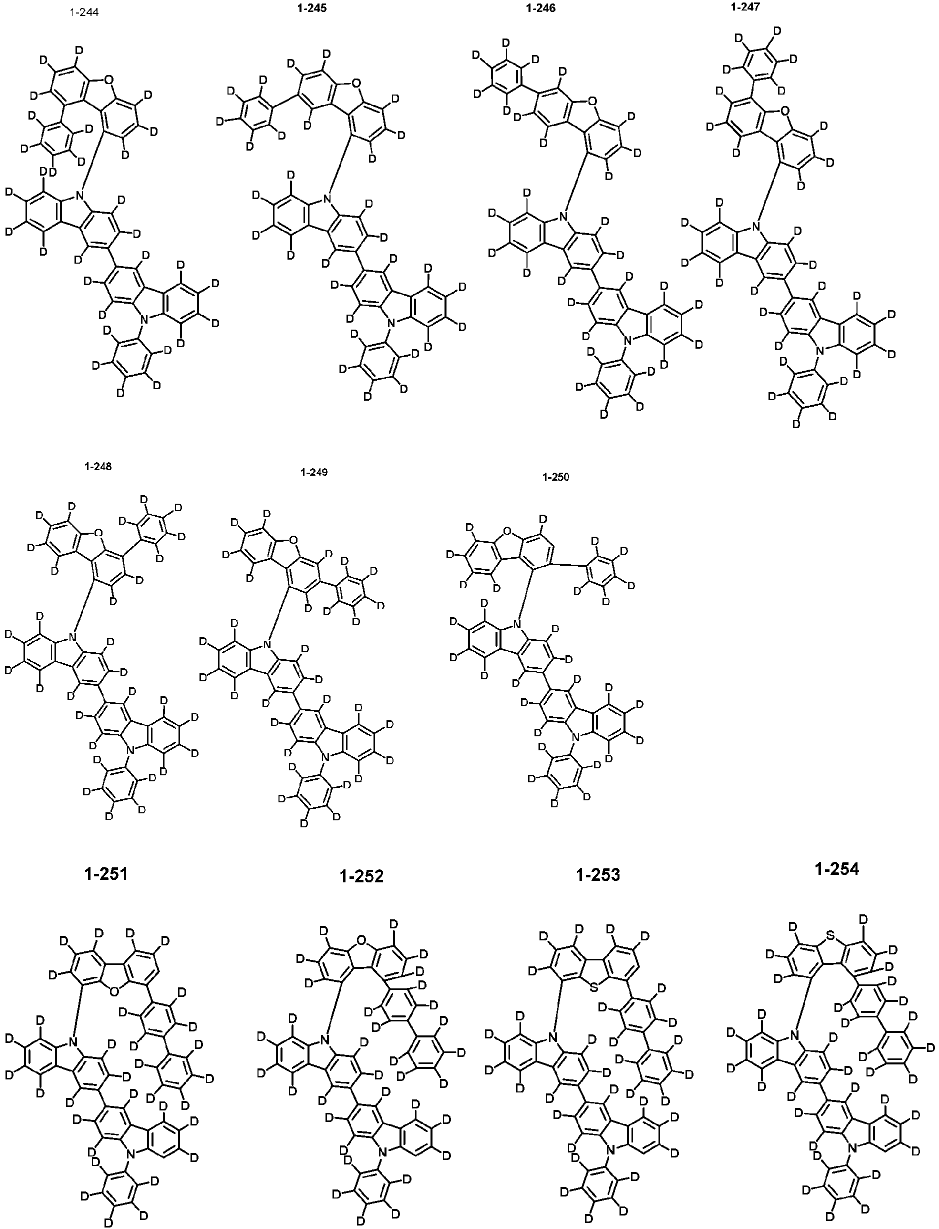

7. Zusammensetzung für organische optoelektronische Einrichtung nach Anspruch 1, wobei
die zweite Verbindung durch eine der Chemischen Formeln 2A bis 2F dargestellt ist: 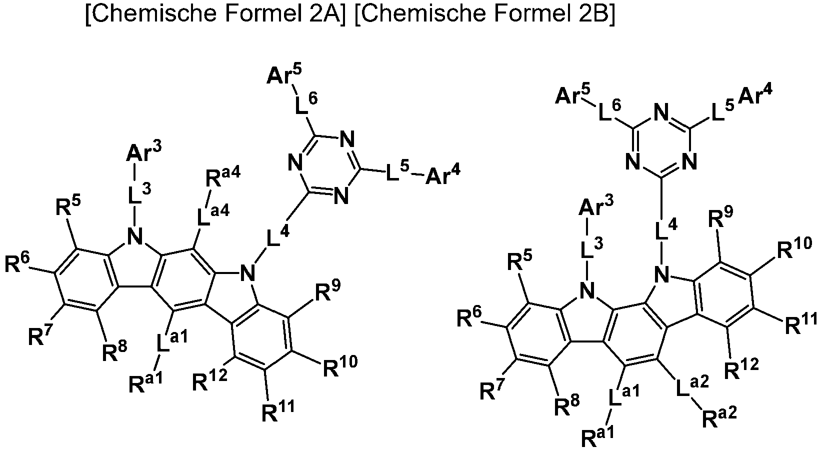 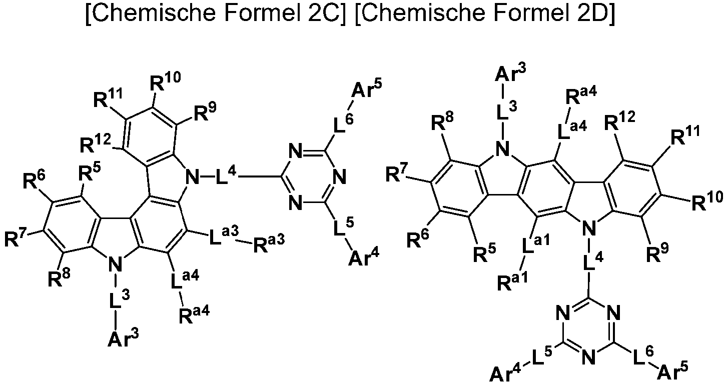 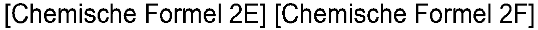 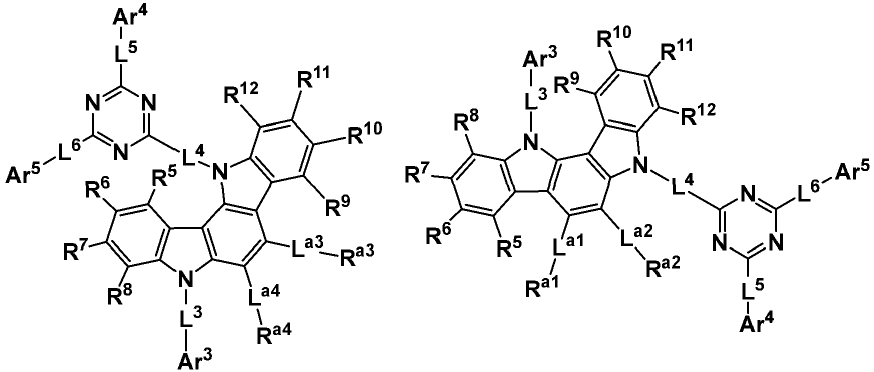
wobei in den Chemischen Formeln 2A bis 2F
Ar³ bis Ar⁵, L³ bis L⁶ und R⁵ bis R¹² wie in Anspruch 1 beschrieben definiert sind,
L^{a1} bis L^{a4} der Definition von L^{a} entsprechen und
R^{a1} bis R^{a4} der Definition von R^{a} entsprechen.

8. Zusammensetzung für organische optoelektronische Einrichtung nach Anspruch 1, wobei
R⁵ bis R¹² in den Chemischen Formeln 2 und 3 jeweils unabhängig Wasserstoff, Deuterium oder eine Cyanogruppe sind,
L³ bis L⁶ eine Einfachbindung, eine substituierte oder unsubstituierte Phenylengruppe, eine substituierte oder unsubstituierte Biphenylengruppe oder eine substituierte oder unsubstituierte Pyridinylengruppe sind und
Ar³ bis Ar⁵ jeweils unabhängig eine substituierte oder unsubstituierte Phenylgruppe, eine substituierte oder unsubstituierte Biphenylgruppe, eine substituierte oder unsubstituierte Triphenylengruppe, eine substituierte oder unsubstituierte Fluorenylgruppe, eine substituierte oder unsubstituierte Dibenzofuranylgruppe oder eine substituierte oder unsubstituierte Dibenzothiophenylgruppe sind.

9. Zusammensetzung für organische optoelektronische Einrichtung nach Anspruch 1, wobei
die erste Verbindung durch die Chemische Formel 1-8a dargestellt ist und
die zweite Verbindung durch die Chemische Formel 2B dargestellt ist: 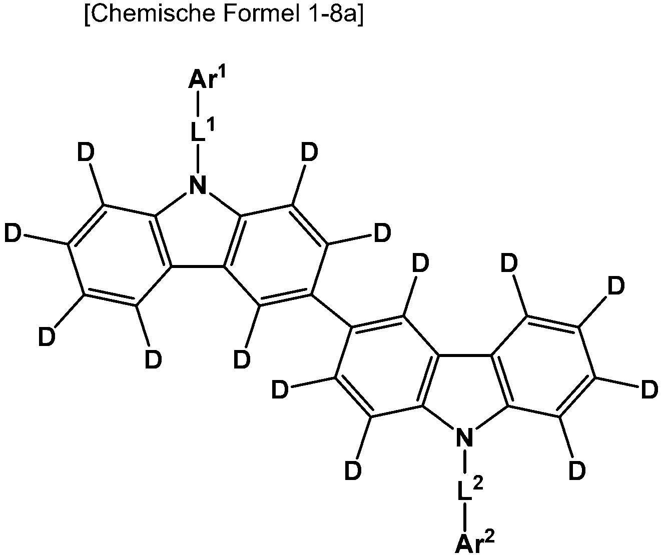
wobei in der Chemischen Formel 1-8a
L' und L² eine Einfachbindung oder eine substituierte oder unsubstituierte Phenylengruppe sind und
Ar¹ und Ar² jeweils eine mit Deuterium substituierte Phenylgruppe, eine mit Deuterium substituierte Biphenylgruppe, eine mit Deuterium substituierte Terphenylgruppe oder eine mit Deuterium substituierte Triphenylengruppe sind, 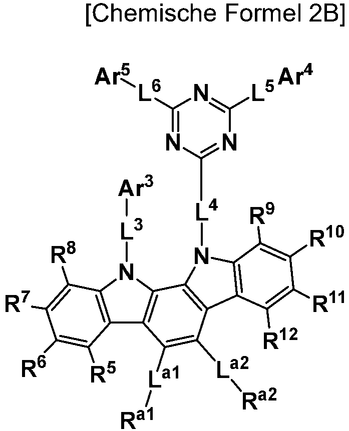
wobei in der Chemischen Formel 2B
L^{a1} und L^{a2} eine Einfachbindung sind,
L³ bis L⁶ jeweils unabhängig eine Einfachbindung oder eine substituierte oder unsubstituierte C6- bis C12-Arylengruppe sind,
R⁵ bis R¹², R^{a1} und R^{a2} jeweils unabhängig Wasserstoff, Deuterium, eine Cyanogruppe oder eine substituierte oder unsubstituierte Phenylgruppe sind und
Ar³ bis Ar⁵ jeweils unabhängig eine substituierte oder unsubstituierte Phenylgruppe, eine substituierte oder unsubstituierte Biphenylgruppe oder eine substituierte oder unsubstituierte Triphenylengruppe sind.

10. Organische optoelektronische Einrichtung, aufweisend
eine Anode und eine Kathode, die einander gegenüberliegen, und
mindestens eine organische Schicht zwischen der Anode und der Kathode,
wobei die organische Schicht die Zusammensetzung für organische optoelektronische Einrichtung nach einem der Ansprüche 1 bis 9 aufweist.

11. Organische optoelektronische Einrichtung nach Anspruch 10, wobei
die organische Schicht eine lichtemittierende Schicht aufweist und
die lichtemittierende Schicht die Zusammensetzung für organische optoelektronische Einrichtung aufweist.

12. Anzeigeeinrichtung, aufweisend die organische optoelektronische Einrichtung nach Anspruch 10.

## Revendications

1. Composition pour un dispositif optoélectronique organique, comprenant
un premier composé représenté par la Formule Chimique 1, et
un deuxième composé représenté par une combinaison de Formule Chimique 2 et de Formule Chimique 3: 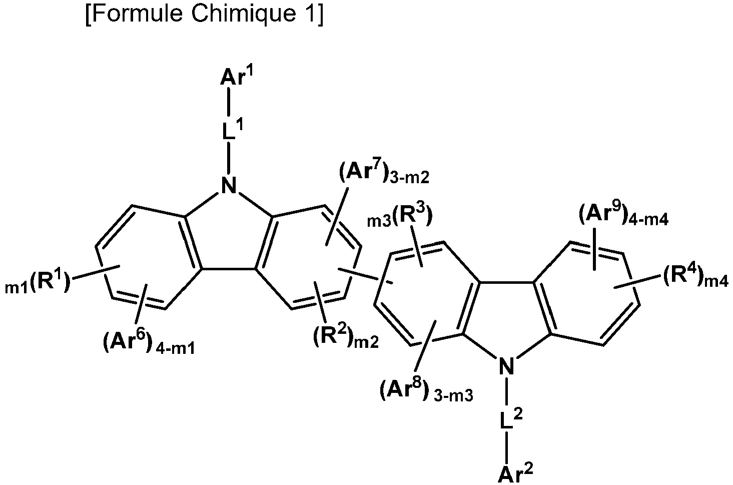
dans laquelle, dans la Formule Chimique 1,
L¹ et L² représentent chacun indépendamment une liaison simple ou un groupe arylène C6 à C30 substitué ou non substitué,
Ar¹ et Ar² représentent chacun indépendamment un groupe aryle C6 à C30 substitué ou non substitué ou un groupe hétérocyclique C2 à C30 substitué ou non substitué,
R¹ à R⁴ représentent chacun indépendamment de l'hydrogène, du deutérium, ou un groupe aryle C6 à C30 substitué ou non substitué,
Ar⁶ à Ar⁹ représentent chacun indépendamment de l'hydrogène ou un groupe aryle C6 à C30 substitué ou non substitué, et
m1 et m4 représentent chacun indépendamment l'un des entiers de 1 à 4,
m2 et m3 représentent chacun indépendamment l'un des entiers de 1 à 3, et
la Formule Chimique 1 remplit simultanément les conditions suivantes (i) et (ii),
(i) au moins l'un parmi Ar¹ et Ar² est un groupe aryle C6 à C30 substitué par au moins du deutérium ou un groupe hétérocyclique C2 à C30 substitué par au moins du deutérium, et
(ii) au moins l'un parmi R¹ à R⁴ est du deutérium; 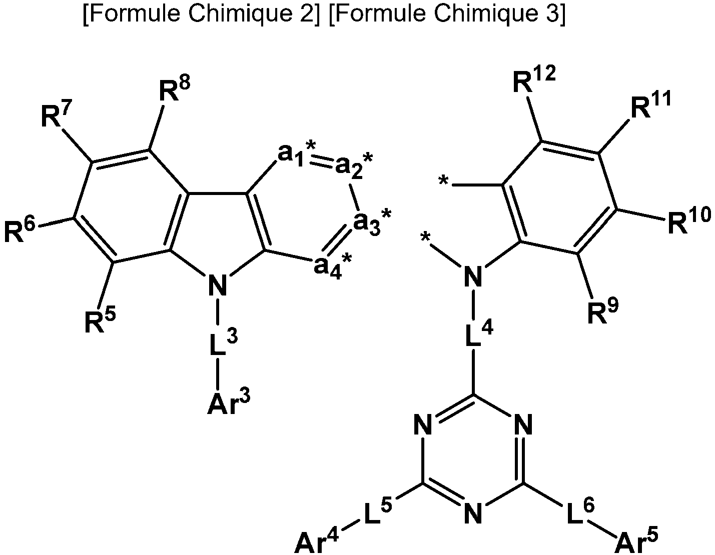
dans lesquelles, dans la Formule Chimique 2 et la Formule Chimique 3,
Ar³ à Ar⁵ représentent chacun indépendamment un groupe aryle C6 à C20 substitué ou non substitué ou un groupe hétérocyclique C2 à C30 substitué ou non substitué,
a₁* à a₄* représentent chacun indépendamment un carbone de liaison (C) ou C-L^{a}-R^{a},
les deux éléments adjacents de a₁* à a₄* dans la Formule Chimique 2 sont liés à * dans la Formule Chimique 3,
L^{a}, L³, à L⁶ représentent chacun indépendamment une liaison simple, un groupe arylène C6 à C20 substitué ou non substitué ou un groupe hétéroarylène C2 à C20 substitué ou non substitué, et
R^{a} et R⁵ à R¹² représentent chacun indépendamment de l'hydrogène, du deutérium, un groupe cyano, un halogène, un groupe amino substitué ou non substitué, un groupe alkyle C1 à C30 substitué ou non substitué, un groupe aryle C6 à C30 substitué ou non substitué, ou un groupe hétérocyclique C2 à C30 substitué ou non substitué,
dans le cas où le premier composé est choisi parmi l'un des composés suivants D1-1 à D1-8 et le deuxième composé est choisi parmi l'un des composés suivants D2-1 à D2-7, les combinaisons suivantes sont exclues
D1-1 avec D2-1, D1-2 avec D2-1, D1-3 avec D2-1, D1-4 avec D2-1, D1-5 avec D2-1,
D1-1 avec D2-2, D1-2 avec D2-2, D1-3 avec D2-2, D1-4 avec D2-2, D1-5 avec D2-2,
D1-1 avec D2-3, D1-2 avec D2-3, D1-3 avec D2-3, D1-4 avec D2-3, D1-5 avec D2-3,
D1-6 avec D2-4, D1-7 avec D2-5, D1-8 avec D2-6 et D1-6 avec D2-7

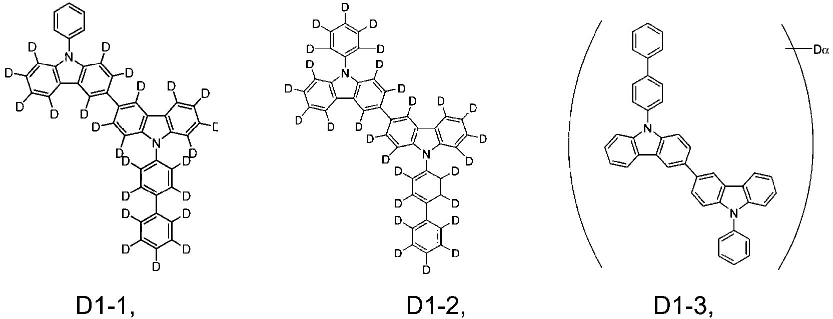

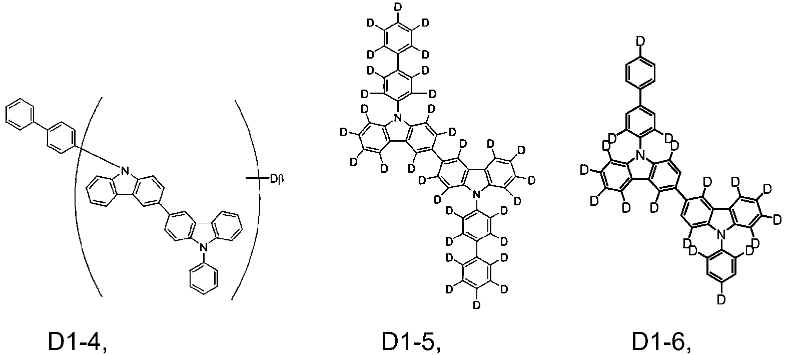

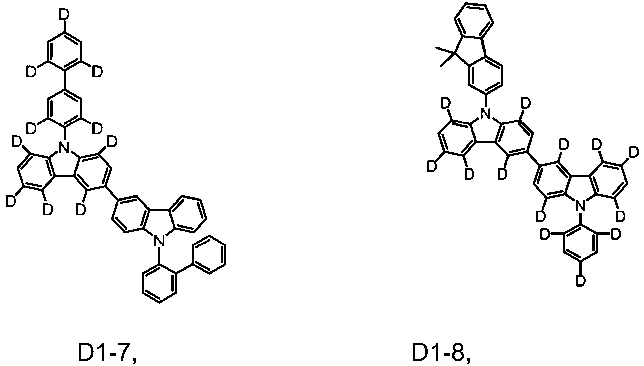

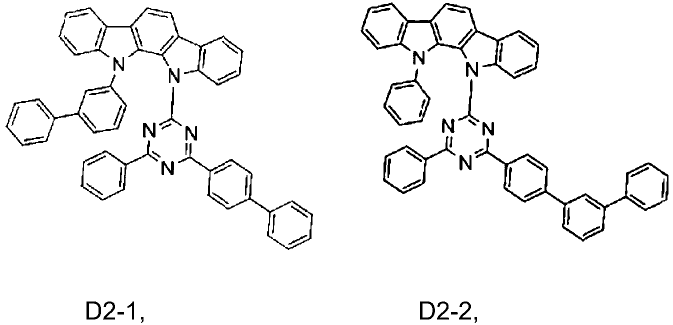

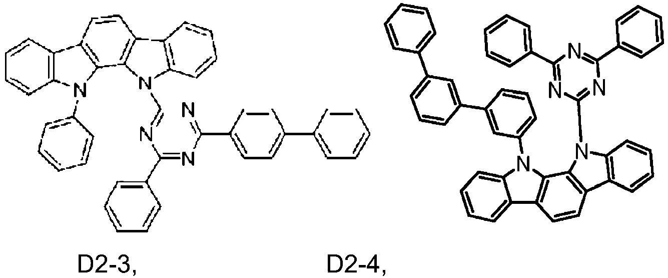

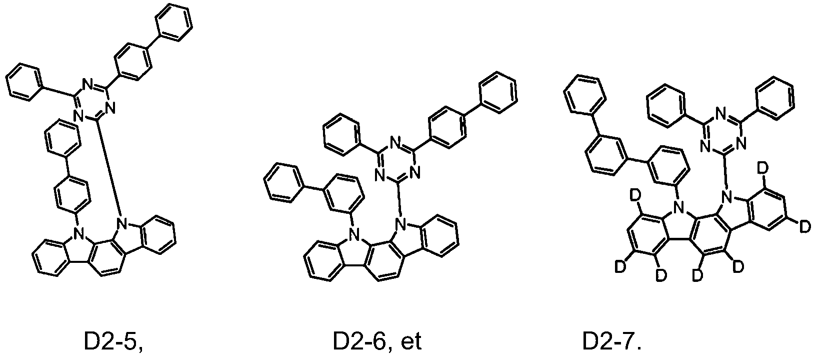

2. Composition pour le dispositif optoélectronique organique selon la revendication 1, dans laquelle
le premier composé est représenté par l'une parmi la Formule Chimique 1-1 à la Formule Chimique 1-10: 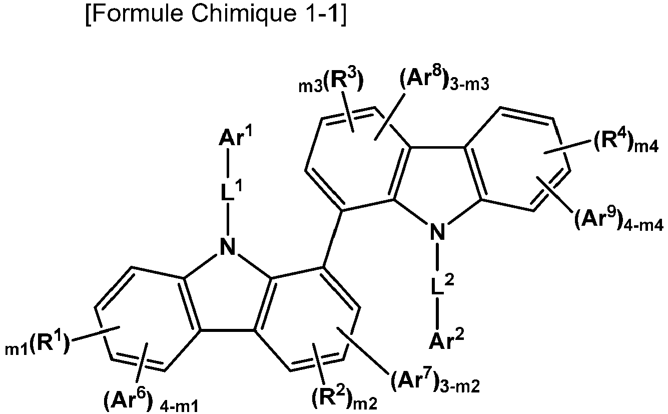 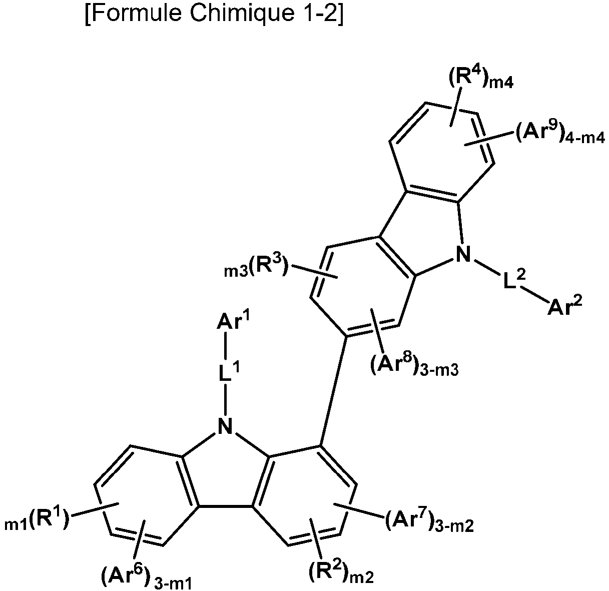 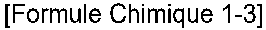 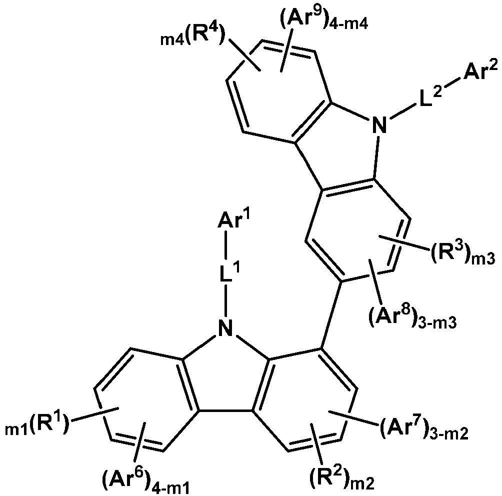 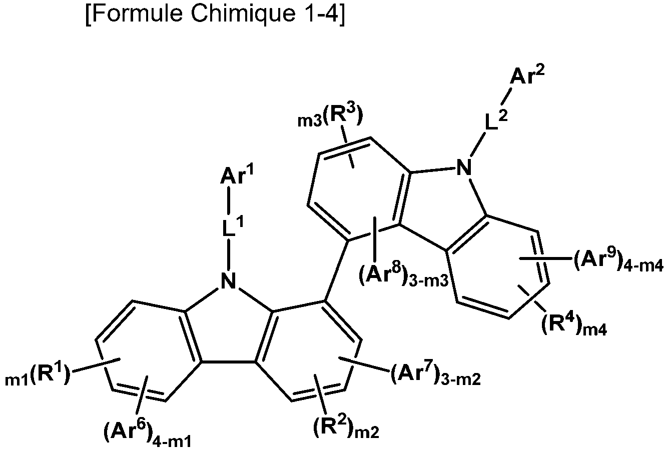 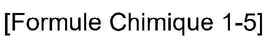 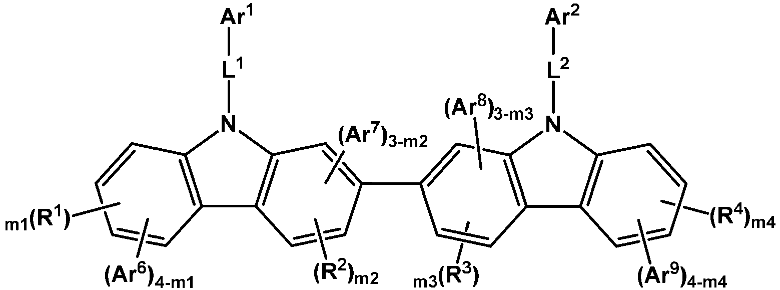 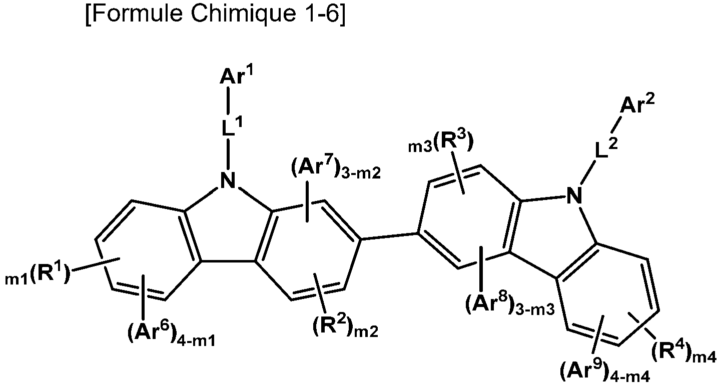 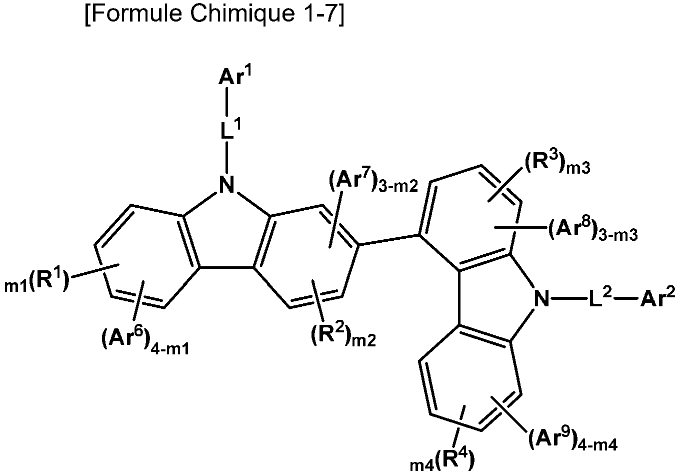 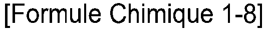 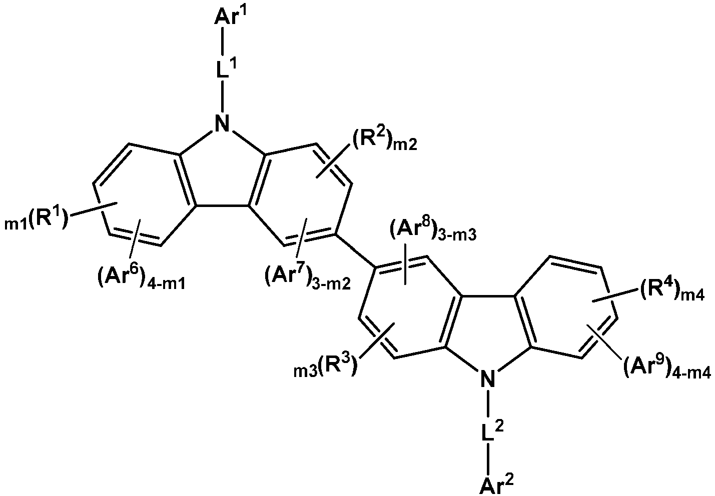 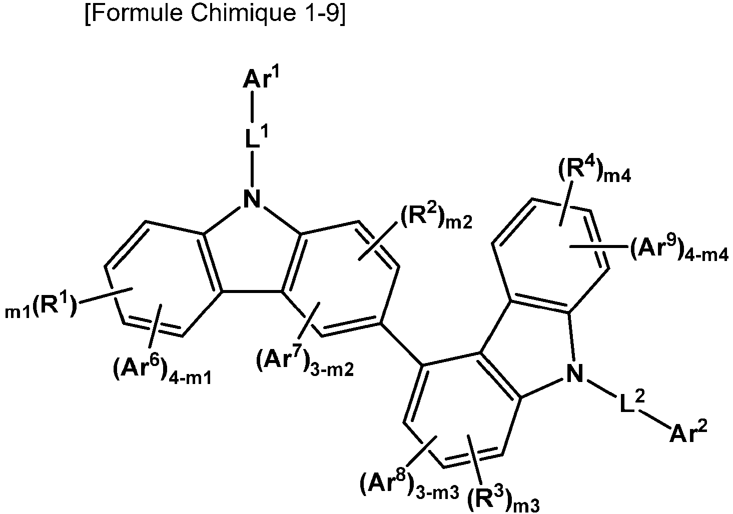 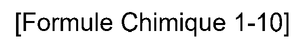 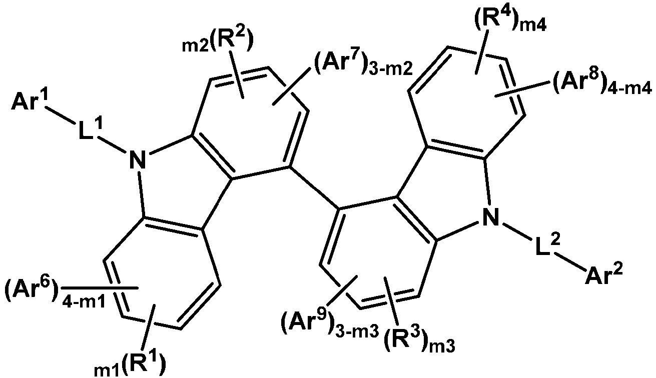
dans laquelle, dans la Formule Chimique 1-1 à la Formule Chimique 1-10,
L¹, L², Ar¹, Ar², Ar⁶ à Ar⁹, R¹ à R⁴, et m¹ à m⁴ sont les mêmes que ceux décrits dans la revendication 1.

3. Composition pour le dispositif optoélectronique organique selon la revendication 1, dans laquelle
au moins l'un parmi Ar¹ et Ar² de la Formule Chimique 1 est un groupe phényle substitué par au moins du deutérium, un groupe biphényle substitué par au moins du deutérium, un groupe terphényle substitué par au moins du deutérium, un groupe naphtyle substitué par au moins du deutérium, un groupe anthracényle substitué par au moins du deutérium, un groupe phénanthrényle substitué par au moins du deutérium, un groupe triphénylène substitué par au moins du deutérium, un groupe fluorényle substitué par au moins du deutérium, groupe dibenzofuranyle substitué par au moins du deutérium, ou groupe dibenzothiophényle substitué par au moins du deutérium.

4. Composition pour le dispositif optoélectronique organique selon la revendication 1, dans laquelle
L¹-Ar¹ et L²-Ar² de la Formule Chimique 1 sont chacun indépendamment choisis parmi les substituants énumérés dans le Groupe I -1 et le Groupe I -2, et
au moins l'un parmi L¹-Ar¹ et L²-Ar² est choisi parmi les substituants énumérés dans le Groupe I -2: 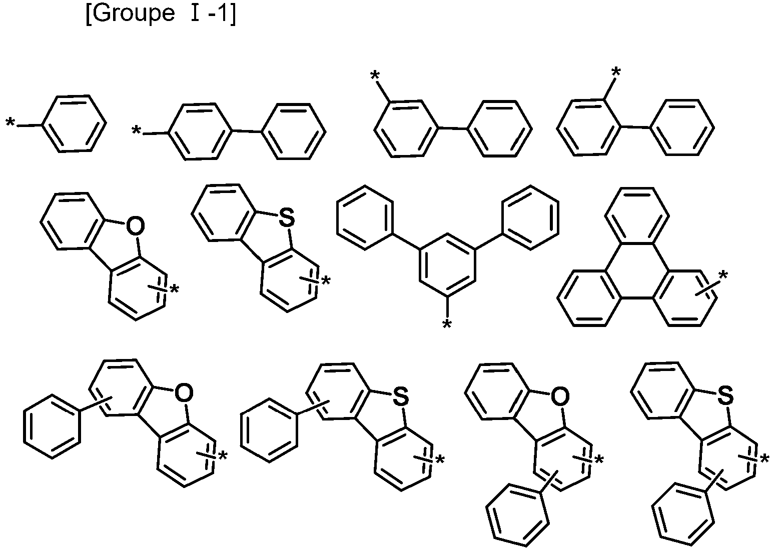 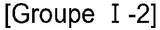 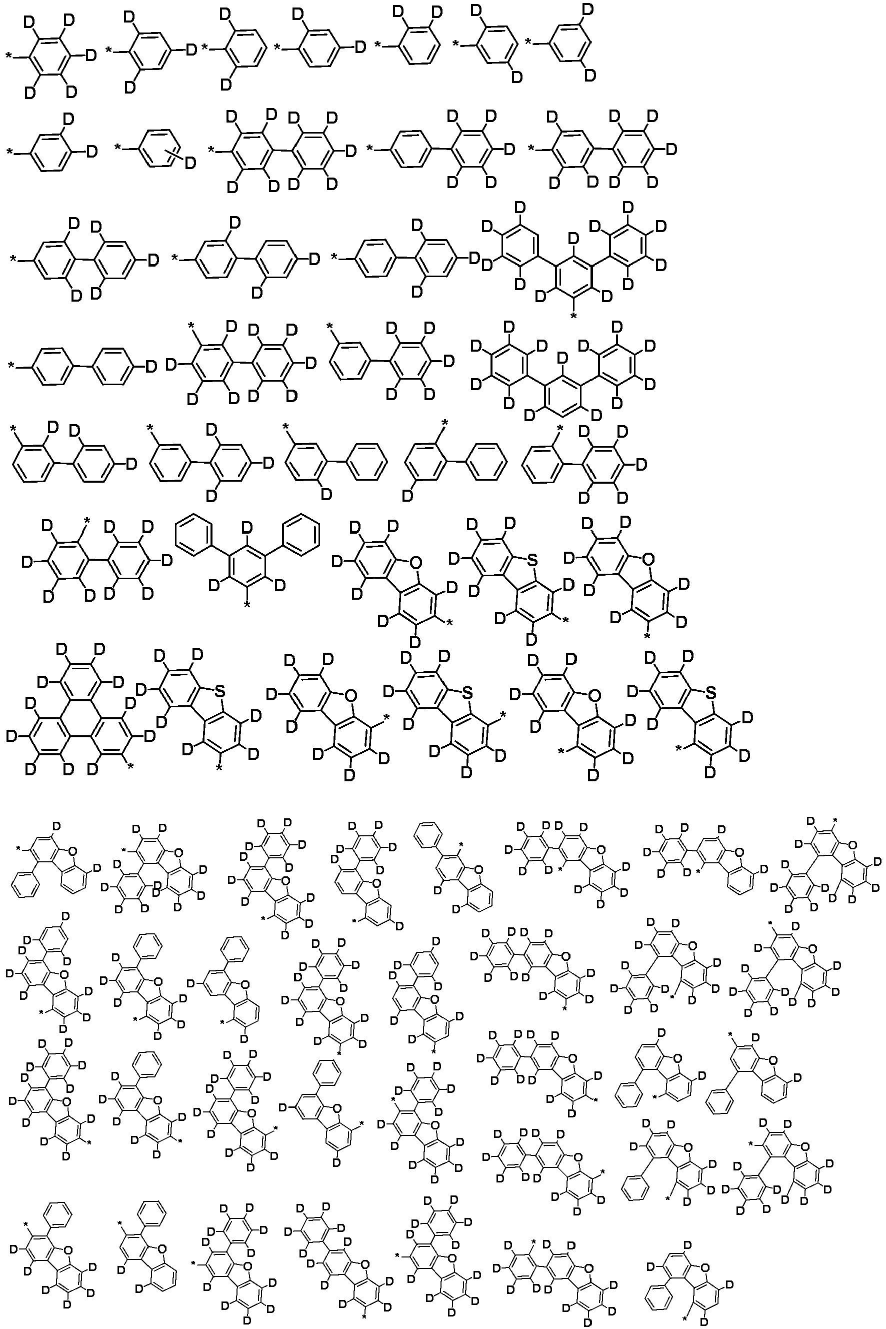 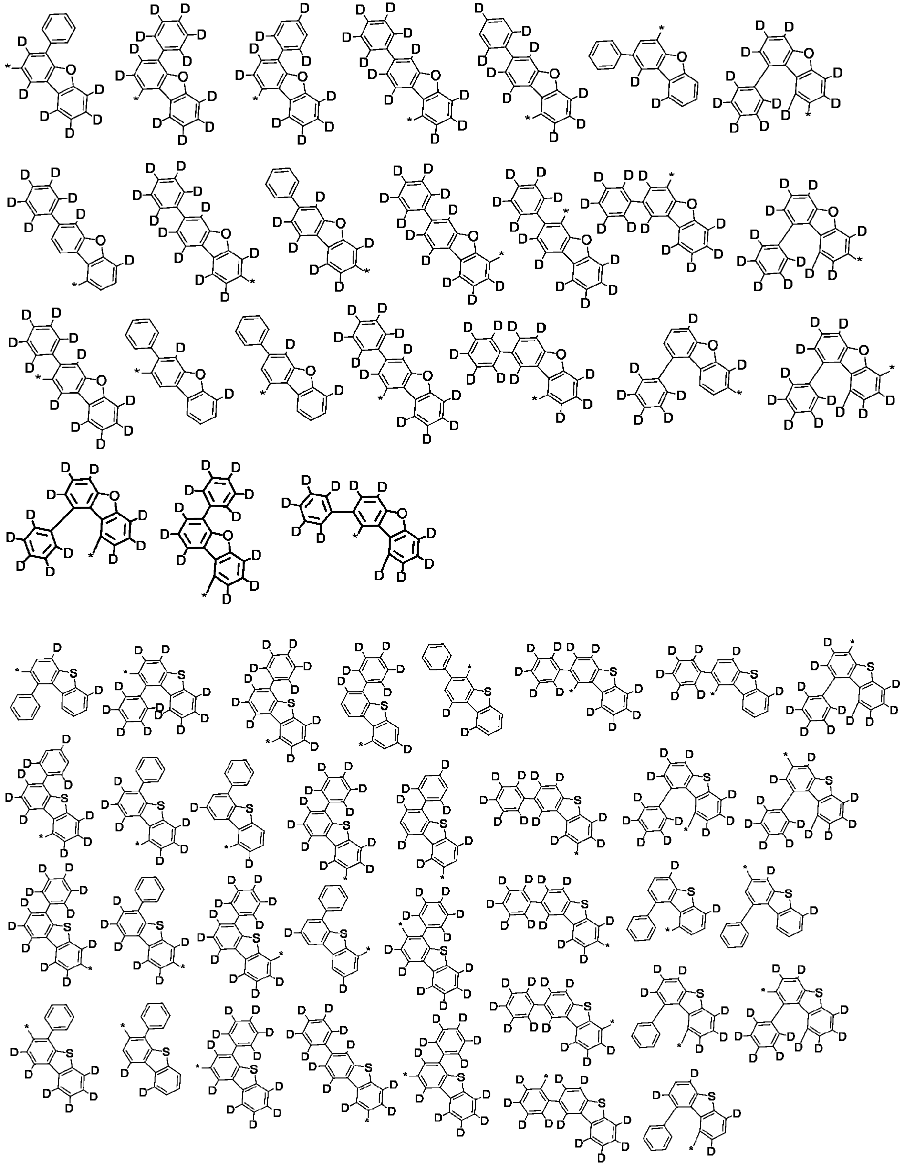 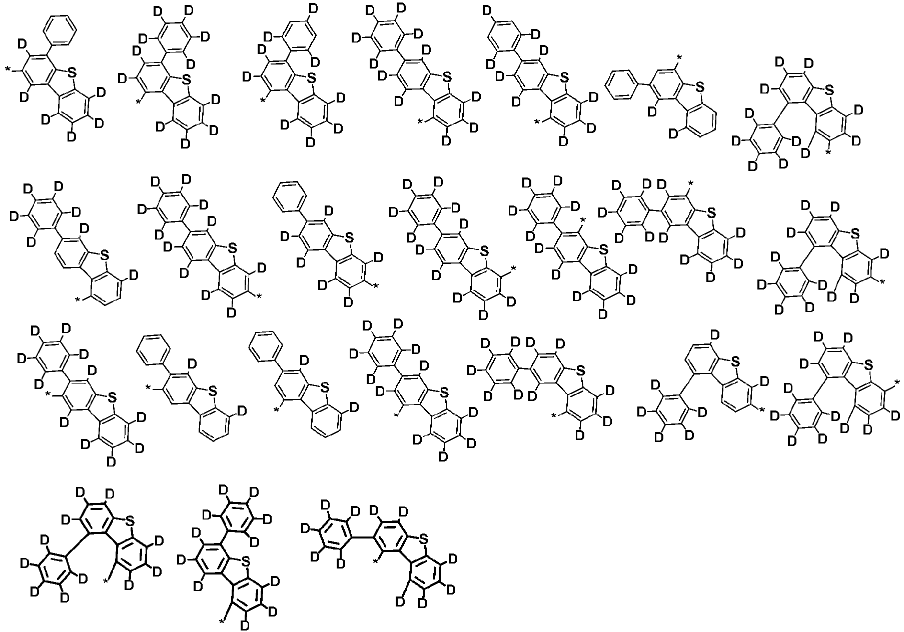
dans laquelle, dans le Groupe I -1 et le Groupe I -2, * est un point de liaison.

5. Composition pour le dispositif optoélectronique organique selon la revendication 1, dans laquelle
le premier composé est représenté par la Formule Chimique 1-8a: 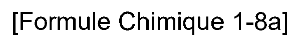 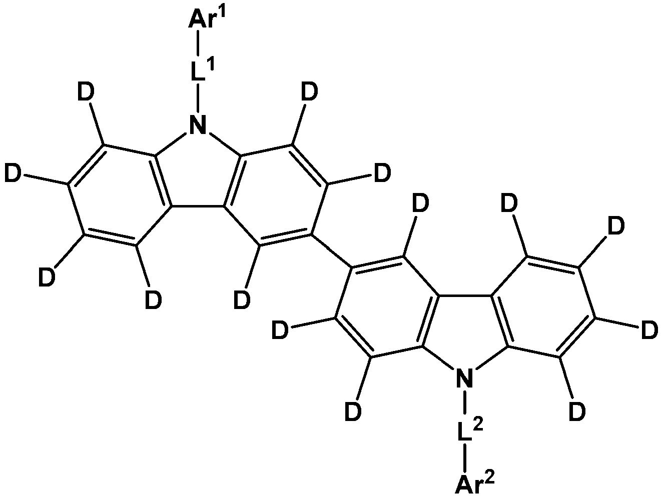
dans laquelle, dans la Formule Chimique 1-8a,
L¹, L², Ar¹ et Ar² sont les mêmes que ceux décrits dans la revendication 1.

6. Composition pour le dispositif optoélectronique organique selon la revendication 1, dans laquelle
le premier composé est choisi parmi les composés énumérés dans le Groupe 1:

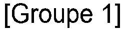

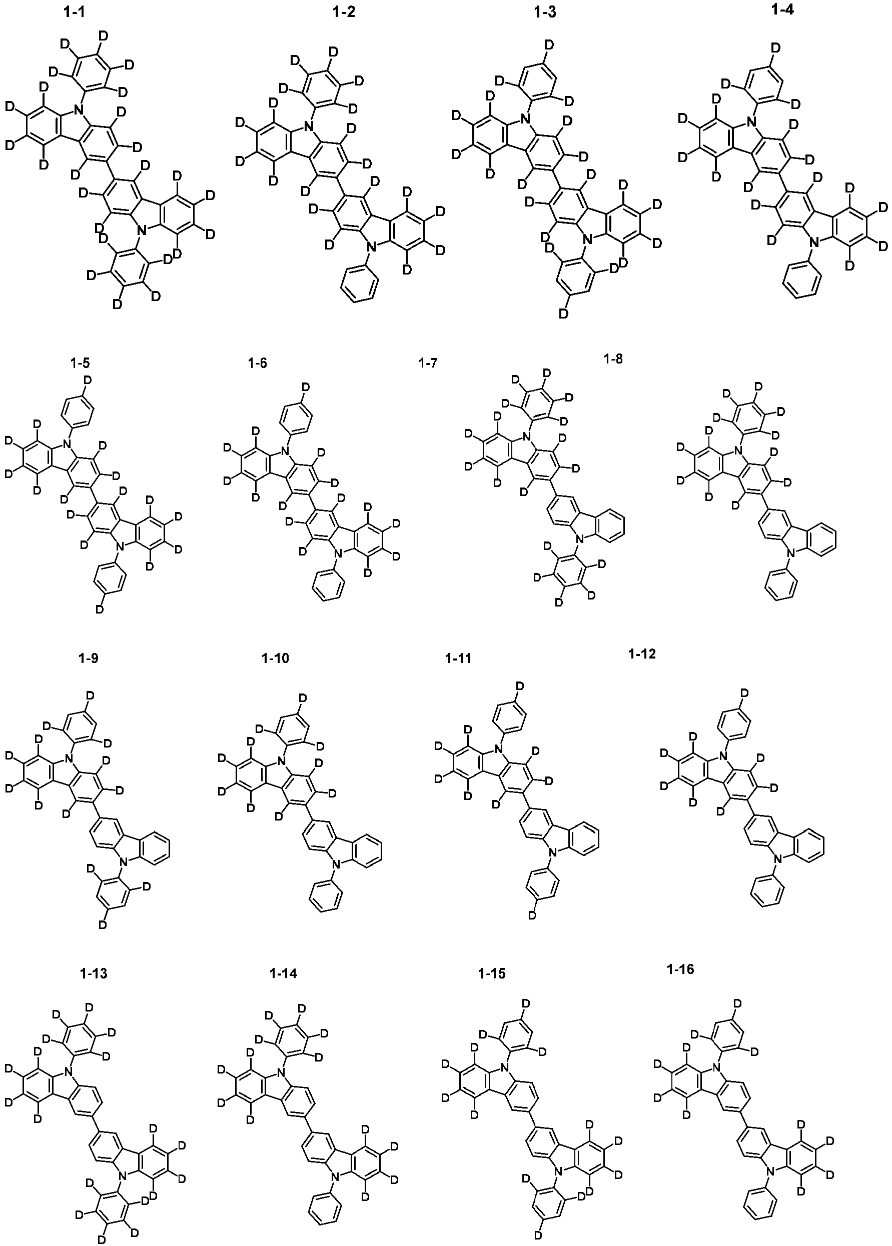

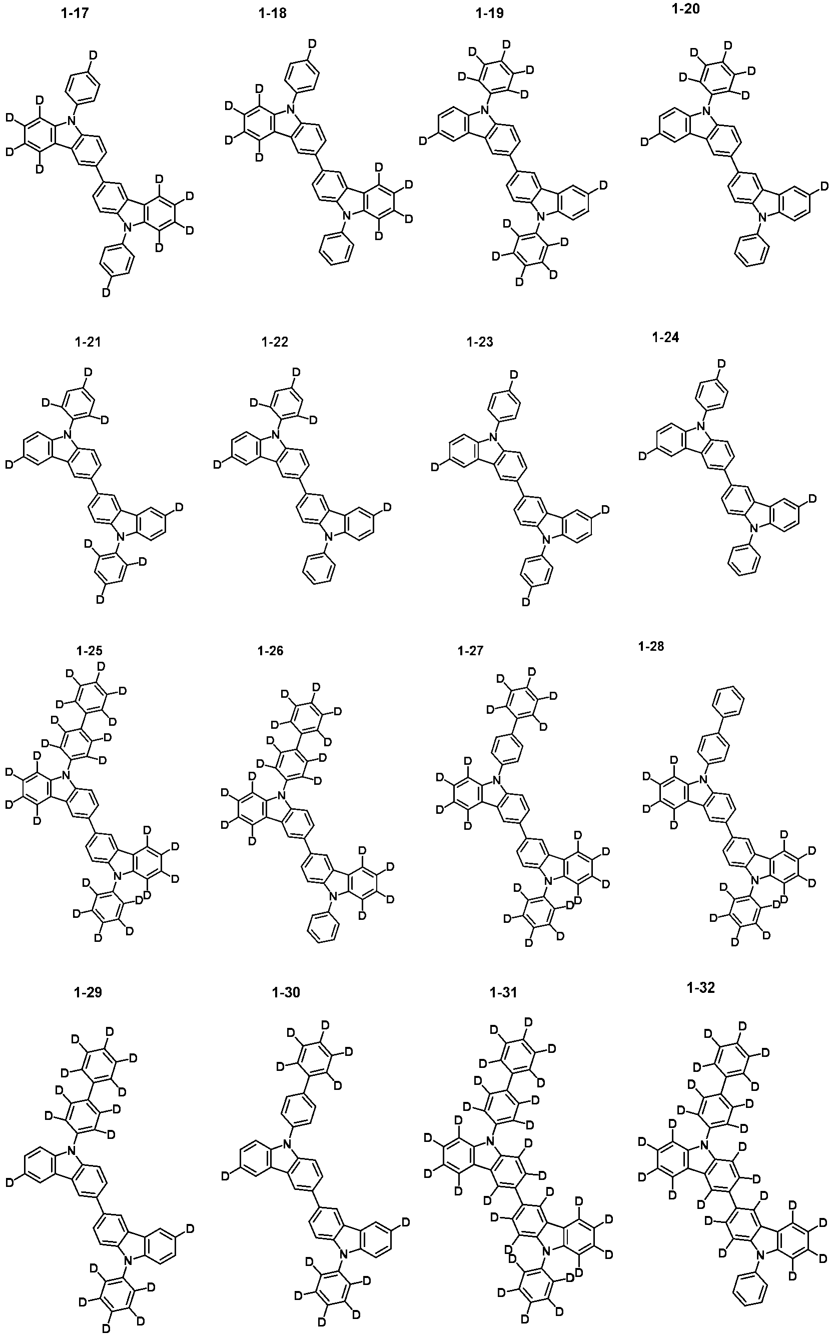

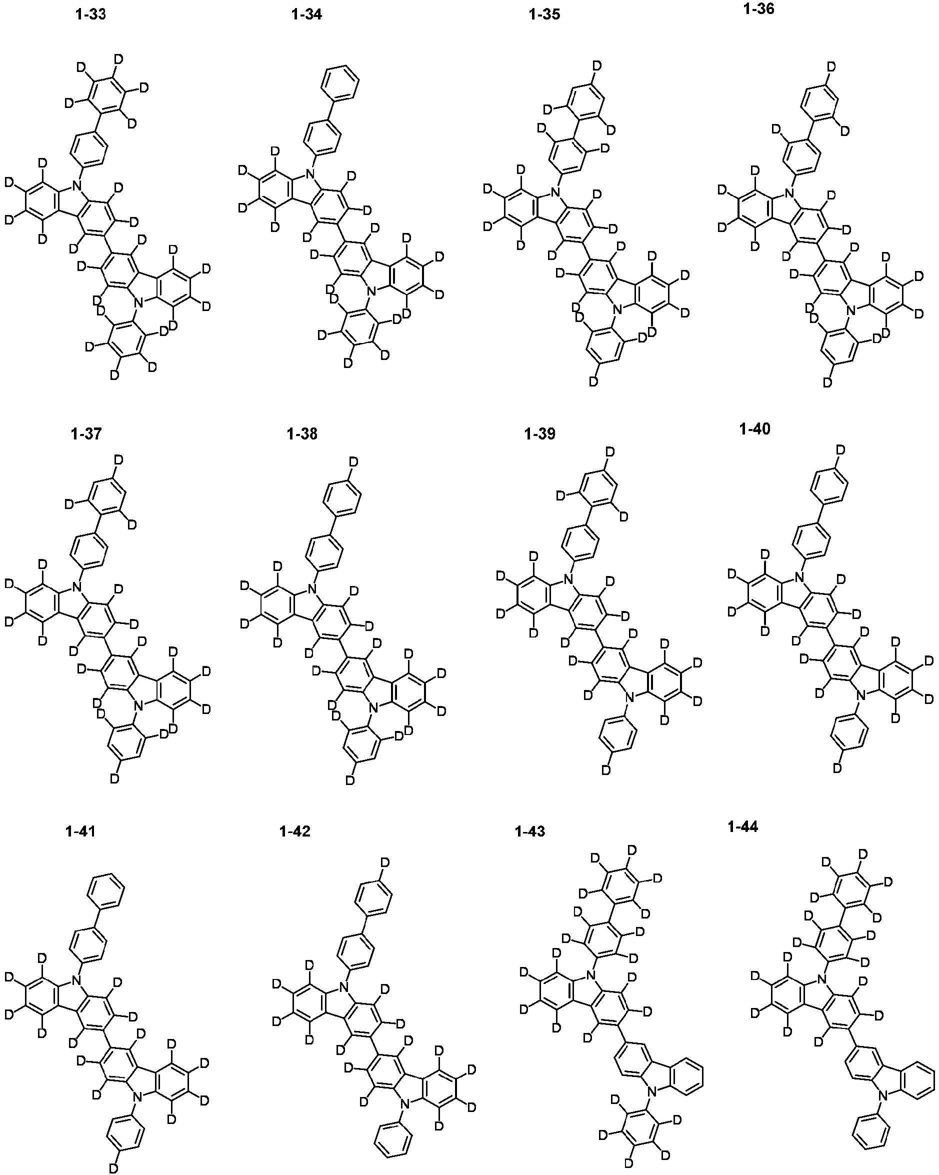

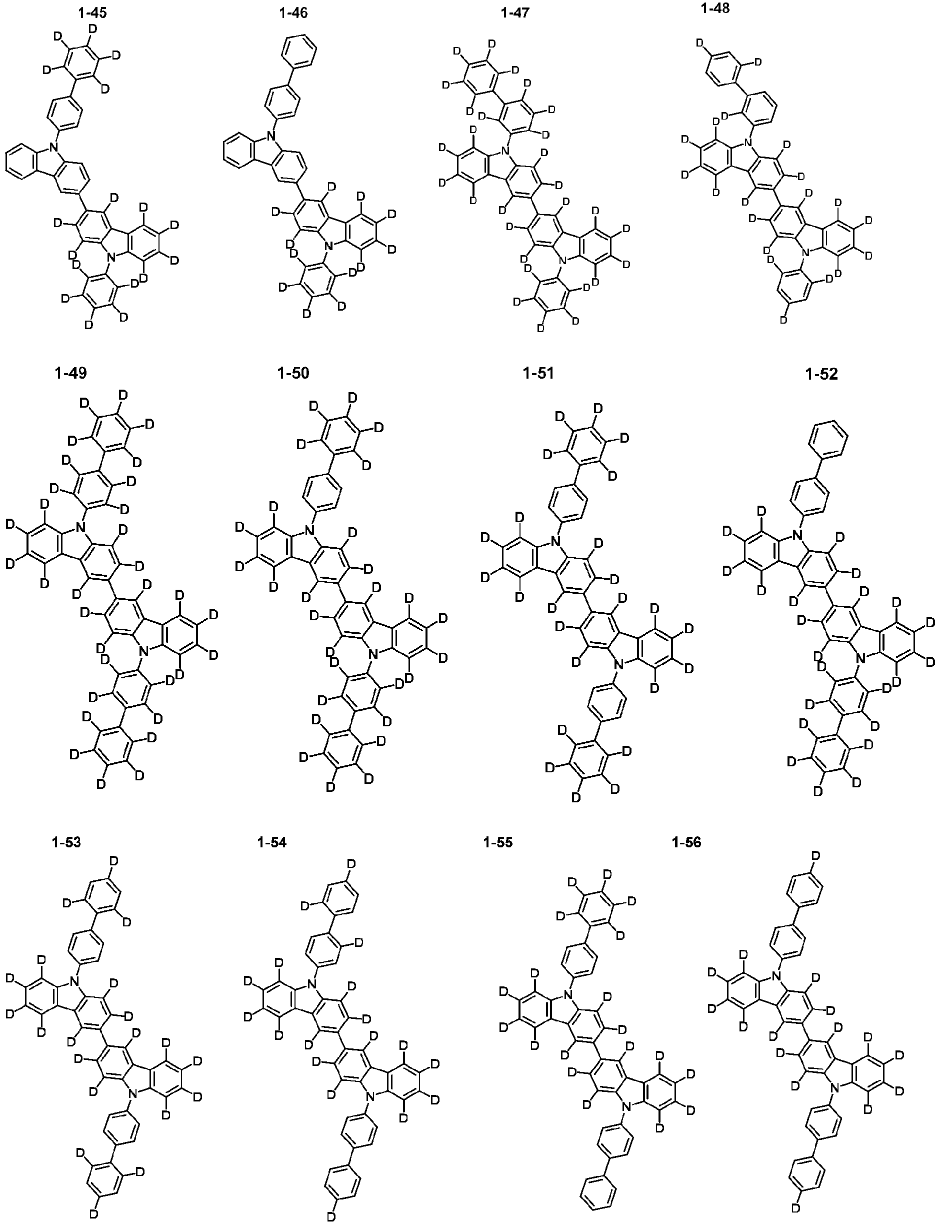

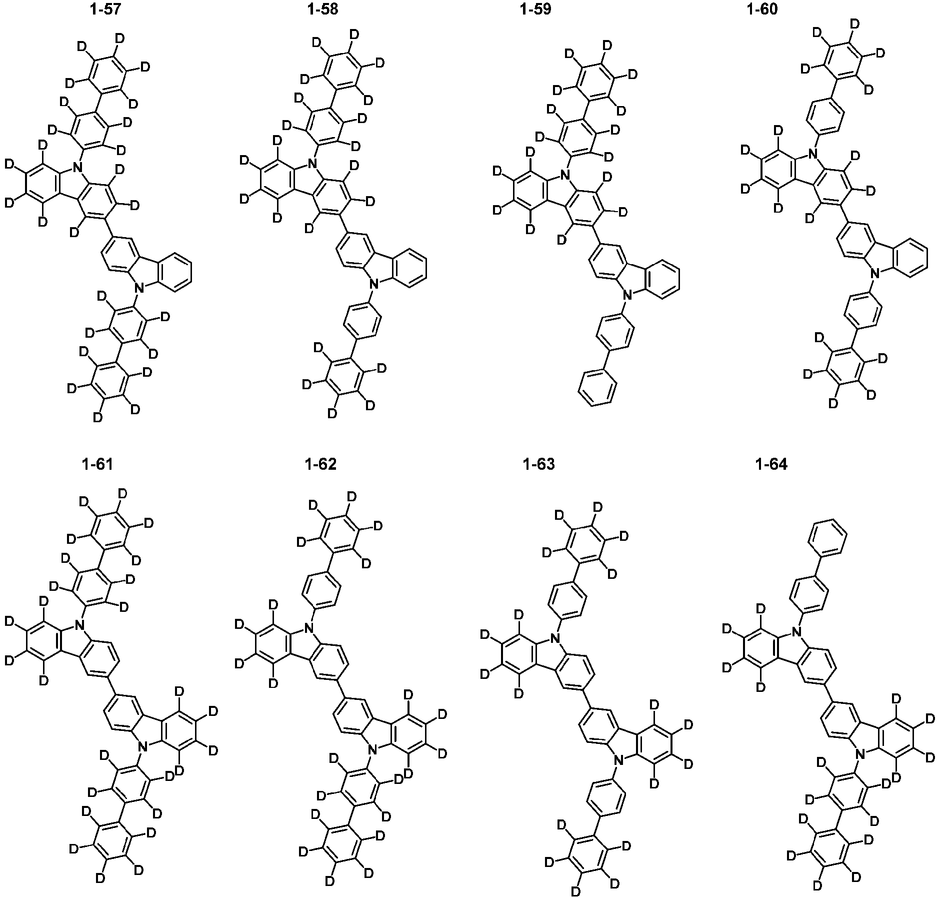

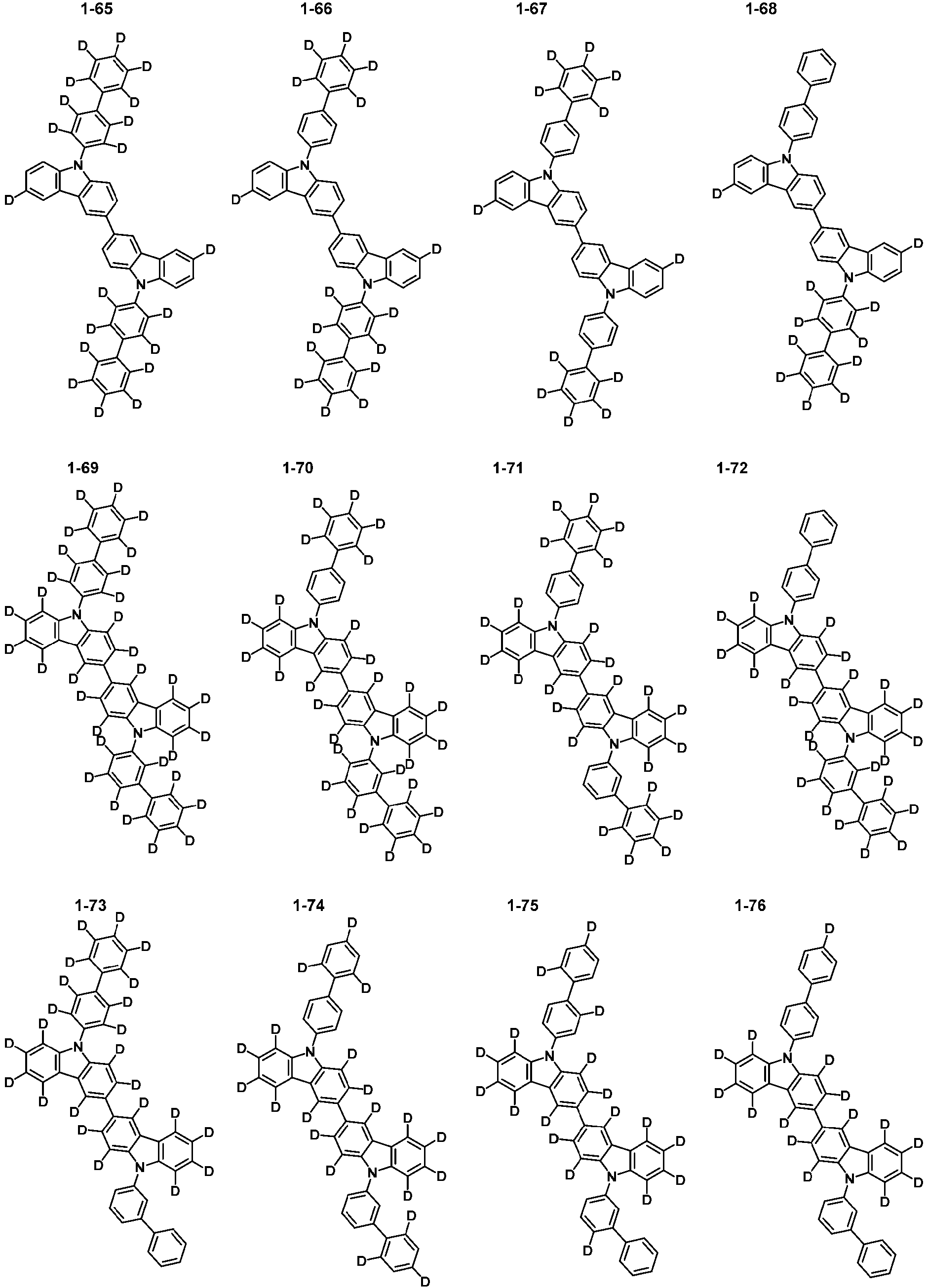

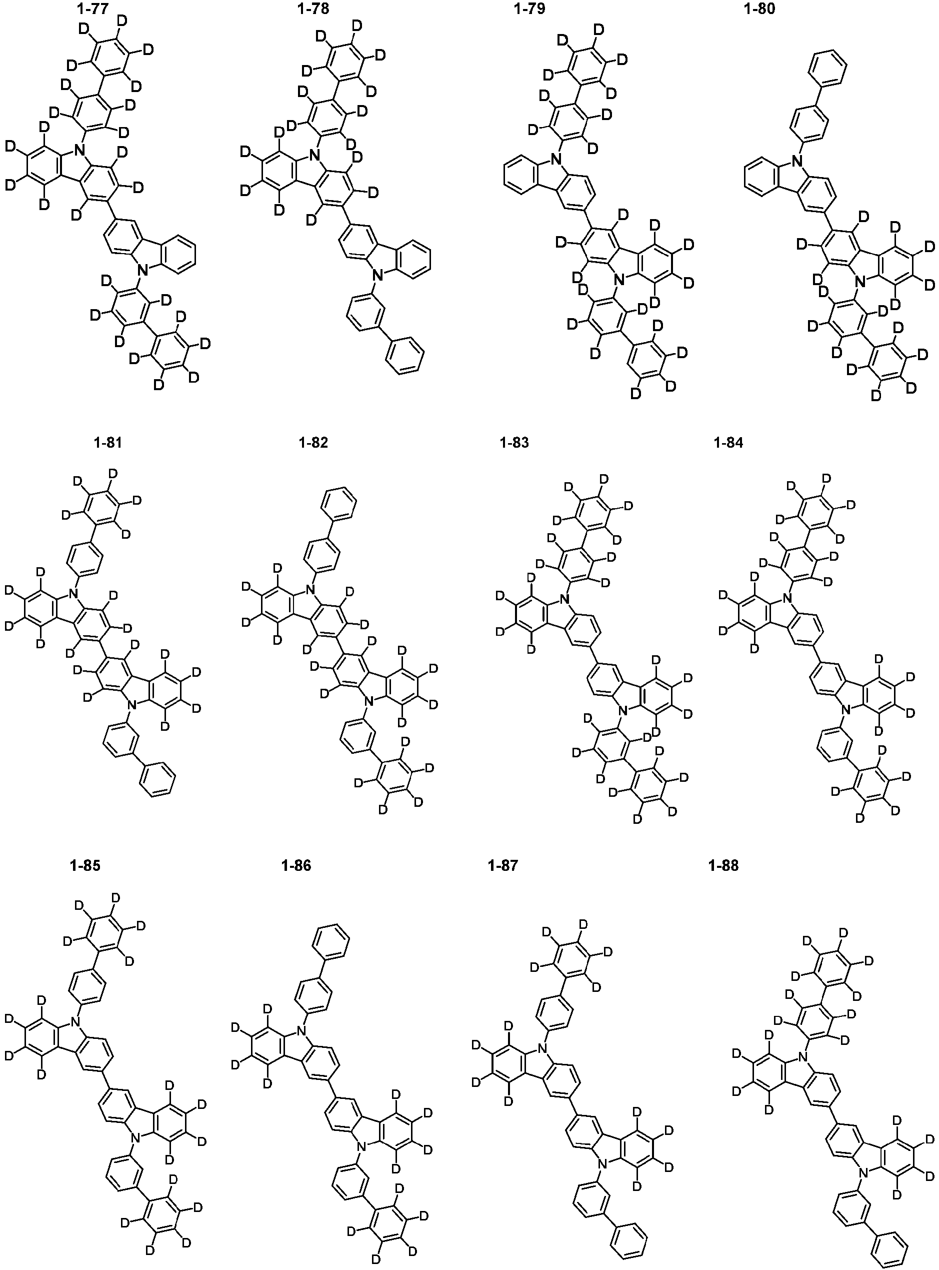

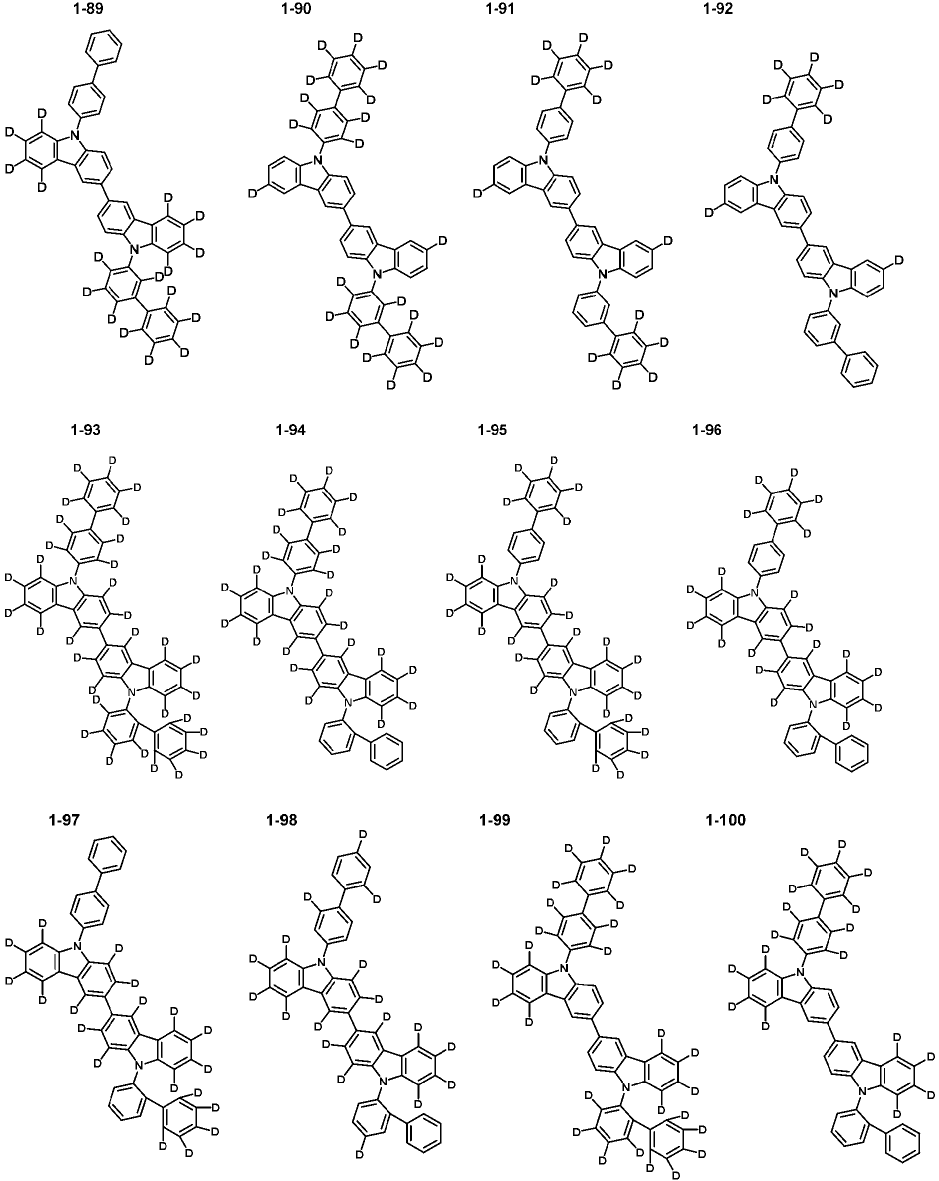

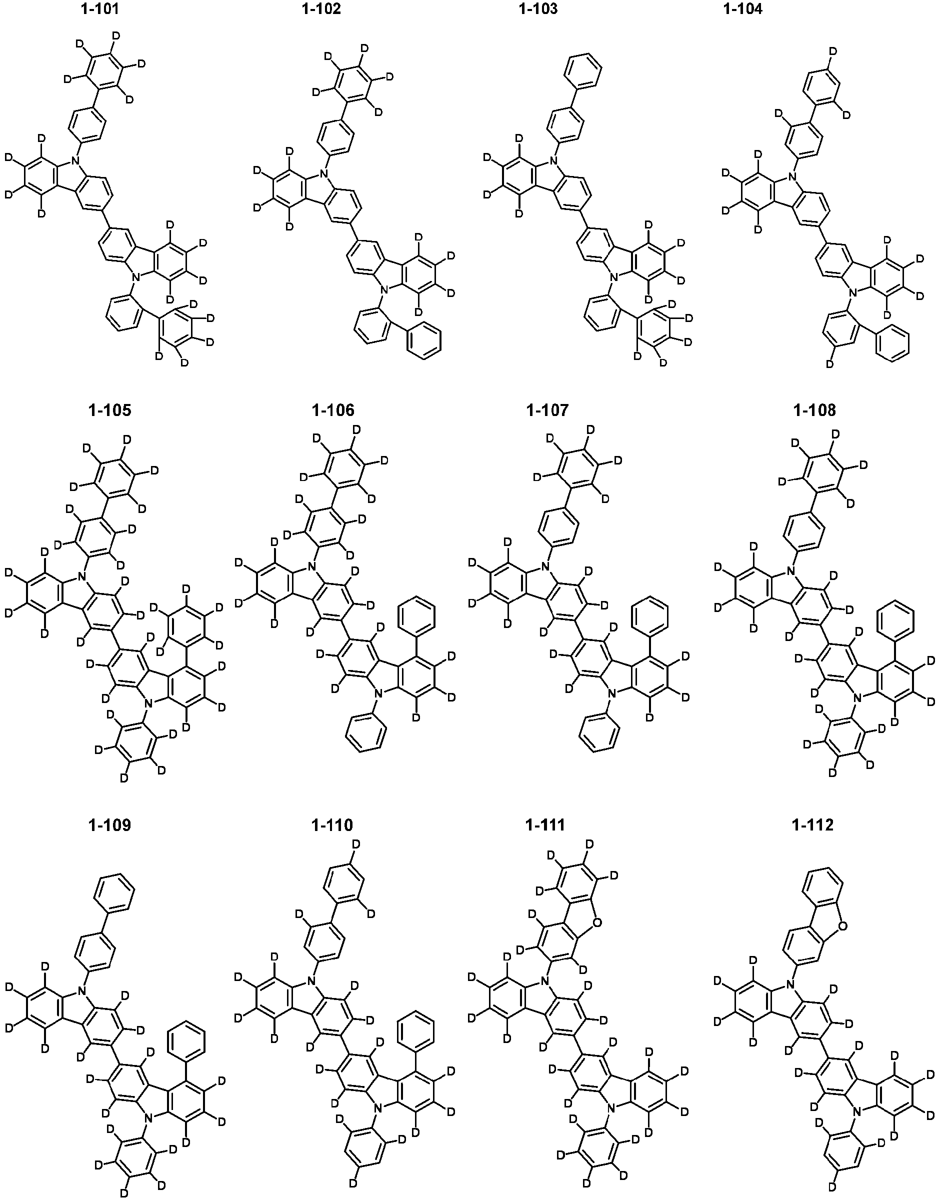

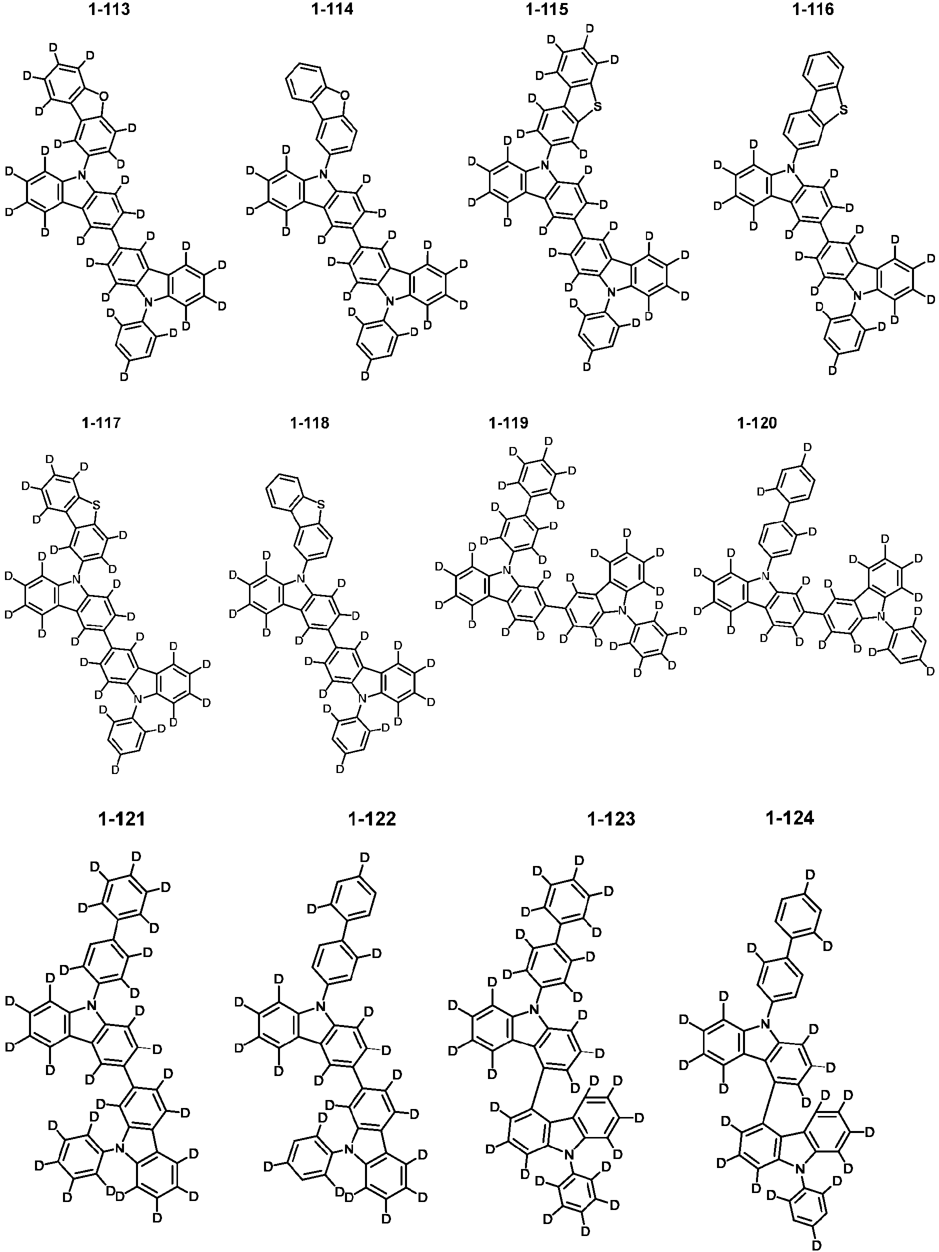

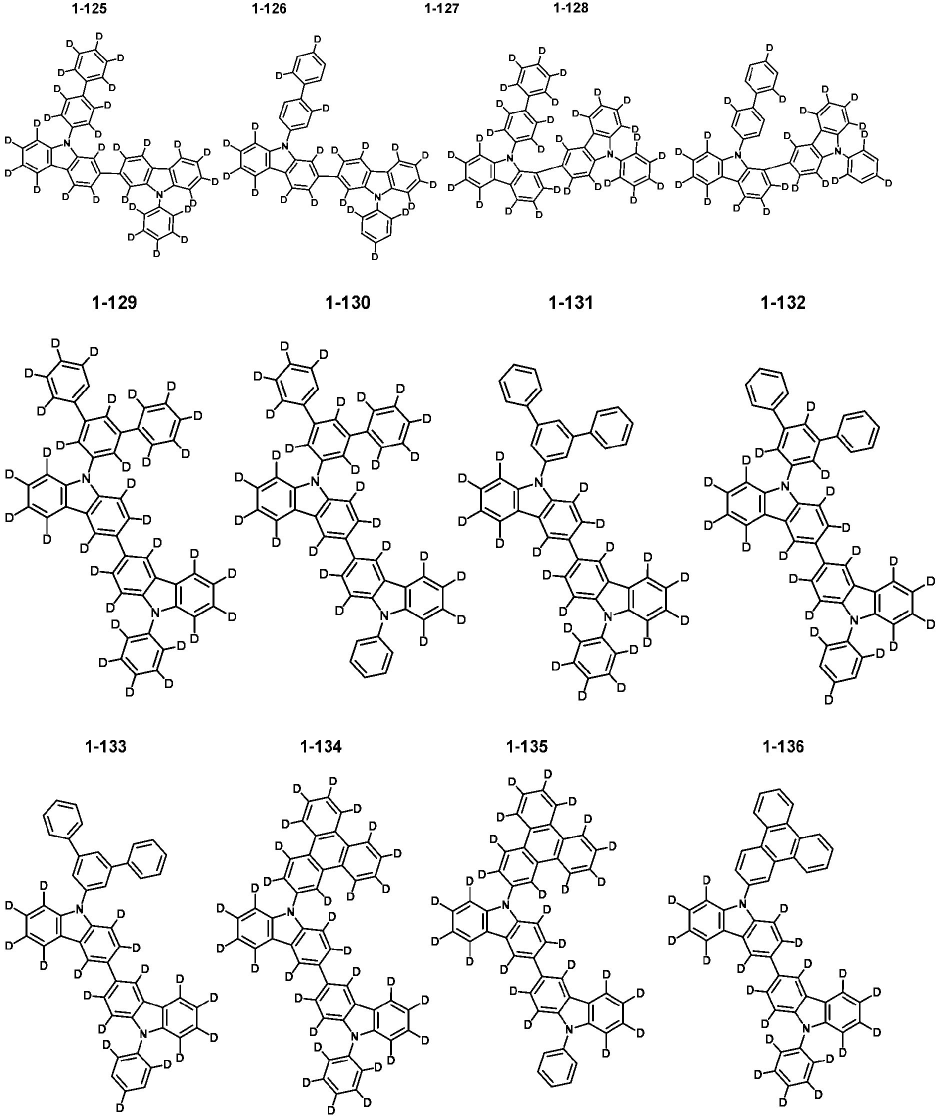

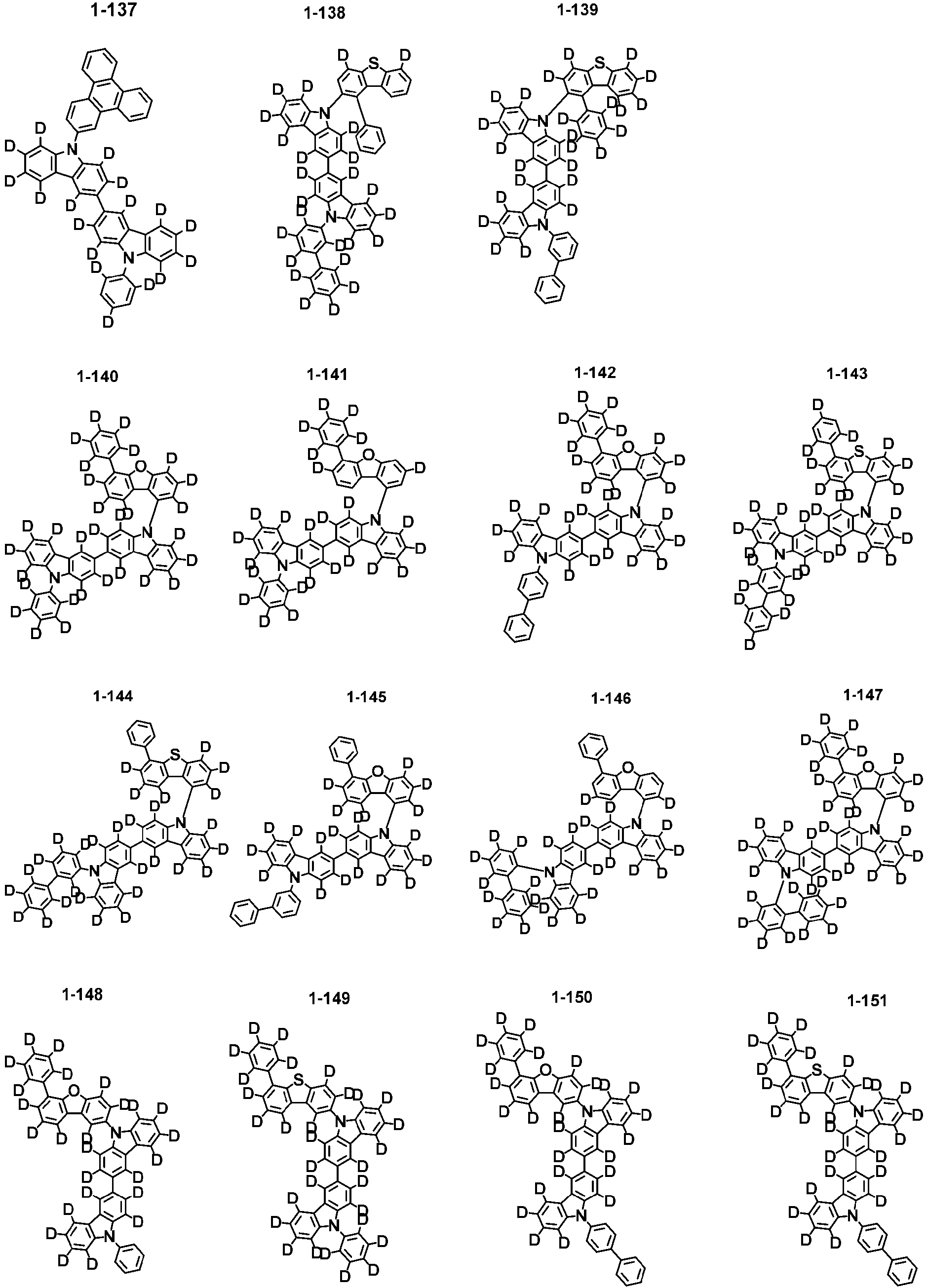

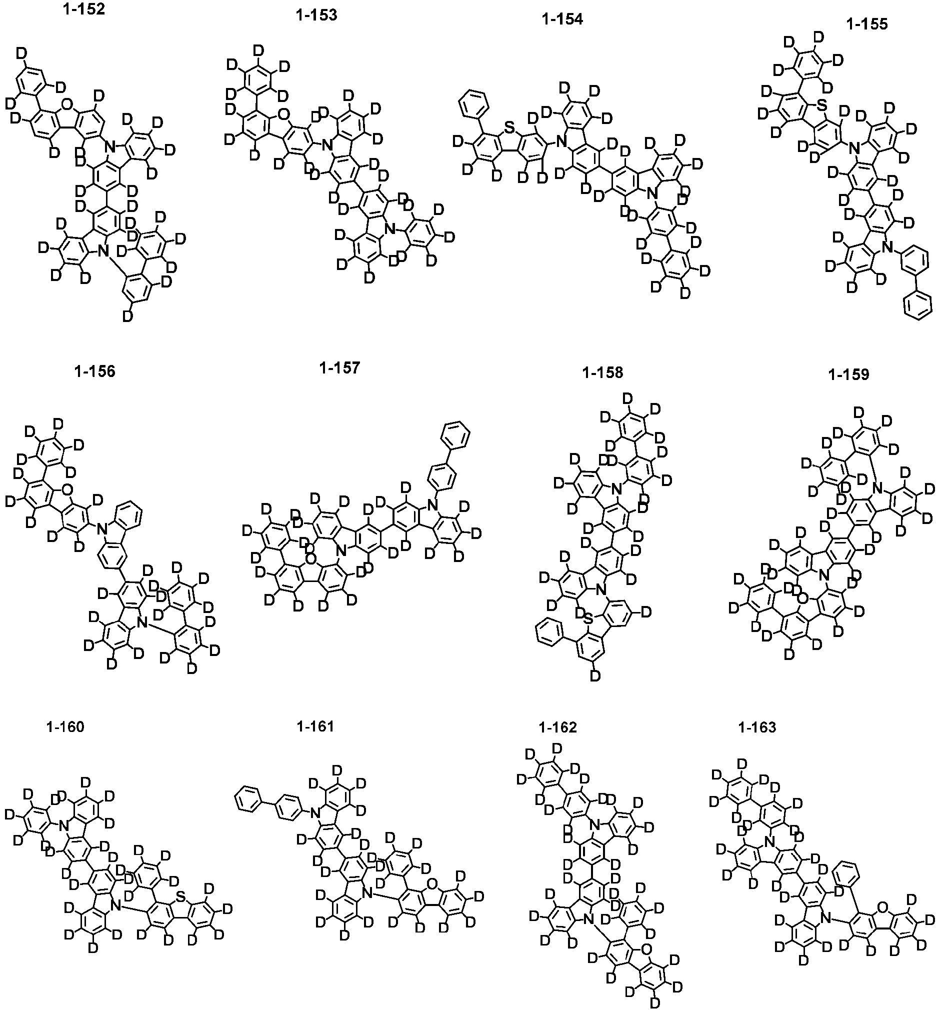

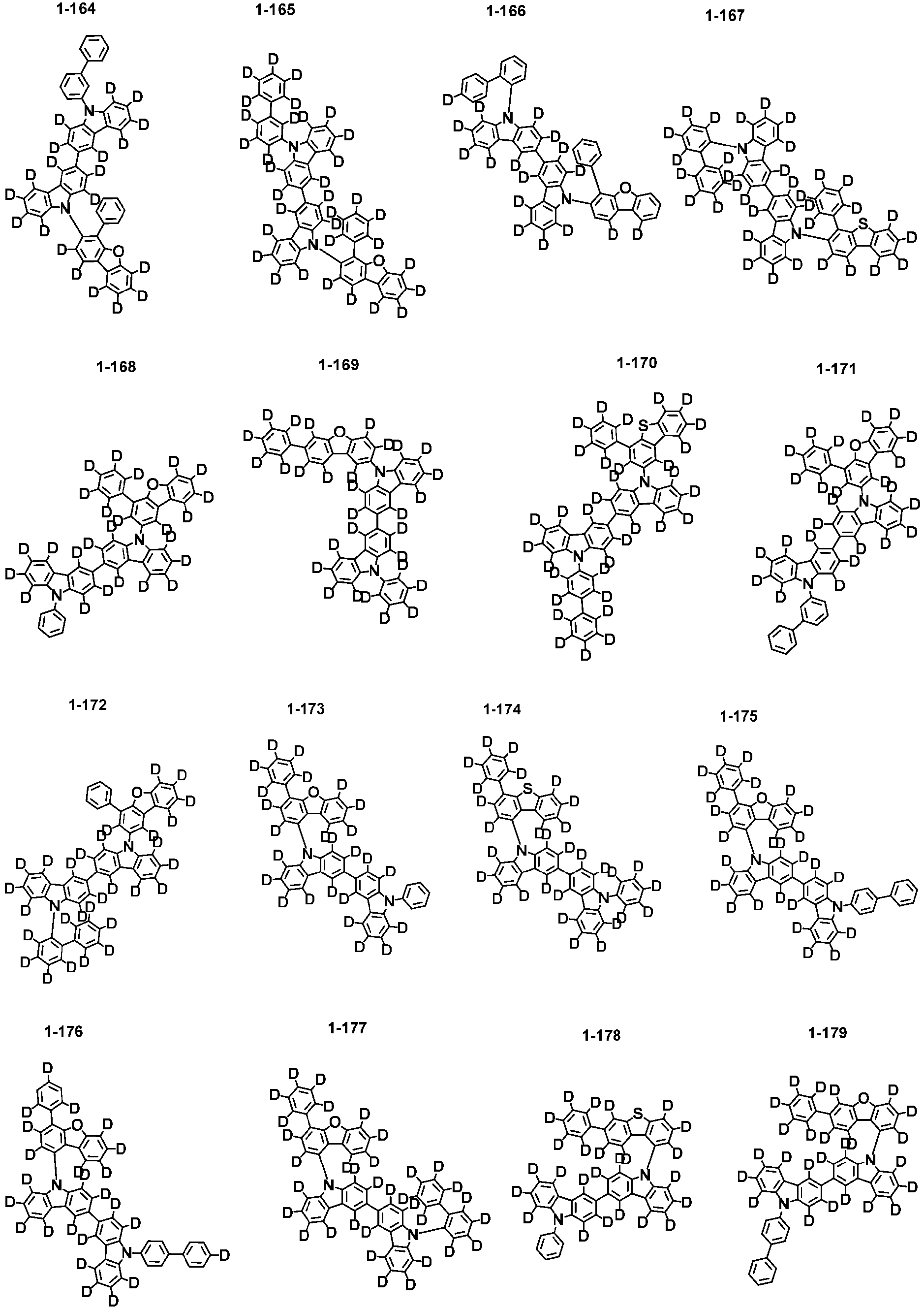

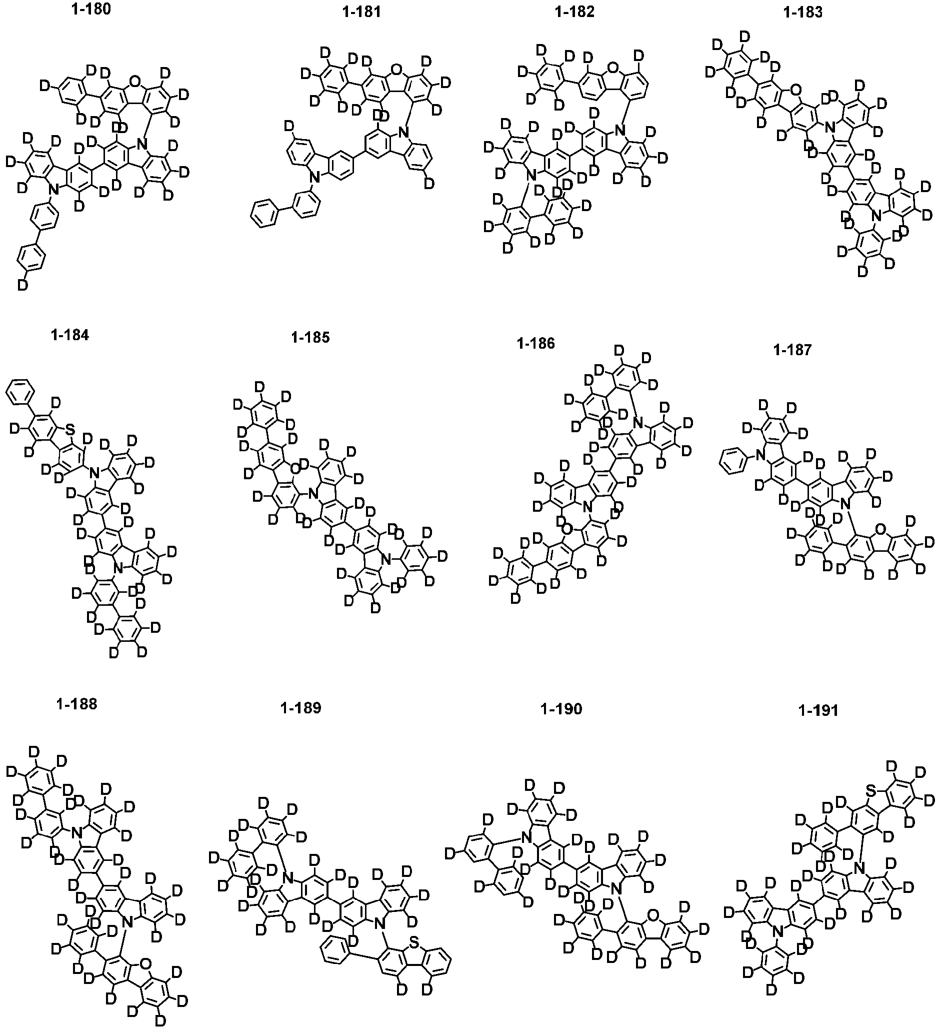

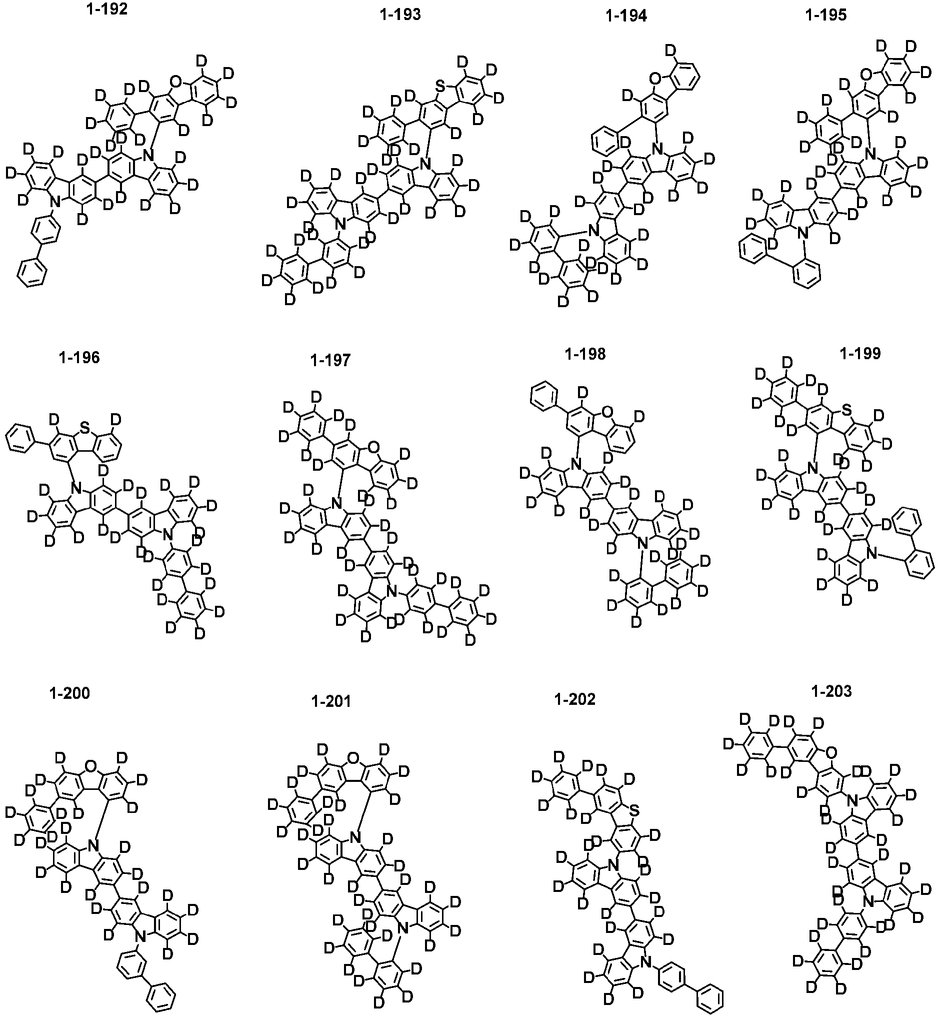

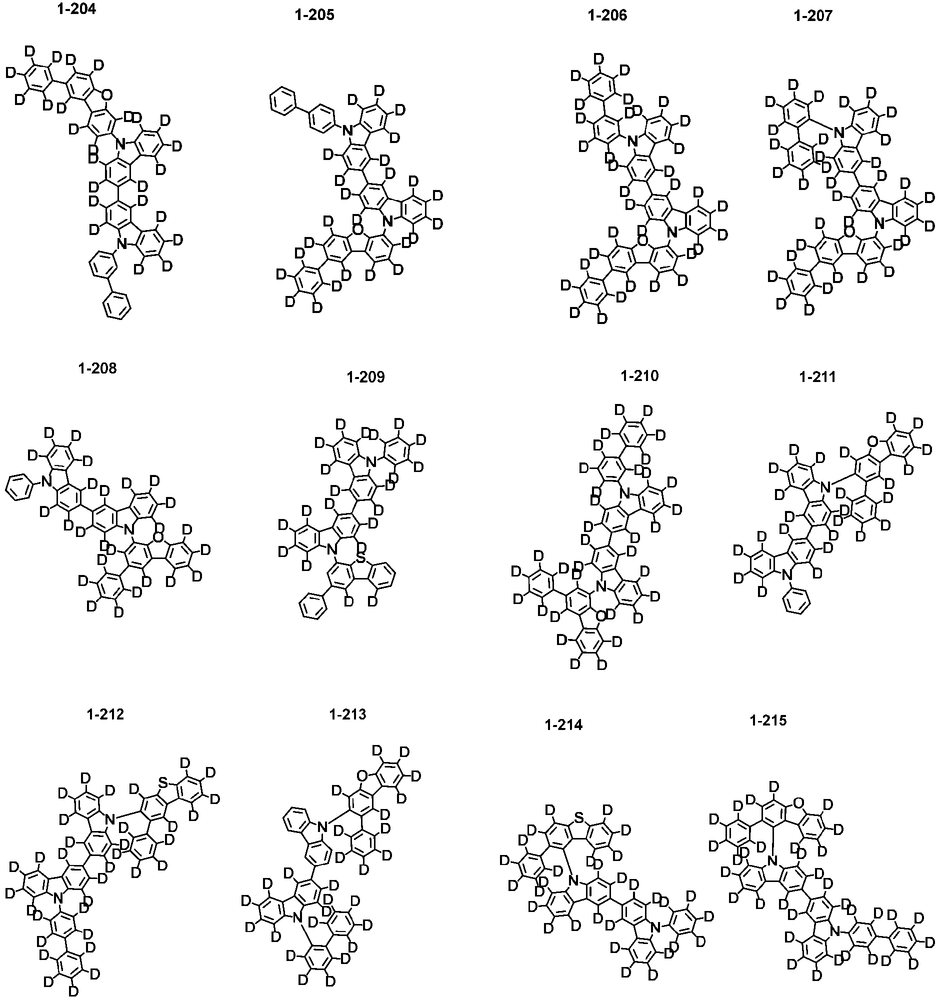

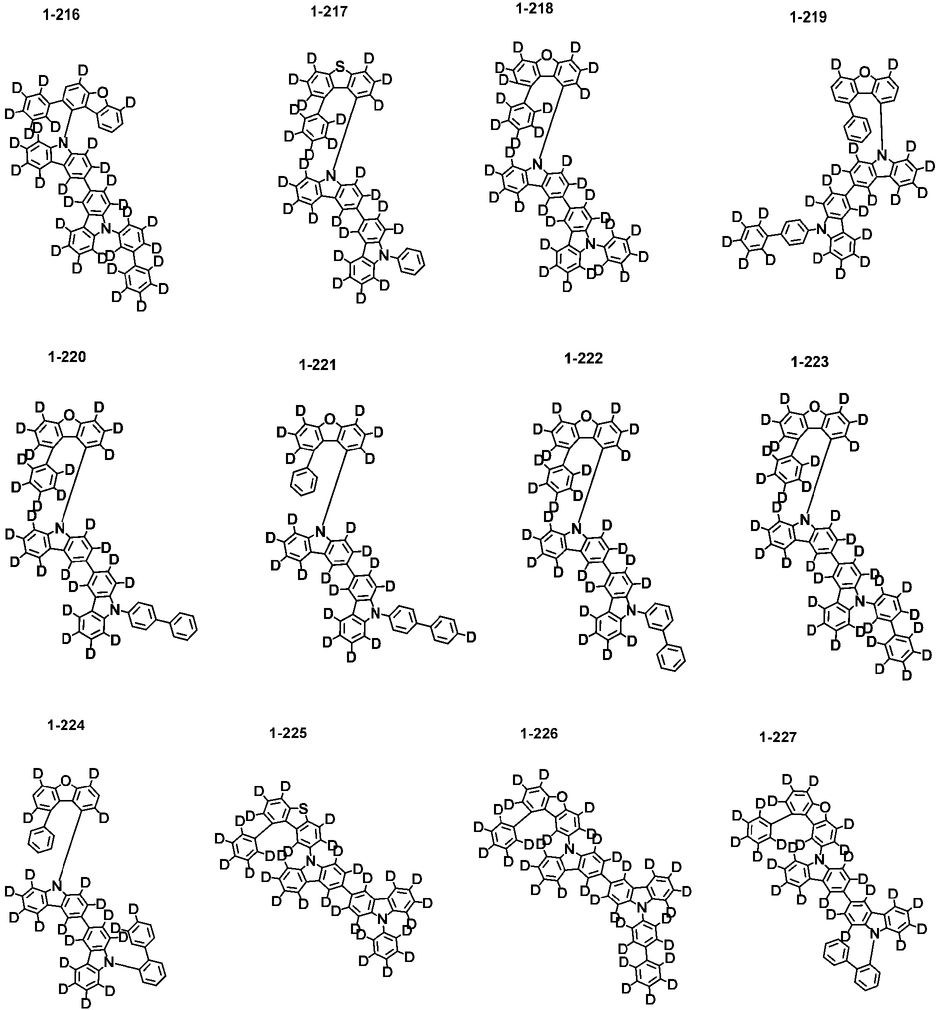

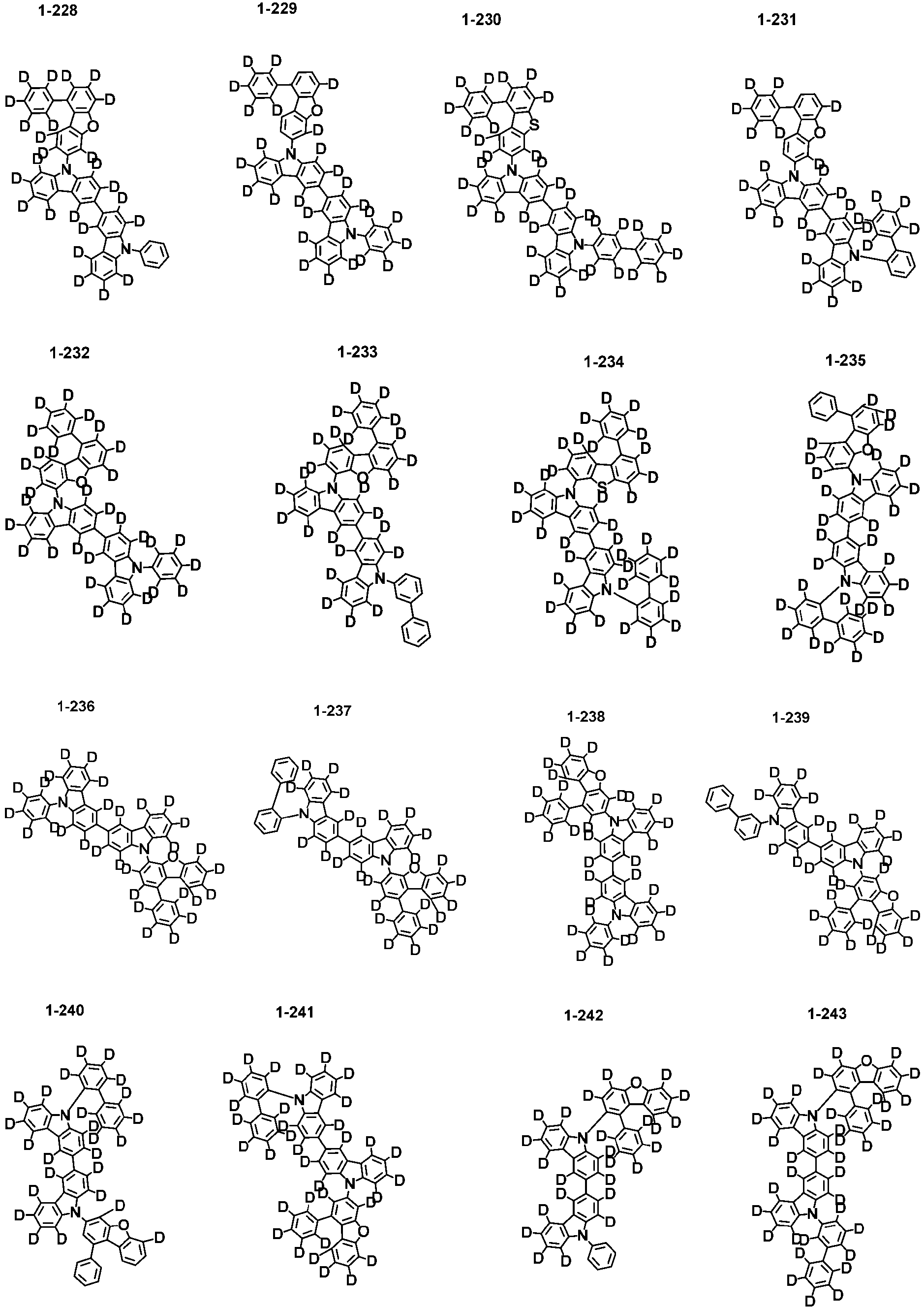

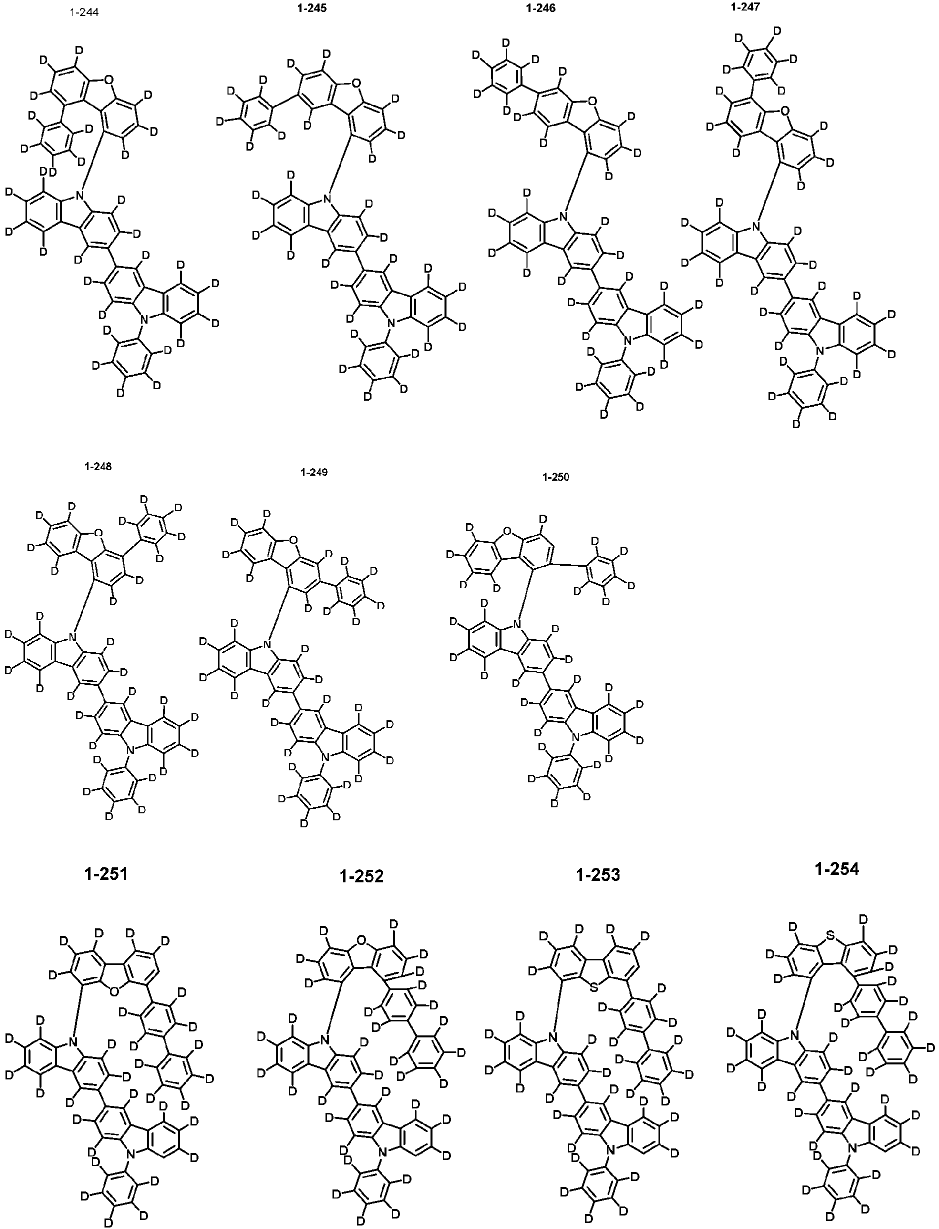

7. Composition pour le dispositif optoélectronique organique selon la revendication 1, dans laquelle
le deuxième composé est représenté par l'une parmi la Formule Chimique 2A à la Formule Chimique 2F: 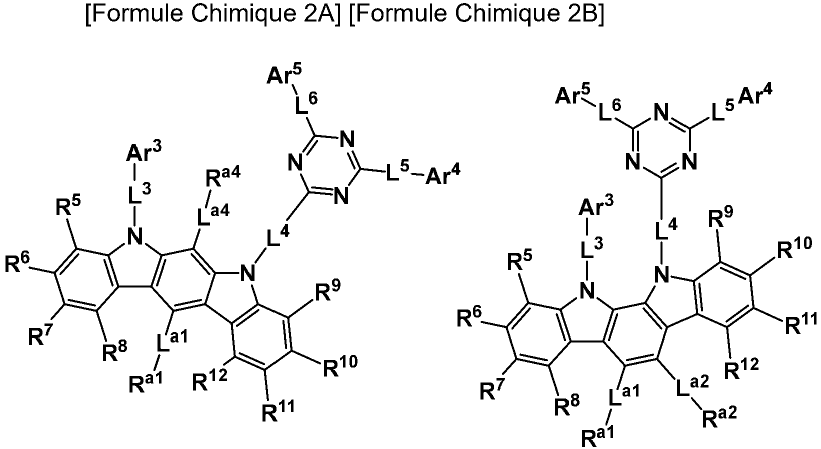 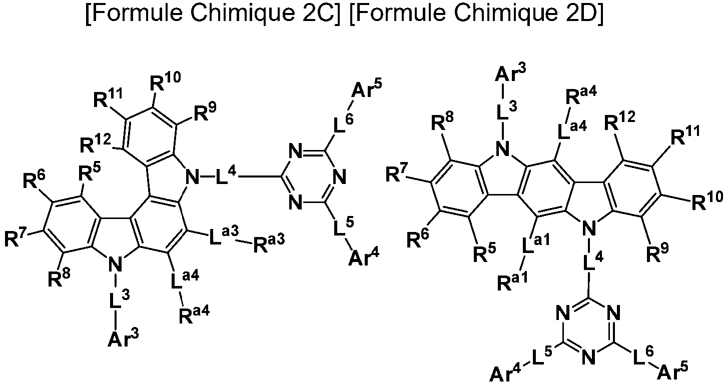 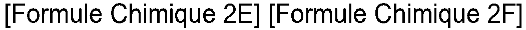 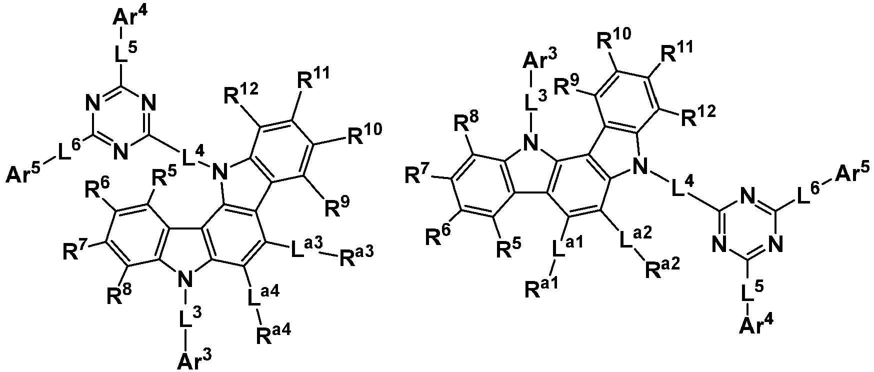
dans laquelle, dans la Formule Chimique 2A à la Formule Chimique 2F,
Ar³ à Ar⁵, L³ à L⁶, et R⁵ à R¹² sont les mêmes que ceux décrits dans la revendication 1,
L^{a1} à L^{a4} sont identiques à la définition de L^{a}, et
R^{a1} à R^{a4} sont identiques à la définition de R^{a}.

8. Composition pour le dispositif optoélectronique organique selon la revendication 1, dans laquelle
R⁵ à R¹² dans les Formules Chimiques 2 et 3 représentent chacun indépendamment de l'hydrogène, du deutérium ou un groupe cyano,
L³ à L⁶ représentent une liaison simple, un groupe phénylène substitué ou non substitué, un groupe biphénylène substitué ou non substitué, ou un groupe pyridinylène substitué ou non substitué, et
Ar³ à Ar⁵ représentent chacun indépendamment un groupe phényle substitué ou non substitué, un groupe biphényle substitué ou non substitué, un groupe triphénylène substitué ou non substitué, un groupe fluorényle substitué ou non substitué, un groupe dibenzofurannyle substitué ou non substitué, ou un groupe dibenzothiophényle substitué ou non substitué.

9. Composition pour le dispositif optoélectronique organique selon la revendication 1, dans laquelle
le premier composé est représenté par la Formule Chimique 1-8a, et
le deuxième composé est représenté par la Formule Chimique 2B: 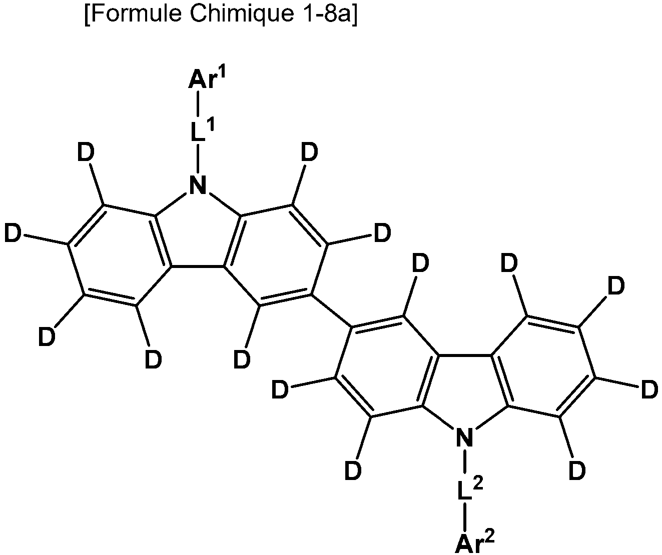
dans laquelle, dans la Formule Chimique 1-8a,
L¹ et L² sont une liaison simple ou un groupe phénylène substitué ou non substitué, et
Ar¹ et Ar² représentent chacun un groupe phényle substitué par du deutérium, un groupe biphényle substitué par du deutérium, un groupe terphényle substitué par du deutérium, ou un groupe triphénylène substitué par du deutérium, 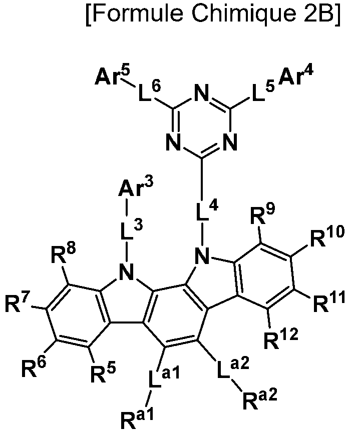
dans laquelle, dans la Formule Chimique 2B,
L^{a1} et L^{a2} représentent une liaison simple,
L³ à L⁶ représentent chacun indépendamment une liaison simple ou un groupe arylène C6 à C12 substitué ou non substitué,
R⁵ à R¹², R^{a1}, et R^{a2} représentent chacun indépendamment de l'hydrogène, du deutérium, un groupe cyano, ou un groupe phényle substitué ou non substitué, et
Ar³ à Ar⁵ représentent chacun indépendamment un groupe phényle substitué ou non substitué, un groupe biphényle substitué ou non substitué, ou un groupe triphénylène substitué ou non substitué.

10. Dispositif optoélectronique organique, comprenant
une anode et une cathode se faisant face,
au moins une couche organique entre l'anode et la cathode, et
la couche organique comprend la composition pour le dispositif optoélectronique organique selon l'une quelconque des revendications 1 à 9.

11. Dispositif optoélectronique organique selon la revendication 10, dans lequel
la couche organique comprend une couche électroluminescente, et
la couche électroluminescente comprend la composition pour un dispositif optoélectronique organique.

12. Dispositif d'affichage comprenant le dispositif optoélectronique organique selon la revendication 10.
